(19) Europäisches Patentamt
European Patent Office
Office européen des brevets

(11) EP 4 450 496 A1

(12) # EUROPEAN PATENT APPLICATION
published in accordance with Art. 153(4) EPC

(43) Date of publication:
**23.10.2024 Bulletin 2024/43**

(21) Application number: **22906689.9**

(22) Date of filing: **16.12.2022**

(51) International Patent Classification (IPC):
***C07D 403/12*** (2006.01) ***A61K 31/4427*** (2006.01)
***A61P 35/00*** (2006.01)

(52) Cooperative Patent Classification (CPC):
**A61K 31/4427; A61P 35/00; C07D 403/12**

(86) International application number:
**PCT/CN2022/139570**

(87) International publication number:
**WO 2023/109944 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.12.2021 CN 202111544384**
**02.06.2022 CN 202210624727**
**08.12.2022 CN 202211573268**

(71) Applicant: **Duality Biologics (Suzhou) Co., Ltd.**
**Suzhou, Jiangsu 215000 (CN)**

(72) Inventors:
- **ZHANG, Yu**
**Shanghai 201204 (CN)**
- **LI, Bing**
**Shanghai 201204 (CN)**
- **LI, Xi**
**Shanghai 201204 (CN)**
- **HUA, Haiqing**
**Shanghai 201204 (CN)**
- **ZHU, Zhongyuan**
**Shanghai 201204 (CN)**

(74) Representative: **Vossius & Partner**
**Patentanwälte Rechtsanwälte mbB**
**Siebertstraße 3**
**81675 München (DE)**

(54) **TLR MODULATOR AND USE THEREOF**

(57) The present application relates to a TLR modulator and the use thereof. Specifically, the present application relates to a compound for modulating TLR activity, or a tautomer, a mesomer, a racemate, an enantiomer, a diastereoisomer, or a mixture form thereof, or a pharmaceutically acceptable salt thereof. The present application further relates to a method for preparing the compound of the present application and the use thereof.

EP 4 450 496 A1

## Description

### TECHNICAL FIELD

The present application relates to the field of biopharmaceuticals, and in particular, to a compound as a TLR modulator and use thereof.

### BACKGROUND

The highly conserved pattern recognition receptor protein Toll-like receptor (TLR) family is believed to be involved in innate immunity as receptors of pathogen-associated molecular patterns (PAMPs). Related compounds that influence TLR activity may affect therapies for diseases including autoimmunity, inflammation, allergy, asthma, transplant rejection, graft-versus-host disease, infection, cancer, or immunodeficiency. There is therefore an urgent need in the art for compounds capable of influencing TLR activity.

### SUMMARY

The present application provides a compound or a tautomer, a mesomer, a racemate, an enantiomer or a diastereoisomer thereof, or a mixture thereof, or a pharmaceutically acceptable salt thereof. The compound may have an effect selected from the group consisting of: inhibiting tumor growth, influencing Toll-like receptor (TLR) functionality, and influencing immune system functionality. The present application provides a compound or a tautomer, a mesomer, a racemate, an enantiomer or a diastereoisomer thereof, or a mixture thereof, or a pharmaceutically acceptable salt thereof, wherein the compound comprises a structure of formula (II-a):

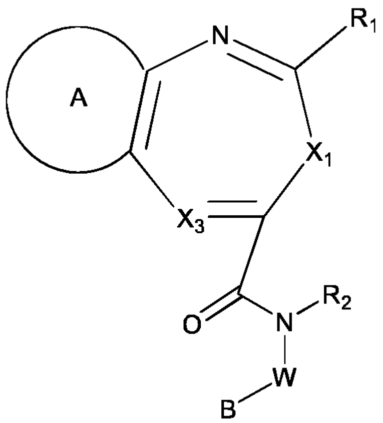

(II-a),

wherein R1 and R2 are each independently any group, X1, X2, and X3 are each independently any atom optionally substituted by any group, A is an optionally substituted cyclic structure, W is absent or is any group, and B is an optionally substituted aromatic ring,
wherein B is substituted with one or more optionally substituted amino groups.

In another aspect, the present application provides a conjugate comprising the compound or the tautomer, the mesomer, the racemate, the enantiomer or the diastereoisomer thereof, or the mixture thereof, or the pharmaceutically acceptable salt thereof of the present application.

In another aspect, the present application provides a pharmaceutical composition comprising the compound or the tautomer, the mesomer, the racemate, the enantiomer or the diastereoisomer thereof, or the mixture thereof, or the pharmaceutically acceptable salt thereof of the present application and/or the conjugate of the present application, and optionally a pharmaceutically acceptable carrier. In another aspect, the present application provides a kit comprising the compound or the tautomer, the mesomer, the racemate, the enantiomer or the diastereoisomer thereof, or the mixture thereof, or the pharmaceutically acceptable salt thereof of the present application, the conjugate of the present application, and/or the pharmaceutical composition of the present application.

In another aspect, the present application provides a method for influencing Toll-like receptor (TLR) functionality, comprising administering the compound or the tautomer, the mesomer, the racemate, the enantiomer or the diastereoisomer thereof, or the mixture thereof, or the pharmaceutically acceptable salt thereof of the present application, the conjugate of the present application, the pharmaceutical composition of the present application, and/or the kit of the

present application.

In another aspect, the present application provides a method for modulating immune system functionality, comprising administering the compound or the tautomer, the mesomer, the racemate, the enantiomer or the diastereoisomer thereof, or the mixture thereof, or the pharmaceutically acceptable salt thereof of the present application, the conjugate of the present application, the pharmaceutical composition of the present application, and/or the kit of the present application.

In another aspect, the present application provides use of the compound or the tautomer, the mesomer, the racemate, the enantiomer or the diastereoisomer thereof, or the mixture thereof, or the pharmaceutically acceptable salt thereof of the present application, the conjugate of the present application, the pharmaceutical composition of the present application, and/or the kit of the present application in preparing a medicament for preventing and/or treating a disease and/or a symptom. Other aspects and advantages of the present application will be readily apparent to those skilled in the art from the following detailed description. Only exemplary embodiments of the present application have been shown and described in the following detailed description. As those skilled in the art will recognize, the content of the present application enables those skilled in the art to make changes to the specific embodiments disclosed without departing from the spirit and scope of the invention to which the present application pertains. Accordingly, descriptions in the specification are only illustrative rather than restrictive.

## DETAILED DESCRIPTION

The embodiments of the present invention are described below with reference to specific examples, and other advantages and effects of the present invention will be readily apparent to those skilled in the art from the disclosure of the present specification.

### Definitions of Terms

In the present application, the terms "Toll-like receptor" and "TLR" generally refer to any member of the highly conserved mammal pattern recognition receptor family that recognizes pathogen-associated molecular patterns (PAMPs) and serves as a key signaling element in innate immunity. TLR polypeptides share a characteristic structure, comprising an extracellular domain with leucine-rich repeats, a transmembrane domain, and an intracellular domain involved in TLR signaling. TLR includes, but is not limited to, human TLR.

In the present application, the terms "Toll-like receptor 7" and "TLR7" refer to a nucleic acid or polypeptide having at least 70%, 80%, 90%, 95%, 96%, 97%, 98%, 99%, or higher sequence identity with published TLR7 sequences, such as human TLR7 polypeptide of GenBank Accession No. AAZ99026, or mouse TLR7 polypeptide of GenBank Accession No. AAK62676.

In the present application, the terms "Toll-like receptor 8" and "TLR8" refer to a nucleic acid or polypeptide having at least 70%, 80%, 90%, 95%, 96%, 97%, 98%, 99%, or higher sequence identity with published TLR8 sequences, such as human TLR8 polypeptide of GenBank Accession No. AAZ95441, or mouse TLR8 polypeptide of GenBank Accession No. AAK62677.

In the present application, the term "TLR agonist" generally refers to an agent that, either directly or indirectly, binds to TLR (e.g., TLR7 and/or TLR8) to induce TLR signaling. Any detectable difference in TLR signaling may indicate that the agonist stimulates or activates TLR. Differences in signaling may be represented by changes in target gene expression, changes in the phosphorylation of signaling components, changes in the intracellular localization of downstream elements such as nuclear factor-κB(NP-κB), changes in the association of certain components such as IL-1 receptor-associated kinase (IRAK) with other proteins or intracellular structures, or changes in the biochemical activity of components such as kinases (e.g., mitogen-activated protein kinase (MAPK)).

In the present application, the term "halogen" generally refers to fluorine, chlorine, bromine, or iodine, and it may be, for example, fluorine or chlorine.

In the present application, the term "alkyl" generally refers to a residue derived from an alkane by the removal of a hydrogen atom. The alkyl may be substituted or unsubstituted, or replaced or unreplaced. The term "alkyl" generally refers to a saturated linear or branched aliphatic hydrocarbon group having a residue derived from a parent alkane by the removal of hydrogen atoms from the same carbon atom or two different carbon atoms, and it may be a linear or branched group containing 1 to 20 carbon atoms, e.g., 1 to 12 carbon atoms, such as alkyl containing 1 to 6 carbon atoms. Non-limiting examples of alkyl include but are not limited to methyl, ethyl, propyl, propyl, butyl, etc. The alkyl may be substituted or unsubstituted, or replaced or unreplaced. For example, when the alkyl is substituted, substitution with a substituent may be performed at any available linking site, and the substituent may be independently optionally selected from one or more of alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo, and the substituent may, e.g., be hydrogen, protium, deuterium, tritium, halogen, $-NO_2$, -CN, -OH, -SH, - $NH_2$, -C(O)H,

$-CO_2H$, $-C(O)C(O)H$, $-C(O)CH_2C(O)H$, $-S(O)H$, $-S(O)_2H$, $-C(O)NH_2$, $-SO_2NH_2$, - OC(O)H, $-N(H)SO_2H$, or a $C_{1-6}$ aliphatic group.

In the present application, the term "alkylene" generally refers to a saturated linear or branched aliphatic hydrocarbon group having 2 residues derived from a parent alkane by the removal of two hydrogen atoms from the same carbon atom or two different carbon atoms, and it may be a linear or branched group containing 1 to 20 carbon atoms; for example, the term "methylene" may refer to a residue derived from a one-carbon atom group by removal of two hydrogen atoms. Methylene may be substituted or unsubstituted, or replaced or unreplaced; for example, the alkylene contains 1 to 12 carbon atoms, e.g., alkylene containing 1 to 6 carbon atoms. Non-limiting examples of alkylene include but are not limited to methylene ($-CH_2-$), 1,1-ethylene ($-CH(CH_3)-$), 1,2-ethylene ($-CH_2CH_2-$), 1,1-propylene ($-CH(CH_2CH_3)-$), 1,2-propylene ($-CH_2CH(CH_3)-$), 1,3-propylene ($-CH_2CH_2CH_2-$), 1,4-butylene ($-CH_2CH_2CH_2CH_2-$), 1,5-butylene ($-CH_2CH_2CH_2CH_2CH_2-$), etc. The alkylene may be substituted or unsubstituted, or replaced or unreplaced. For example, when substituted, the alkylene may be substituted at any available connection site with a substituent that may be independently optionally selected from one or more of alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo, and, for example, may be hydrogen, protium, deuterium, tritium, halogen, $-NO_2$, -CN, -OH, -SH, $-NH_2$, - C(O)H, $-CO_2H$, $-C(O)C(O)H$, $-C(O)CH_2C(O)H$, $-S(O)H$, $-S(O)_2H$, $-C(O)NH_2$, $-SO_2NH_2$, -OC(O)H, $-N(H)SO_2H$, or $C_{1-6}$ aliphatic group. Methylene or the alkylene may be substituted or unsubstituted. In the present application, the term "alkenyl" generally refers to a linear or branched hydrocarbon group containing one or more double bonds. Exemplary examples of alkenyl include allyl, homoallyl, vinyl, crotyl, butenyl, pentenyl, hexenyl, etc. Exemplary instances of C2-6 alkenyl containing one or more double bonds include butadienyl, pentadienyl, hexadienyl, and hexatrienyl, as well as branched forms thereof. The positions of the unsaturated bonds (double bonds) may be any positions in the carbon chain. The alkenyl may be substituted or unsubstituted.

In the present application, the term "alkenylene" generally refers to a residue derived from an alkene by the removal of two hydrogen atoms from a carbon atom. For example, the alkenylene may be acrol, vinylene, butenylene, pentenylene, hexenylene, etc. The alkenylene may be substituted or unsubstituted.

In the present application, the term "alkynyl" generally refers to unsaturated linear or branched alkynyl, e.g., ethynyl, 1-propynyl, propargyl, or butynyl. The alkynyl may be substituted or unsubstituted.

In the present application, the term "alkynylene" generally refers to a residue derived from an alkyne by the removal of two hydrogen atoms from a carbon atom. For example, the alkynylene may be ethynylene, propynylene, propargylene, butynylene, etc. The alkynylene may be substituted or unsubstituted.

In the present application, the term "aryl" generally refers to a residue derived from an aromatic ring by the removal of a hydrogen atom. The term "aromatic ring" may refer to a 6- to 14-membered all-carbon monocyclic ring or fused polycyclic rings (i.e., rings that share a pair of adjacent carbon atoms) having a conjugated $\pi$-electron system, and may be 6- to 10-membered, such as benzene and naphthalene. The aromatic ring can be fused to a heteroaryl, heterocyclyl, or cycloalkyl ring, wherein the ring connected to the parent structure is the aryl ring. The aryl may be substituted or unsubstituted, and when it is substituted, the substituent may be one or more of the following groups independently selected from the group consisting of: alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio. The aryl may be substituted or unsubstituted.

In the present application, the term "arylene" generally refers to a residue derived from an aromatic ring by the removal of two hydrogen atoms from carbon atoms. For example, the arylene may be phenylene and naphthylene. The arylene may be substituted or unsubstituted.

In the present application, the term "heteroaryl" generally refers to a residue derived from a heteroaromatic ring by the removal of a hydrogen atom from a carbon atom. The term "heteroaromatic ring" refers to a heteroaromatic system comprising 1 to 4 heteroatoms and 5 to 14 ring atoms, wherein the heteroatoms may be selected from the group consisting of: oxygen, sulfur, and nitrogen. The heteroaryl may be 5- to 10-membered, such as 5- or 6-membered heteroaryl, e.g., furanyl, thienyl, pyridinyl, pyrrolyl, N-alkylpyrrolyl, pyrimidinyl, pyrazinyl, imidazolyl, and tetrazolyl. The heteroaryl ring can be fused to an aryl, heterocyclyl, or cycloalkyl ring, wherein the ring connected to the parent structure is the heteroaryl ring. The heteroaryl may be optionally substituted or unsubstituted, and when it is substituted, the substituent may be one or more of the following groups independently selected from the group consisting of: alkyl, alkenyl, alkynyl, alkoxy, alkylthio, alkylamino, halogen, sulfhydryl, hydroxy, carboxy, nitro, cyano, cycloalkyl, heterocycloalkyl, aryl, heteroaryl, cycloalkoxy, heterocycloalkoxy, cycloalkylthio, and heterocycloalkylthio. The heteroaryl may be substituted or unsubstituted.

In the present application, the term "heteroarylene" generally refers to a residue derived from a heteroaromatic ring by the removal of two hydrogen atoms from carbon atoms. For example, the heteroarylene may be furanylene, thienylene, pyridinylene, pyrrolylene, pyrimidinylene, pyrazinylene, imidazolylene, tetrazolylene, etc. The heteroarylene may be substituted or unsubstituted.

In the present application, the term "alcyl" generally refers to a residue derived from an aliphatic ring by the

removal of a hydrogen atom from the same carbon atom or from a plurality of different carbon atoms. The term "cycloalkane" or "cycloalkyl" generally refers to a saturated or partially unsaturated monocyclic or polycyclic hydrocarbon, and the carbon ring contains 3 to 20 carbon atoms, may contain 3 to 12 carbon atoms, may contain 3 to 10 carbon atoms, and may contain 3 to 8 carbon atoms. Non-limiting examples of alcyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic carbon rings may include spiro, fused, and bridged carbon rings. The alcyl may be substituted or unsubstituted. In the present application, the term "carbocyclyl" generally refers to a residue derived from a carbon ring by the removal of a hydrogen atom from a carbon atom. The term "carbon ring" generally refers to a saturated or partially unsaturated monocyclic or polycyclic hydrocarbon, and contains 3 to 20 carbon atoms, may contain 3 to 12 carbon atoms, may contain 3 to 10 carbon atoms, and may contain 3 to 8 carbon atoms. Non-limiting examples of monocyclic carbon rings include cyclopropane, cyclobutane, cyclopentane, cyclopentene, cyclohexane, cyclohexene, cyclohexadiene, cycloheptane, cycloheptatriene, cyclooctane, etc.; polycyclic carbon rings may include spiro, fused and bridged carbon rings. The carbocyclyl may be substituted or unsubstituted. In some instances, alicyclic and carbocyclic rings are used interchangeably. The cycloalkyl may be optionally substituted with one or more substituents selected from $C_1$-$C_6$ alkyl, cyano, amino, -NH($C_1$-$C_6$ alkyl), -N($C_1$-$C_6$ alkyl)$_2$, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, -$C_1$-$C_6$ alkylhydroxy, -$C_1$-$C_6$ alkyl-$C_1$-$C_6$ alkoxy, halogen, halogenated $C_1$-$C_6$ alkyl, halogenated $C_1$-$C_6$ alkoxy, -S(O)$C_1$-$C_6$ alkyl, -S(O)$_2C_1$-$C_6$ alkyl, - S(O)$_2NR_eR_f$, or -C(O)$NR_eR_f$, or substituted with one or more substituents selected from halogen, $C_1$-$C_6$ alkyl, $C_1$-$C_6$ alkoxy, -$C_1$-$C_6$ alkylhydroxy, hydroxy, oxo, amino, -NH($C_1$-$C_6$ alkyl), -N($C_1$-$C_6$ alkyl)$_2$, -O-phenyl, or phenyl, wherein $R_e$ and $R_f$ are each independently selected from H, $C_1$-$C_6$ alkyl, or -$C_1$-$C_6$ alkylhydroxy, or $R_e$ and $R_f$, together with the nitrogen atom to which they are connected, form a 4- to 8-membered nitrogen-containing heterocyclyl or 5- or 6-membered nitrogen-containing heteroaryl.

In the present application, the term "partially unsaturated" generally means that the cyclic structure contains at least one double or triple bond in the ring molecule. The term "partially unsaturated" encompasses cyclic structures having multiple sites of unsaturation, but is not intended to include aromatic or heteroaromatic rings defined herein. The term "unsaturated" means that the moiety has one or more degrees of unsaturation.

In the present application, the term "alcylene" generally refers to a residue derived from an alicyclic ring by the removal of two hydrogen atoms from carbon atoms. For example, the alcylene may be cyclopropylene, cyclobutylene, cyclopentylene, cyclopentenylene, cyclohexylene, cyclohexenylene, cyclohexadienylene, cycloheptylene, cycloheptatrienylene, cyclooctylene, etc.; polycyclic carbon rings may include spiro, fused and bridged carbon rings. The alcylene may be substituted or unsubstituted.

In the present application, the term "aliphatic heterocyclyl" or "heterocyclyl" generally refers to a stable, non-aromatic 3- to 12-membered heterocyclyl, such as a 3- to 8-membered, 4- to 8-membered, or 4- to 10-membered heterocyclyl, e.g., a 4- to 8-membered or 4- to 10-membered nitrogen-containing heterocyclyl, preferably a 3- to 7-membered heteromonocyclic structure, a fused 7- to 10-membered heterobicyclic structure, or a bridged 6- to 10-membered heterobicyclic structure. Such cyclic structures may be saturated or partially saturated and further contain one or more heteroatoms in addition to carbon atoms, wherein the heteroatoms may be selected from the group consisting of: oxygen, sulfur, and nitrogen. For example, the cyclic structures contain 1 to 4 heteroatoms as defined above. When used to refer to atoms on an aliphatic heterocyclic structure, the term "nitrogen" may include nitrogen that undergoes a substitution reaction. For example, the aliphatic heterocyclyl may comprise "heterocycloalkyl" that may refer to a 3- to 7-membered monocyclic alkyl structure, a fused 7- to 10-membered heterobicyclic structure, or a bridged 6- to 10-membered heterobicyclic structure that is stable and non-aromatic. Such cyclic structures further contain one or more heteroatoms in addition to carbon atoms, wherein the heteroatoms may be selected from the group consisting of: oxygen, sulfur, and nitrogen. For example, the cyclic structures contain 1 to 4 heteroatoms as defined above. The heterocycloalkyl may be substituted or unsubstituted. The aliphatic heterocyclyl may be substituted or unsubstituted. Examples of heterocyclyl include, but are not limited to, the following groups: tetrahydropyrrolyl, piperidinyl, morpholinyl, piperazinyl, azetidinyl,

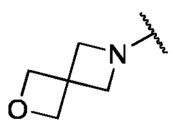,

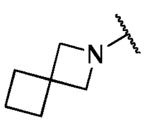, 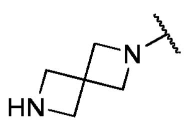, 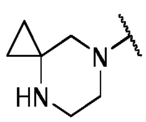, 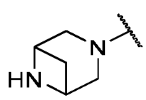, 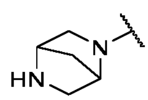, 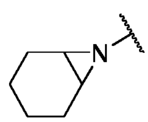, 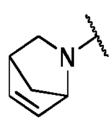,

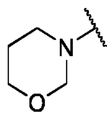, 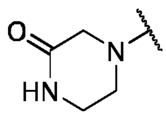, 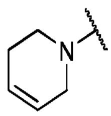, 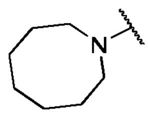, 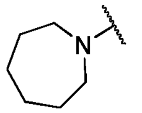, 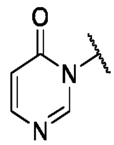, 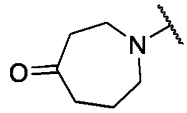,

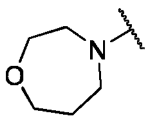, 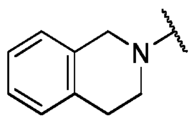, 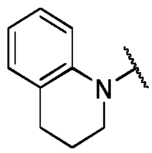, 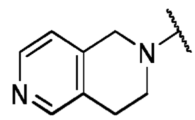, and 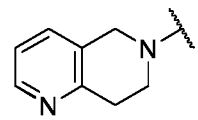.

Moreover, the heterocyclyl may be optionally substituted with one or more substituents selected from $C_1$-$C_6$ alkyl, cyano, amino, -NH($C_1$-$C_6$ alkyl), -N($C_1$-$C_6$ alkyl)$_2$, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, -$C_1$-$C_6$ alkylhydroxy, -$C_1$-$C_6$ alkyl-$C_1$-$C_6$ alkoxy, halogen, halogenated $C_1$-$C_6$ alkyl, halogenated $C_1$-$C_6$ alkoxy, -S(O)$C_1$-$C_6$ alkyl, -S(O)$_2$$C_1$-$C_6$ alkyl, -S(O)$_2$N$R_e$$R_f$, or -C(O)N$R_e$$R_f$, or substituted with one or more substituents selected from halogen, $C_1$-$C_6$ alkyl, $C_1$-$C_6$ alkoxy, -$C_1$-$C_6$ alkylhydroxy, hydroxy, oxo, amino, -NH($C_1$-$C_6$ alkyl), - N($C_1$-$C_6$ alkyl)$_2$, -O-phenyl, or phenyl, wherein $R_e$ and $R_f$ are each independently selected from H, $C_1$-$C_6$ alkyl, or -$C_1$-$C_6$ alkylhydroxy, or $R_e$ and $R_f$, together with the nitrogen atom to which they are connected, form a 4- to 8-membered nitrogen-containing heterocyclyl or 5- or 6-membered nitrogen-containing heteroaryl.

In the present application, the term "aliphatic heterocyclylene" generally refers to a residue derived from an alicyclic ring by the removal of two hydrogen atoms from carbon atoms. The aliphatic heterocyclylene may be substituted or unsubstituted.

In the present application, the term "ring-forming atom" generally refers to an atom contained in a cyclic structure. For example, a ring-forming atom may be a carbon atom in a benzene ring or may be a nitrogen atom in a pyridine ring. When a hydrogen atom is connected to a ring-forming atom, the ring-forming atom may be substituted or unsubstituted.

In the present application, the term "each independently" generally means that a variable applies in any case irrespective of the presence or absence of variables having the same or different definitions in the same compound. For example, the variable may refer to the type or number of substituents in the compound, the type of atoms in the compound, and so on. For example, where R occurs twice in a compound and R is defined as "independently carbon or nitrogen", both R may be carbon, both R may be nitrogen, or one R may be carbon while the other R is nitrogen.

In the present application, the term "optional" or "optionally" generally means that the event or circumstance subsequently described may, but not necessarily occur, and that the description includes instances where the event or circumstance occurs or does not occur. For example, "heterocyclyl group optionally substituted with alkyl" means that alkyl may be, but not necessarily present, and that the description may include instances where the heterocyclyl group is substituted or not substituted with alkyl.

In the present application, the term "substituted" generally means that one or more hydrogen atoms in the group, for example, up to 5 (e.g., 1 to 3) hydrogen atoms, are each independently substituted by a corresponding number of substituents. A substituent is only in its possible chemical position, and those skilled in the art will be able to determine (by experiments or theories) possible or impossible substitution without undue efforts. For example, it may be unstable when amino or hydroxy having a free hydrogen is bound to a carbon atom having an unsaturated (such as olefin) bond.

In the present application, the term "0 or more (e.g., 0 or 1 or more, 0 or 1, or 0) methylene units are replaced" generally means that when the structure comprises one or more methylene units, the one or more methylene units may be unreplaced, or may be replaced by one or more groups that are not methylene (e.g., -NHC(O)-, -C(O)NH-, -C(O)-, -OC(O)-, -C(O)O-, -NH-, -O-, -S-, -SO-, -$SO_2$-, -PH-, -P(=O)H-, -$NHSO_2$-, -$SO_2$NH-, -C(=S)-, -C(=NH)-, -N=N-, -C=N-, -N=C- or -C(=$N_2$)-).

In the present application, the "linking" of group X to group Y may generally be in any orientation, which generally means that when group X is used for linker Y and group Z, two or more linking sites of group X may be linked arbitrarily to either group Y or group Z.

In the present application, the term "compound" generally refers to a substance having two or more different elements. For example, the compound of the present application may be an organic compound. For example, the compound described herein may be a compound having a molecular weight of 500 or greater, a compound having a molecular weight of 1000 or less, a compound having a molecular weight of 1000 or greater, or a compound having a molecular weight of 10,000 or greater, or 100,000 or greater. In the present application, the compound may further refer to a compound that involves linking by a chemical bond, for example, a compound where one or more molecules having a molecular weight of 1000 or less are linked, via a chemical bond, to a biological macromolecule, wherein the biological macromolecule may be polysaccharide, protein, nucleic acid, polypeptide, and the like. For example, the compound of

the present application may be a compound comprising a protein and one or more molecules having a molecular weight of 1000 or less linked to the protein, a compound comprising a protein and one or more molecules having a molecular weight of 10,000 or less linked to the protein, or a compound comprising a protein and one or more molecules having a molecular weight of 100,000 or less linked to the protein.

In the present application, the terms such as "alkyl", "alkenyl" and "cycloalkyl" may be preceded by a notation to indicate the number of atoms present in the group under particular circumstances as in $C_1$-$C_4$ alkyl, $C_3$-$C_7$ cycloalkoxy, and $C_1$-$C_4$ alkylcarbonylamino and the like, as known to those skilled in the art, and the subscript numeral following "C" indicates the number of carbon atoms present in the group. For example, $C_3$ alkyl refers to an alkyl group containing three carbon atoms (e.g., n-propyl or isopropyl); in $C_{1-10}$, members of the group may contain any number of carbon atoms within the range of 1-10.

One or more hydrogen atoms in the group, for example, up to 5 (e.g., 1 to 3) hydrogen atoms, are each independently substituted by a corresponding number of substituents. A substituent is only in its possible chemical position, and those skilled in the art will be able to determine (by experiments or theories) possible or impossible substitution without undue efforts. For example, it may be unstable when amino or hydroxy having a free hydrogen is bound to a carbon atom having an unsaturated (such as olefin) bond.

In the present application, the compound of the present application includes tautomers, mesomers, racemates, enantiomers, and/or diastereoisomers thereof. In the present application, the term "diastereoisomer" generally refers to a stereoisomer that has two or more chiral centers and whose molecules are not mirror images of each other. Diastereoisomers may have different physical properties, e.g., melting points, boiling points, spectral properties, and reactivities. In the present application, the terms "tautomer" and "tautomeric form" are used interchangeably and generally refer to structural isomers of different energies that can be converted into each other by crossing a low energy barrier. For example, proton tautomers (also known as prototropic tautomers) include interconversions by proton migration, such as keto-enol isomerization and imine-enamine isomerization. Valence tautomers include interconversions by recombination of some bonding electrons. In the present application, the term "mesomer" generally means that the molecule contains asymmetric atoms but the total optical rotation is zero due to the presence of symmetric factors. The term "racemate" or "racemic mixture" refers to a composition of two enantiomeric substances in equimolar amounts.

In the present application, certain atoms of the compound of the present application may be present in one or more isotopic forms. For example, hydrogen may occur as protium ($^1H$), deuterium ($^2H$), and tritium ($^3H$), and carbon may naturally occur as three different isotopes ($^{12}C$, $^{13}C$, and $^{14}C$). Examples of isotopes that can be incorporated into the compound of the present application also include but are not limited to $^{15}N$, $^{18}O$, $^{17}O$, $^{18}F$, $^{32}P$, $^{33}P$, $^{129}I$, $^{131}I$, $^{123}I$, $^{124}I$, $^{125}I$, or similar isotopes. Thus, one or more of such isotopes may be enriched in the compounds of the present application relative to the natural abundance of such isotopes. Such isotopically enriched compounds can be used for a variety of purposes, as known to those skilled in the art. For example, replacement with heavy isotopes such as deuterium ($^2H$) may offer certain therapeutic advantages, possibly due to higher metabolic stability. For example, the natural abundance of deuterium ($^2H$) is about 0.015%. Accordingly, one out of about 6500 hydrogen atoms is a deuterium atom. Accordingly, the deuterium abundance of one or more sites (as the case may be) in a deuterium-containing compound of the present application is greater than 0.015%. Unless otherwise indicated, the structures described herein may also include compounds that differ only in the presence or absence of one or more isotopically enriched atoms. For example, compounds having a structure identical to the structure disclosed herein except for the substitution of the hydrogen atom by deuterium or tritium or the substitution of the carbon atom by carbon 13 or carbon 14 shall fall within the scope of the present application.

In the present application, the term "pharmaceutical composition" generally refers to a mixture containing one or more of the compounds described herein or a physiologically/pharmaceutically acceptable salt or pro-drug thereof and other chemical components, and other components such as physiologically/pharmaceutically acceptable carriers and excipients. The pharmaceutical composition may promote the administration to an organism and facilitate the absorption of the active ingredient, thereby exerting biological activities. For the preparation of conventional pharmaceutical compositions, reference can be made to Chinese Pharmacopoeia. The pharmaceutical composition may be in the form of a sterile aqueous or oily suspension for injection for intramuscular and subcutaneous administration. The suspension may be prepared according to a known technique using those suitable dispersing agents or wetting agents and suspending agents described above. The sterile formulation for injection may also be a sterile solution or suspension for injection prepared in a parenterally acceptable non-toxic diluent or solvent, e.g., a solution prepared in 1,3-butanediol. In addition, a sterile fixed oil may be conveniently used as a solvent or a suspending medium. For example, any blend fixed oil including synthetic mono- or di-glycerides can be used. In addition, fatty acids such as oleic acid may also be used in the preparation of injections.

In the present application, the term "pharmaceutically acceptable salt" generally refers to a salt of the compound of the present application or a ligand-drug conjugate, or a salt of the compound described herein. Such salts may possess safety and/or efficacy when used in mammals and may possess the required biological activity, and the antibody-antibody drug conjugate compound of the present application may form a salt with an acid. Non-limiting examples of pharmaceutically acceptable salts include: hydrochloride, hydrobromide, hydroiodide, sulfate, bisulfate, citrate, acetate, succinate, ascorbate, oxalate, nitrate, sorbate, hydrogen phosphate, dihydrogen phosphate, salicylate, hydrogen citrate, tartrate, maleate, fumarate, formate, benzoate, mesylate, ethanesulfonate, benzenesulphonate, and p-toluenesulfonate.

In the present application, the term "conjugate" generally refers to a compound prepared by subjecting the compounds of the present application to one or more chemical reactions or by linking the compounds to one another via one or more linking structures such as bridges, spacers, or linkers.

In the present application, the term "pharmaceutically acceptable carrier" generally refers to a carrier or vehicle for providing therapeutic agents, such as antibodies or polypeptides, genes, and other therapeutic agents. The term refers to any pharmaceutical carrier that does not itself induce the production of antibodies harmful to the individual receiving the composition and can be given without producing undue toxicity. Suitable carriers may be macromolecules that are large and metabolized slowly, such as proteins, polysaccharides, polylactic acids, polyglycolic acids, poly(amino acid)s, amino acid copolymers, lipid aggregates, and inactivated virus particles. Such carriers are well known to those skilled in the art. Pharmaceutically acceptable carriers in therapeutic compositions may include liquids such as water, saline, glycerol, and ethanol. Auxiliary substances, such as wetting or emulsifying agents, or pH buffering substances, may also be present in these carriers.

In the present application, the term "*in vivo*" generally refers to an event that occurs in a subject’s body. In the present application, the term "*in vitro*" generally refers to an event that occurs outside a subject’s body. For example, *in vitro* assays include any assay conducted outside of a subject. *In vitro* assays include cell-based assays in which cells, alive or dead, are employed. *In vitro* assays also include a cell-free assay in which no intact cells are employed.

In the present application, the terms "treatment" and "treating" generally refer to a method for achieving beneficial or desired results, including but not limited to therapeutic benefits. Therapeutic benefits include but are not limited to eradication, inhibition, reduction, or amelioration of the underlying disorder being treated. In addition, therapeutic benefits are achieved by eradicating, inhibiting, reducing, or ameliorating one or more physiological symptoms associated with the underlying disorder, and thus improvements are observed in the patient, but the patient may still be afflicted with the underlying disorder.

In the present application, the terms "prevention" and "preventing" generally refer to a method for achieving beneficial or desired outcomes, including but not limited to prophylactic benefits. For the purpose of prophylactic benefits, a pharmaceutical composition can be administered to a patient at risk of developing a particular disease, or to a patient who reports he/she has one or more physiological symptoms of the disease, even if the patient has not yet been diagnosed with the disease. In the present application, the term "subject" or "patient" generally refers to a human (i.e., a male or female in any age group, e.g., a pediatric subject (e.g., an infant, a child, or an adolescent) or an adult subject (e.g., a young adult, a middle-aged adult or a senior adult)) and/or other primates (e.g., a cynomolgus monkey or a rhesus monkey); a mammal, including commercially relevant mammals, such as cows, pigs, horses, sheep, goats, cats and/or dogs; and/or birds, including commercially relevant birds such as chickens, ducks, geese, quail and/or turkeys.

In the present application, the term "comprise" "comprising", "contain" or "containing" is generally intended to include the explicitly specified features without excluding other elements. The terms "or more/greater" and "or less" generally refer to situations where the number itself is included.

In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% above or below the stated value.

**Detailed Description of the Invention**

In one aspect, the present application provides a compound or a tautomer, a mesomer, a racemate, an enantiomer or a diastereoisomer thereof, or a mixture thereof, or a pharmaceutically acceptable salt thereof, wherein the compound may comprise the structure of formula (II):

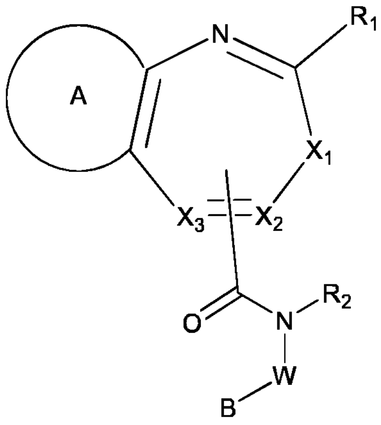

(II),

wherein $R_1$ and $R_2$ may each independently be any group, $X_1$, $X_2$, and $X_3$ may each independently be any atom optionally substituted by any group, A may be an optionally substituted cyclic structure, W is absent or may be any group, and B may be an optionally substituted aromatic ring,

wherein B may be substituted with one or more optionally substituted amino groups. For example, the substitution with the group comprising $R_2$ and W-B may be positioned on $X_1$, $X_2$, and/or $X_3$.

In one aspect, the present application provides a compound or a tautomer, a mesomer, a racemate, an enantiomer or a diastereoisomer thereof, or a mixture thereof, or a pharmaceutically acceptable salt thereof, wherein the compound may comprise the structure of formula (II-a):

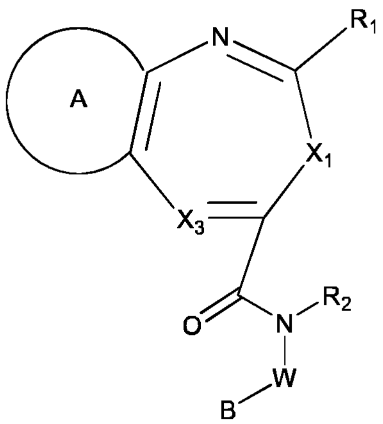

(II-a),

wherein $R_1$ and $R_2$ may each independently be any group, $X_1$ and $X_3$ may each independently be any atom optionally substituted by any group, A may be an optionally substituted cyclic structure, W is absent or may be any group, and B may be an optionally substituted aromatic ring,

wherein B may be substituted with one or more optionally substituted amino groups.

For example, in the compound comprising the structure of formula (II) or formula (II-a), $R_1$ may be an optionally substituted amino.

For example, in the compound comprising the structure of formula (II) or formula (II-a), $X_1$ may be an optionally substituted $-CH_2-$.

For example, in the compound comprising the structure of formula (II) or formula (II-a), $X_1$ may be substituted with one or more $R_{X1-1}$, wherein the one or more $R_{X1-1}$ may each independently be selected from any group. For example, $X_1$ may be substituted with 1, 2, 3, 4, or 5 $R_{X1-1}$.

For example, in the compound comprising the structure of formula (II) or formula (II-a), the one or more $R_{X1-1}$ may each independently be selected from hydrogen and optionally substituted $C_1$-$C_6$ alkyl, such as optionally substituted methyl, optionally substituted ethyl, or optionally substituted propyl; when the one or more $R_{X1-1}$ may comprise methylene units, the methylene units of the one or more $R_{X1-1}$ may each independently be unreplaced, or each independently be replaced by any structure. For example, the methylene unit of the 1, 2, 3, 4, or 5 $R_{X1-1}$ may each independently be replaced by any structure.

For example, in the compound comprising the structure of formula (II) or formula (II-a), the two $R_{X1-1}$ may, together with the atom to which they are connected, form an optionally substituted cyclic structure. For example, in the compound comprising the structure of formula (II) or formula (II-a), the two $R_{X1-1}$ may, together with the atom to which they are connected, form an optionally substituted alcyl. For example, in the compound comprising the structure of formula (II) or formula (II-a), the two $R_{X1-1}$ may, together with the atom to which they are connected, form an optionally substituted cyclopropyl.

For example, in the compound comprising the structure of formula (II), $X_2$ may be selected from optionally substituted -CH- or -N-.

For example, in the compound comprising the structure of formula (II), $X_2$ may be substituted with $R_{X2-1}$, wherein $R_{X2-1}$ is selected from any group.

For example, in the compound comprising the structure of formula (II), $R_{X2-1}$ may be selected from hydrogen and optionally substituted $C_1$-$C_6$ alkyl, such as optionally substituted methyl, optionally substituted ethyl, or optionally substituted propyl; when $R_{X2-1}$ may comprise methylene units, the methylene units of $R_{X2-1}$ may each independently be unreplaced, or each independently be replaced by any structure.

For example, in the compound comprising the structure of formula (II), $R_{X2-1}$ may comprise an optionally substituted ethyl.

For example, in the compound comprising the structure of formula (II), the methylene unit that $R_{X2-1}$ may comprise may be unreplaced, or may be replaced by -C(=O)- and/or optionally substituted - NH-.

For example, in the compound comprising the structure of formula (II), $R_{X2-1}$ may comprise an optionally substituted $-C(=O)NH_2$.

For example, in the compound comprising the structure of formula (II), $R_{X2-1}$ may be substituted by $-R_2$ and -W-B. For example, $-R_2$ and -W-B may be $-R_2$ and -W-B in the general formula. For example, when $R_{X2-1}$ may be substituted with $-R_2$ and -W-B, the substitution with $-R_2$ and -W-B in the general formula may no longer be positioned on $X_1$ and/or $X_3$. For example, when $X_2$ may be substituted with $-R_2$ and -W-B, the substitution with $-R_2$ and -W-B in the general formula may no longer be positioned on $X_1$ and/or $X_3$.

For example, in the compound comprising the structure of formula (II) or formula (II-a), $X_3$ may be selected from optionally substituted -CH- or -N-.

For example, in the compound comprising the structure of formula (II) or formula (II-a), $X_3$ may be substituted with $R_{X3-1}$, wherein $R_{X3-1}$ is selected from any group.

For example, in the compound comprising the structure of formula (II) or formula (II-a), $R_{X3-1}$ may be selected from hydrogen and optionally substituted $C_1$-$C_6$ alkyl, such as optionally substituted methyl, optionally substituted ethyl, or optionally substituted propyl; when $R_{X3-1}$ may comprise methylene units, the methylene units of $R_{X3-1}$ may each independently be unreplaced, or each independently be replaced by any structure.

For example, in the compound comprising the structure of formula (II) or formula (II-a), $R_{X3-1}$ may comprise an optionally substituted methyl.

For example, in the compound comprising the structure of formula (II) or formula (II-a), $R_2$ may each independently be selected from hydrogen and optionally substituted $C_1$-$C_6$ alkyl; when $R_2$ may comprise methylene units, the methylene units of $R_2$ may each independently be unreplaced, or each independently be replaced by any structure.

For example, in the compound comprising the structure of formula (II) or formula (II-a), $R_2$ may each independently be selected from optionally substituted methyl, optionally substituted ethyl, optionally substituted propyl, optionally substituted butyl, and optionally substituted pentyl.

For example, in the compound comprising the structure of formula (II) or formula (II-a), the methylene unit of $R_2$ may each independently be unreplaced or replaced by -O-, -C(=O)-, and/or optionally substituted -NH-.

For example, in the compound comprising the structure of formula (II) or formula (II-a), $R_2$ is selected from the group consisting of: optionally substituted

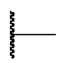,

optionally substituted

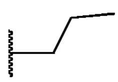,

optionally substituted

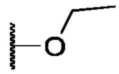

,

optionally substituted

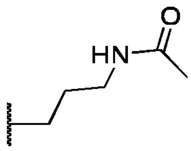

,

optionally substituted

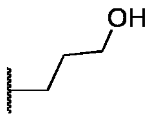

,

optionally substituted

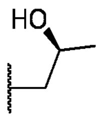

,

optionally substituted

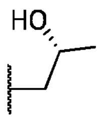

,

optionally substituted

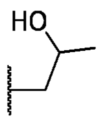

,

optionally substituted

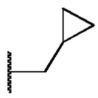

,

optionally substituted

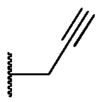

,

optionally substituted

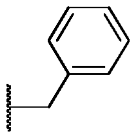,

and optionally substituted

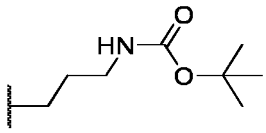.

For example, in the compound comprising the structure of formula (II) or formula (II-a), $R_2$ may be selected from the group consisting of: optionally substituted

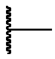,

optionally substituted

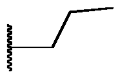,

optionally substituted

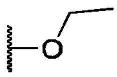,

and optionally substituted

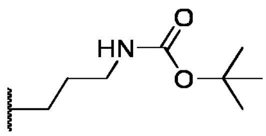.

For example, in the compound comprising the structure of formula (II) or formula (II-a), $R_2$ is selected from the group consisting of: optionally substituted

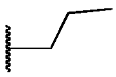,

optionally substituted

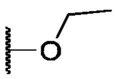,

optionally substituted

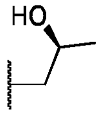

,

optionally substituted

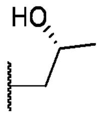

,

and optionally substituted

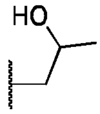

.

For example, in the compound comprising the structure of formula (II) or formula (II-a), $R_2$ is selected from the group consisting of: optionally substituted

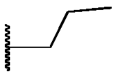

,

optionally substituted

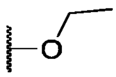

,

and optionally substituted

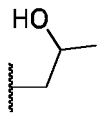

.

For example, in the compound comprising the structure of formula (II) or formula (II-a), $R_2$ is selected from the group consisting of: optionally substituted

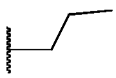

and optionally substituted

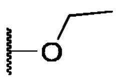

.

For example, in the compound comprising the structure of formula (II) or formula (II-a), $R_2$ may each independently be substituted with one or more $R_{2-1}$, wherein the one or more $R_{2-1}$ may be selected from any group. For example, $R_2$ may be substituted with 1, 2, 3, 4, or 5 $R_{2-1}$.

For example, in the compound comprising the structure of formula (II) or formula (II-a), the one or more $R_{2-1}$ may be selected from the group consisting of: hydrogen, halogen, optionally substituted alkyl, optionally substituted

alcyl, and an optionally substituted aryl; when the one or more $R_{2-1}$ may comprise methylene units, the methylene units of the one or more $R_{2-1}$ may be each independently unreplaced, or may each independently be replaced by any structure. For example, the methylene unit of the 1, 2, 3, 4, or 5 $R_{2-1}$ may each independently be replaced by any structure.

For example, in the compound comprising the structure of formula (II) or formula (II-a), the one or more $R_{2-1}$ may be selected from the group consisting of: hydrogen, fluorine, optionally substituted methyl, optionally substituted cyclopropyl, and optionally substituted phenyl.

For example, in the compound comprising the structure of formula (II) or formula (II-a), W is absent, or may each independently be selected from hydrogen and optionally substituted $C_1$-$C_6$ alkylene; when W may comprise methylene units, the methylene units of W may each independently be unreplaced, or may each independently be replaced by any structure.

For example, in the compound comprising the structure of formula (II) or formula (II-a), W may each independently be selected from optionally substituted methylene, optionally substituted ethylene, optionally substituted propylene, optionally substituted butylene, and optionally substituted pentylene.

For example, in the compound comprising the structure of formula (II) or formula (II-a), the methylene unit of W may each independently be unreplaced or replaced by -O-, -C(=O)-, and/or optionally substituted -NH-.

For example, in the compound comprising the structure of formula (II) or formula (II-a), W may be selected from the group consisting of: optionally substituted

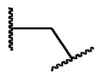
,

optionally substituted

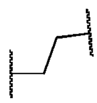
,

optionally substituted

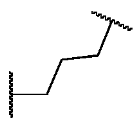
,

optionally substituted

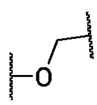
,

and optionally substituted

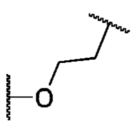
.

For example, in the compound comprising the structure of formula (II) or formula (II-a), W may be selected from the group consisting of: optionally substituted

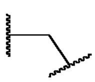

and optionally substituted

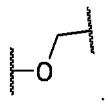

For example, in the compound comprising the structure of formula (II) or formula (II-a), B may be selected from the group consisting of: an optionally substituted aryl and optionally substituted heteroaryl.

For example, in the compound comprising the structure of formula (II) or formula (II-a), B may be selected from the group consisting of: optionally substituted phenyl, optionally substituted naphthyl, optionally substituted pyridinyl, optionally substituted pyrrolyl, optionally substituted thienyl, optionally substituted furanyl, and optionally substituted pyrazinyl.

For example, in the compound comprising the structure of formula (II) or formula (II-a), B may be substituted with one or more $R_{B-1}$, wherein the one or more $R_{B-1}$ may each independently be selected from any group. For example, B may be substituted with 1, 2, 3, 4, or 5 $R_{B-1}$.

For example, in the compound comprising the structure of formula (II) or formula (II-a), the one or more $R_{B-1}$ may each independently be selected from the group consisting of: hydrogen, halogen, and optionally substituted $C_1$-$C_6$ alkyl, such as optionally substituted methyl, optionally substituted ethyl, or optionally substituted propyl; when the one or more $R_{B-1}$ may comprise methylene units, the methylene units of the one or more $R_{B-1}$ may each independently be unreplaced, or each independently be replaced by any structure. For example, the methylene unit of the 1, 2, 3, 4, or 5 $R_{B-1}$ may each independently be replaced by any structure.

For example, in the compound comprising the structure of formula (II) or formula (II-a), the one or more $R_{B-1}$ may each independently be selected from the group consisting of: hydrogen, fluorine, and optionally substituted methyl.

For example, in the compound comprising the structure of formula (II) or formula (II-a), B may be substituted with one or more optionally substituted amino groups, wherein the one or more optionally substituted amino groups may be each independently substituted with any structure. For example, B may be substituted with 1, 2, 3, 4, or 5 amino groups.

For example, in the compound comprising the structure of formula (II) or formula (II-a), B may be substituted with one or more optionally substituted amino groups, wherein the one or more amino groups may each independently be substituted with hydrogen or optionally substituted $C_1$-$C_6$ alkyl, e.g., optionally substituted methyl, optionally substituted ethyl, or optionally substituted propyl. For example, in the compound comprising the structure of formula (II) or formula (II-a), A may be selected from the group consisting of: optionally substituted alcyl, optionally substituted aliphatic heterocyclyl, optionally substituted aryl, and optionally substituted heteroaryl.

For example, in the compound comprising the structure of formula (II) or formula (II-a), A may be selected from the group consisting of: optionally substituted phenyl, optionally substituted pyridinyl, optionally substituted pyrrolyl, optionally substituted thienyl, optionally substituted furanyl, and optionally substituted pyrazinyl. For example, in the compound comprising the structure of formula (II) or formula (II-a), A may be selected from optionally substituted phenyl.

For example, in the compound comprising the structure of formula (II) or formula (II-a), A may be substituted with one or more $R_{A-1}$, wherein the one or more $R_{A-1}$ may each independently be selected from any group. For example, A may be substituted with 1, 2, 3, 4, or 5 $R_{A-1}$.

For example, in the compound comprising the structure of formula (II) or formula (II-a), $R_{A-1}$ is absent or may be selected from optionally substituted $C_1$-$C_6$ alkyl; when $R_{A-1}$ may comprise methylene units, the methylene units of $R_{A-1}$ may each independently be unreplaced, or each independently be replaced by any structure.

For example, in the compound comprising the structure of formula (II) or formula (II-a), $R_{A-1}$ may be selected from optionally substituted methyl, optionally substituted ethyl, optionally substituted propyl, or optionally substituted butyl.

For example, in the compound comprising the structure of formula (II) or formula (II-a), the methylene unit that $R_{A-1}$ may comprise may be unreplaced, or may be replaced by a group selected from the group consisting of: -S(=O)-, -C(=O)-, $-S(=O)_2-$, -O-, -S-, optionally substituted -NH-, optionally substituted alkylene, optionally substituted alkenylene, optionally substituted alkynylene, optionally substituted alcylene, optionally substituted aliphatic heterocyclylene, optionally substituted arylene, and optionally substituted heteroarylene.

For example, in the compound comprising the structure of formula (II) or formula (II-a), $R_{A-1}$ is absent, or may be selected from the group consisting of: optionally substituted $CH_3$-, optionally substituted $CH(=CH_2)$-, optionally substituted HC(=O)-, optionally substituted HOC(=O)-, optionally substituted HC(=O)O-, optionally substituted $NH_2$-, optionally substituted HO-, optionally substituted HS-, optionally substituted HS(=O)-, optionally substituted $HS(=O)_2$-, optionally substituted $CH_3CH_2$-, optionally substituted $CH_2$=CH-, optionally substituted HC=C-, optionally substituted $HOCH_2$-, optionally substituted $CH_3O$-, optionally substituted $CH_3NH$-, optionally substituted $NH_2CH_2$-, optionally substituted $HS(=O)_2$-NH-, optionally substituted $NH_2$-$S(=O)_2$-, optionally substituted HS-$CH_2$-, optionally substituted $CH_3S$-, optionally substituted HS(=O)-$CH_2$-, optionally substituted $CH_3$-S(=O)-, optionally substituted $HS(=O)_2$-$CH_2$-, optionally sub-

stituted $CH_3$-S(=O)$_2$-, optionally substituted $NH_2C$(=O)-, optionally substituted HC(=O)NH-, optionally substituted HC(=O)$CH_2$-, optionally substituted HC(=O)$NHCH_2$-, optionally substituted $CH_3$NHC(=O)-, optionally substituted

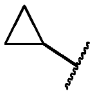,

optionally substituted

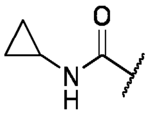,

optionally substituted

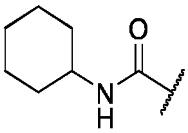,

optionally substituted

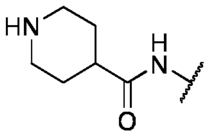,

optionally substituted

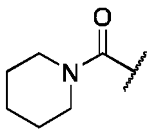,

optionally substituted

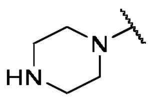,

optionally substituted

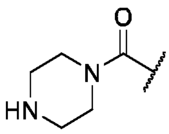,

optionally substituted

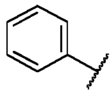,

optionally substituted

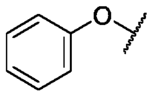,

optionally substituted

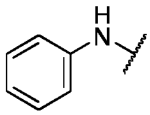,

optionally substituted

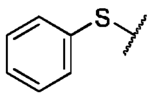,

optionally substituted

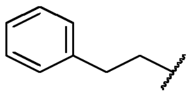,

optionally substituted

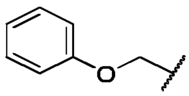,

optionally substituted

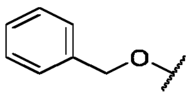,

optionally substituted

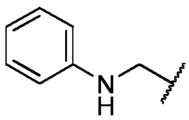,

optionally substituted

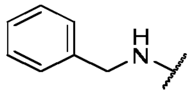,

optionally substituted

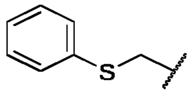,

optionally substituted

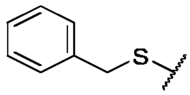,

optionally substituted

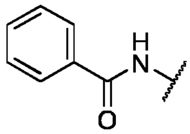,

optionally substituted

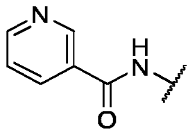,

optionally substituted

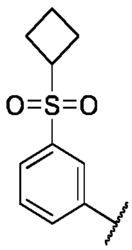,

optionally substituted

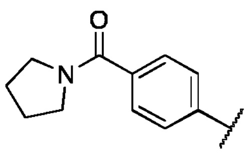,

optionally substituted

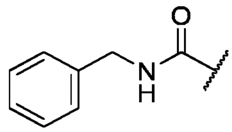,

optionally substituted

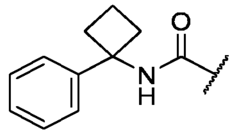,

optionally substituted

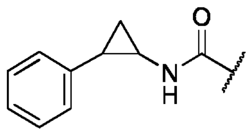,

optionally substituted

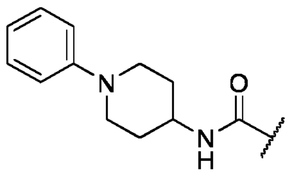,

optionally substituted

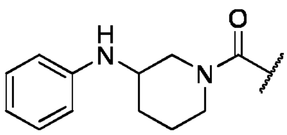,

optionally substituted

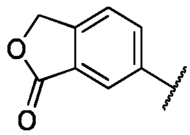,

optionally substituted

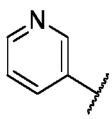,

and optionally substituted

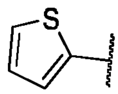

For example, in the compound comprising the structure of formula (II) or formula (II-a), $R_{A-1}$ comprises optionally substituted

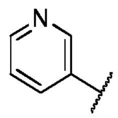

For example, in the compound comprising the structure of formula (II) or formula (II-a), the one or more $R_{A-1}$ may each independently be substituted with one or more $R_{A-2}$, wherein the one or more $R_{A-2}$ may each independently be selected from any group. For example, $R_{A-1}$ may be substituted with 1, 2, 3, 4, or 5 $R_{A-2}$.

For example, in the compound comprising the structure of formula (II) or formula (II-a), $R_{A-2}$ may be absent or may be selected from optionally substituted $C_1$-$C_6$ alkyl, such as optionally substituted methyl, optionally substituted ethyl, or optionally substituted propyl; when $R_{A-2}$ may comprise methylene units, the methylene units of $R_{A-2}$ may each independently be unreplaced, or each independently be replaced by any structure.

For example, in the compound comprising the structure of formula (II) or formula (II-a), $R_{A-2}$ may comprise hydrogen, halogen, optionally substituted methyl, and/or optionally substituted hydroxy. For example, in the compound comprising the structure of formula (II) or formula (II-a), the one or more $R_{A-2}$ may each independently be substituted with one or more $R_{A-3}$, wherein the one or more $R_{A-3}$ may each independently be selected from any group.

For example, in the compound comprising the structure of formula (II) or formula (II-a), $R_{A-3}$ may comprise optionally substituted methyl and/or optionally substituted hydroxy.

In one aspect, the present application provides a compound or a tautomer, a mesomer, a racemate, an enantiomer or a diastereoisomer thereof, or a mixture thereof, or a pharmaceutically acceptable salt thereof, wherein the compound may comprise the structure of formula (II):

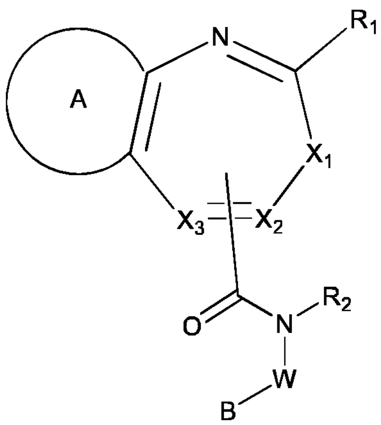

(II),

wherein $R_1$ may be optionally substituted amino; $X_1$ may be selected from optionally substituted - $CH_2$-; $X_2$ may be selected from optionally substituted -CH- or -N-, and $X_2$ may be substituted with $R_{X2-1}$, wherein $R_{X2-1}$ may comprise optionally substituted -C(=O)$NH_2$, and $R_{X2-1}$ may be substituted with -$R_2$ and -W-B; $X_3$ may be selected from optionally substituted -CH- or -N-;

$R_2$ may be selected from the group consisting of: optionally substituted

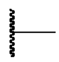,

optionally substituted

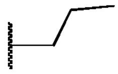

,

optionally substituted

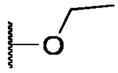

,

and optionally substituted

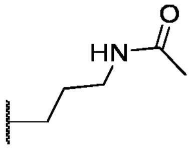

,

and $R_2$ may each independently be substituted with one or more $R_{2-1}$, wherein the one or more $R_{2-1}$ may be selected from the group consisting of: hydrogen, fluorine, optionally substituted methyl, optionally substituted cyclopropyl, and optionally substituted phenyl;

W may be selected from the group consisting of: optionally substituted

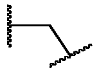

,

optionally substituted

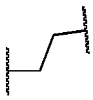

,

optionally substituted

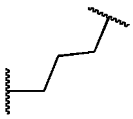

,

optionally substituted

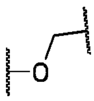

,

and optionally substituted

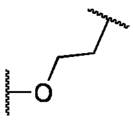

;

B may be selected from the group consisting of: optionally substituted phenyl, optionally substituted naphthyl, optionally substituted pyridinyl, optionally substituted pyrrolyl, optionally substituted thienyl, optionally substituted furanyl, and optionally substituted pyrazinyl, and B may be substituted with one or more optionally substituted amino groups;

A may be selected from the group consisting of: optionally substituted phenyl, optionally substituted pyridinyl, optionally substituted pyrrolyl, optionally substituted thienyl, optionally substituted furanyl, and optionally substituted pyrazinyl; A may be substituted with one or more $R_{A-1}$, wherein $R_{A-1}$ is absent or may comprise: optionally substituted $CH_3$-, optionally substituted $CH(=CH_2)$-, optionally substituted HC(=O)-, optionally substituted HOC(=O)-, optionally substituted HC(=O)O-, optionally substituted $NH_2$-, optionally substituted HO-, optionally substituted HS-, optionally substituted HS(=O)-, optionally substituted $HS(=O)_2$-, optionally substituted $CH_3CH_2$-, optionally substituted $CH_2$=CH-, optionally substituted HC=C-, optionally substituted $HOCH_2$-, optionally substituted $CH_3O$-, optionally substituted $CH_3NH$-, optionally substituted $NH_2CH_2$-, optionally substituted $HS(=O)_2$-NH-, optionally substituted $NH_2$-$S(=O)_2$-, optionally substituted HS-$CH_2$-, optionally substituted $CH_3S$-, optionally substituted HS(=O)-$CH_2$-, optionally substituted $CH_3$-S(=O)-, optionally substituted $HS(=O)_2$-$CH_2$-, optionally substituted $CH_3$-$S(=O)_2$-, optionally substituted $NH_2C(=O)$-, optionally substituted HC(=O)NH-, optionally substituted HC(=O)$CH_2$-, optionally substituted HC(=O)$NHCH_2$-, optionally substituted $CH_3NHC(=O)$-, optionally substituted

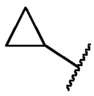,

optionally substituted

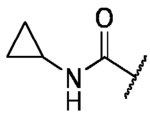,

optionally substituted

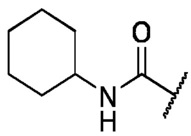,

optionally substituted

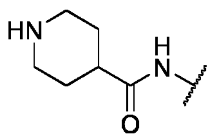,

optionally substituted

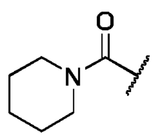,

optionally substituted

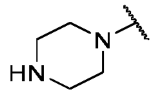,

optionally substituted

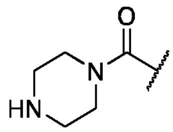,

optionally substituted

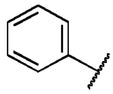,

optionally substituted

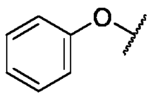,

optionally substituted

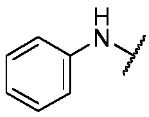,

optionally substituted

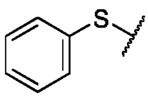,

optionally substituted

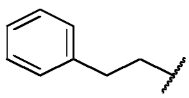,

optionally substituted

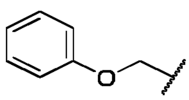,

optionally substituted

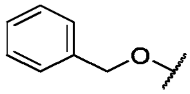,

optionally substituted

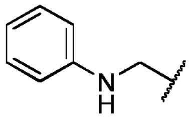,

optionally substituted

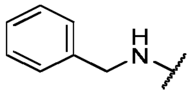,

optionally substituted

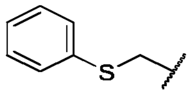,

optionally substituted

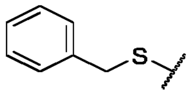,

optionally substituted

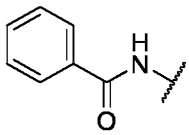,

optionally substituted

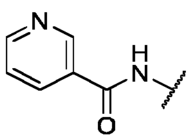,

optionally substituted

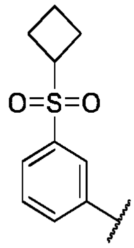,

optionally substituted

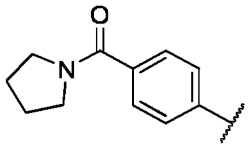,

optionally substituted

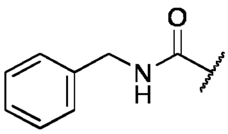,

optionally substituted

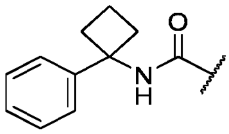,

optionally substituted

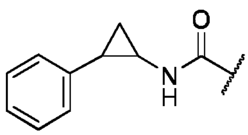,

optionally substituted

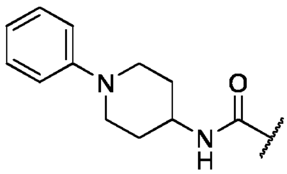,

optionally substituted

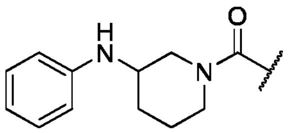

,

optionally substituted

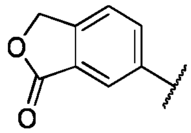

,

optionally substituted

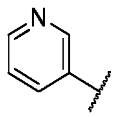

,

and optionally substituted

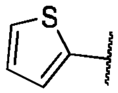

;

one or more

$R_{A-1}$ may each independently be substituted with one or more $R_{A-2}$, wherein $R_{A-2}$ may comprise hydrogen, halogen, optionally substituted methyl, and/or optionally substituted hydroxy; the one or more $R_{A-2}$ may each independently be substituted with one or more $R_{A-3}$, wherein $R_{A-3}$ may comprise optionally substituted methyl and/or optionally substituted hydroxy.

In one aspect, the present application provides a compound or a tautomer, a mesomer, a racemate, an enantiomer or a diastereoisomer thereof, or a mixture thereof, or a pharmaceutically acceptable salt thereof, wherein the compound may comprise the structure of formula (II-a):

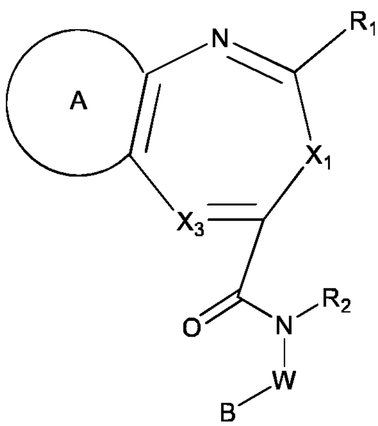

(II-a),

$R_1$ may be optionally substituted amino; $X_1$ may be selected from optionally substituted $-CH_2-$; $X_3$ may be selected from optionally substituted -CH- or -N-;

$R_2$ may be selected from the group consisting of: optionally substituted

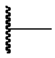

,

optionally substituted

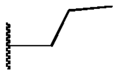

,

optionally substituted

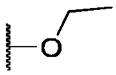

,

and optionally substituted

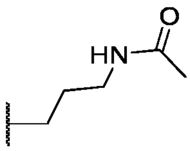

,

and $R_2$ may each independently be substituted with one or more $R_{2-1}$, wherein the one or more $R_{2-1}$ may be selected from the group consisting of: hydrogen, fluorine, optionally substituted methyl, optionally substituted cyclopropyl, and optionally substituted phenyl;

W may be selected from the group consisting of: optionally substituted

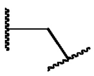

,

optionally substituted

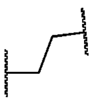

,

optionally substituted

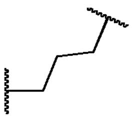

,

optionally substituted

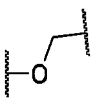

,

and optionally substituted

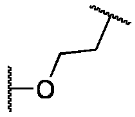

;

B may be selected from the group consisting of: optionally substituted phenyl, optionally substituted naphthyl, optionally substituted pyridinyl, optionally substituted pyrrolyl, optionally substituted thienyl, optionally substituted furanyl, and optionally substituted pyrazinyl, and B may be substituted with one or more optionally substituted amino groups;

A may be selected from the group consisting of: optionally substituted phenyl, optionally substituted pyridinyl, optionally substituted pyrrolyl, optionally substituted thienyl, optionally substituted furanyl, and optionally substituted pyrazinyl; A may be substituted with one or more $R_{A-1}$, wherein $R_{A-1}$ is absent or may comprise: optionally substituted $CH_3$-, optionally substituted $CH(=CH_2)$-, optionally substituted HC(=O)-, optionally substituted HOC(=O)-, optionally substituted HC(=O)O-, optionally substituted $NH_2$-, optionally substituted HO-, optionally substituted HS-, optionally substituted HS(=O)-, optionally substituted $HS(=O)_2$-, optionally substituted $CH_3CH_2$-, optionally substituted $CH_2$=CH-, optionally substituted HC=C-, optionally substituted $HOCH_2$-, optionally substituted $CH_3O$-, optionally substituted $CH_3NH$-, optionally substituted $NH_2CH_2$-, optionally substituted $HS(=O)_2$-NH-, optionally substituted $NH_2$-$S(=O)_2$-, optionally substituted HS-$CH_2$-, optionally substituted $CH_3S$-, optionally substituted HS(=O)-$CH_2$-, optionally substituted $CH_3$-S(=O)-, optionally substituted $HS(=O)_2$-$CH_2$-, optionally substituted $CH_3$-$S(=O)_2$-, optionally substituted $NH_2C(=O)$-, optionally substituted HC(=O)NH-, optionally substituted HC(=O)$CH_2$-, optionally substituted HC(=O)NH$CH_2$-, optionally substituted $CH_3$NHC(=O)-, optionally substituted

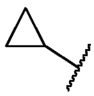

,

optionally substituted

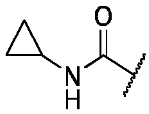

,

optionally substituted

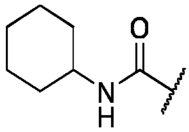

,

optionally substituted

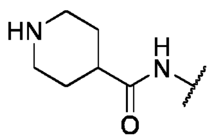

,

optionally substituted

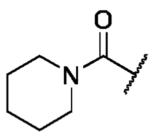

,

optionally substituted

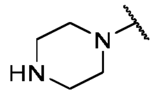,

optionally substituted

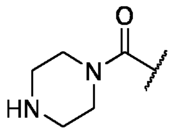,

optionally substituted

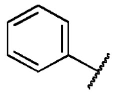,

optionally substituted

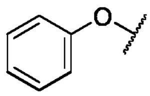,

optionally substituted

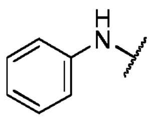,

optionally substituted

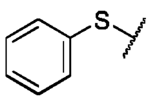,

optionally substituted

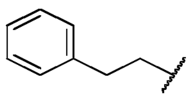,

optionally substituted

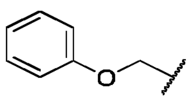,

optionally substituted

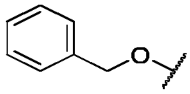,

optionally substituted

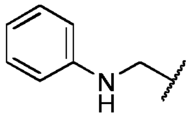,

optionally substituted

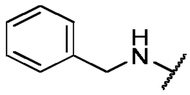,

optionally substituted

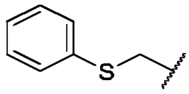,

optionally substituted

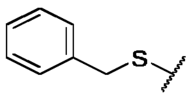,

optionally substituted

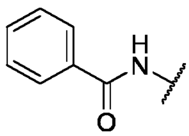,

optionally substituted

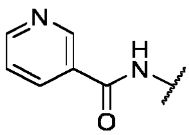,

optionally substituted

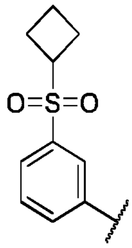

,

optionally substituted

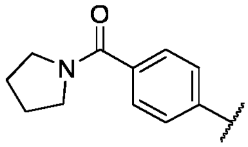

,

optionally substituted

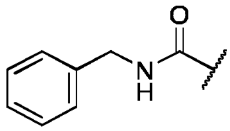

,

optionally substituted

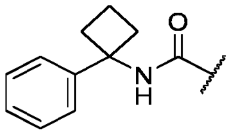

,

optionally substituted

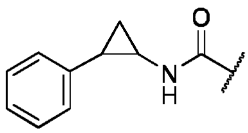

,

optionally substituted

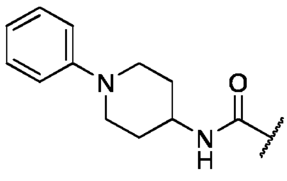

,

optionally substituted

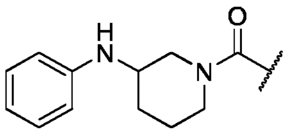,

optionally substituted

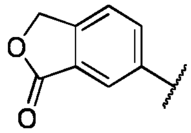,

optionally substituted

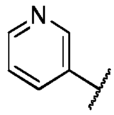,

and optionally substituted

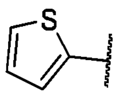;

one or more $R_{A-1}$ may each independently be substituted with one or more $R_{A-2}$, wherein $R_{A-2}$ may comprise hydrogen, halogen, optionally substituted methyl, and/or optionally substituted hydroxy; the one or more $R_{A-2}$ may each independently be substituted with one or more $R_{A-3}$, wherein $R_{A-3}$ may comprise optionally substituted methyl and/or optionally substituted hydroxy.

For example, in the compound comprising the structure of formula (II-a), $R_1$ is amino.

For example, in the compound comprising the structure of formula (II-a), $X_1$ is $-CH_2-$.

For example, in the compound comprising the structure of formula (II-a), $X_3$ is -CH=.

For example, in the compound comprising the structure of formula (II-a), $R_2$ is selected from the group consisting of:

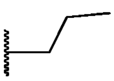, 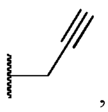, 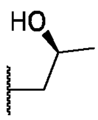, 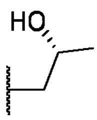, 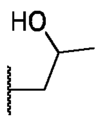, 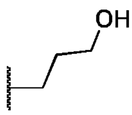, and 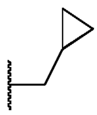.

For example, in the compound comprising the structure of formula (II-a), $R_2$ is selected from

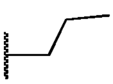 and 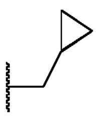.

For example, in the compound comprising the structure of formula (II-a), W may be selected from the group consisting of:

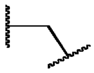 and 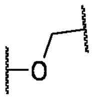.

For example, in the compound comprising the structure of formula (II-a), W is selected from

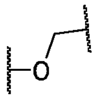.

For example, in the compound comprising the structure of formula (II-a), B is selected from phenyl, and B may be substituted with one or more $R_{B-1}$, wherein the one or more $R_{B-1}$ may each independently be selected from hydrogen, halogen, and $C_1$-$C_6$ alkyl; preferably, $R_{B-1}$ may each independently be selected from hydrogen, fluorine, and methyl.

For example, in the compound comprising the structure of formula (II-a), B is substituted with one or more amino groups, wherein the one or more amino groups may each independently be substituted with hydrogen or $C_1$-$C_6$ alkyl, e.g., methyl, ethyl, or propyl.

For example, in the compound comprising the structure of formula (II-a), A is selected from optionally substituted phenyl or optionally substituted pyridinyl.

For example, in the compound comprising the structure of formula (II-a), A is selected from phenyl, and A may be substituted with one or more $R_{A-1}$, wherein the one or more $R_{A-1}$ may each independently be selected from any group.

For example, in the compound comprising the structure of formula (II-a), A is selected from pyridinyl, and A may be substituted with one or more $R_{A-1}$, wherein the one or more $R_{A-1}$ may each independently be selected from any group.

For example, in the compound comprising the structure of formula (II-a), $R_{A-1}$ is selected from optionally substituted

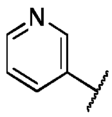,

optionally substituted

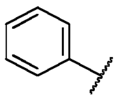,

and optionally substituted $NH_2C(=O)$-.

For example, in the compound comprising the structure of formula (II-a), $R_{A-1}$ is selected from optionally substituted

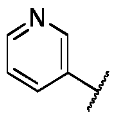.

For example, in the compound comprising the structure of formula (II-a), $R_{A-1}$ is selected from optionally substituted

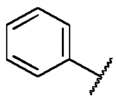.

For example, in the compound comprising the structure of formula (II-a), $R_{A-1}$ is selected from optionally substituted $NH_2C(=O)$-. In one aspect, the present application provides a compound of formula (A) or a tautomer, an enantiomer or a diastereoisomer, or a mixture of isomers, or a pharmaceutically acceptable salt or solvate thereof,

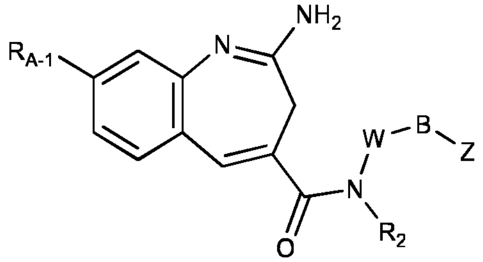

(A)

wherein:

$R_{A-1}$ is selected from $R_{A2-1}$-Lx-;

Lx is selected from single bond, $C_1$-$C_6$ alkylene, -O-, -S-, -C(O)-, -NH-, -C(O)NH-, -NHC(O)-, - C(O)-N($C_1$-$C_6$ alkyl)-, or -N($C_1$-$C_6$ alkylene)-C(O)-;

$R_{A2-1}$ is aryl, heteroaryl, or heterocyclyl, preferably phenyl, pyridinyl, or isobenzofuranonyl, and $R_{A2-1}$ is optionally substituted with one or more substituents $R_{A2-2}$, wherein $R_{A2-2}$ are each independently selected from $C_1$-$C_6$ alkyl, amino, -NH($C_1$-$C_6$ alkyl), -N($C_1$-$C_6$ alkyl)$_2$, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, -$C_1$-$C_6$ alkylhydroxy, -$C_1$-$C_6$ alkyl-$C_1$-$C_6$ alkoxy, halogen, halogenated $C_1$-$C_6$ alkyl, halogenated $C_1$-$C_6$ alkoxy, or - C(O)N$R_e$$R_f$, and wherein $R_e$e and $R_f$ are each independently selected from H or $C_1$-$C_6$ alkyl, or $R_e$ and $R_f$, together with the nitrogen atom to which they are connected, form a 5- or 6-membered nitrogen-containing heterocyclyl, such as tetrahydropyrrolyl or piperidinyl, the 5- or 6-membered nitrogen-containing heterocyclyl being optionally substituted with one or more substituents independently selected from $C_1$-$C_6$ alkyl, $C_1$-$C_6$ alkoxy, hydroxy, oxo, amino, -NH($C_1$-$C_6$ alkyl), or - N($C_1$-$C_6$ alkyl)$_2$, or

$R_{A2-1}$ is phenyl, 5- to 6-membered heteroaryl, or isobenzofuranonyl, and $R_{A2-1}$ is optionally substituted with one or more substituents $R_{A2-2}$, wherein $R_{A2-2}$ is each independently selected from $C_1$-$C_6$ alkyl, cyano, amino, -COOH, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, -$C_1$-$C_6$ alkyl-hydroxy, -$C_1$-$C_6$ alkylphenyl, -phenyl, -O-phenyl, $C_{3-6}$ cycloalkyl, -$C_1$-$C_6$ alkyl-$C_1$-$C_6$ alkoxy, halogen, halogenated $C_1$-$C_6$ alkyl, halogenated $C_1$-$C_6$ alkoxy, -S(O)$C_1$-$C_6$ alkyl, -S(O)$_2$$C_1$-$C_6$ alkyl, -S(O)$_2$N$R_e$$R_f$, -C(O)N$R_e$$R_f$, or -N$R_e$$R_f$, wherein $R_e$ and $R_f$ are each independently selected from H, $C_1$-$C_6$ alkyl or -$C_1$-$C_6$ alkylhydroxy, or $R_e$ and $R_f$, together with the nitrogen atom to which they are connected, form a 4- to 8-membered nitrogen-containing heterocyclyl, e.g., tetrahydropyrrolyl, piperidinyl, morpholinyl, piperazinyl, and azetidinyl, the 4- to 8-membered nitrogen-containing heterocycle being optionally substituted with one or more substituents independently selected from halogen, hydroxy, oxo, amino, $C_1$-$C_6$ alkyl, $C_1$-$C_6$ alkoxy, - $C_1$-$C_6$ alkylhydroxy, -NH($C_1$-$C_6$ alkyl), -N($C_1$-$C_6$ alkyl)$_2$, -O-phenyl, and phenyl; or

$R_{A-1}$ is selected from N($R_{AN-1}$)($R_{AN-2}$)C(O)-, wherein $R_{AN-1}$ and $R_{AN-2}$ are each independently selected from H, $C_1$-$C_6$ alkyl, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, -$C_1$-$C_6$ alkylhydroxy, halogenated $C_1$-$C_6$ alkyl, -$C_1$-$C_6$ alkyl-$C_1$-$C_6$ alkoxy, -$C_1$-$C_6$ alkyl-phenyl, - phenyl, 5- to 10-membered heteroaryl, $C_{3-6}$ cycloalkyl, -$C_{3-6}$ cycloalkyl-phenyl, 7- to 10-membered bicyclic heterocyclyl, and 3- to 8-membered heterocyclyl, wherein the phenyl, 5- to 10-membered heteroaryl, $C_{3-6}$ cycloalkyl, 7- to 10-membered bicyclic heterocyclyl, and 3- to 8-membered heterocyclyl are each independently optionally substituted with one or more substituents selected from $C_1$-$C_6$ alkyl, cyano, amino, -NH($C_1$-$C_6$ alkyl), -N($C_1$-$C_6$ alkyl)$_2$, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, -$C_1$-$C_6$ alkylhydroxy, -$C_1$-$C_6$ alkyl-$C_1$-$C_6$ alkoxy, halogen, halogenated $C_1$-$C_6$ alkyl, halogenated $C_1$-$C_6$ alkoxy, -S(O)$C_1$-$C_6$ alkyl, -S(O)$_2$$C_1$-$C_6$ alkyl, -S(O)$_2$N$R_e$$R_f$, or -C(O)N$R_e$$R_f$, $R_e$ and $R_f$ being each independently selected from H, $C_1$-$C_6$ alkyl, or -$C_1$-$C_6$ alkylhydroxy; or

$R_{AN-1}$ and $R_{AN-2}$, together with the nitrogen atom to which they are connected, form a 4- to 10-membered nitrogen-containing heterocycle, e.g., tetrahydropyrrolyl, piperidinyl, morpholinyl, piperazinyl, azetidinyl,

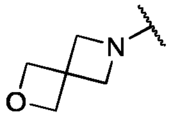 , 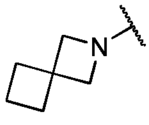 , 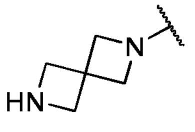 , 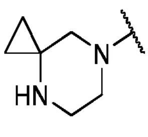 , 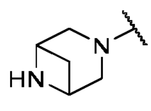 ,

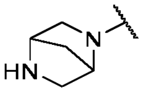, 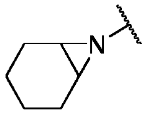, 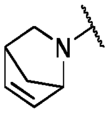, 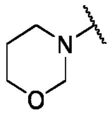, 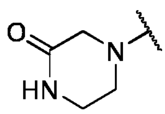, 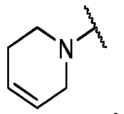, 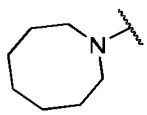, 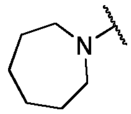,

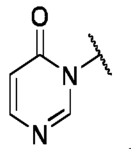, 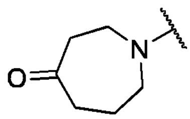, 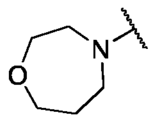, 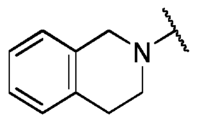, 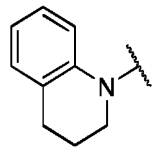, 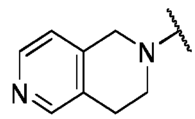

and

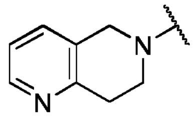,

wherein the 4- to 10-membered nitrogen-containing heterocycle is optionally substituted with one or more substituents independently selected from hydrogen, halogen, $C_1$-$C_6$ alkyl, $C_1$-$C_6$ alkoxy, -$C_1$-$C_6$ alkylhydroxy, hydroxy, oxo, amino, -NH($C_1$-$C_6$ alkyl), -N($C_1$-$C_6$ alkyl$)_2$, -O-phenyl, and phenyl;

$R_2$ is selected from -$C_1$-$C_6$ alkyl, -$C_1$-$C_6$ alkoxy, -$C_1$-$C_6$ alkylenehydroxy, or halogenated $C_1$-$C_6$ alkyl; or $R_2$ is -$C_1$-$C_6$ alkyl-cyclopropyl;

W is selected from -$C_1$-$C_6$ alkylene- and -O-$C_1$-$C_6$ alkylene-; preferably, W is selected from

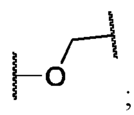;

B is phenyl, and is optionally substituted with one or more substituents selected from: halogen, $C_1$-$C_6$ alkyl, halogenated $C_1$-$C_6$ alkyl, and $C_1$-$C_6$ alkoxy; and

Z is -NH($R_{Z-1}$), $R_{Z-1}$ being selected from H, -$C_1$-$C_6$ alkyl, or halogenated $C_1$-$C_6$ alkyl.

In one preferred embodiment, $R_{A-1}$ is selected from $R_{A2-1}$ or N($R_{AN-1}$)($R_{AN-2}$)C(O)-;

$R_{A2-1}$ is phenyl, 5- to 6-membered heteroaryl, or isobenzofuranonyl, preferably phenyl, pyridinyl, or isobenzofuranonyl, and $R_{A2-1}$ is optionally substituted with one or more substituents $R_{A2-2}$, wherein $R_{A2-2}$ is each independently selected from $C_1$-$C_6$ alkyl, cyano, amino, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl$)_2$, hydroxy, -$C_1$-$C_6$ alkoxy, -$C_1$-$C_6$ alkylhydroxy, -$C_1$-$C_6$ alkylphenyl, -phenyl, -O-phenyl, $C_{3-6}$ cycloalkyl, -$C_1$-$C_6$ alkyl-$C_1$-$C_6$ alkoxy, halogen, halogenated $C_1$-$C_6$ alkyl, halogenated $C_1$-$C_6$ alkoxy, -S(O)$C_1$-$C_6$ alkyl, -S(O$)_2C_1$-$C_6$ alkyl, - S(O$)_2NR_eR_f$, -C(O)$NR_eR_f$, or -$NR_eR_f$, wherein $R_e$ and $R_f$ are each independently selected from H, $C_1$-$C_6$ alkyl or -$C_1$-$C_6$ alkylhydroxy, or $R_e$ and $R_f$, together with the nitrogen atom to which they are connected, form a 4- to 8-membered nitrogen-containing heterocyclyl, e.g., tetrahydropyrrolyl, piperidinyl, morpholinyl, piperazinyl, and azetidinyl, the 4- to 8-membered nitrogen-containing heterocycle being optionally substituted with one or more substituents independently selected from hydrogen, halogen, $C_1$-$C_6$ alkyl, $C_1$-$C_6$ alkoxy, -$C_1$-$C_6$ alkylhydroxy, hydroxy, oxo, amino, -NH($C_1$-$C_6$ alkyl), -N($C_1$-$C_6$ alkyl$)_2$, -O-phenyl, and phenyl;

$R_{AN-1}$ and $R_{AN-2}$ are each independently selected from H, $C_1$-$C_6$ alkyl, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl$)_2$, -$C_1$-$C_6$ alkylhydroxy, halogenated $C_1$-$C_6$ alkyl, -$C_1$-$C_6$ alkyl-$C_1$-$C_6$ alkoxy, -$C_1$-$C_6$ alkyl-phenyl, -phenyl, 5- to 10-membered heteroaryl, $C_{3-6}$ cycloalkyl, -$C_{3-6}$ cycloalkyl-phenyl, 7- to 10-membered bicyclic heterocyclyl, and 3- to 8-membered heterocyclyl, wherein the phenyl, 5- to 10-membered heteroaryl, $C_{3-6}$ cycloalkyl, 7- to 10-membered bicyclic heterocyclyl, and 3- to 8-membered heterocyclyl are each independently optionally substituted with one or more substituents selected from hydrogen, $C_1$-$C_6$ alkyl, cyano, amino, - NH($C_1$-$C_6$ alkyl), -N($C_1$-$C_6$ alkyl$)_2$, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl$)_2$, hydroxy, -$C_1$-$C_6$ alkoxy, -$C_1$-$C_6$ alkylhydroxy, -$C_1$-$C_6$ alkyl-$C_1$-$C_6$ alkoxy, halogen, halogenated $C_1$-$C_6$ alkyl, halogenated

$C_1$-$C_6$ alkoxy, -S(O)$C_1$-$C_6$ alkyl, -S(O)$_2$$C_1$-$C_6$ alkyl, - S(O)$_2$N$R_e$$R_f$, or -C(O)N$R_e$$R_f$, $R_e$ and $R_f$ being each independently selected from H, $C_1$-$C_6$ alkyl, or - $C_1$-$C_6$ alkylhydroxy; or

$R_{AN-1}$ and $R_{AN-2}$, together with the nitrogen atom to which they are connected, form a 4- to 10-membered nitrogen-containing heterocycle, e.g., tetrahydropyrrolyl, piperidinyl, morpholinyl, piperazinyl, azetidinyl,

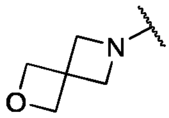, 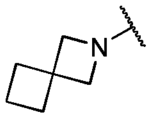, 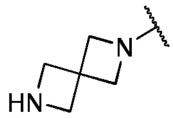, 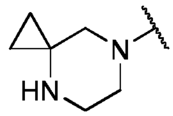, 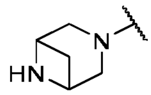,

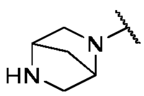, 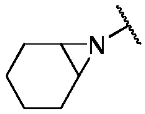, 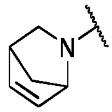, 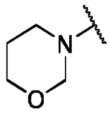, 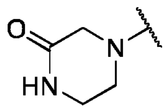, 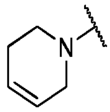, 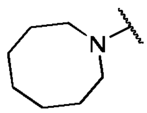, 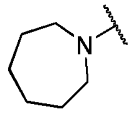,

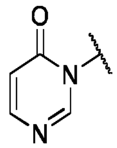, 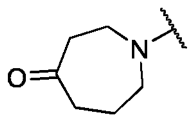, 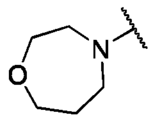, 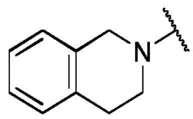, 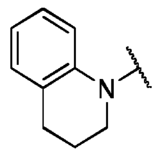, 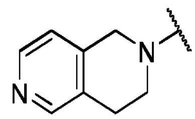

and

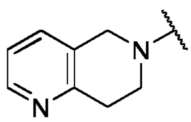,

wherein the 4- to 10-membered nitrogen-containing heterocycle is optionally substituted with one or more substituents independently selected from hydrogen, halogen, $C_1$-$C_6$ alkyl, $C_1$-$C_6$ alkoxy, -$C_1$-$C_6$ alkylhydroxy, hydroxy, oxo, amino, -NH($C_1$-$C_6$ alkyl), -N($C_1$-$C_6$ alkyl)$_2$, -O-phenyl, and phenyl;

$R_2$ is selected from -$C_1$-$C_6$ alkyl, -$C_1$-$C_6$ alkoxy, -$C_1$-$C_6$ alkylenehydroxy, -$C_1$-$C_6$ alkyl-cyclopropyl, or halogenated $C_1$-$C_6$ alkyl; preferably, $R_2$ is selected from

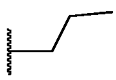, 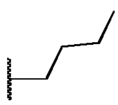, 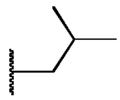, 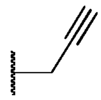, 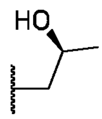,

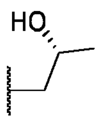, 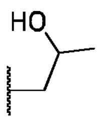, 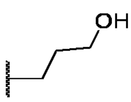, 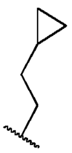, 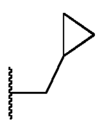, and 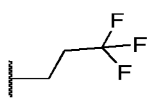;

more preferably, $R_2$ is selected from

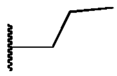 and 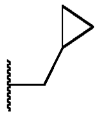 ;

W is selected from $-C_1-C_6$ alkylene- and $-O-C_1-C_6$ alkylene-; preferably, W is selected from

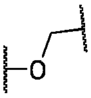 ;

B is phenyl, and is optionally substituted with one or more substituents selected from: hydrogen, halogen, $C_1-C_6$ alkyl, halogenated $C_1-C_6$ alkyl, and $C_1-C_6$ alkoxy, and preferably, B is phenyl; and

Z is $-NH(R_{Z-1})$, wherein $R_{Z-1}$ is selected from H, $-C_1-C_6$ alkyl, and halogenated $C_1-C_6$ alkyl; preferably, $R_{Z-1}$ is selected from H,

$CH_3$, and $-CH_2CH_3$, and more preferably, $R_{Z-1}$ is selected from H.

For example, in one embodiment, a compound of formula (A) or a tautomer, an enantiomer or a diastereoisomer, or a mixture of isomers, or a pharmaceutically acceptable salt or solvate thereof is provided,

wherein $R_{A-1}$ is selected from $R_{A2-1}$ or $N(R_{AN-1})(R_{AN-2})C(O)$-;

$R_{A2-1}$ is phenyl, pyridinyl, or isobenzofuranonyl, and $R_{A2-1}$ is optionally substituted with one or more substituents $R_{A2-2}$, wherein $R_{A2-2}$ is each independently selected from hydrogen, $C_1-C_6$ alkyl, cyano, amino, $-C_1-C_6$ alkylamino, halogen, halogenated $C_1-C_6$ alkyl, $-C_1-C_6$ alkylhydroxy, $-S(O)_2C_1-C_6$ alkyl, $-S(O)_2NR_eR_f$, $-C(O)NR_eR_f$, or $-NR_eR_f$, wherein $R_e$ and $R_f$ are each independently selected from H, $C_1-C_6$ alkyl, or $-C_1-C_6$ alkylhydroxy, or $R_e$ and $R_f$, together with the nitrogen atom to which they are connected, form a 4- to 8-membered nitrogen-containing heterocycle, e.g., tetrahydropyrrolyl, piperidinyl, morpholinyl, piperazinyl, and azetidinyl, the 4- to 8-membered nitrogen-containing heterocycle being optionally substituted with one or more substituents independently selected from hydrogen, halogen, hydroxy, oxo, amino, $C_1-C_6$ alkyl, $C_1-C_6$ alkoxy, or $-C_1-C_6$ alkylhydroxy;

$R_{AN-1}$ and $R_{AN-2}$ are each independently selected from H, $C_1-C_6$ alkyl, $-C_1-C_6$ alkylhydroxy, phenyl, pyridinyl, $C_{3-6}$ cycloalkyl, and 4- to 6-membered heterocyclyl, wherein the phenyl, pyridinyl, $C_{3-6}$ cycloalkyl, and 4- to 6-membered heterocyclyl are each independently optionally substituted with one or more substituents selected from hydrogen, halogen, hydroxy, and $C_1-C_6$ alkyl; or

$R_{AN-1}$ and $R_{AN-2}$, together with a nitrogen atom to which they are connected, form a 4- to 8-membered nitrogen-containing heterocycle, e.g., tetrahydropyrrolyl, piperidinyl, morpholinyl, piperazinyl, and azetidinyl, the 4- to 8-membered nitrogen-containing heterocycle being optionally substituted with one or more substituents independently selected from hydrogen, halogen, hydroxy, oxo, amino, or - $C_1-C_6$ alkylhydroxy.

For example, in one embodiment, a compound of formula (A) or a tautomer, an enantiomer or a diastereoisomer, or a mixture of isomers, or a pharmaceutically acceptable salt or solvate thereof is provided,

wherein $R_{A-1}$ is $R_{A2-1}$;

$R_{A2-1}$ is phenyl, and $R_{A2-1}$ is optionally substituted with one or more substituents $R_{A2-2}$, wherein $R_{A2-2}$ is each independently selected from hydrogen, $C_1-C_6$ alkyl, cyano, amino, $-C_1-C_6$ alkylamino, halogen, halogenated $C_1-C_6$ alkyl, $-C_1-C_6$ alkylhydroxy, $-S(O)_2C_1-C_6$ alkyl, $-S(O)_2NR_eR_f$, - $C(O)NR_eR_f$, or $-NR_eR_f$, wherein $R_e$ and $R_f$ are each independently selected from H, $C_1-C_6$ alkyl, or - $C_1-C_6$ alkylhydroxy, or $R_e$ and $R_f$, together with the nitrogen atom to which they are connected, form a 4- to 8-membered nitrogen-containing heterocycle, e.g., tetrahydropyrrolyl, piperidinyl, morpholinyl, piperazinyl, and azetidinyl, the 4- to 8-membered nitrogen-containing heterocycle being optionally substituted with one or more substituents independently selected from hydrogen, halogen, hydroxy, oxo, amino, $C_1-C_6$ alkyl, $C_1-C_6$ alkoxy, or $-C_1-C_6$ alkylhydroxy.

For example, in one embodiment, a compound of formula (A) or a tautomer, an enantiomer or a diastereoisomer, or a mixture of isomers, or a pharmaceutically acceptable salt or solvate thereof is provided,

wherein $R_{A-1}$ is $R_{A2-1}$;

$R_{A2-1}$ is phenyl, and $R_{A2-1}$ is optionally substituted with one or more substituents $R_{A2-2}$, wherein $R_{A2-2}$ is each

independently selected from $-C(O)NR_eR_f$ and $-S(O)_2NR_eR_f$, wherein $R_e$ and $R_f$, together with a nitrogen atom to which they are connected, form a 4- to 8-membered nitrogen-containing heterocycle, e.g., tetrahydropyrrolyl, piperidinyl, morpholinyl, piperazinyl, and azetidinyl, the 4- to 8-membered nitrogen-containing heterocycle being optionally substituted with one or more substituents independently selected from hydrogen, halogen, hydroxy, or $-C_1-C_6$ alkylhydroxy.

For example, in one embodiment, a compound of formula (A) or a tautomer, an enantiomer or a diastereoisomer, or a mixture of isomers, or a pharmaceutically acceptable salt or solvate thereof is provided, wherein $R_{A-1}$ is $R_{A2-1}$; $R_{A2-1}$ is pyridinyl, and $R_{A2-1}$ is optionally substituted with one or more substituents $R_{A2-2}$, wherein $R_{A2-2}$ is each independently selected from hydrogen, $C_1-C_6$ alkyl, cyano, amino, $-C_1-C_6$ alkylamino, halogen, halogenated $C_1-C_6$ alkyl, $-C_1-C_6$ alkylhydroxy, $-S(O)_2C_1-C_6$ alkyl, $-S(O)_2NR_eR_f$, $-C(O)NR_eR_f$, or $-NR_eR_f$, wherein $R_e$ and $R_f$ are each independently selected from H, $C_1-C_6$ alkyl, or $-C_1-C_6$ alkylhydroxy, or $R_e$ and $R_f$, together with the nitrogen atom to which they are connected, form a 4- to 8-membered nitrogen-containing heterocycle, e.g., tetrahydropyrrolyl, piperidinyl, morpholinyl, piperazinyl, and azetidinyl, the 4- to 8-membered nitrogen-containing heterocycle being optionally substituted with one or more substituents independently selected from hydrogen, halogen, hydroxy, oxo, amino, $C_1-C_6$ alkyl, $C_1-C_6$ alkoxy, or $-C_1-C_6$ alkylhydroxy.

For example, in one embodiment, a compound of formula (A) or a tautomer, an enantiomer or a diastereoisomer, or a mixture of isomers, or a pharmaceutically acceptable salt or solvate thereof is provided,

wherein $R_{A-1}$ is $R_{A2-1}$;
$R_{A2-1}$ is pyridinyl, and $R_{A2-1}$ is optionally substituted with one or more substituents $R_{A2-2}$, wherein $R_{A2-2}$ is each independently selected from hydrogen, $C_1-C_6$ alkyl, cyano, amino, $-C_1-C_6$ alkylamino, halogen, halogenated $C_1-C_6$ alkyl, $-C_1-C_6$ alkylhydroxy, $-S(O)_2C_1-C_6$ alkyl, $-S(O)_2NR_eR_f$, or $-NR_eR_f$, wherein $R_e$ and $R_f$, together with the nitrogen atom to which they are connected, form a 4- to 8-membered nitrogen-containing heterocycle, e.g., tetrahydropyrrolyl, piperidinyl, morpholinyl, piperazinyl, and azetidinyl, the 4- to 8-membered nitrogen-containing heterocycle being optionally substituted with one or more substituents independently selected from hydrogen, halogen, hydroxy, or $-C_1-C_6$ alkylhydroxy.

For example, in one embodiment, a compound of formula (A) or a tautomer, an enantiomer or a diastereoisomer, or a mixture of isomers, or a pharmaceutically acceptable salt or solvate thereof is provided,

wherein $R_{A-1}$ is $N(R_{AN-1})(R_{AN-2})C(O)-$;
$R_{AN-1}$ and $R_{AN-2}$ are each independently selected from H, $C_1-C_6$ alkyl, $-C_1-C_6$ alkylhydroxy, phenyl, pyridinyl, $C_{3-6}$ cycloalkyl, and 4- to 6-membered heterocyclyl, wherein the phenyl, pyridinyl, $C_{3-6}$ cycloalkyl, and 4- to 6-membered heterocyclyl are each independently optionally substituted with one or more substituents selected from hydrogen, halogen, hydroxy, and $C_1-C_6$ alkyl; or
$R_{AN-1}$ and $R_{AN-2}$, together with a nitrogen atom to which they are connected, form a 4- to 8-membered nitrogen-containing heterocycle, e.g., tetrahydropyrrolyl, piperidinyl, morpholinyl, piperazinyl, and azetidinyl, the 4- to 8-membered nitrogen-containing heterocycle being optionally substituted with one or more substituents independently selected from hydrogen, halogen, hydroxy, oxo, amino, or $-C_1-C_6$ alkylhydroxy.

For example, in one embodiment, a compound of formula (A) or a tautomer, an enantiomer or a diastereoisomer, or a mixture of isomers, or a pharmaceutically acceptable salt or solvate thereof is provided,

wherein $R_{A-1}$ is $N(R_{AN-1})(R_{AN-2})C(O)-$;
$R_{AN-1}$ and $R_{AN-2}$ are each independently selected from H, pyridinyl, $C_{3-6}$ cycloalkyl, and 5- to 7-membered heterocyclyl, wherein the pyridinyl, $C_{3-6}$ cycloalkyl, and 5- to 7-membered heterocyclyl are each independently optionally substituted with one or more substituents selected from hydrogen, halogen, hydroxy, amino, cyano, oxo, $C_1-C_6$ alkyl, or $-C_1-C_6$ alkylhydroxy; or
$R_{AN-1}$ and $R_{AN-2}$, together with a nitrogen atom to which they are connected, form a 4- to 8-membered nitrogen-containing heterocycle, e.g., tetrahydropyrrolyl, piperidinyl, morpholinyl, piperazinyl, and azetidinyl, the 4- to 8-membered nitrogen-containing heterocycle being optionally substituted with one or more substituents independently selected from hydrogen, halogen, hydroxy, oxo, amino, or $-C_1-C_6$ alkylhydroxy.

For example, in the compound of formula (II-a) or formula (A) or the tautomer, the enantiomer or the diastereoisomer, or the mixture of isomers, or the pharmaceutically acceptable salt or solvate thereof,

$R_{A-1}$ is selected from $R_{A2-1}$ or $N(R_{AN-1})(R_{AN-2})C(O)-$;

$R_{A2-1}$ is selected from

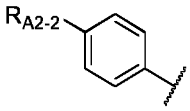, 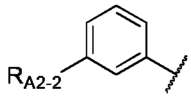, 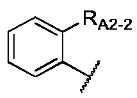, 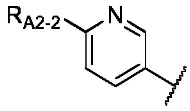, 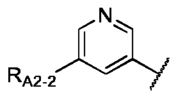,

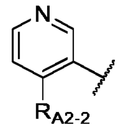, 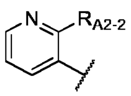, 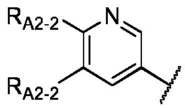, 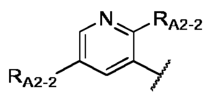, 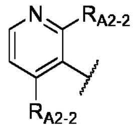, 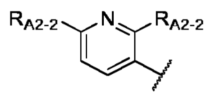,

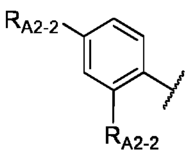, 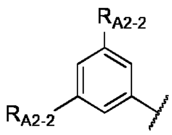, or 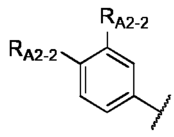;

$R_{A2-2}$, $R_{AN-1}$, and $R_{AN-2}$ are each independently as defined in any one of formula (A).

For example, in the compound of formula (II-a) or formula (A) or the tautomer, the enantiomer or the diastereoisomer, or the mixture of isomers, or the pharmaceutically acceptable salt or solvate thereof,

$R_{A-1}$ is selected from $R_{A2-1}$ or $N(R_{AN-1})(R_{AN-2})C(O)$-;

$R_{A2-1}$ is selected from

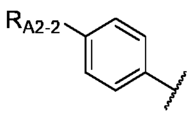, 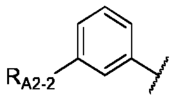, 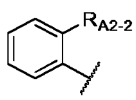, 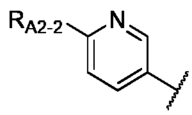, 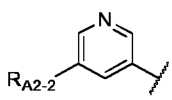,

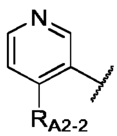, 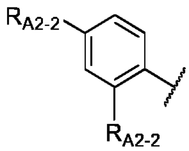, 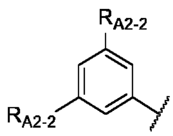, or 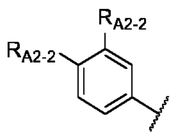;

$R_{A2-2}$, $R_{AN-1}$, and $R_{AN-2}$ are each independently as defined in any one of formula (A).

For example, in the compound of formula (II-a) or formula (A) or the tautomer, the enantiomer or the diastereoisomer, or the mixture of isomers, or the pharmaceutically acceptable salt or solvate thereof,

$R_{A-1}$ is selected from $R_{A2-1}$ or $N(R_{AN-1})(R_{AN-2})C(O)$-;

$R_{A2-1}$ is selected from

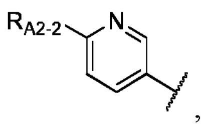, 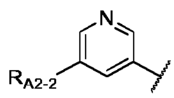, or 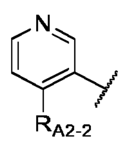;

$R_{A2-2}$, $R_{AN-1}$, and $R_{AN-2}$ are each independently as defined in any one of formula (A).

For example, in the compound of formula (II-a) or formula (A) or the tautomer, the enantiomer or the diastereoisomer, or the mixture of isomers, or the pharmaceutically acceptable salt or solvate thereof,

B is selected from

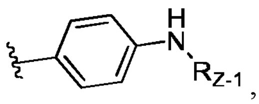, 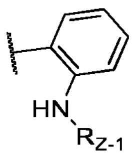, and 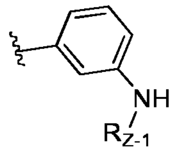,

and $R_{Z-1}$ is selected from H or -$C_1$-$C_6$ alkyl; preferably, $R_{Z-1}$ is selected from H, $CH_3$, or -$CH_2CH_3$; more preferably, $R_{Z-1}$ is selected from H.

For example, in the compound of formula (II-a) or formula (A) or the tautomer, the enantiomer or the diastereoisomer, or the mixture of isomers, or the pharmaceutically acceptable salt or solvate thereof, B is selected from

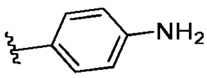.

For example, in the compound of formula (II-a) or formula (A) or the tautomer, the enantiomer or the diastereoisomer, or the mixture of isomers, or the pharmaceutically acceptable salt or solvate thereof, -W-B is selected from

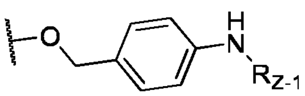, 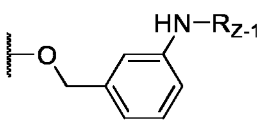, and 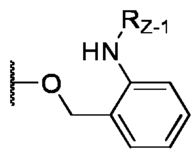,

and $R_{Z-1}$ is selected from H or -$C_1$-$C_6$ alkyl; preferably, $R_{Z-1}$ is selected from H, $CH_3$, or -$CH_2CH_3$; more preferably, $R_{Z-1}$ is selected from H.

For example, in the compound of formula (II-a) or formula (A) or the tautomer, the enantiomer or the diastereoisomer, or the mixture of isomers, or the pharmaceutically acceptable salt or solvate thereof, -W-B is selected from

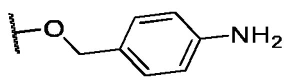.

In some embodiments, the compound or the tautomer, the enantiomer or the diastereoisomer, or the mixture of isomers, or the pharmaceutically acceptable salt or solvate thereof of the present invention is selected from:

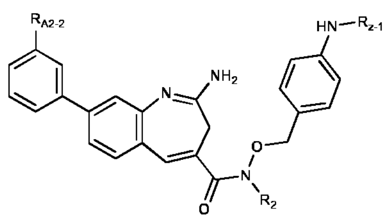,

(II-a-1)

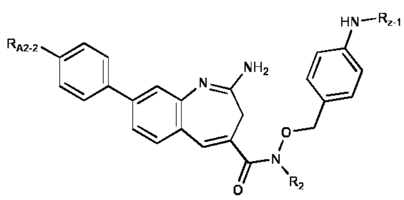,

(II-a-2)

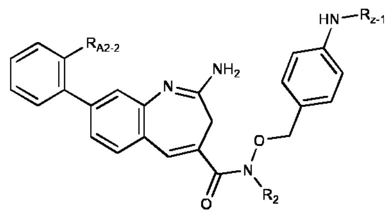,

(II-a-3)

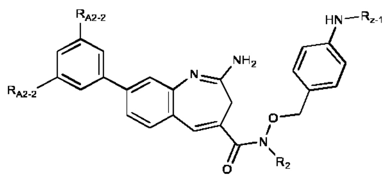,

(II-a-4)

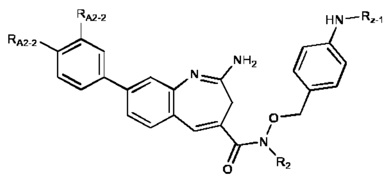,

(II-a-5)

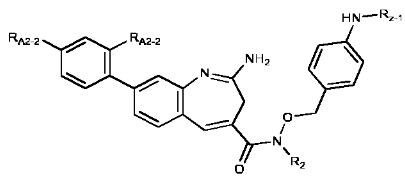,

(II-a-6)

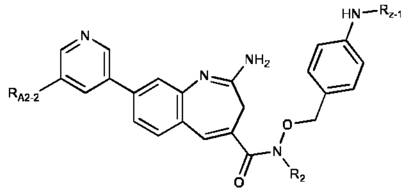,

(II-a-7)

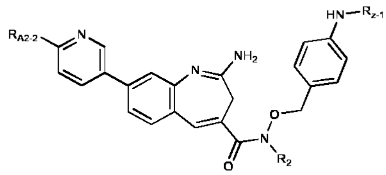,

(II-a-8)

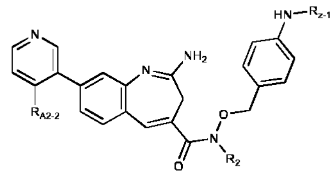,

(II-a-9)

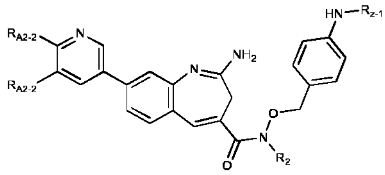,

(II-a-10)

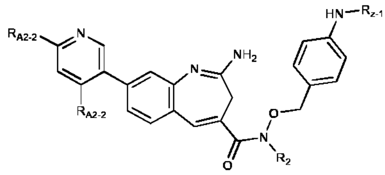,

(II-a-11)

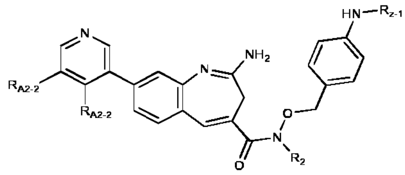,

(II-a-12)

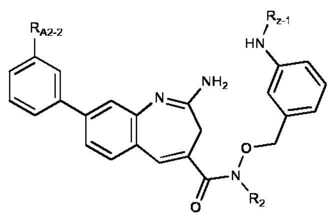,

(II-a-13)

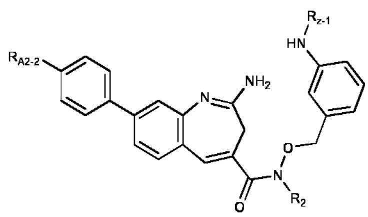,

(II-a-14)

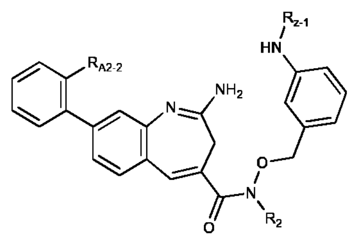,

(II-a-15)

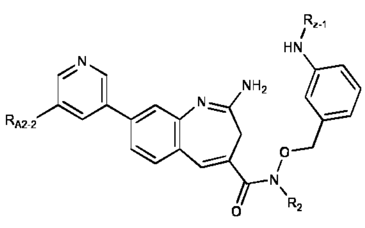,

(II-a-16)

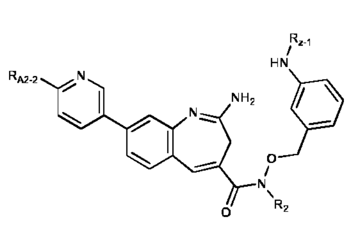,

(II-a-17)

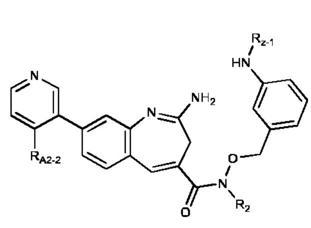,

(II-a-18)

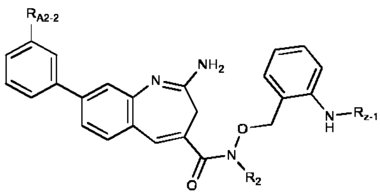,
(II-a-1)

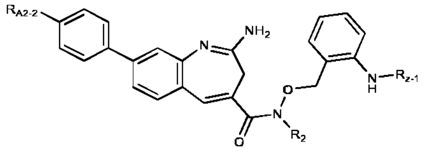,
(II-a-2)

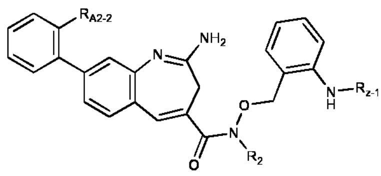,
(II-a-3)

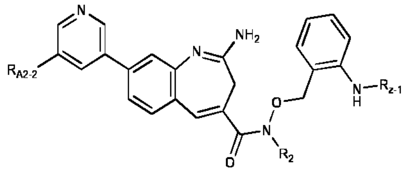,
(II-a-19)

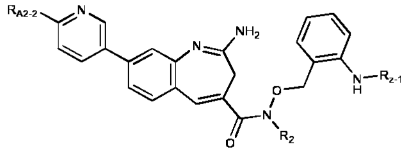,
(II-a-20)

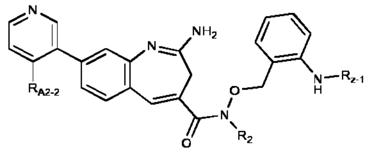,
(II-a-21)

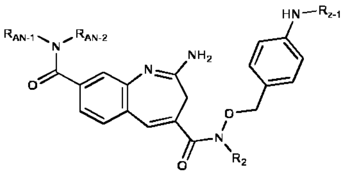,
(II-a-22)

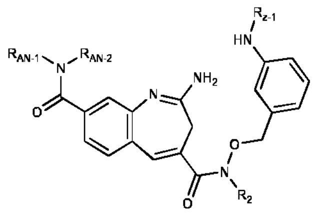,
(II-a-23)

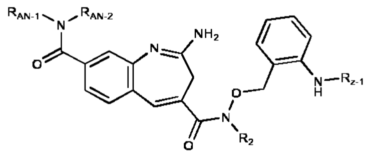,
(II-a-24)

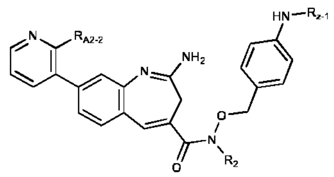
(II-a-25),

wherein,

$R_{A2-2}$ is each independently selected from $C_1$-$C_6$ alkyl, cyano, amino, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, -$C_1$-$C_6$ alkylhydroxy, -$C_1$-$C_6$ alkylphenyl, -phenyl, -O-phenyl, $C_{3-6}$ cycloalkyl, -$C_1$-$C_6$ alkyl-$C_1$-$C_6$ alkoxy, halogen, halogenated $C_1$-$C_6$ alkyl, halogenated $C_1$-$C_6$ alkoxy, -S(O)$C_1$-$C_6$ alkyl, -S(O)$_2$$C_1$-$C_6$ alkyl, - S(O)$_2$N$R_e$$R_f$, -C(O)N$R_e$$R_f$, or -N$R_e$$R_f$, wherein $R_e$ and $R_f$ are each independently selected from H, $C_1$-$C_6$ alkyl or -$C_1$-$C_6$ alkylhydroxy, or $R_e$ and $R_f$, together with the nitrogen atom to which they are connected, form a 4- to 8-membered nitrogen-containing heterocyclyl, e.g., tetrahydropyrrolyl, piperidinyl, morpholinyl, piperazinyl, and azetidinyl, the 4- to 8-membered nitrogen-containing heterocycle being optionally substituted with one or more substituents independently selected from halogen, hydroxy, oxo, amino, $C_1$-$C_6$ alkyl, $C_1$-$C_6$ alkoxy, -$C_1$-$C_6$ alkylhydroxy, -NH($C_1$-$C_6$ alkyl), - N($C_1$-$C_6$ alkyl)$_2$, -O-phenyl, and phenyl;

$R_{AN-1}$ and $R_{AN-2}$ are each independently selected from H, $C_1$-$C_6$ alkyl, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, -$C_1$-$C_6$ alkylhydroxy, halogenated $C_1$-$C_6$ alkyl, -$C_1$-$C_6$ alkyl-$C_1$-$C_6$ alkoxy, -$C_1$-$C_6$ alkyl-phenyl, -phenyl, 5- to 10-membered heteroaryl, $C_{3-6}$ cycloalkyl, -$C_{3-6}$ cycloalkyl-phenyl, 7- to 10-membered bicyclic heterocyclyl, and 3- to 8-membered heterocyclyl, wherein the phenyl, 5- to 10-membered heteroaryl, $C_{3-6}$ cycloalkyl, 7- to 10-membered bicyclic heterocyclyl, and 3- to 8-membered heterocyclyl are each independently optionally substituted with one or more substituents selected from $C_1$-$C_6$ alkyl, cyano, amino, -NH($C_1$-$C_6$ alkyl), -N($C_1$-$C_6$ alkyl)$_2$, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, -$C_1$-$C_6$ alkylhydroxy, -$C_1$-$C_6$ alkyl-$C_1$-$C_6$ alkoxy, halogen, halogenated $C_1$-$C_6$ alkyl, halogenated $C_1$-$C_6$ alkoxy,

-S(O)$C_1$-$C_6$ alkyl, -S(O)$_2$$C_1$-$C_6$ alkyl, -S(O)$_2$N$R_eR_f$, or - C(O)N$R_eR_f$, $R_e$ and $R_f$ being each independently selected from H, $C_1$-$C_6$ alkyl, or -$C_1$-$C_6$ alkylhydroxy; or

$R_{AN-1}$ and $R_{AN-2}$, together with the nitrogen atom to which they are connected, form a 4- to 10-membered nitrogen-containing heterocycle, e.g., tetrahydropyrrolyl, piperidinyl, morpholinyl, piperazinyl, azetidinyl,

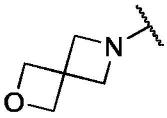, 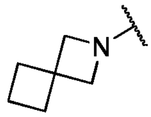, 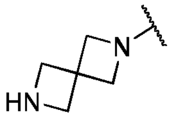, 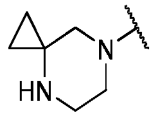, 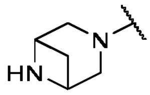,

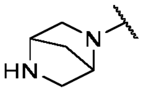, 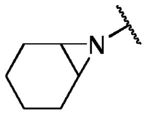, 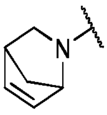, 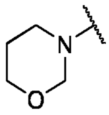, 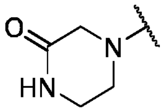, 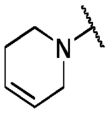, 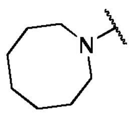, 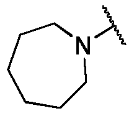,

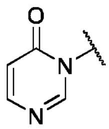, 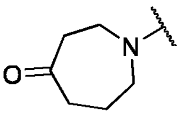, 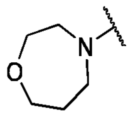, 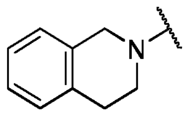, 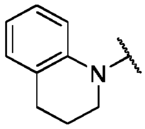, 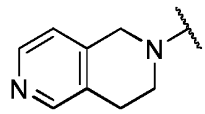 and

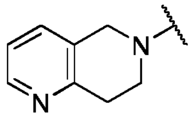,

wherein the 4- to 10-membered nitrogen-containing heterocycle is optionally substituted with one or more substituents independently selected from hydrogen, halogen, $C_1$-$C_6$ alkyl, $C_1$-$C_6$ alkoxy, -$C_1$-$C_6$ alkylhydroxy, hydroxy, oxo, amino, -NH($C_1$-$C_6$ alkyl), -N($C_1$-$C_6$ alkyl)$_2$, -O-phenyl, and phenyl;

$R_2$ is selected from -$C_1$-$C_6$ alkyl, -$C_1$-$C_6$ alkoxy, -$C_1$-$C_6$ alkylenehydroxy, -$C_1$-$C_6$ alkyl-cyclopropyl, or halogenated $C_1$-$C_6$ alkyl; preferably, $R_2$ is selected from

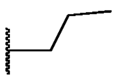, 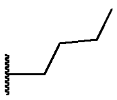, 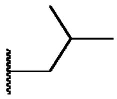, 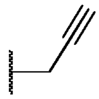,

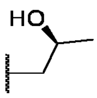, 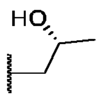, 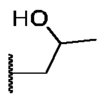, 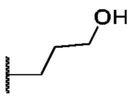, 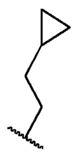, 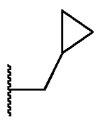, and 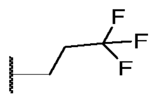;

more preferably, $R_2$ is selected from

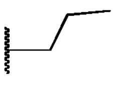 and 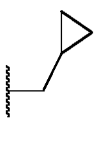;

$R_{Z-1}$ is selected from H, -$C_1$-$C_6$ alkyl, and halogenated $C_1$-$C_6$ alkyl; preferably, $R_{Z-1}$ is selected from H, $CH_3$, and -$CH_2CH_3$; more preferably, $R_{Z-1}$ is selected from H.

For example, in the compounds of formulae (II-a-1) to (II-a-25) or the tautomer, the enantiomer or the diastereoisomer, or the mixture of isomers, or the pharmaceutically acceptable salt or solvate thereof, $R_{Z-1}$, $R_2$, $R_{A2-2}$, $R_{AN-1}$, and $R_{AN-2}$ are each independently as defined in any one of formula (A). For example, in the compounds of formula (II-a), formula (A), or formulae (II-a-1) to (II-a-25) or the tautomers, the enantiomers or the diastereoisomers, or the mixtures of isomers thereof, or the pharmaceutically acceptable salts or solvates thereof, wherein

$R_{A-1}$ is selected from

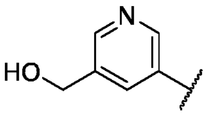, 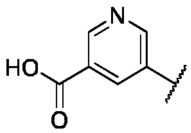, 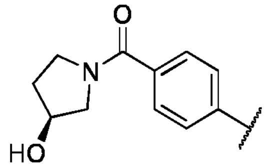,

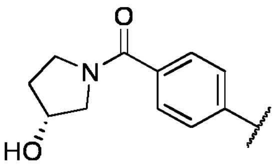, 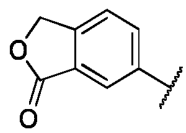, 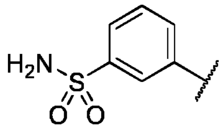, 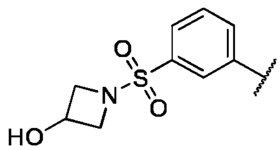,

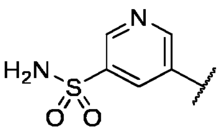, 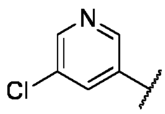, 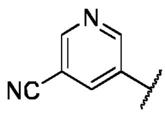, 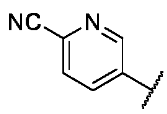, 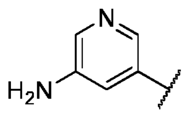, 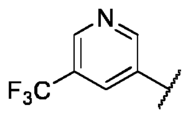,

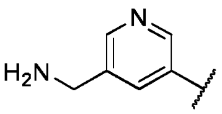, 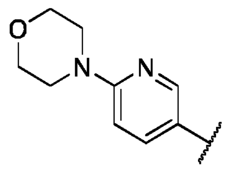, 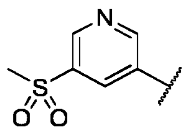, 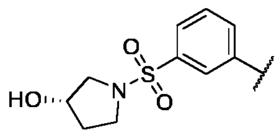,

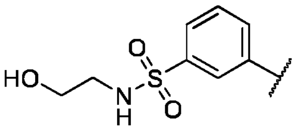, 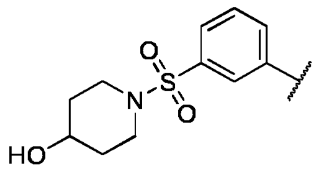, 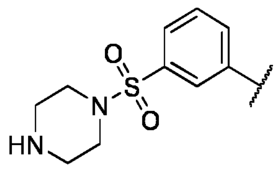,

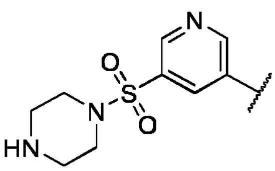, 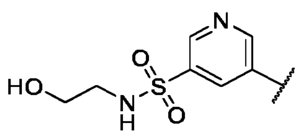, 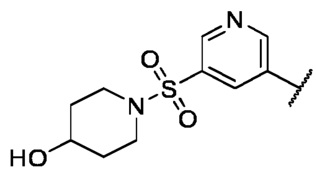,

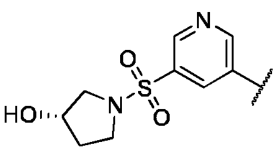, 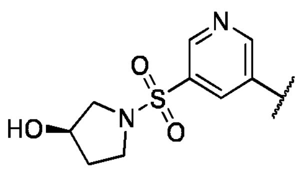, 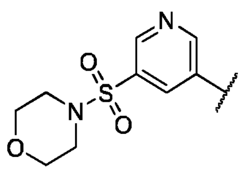, 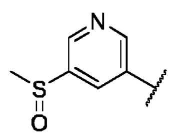, 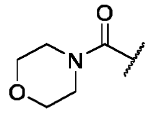,

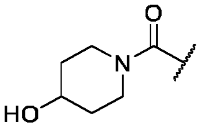, 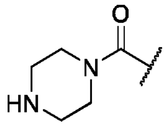, 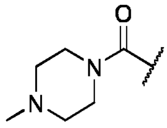, 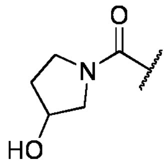, 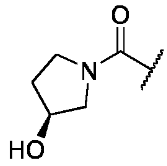, 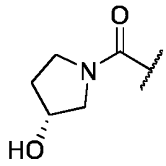,

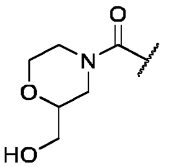, 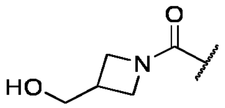, 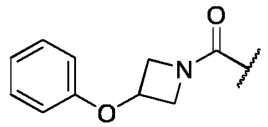, 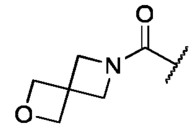, 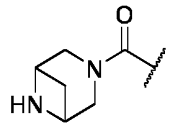,

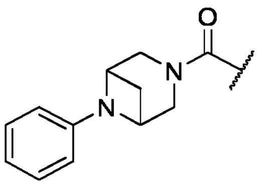, 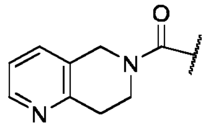, 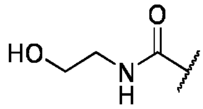, 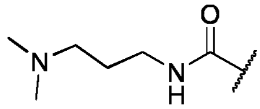, 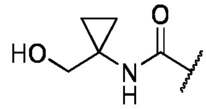,

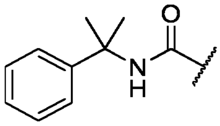, 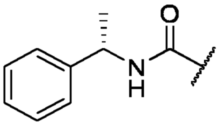, 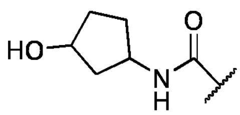, 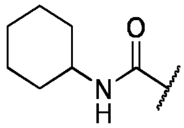, 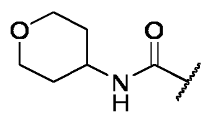,

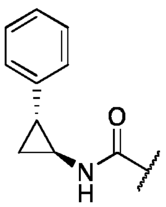, 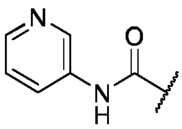, 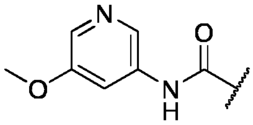, 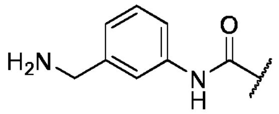, 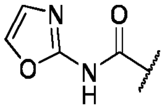,

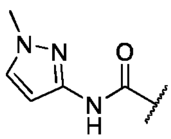, 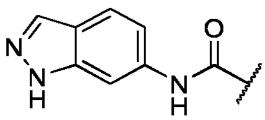, and 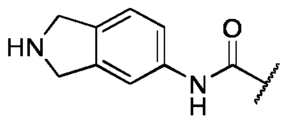.

In one particularly preferred embodiment, the present invention provides a compound or a tautomer, an enantiomer or a diastereoisomer, or a mixture of isomers, or a pharmaceutically acceptable salt or solvate thereof selected from:

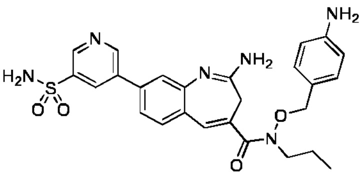 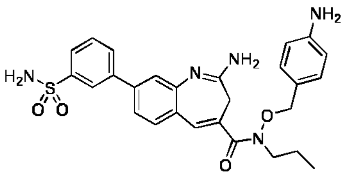 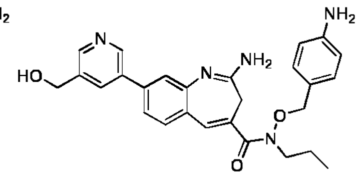

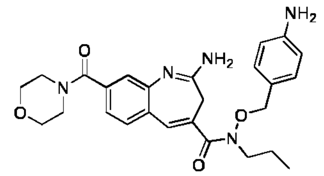 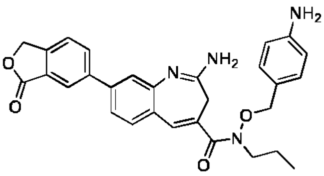 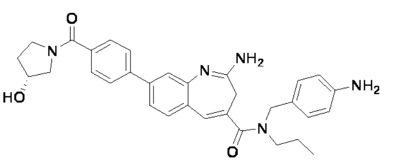

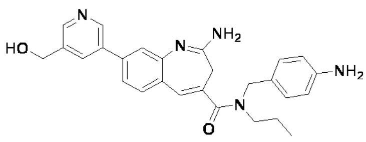
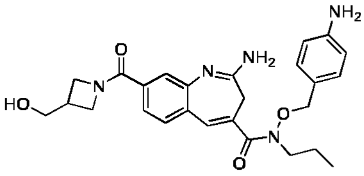
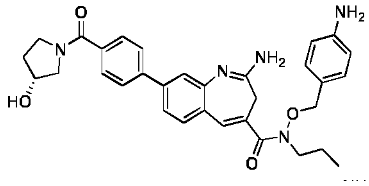

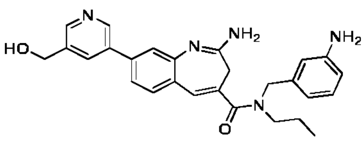
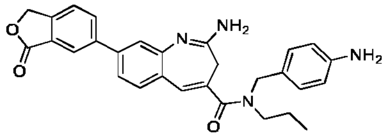
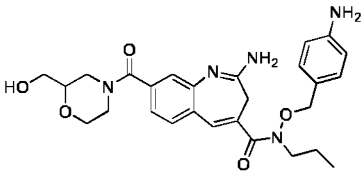

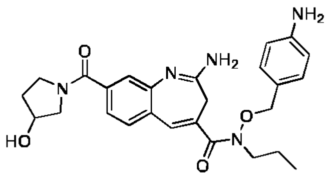
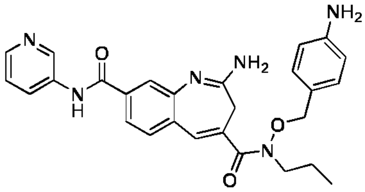
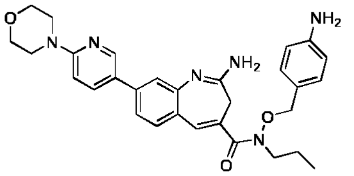

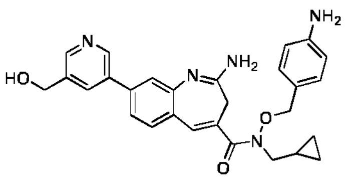
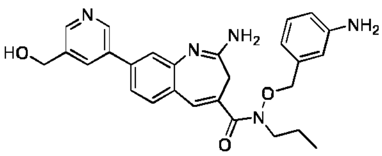
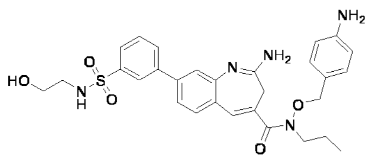

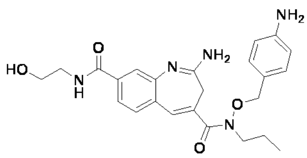
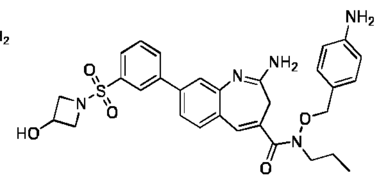
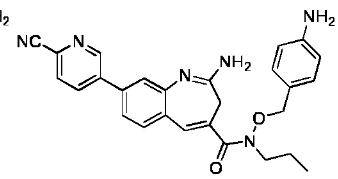

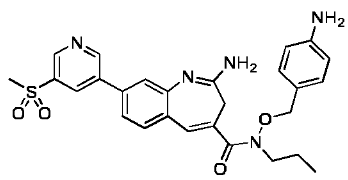
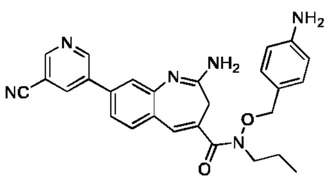
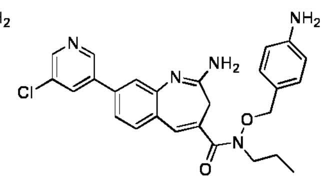

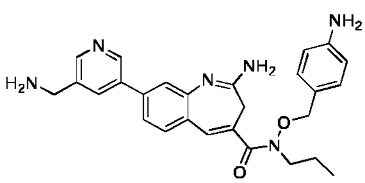
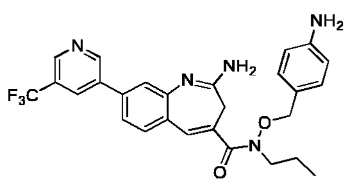
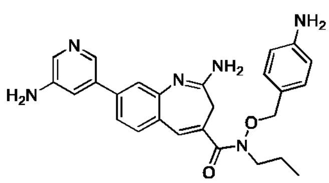

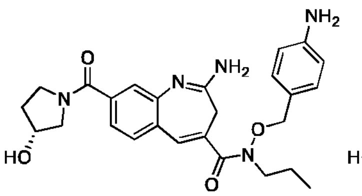
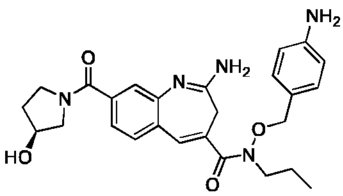
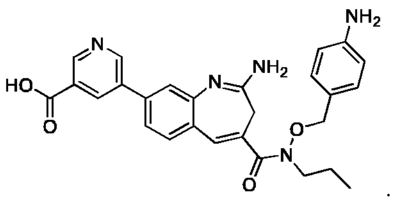

In one particularly preferred embodiment, the present invention provides a compound or a tautomer, an enantiomer or a diastereoisomer, or a mixture of isomers, or a pharmaceutically acceptable salt or solvate thereof selected from:

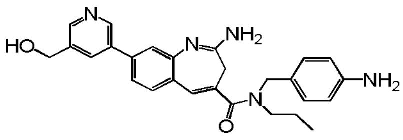
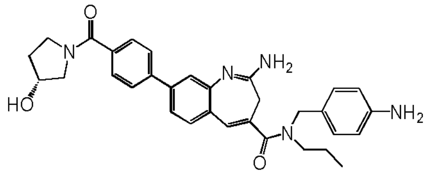
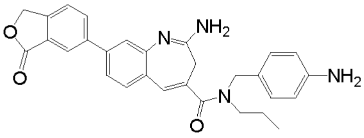
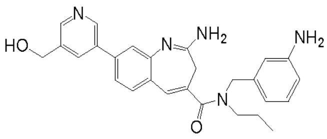
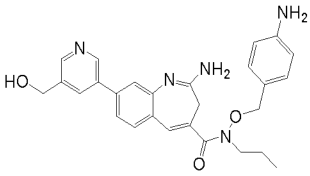
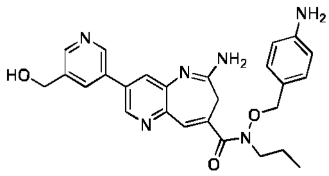
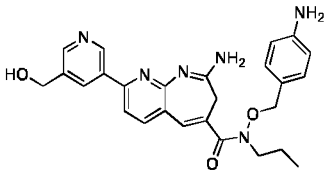
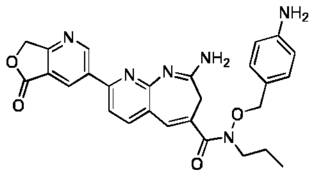
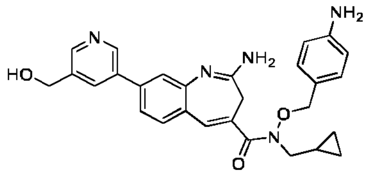
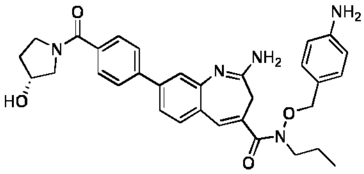
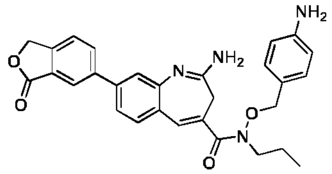
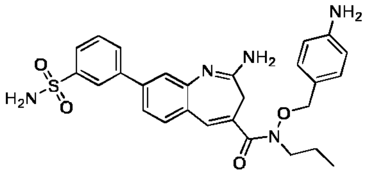
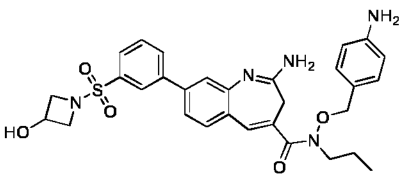
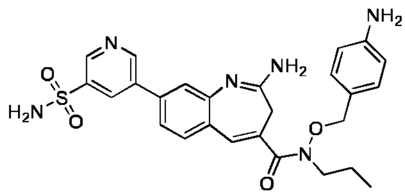
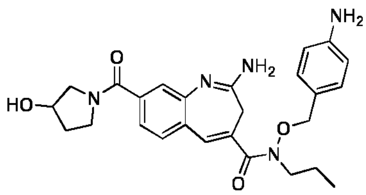
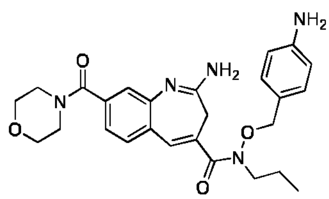
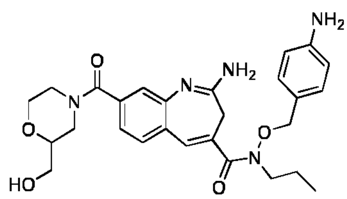
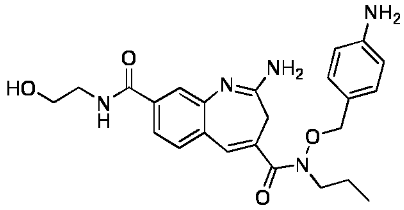
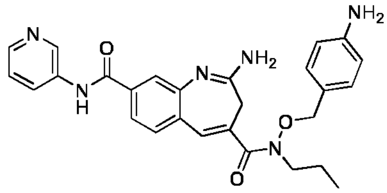
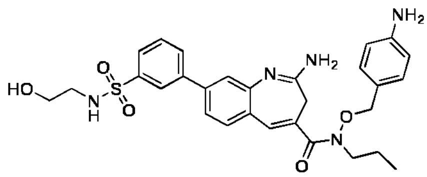
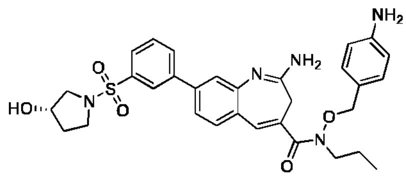

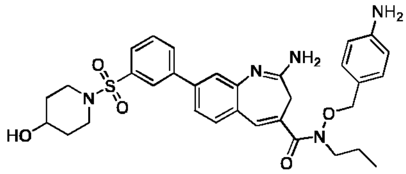 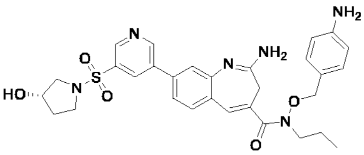 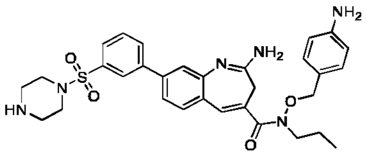

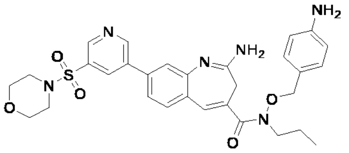 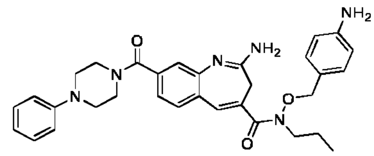 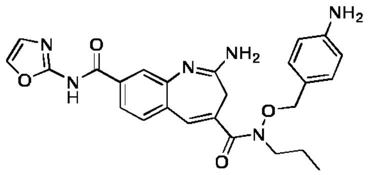

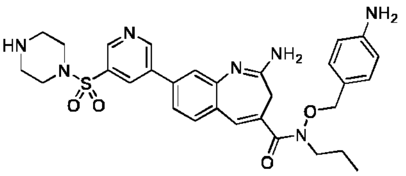 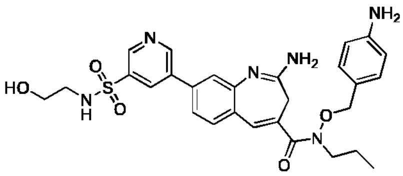 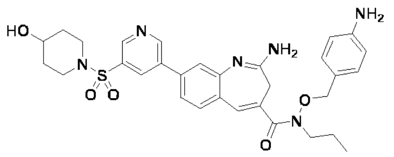

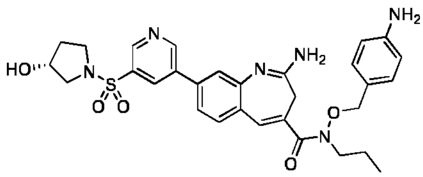 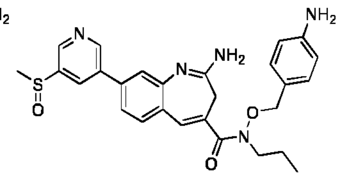 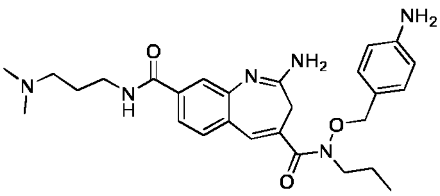

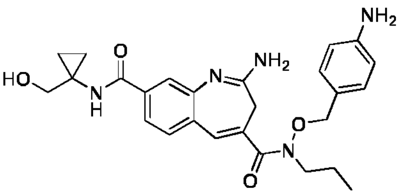 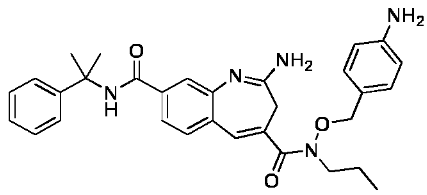 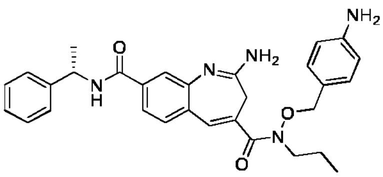

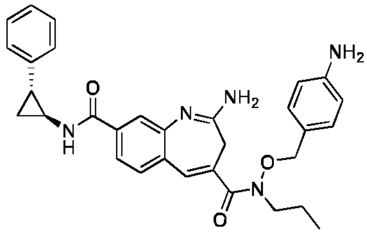 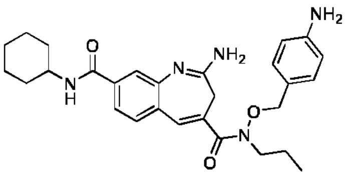 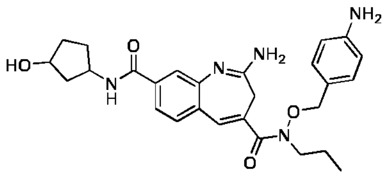

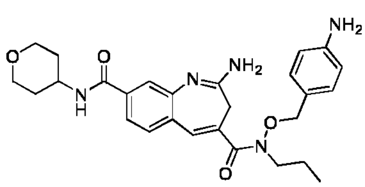 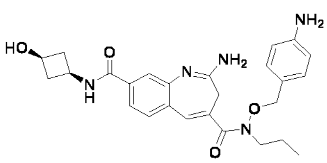 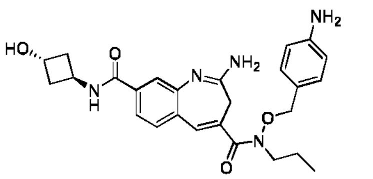

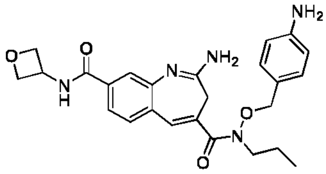
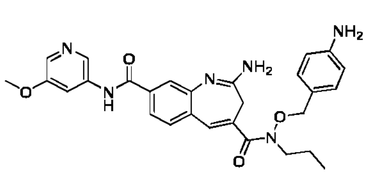
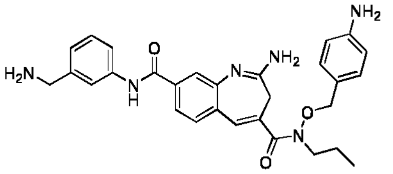

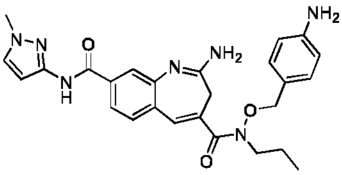
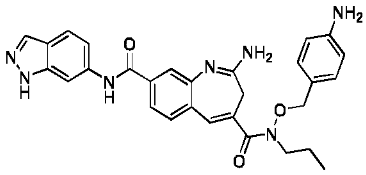
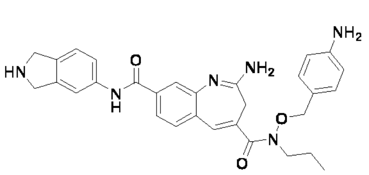

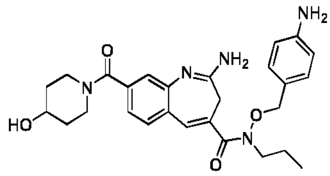
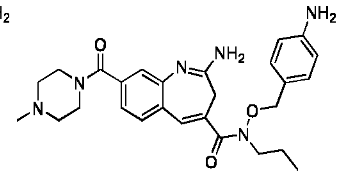
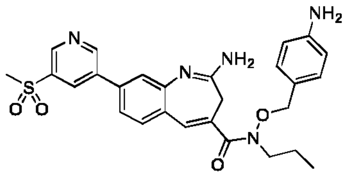

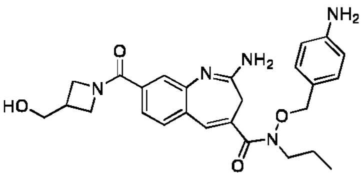
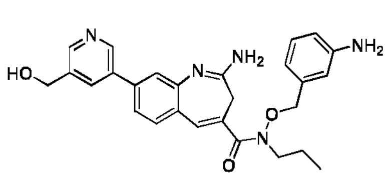
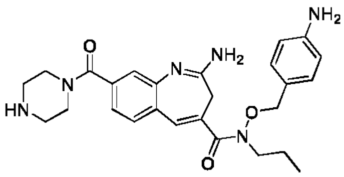

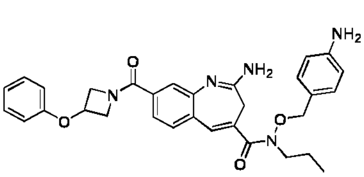
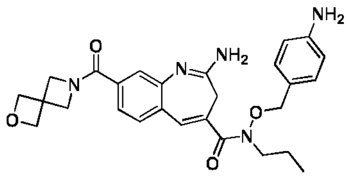
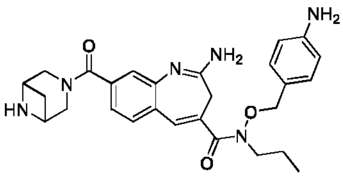

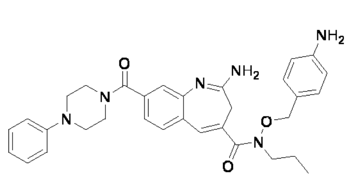
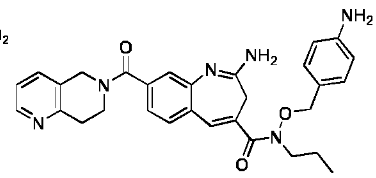
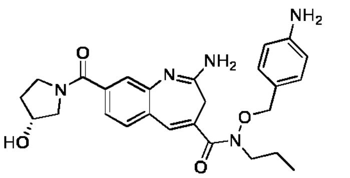

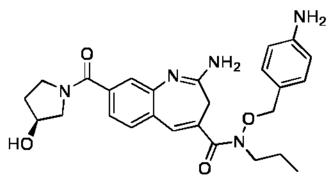
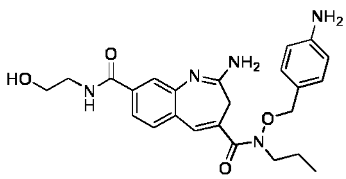
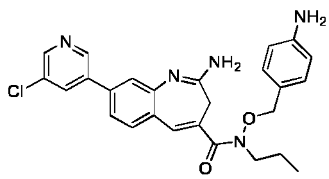

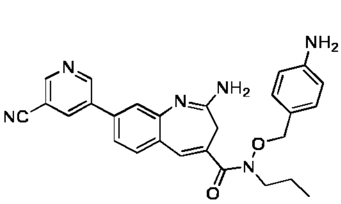
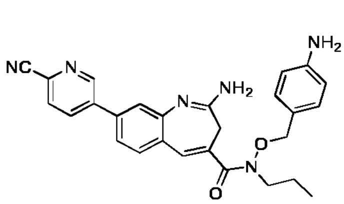
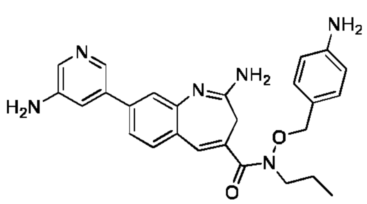

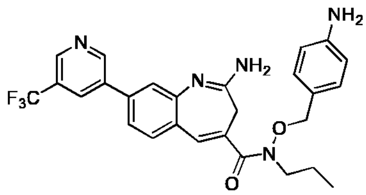 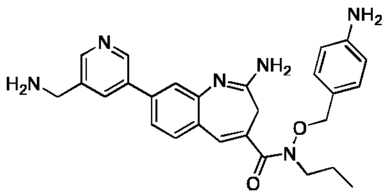 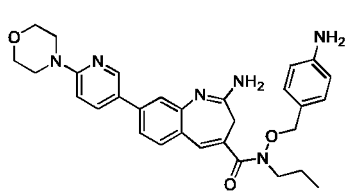

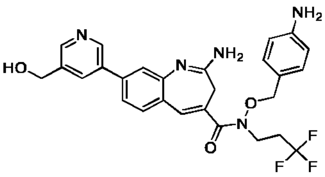 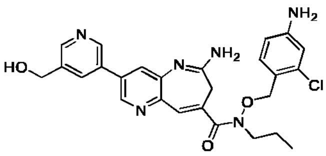 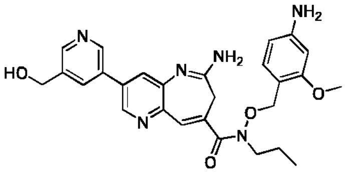.

The present application provides a compound or a tautomer, a mesomer, a racemate, an enantiomer or a diastereoisomer thereof, or a mixture thereof, or a pharmaceutically acceptable salt thereof, wherein the compound comprises a structure that may be selected from the group consisting of:

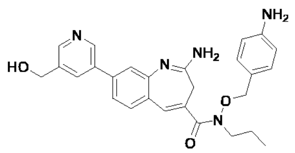 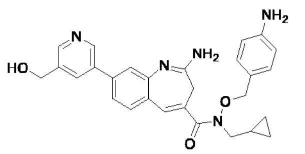 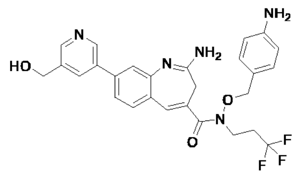 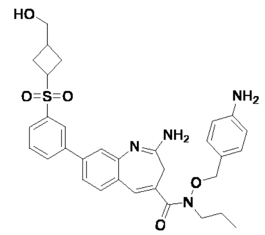

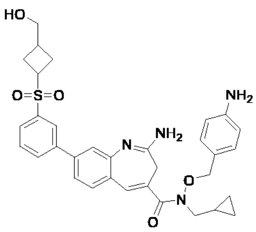 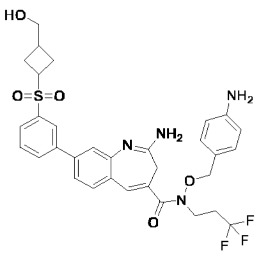 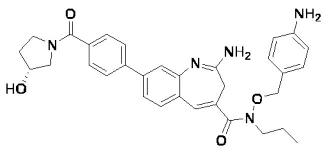 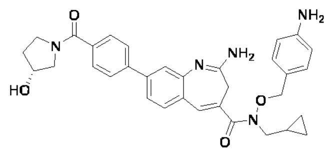

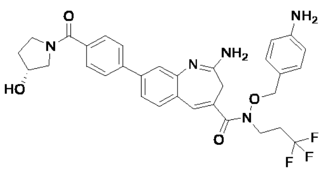 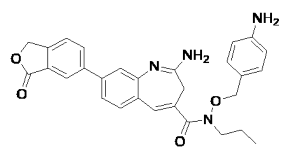 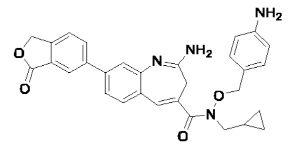 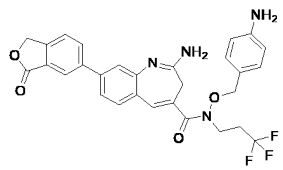

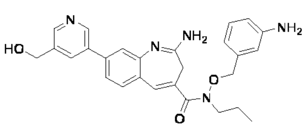 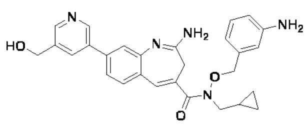 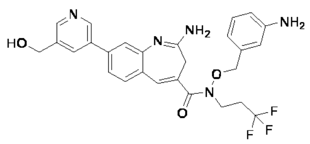 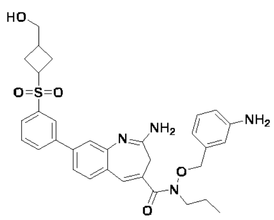

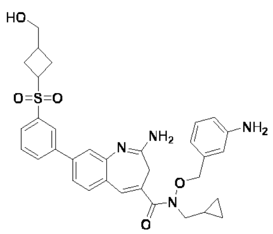 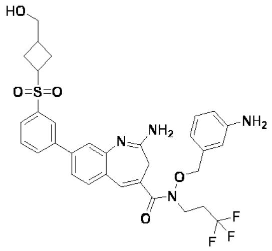 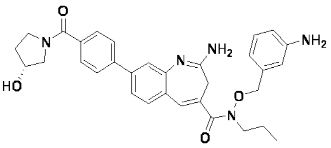 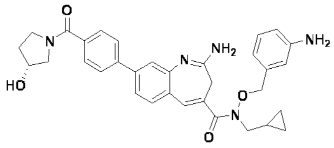

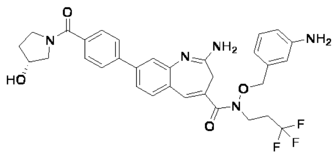 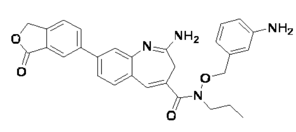 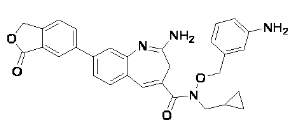 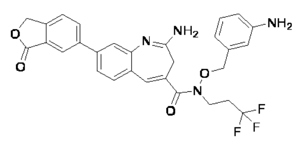

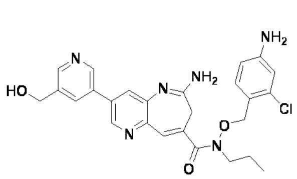 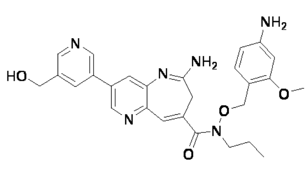 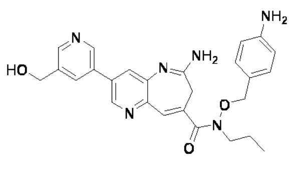 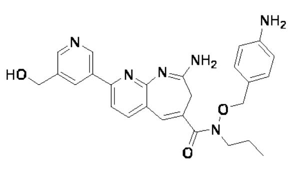

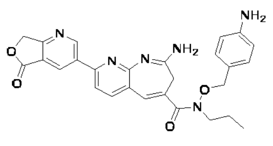 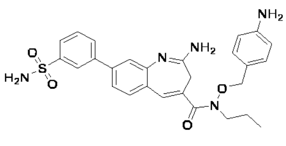 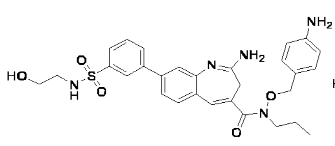 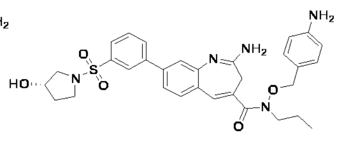

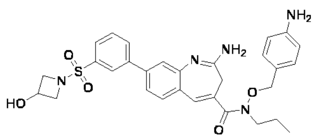 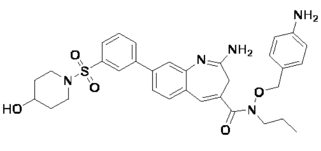 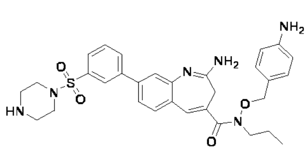 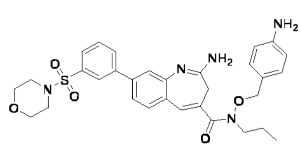

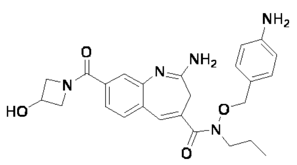 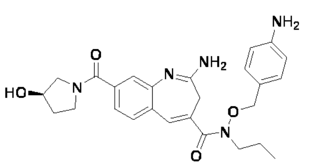 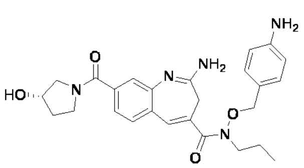 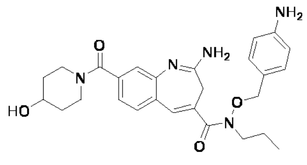

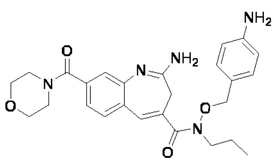 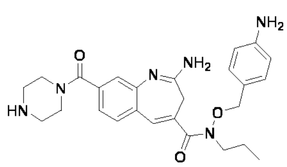

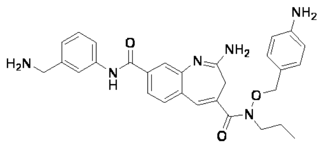 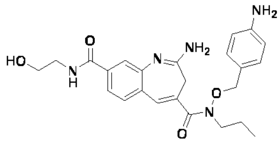 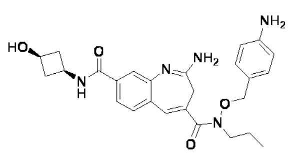 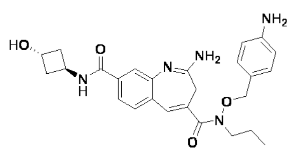

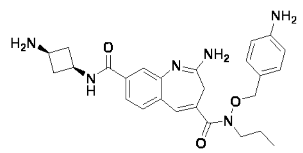
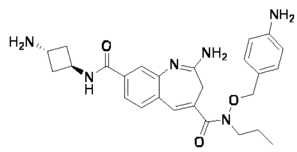
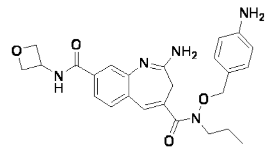
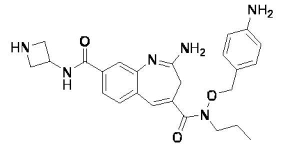
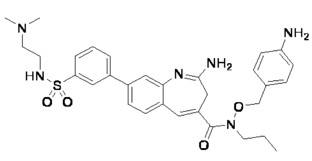
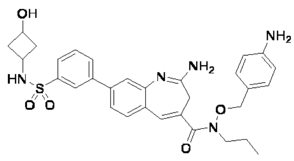
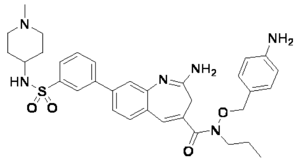
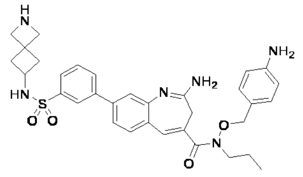
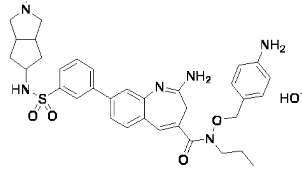
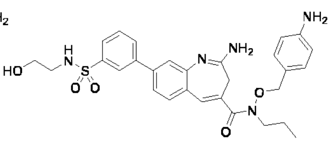
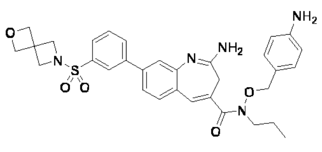
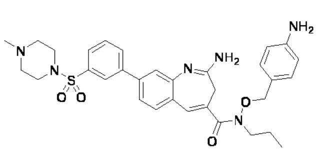
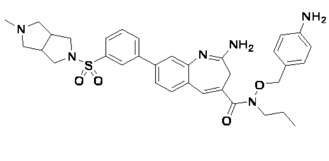
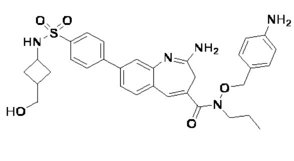
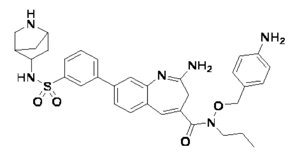
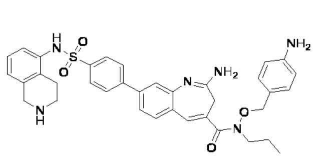
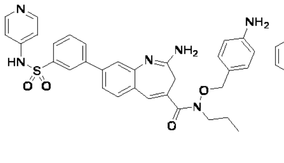
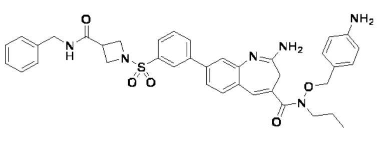
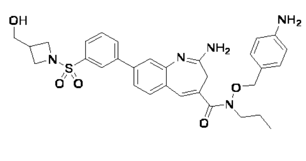
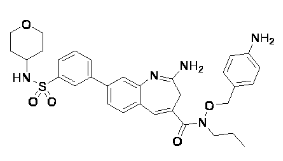
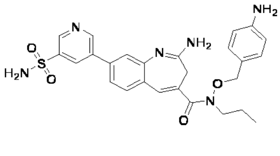
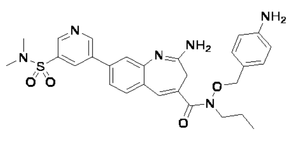
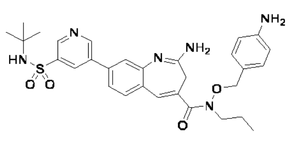
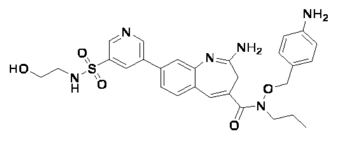
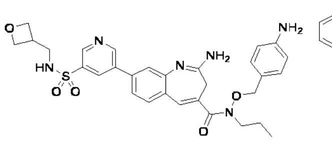
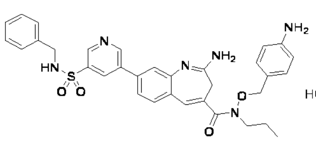
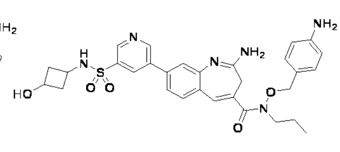
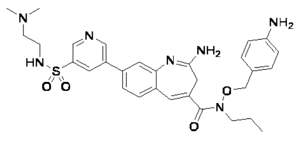
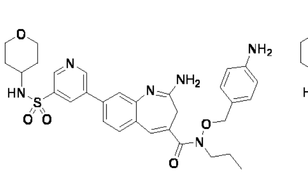
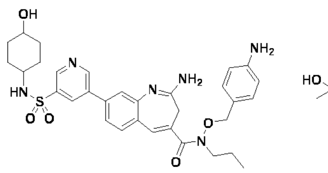
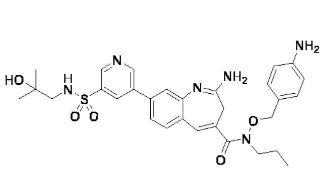
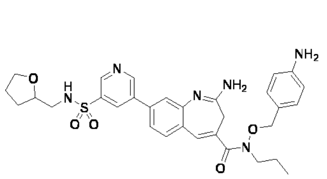
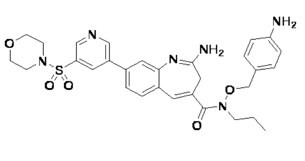
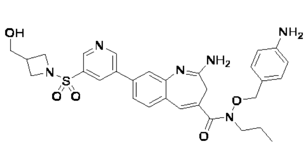
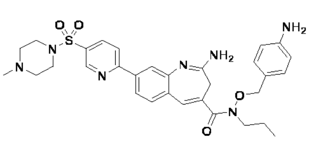
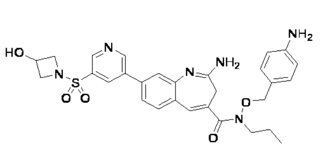

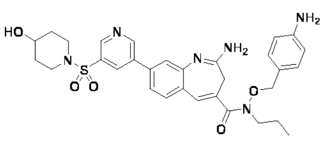
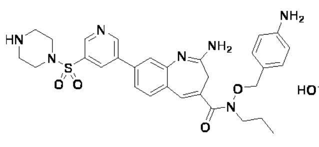
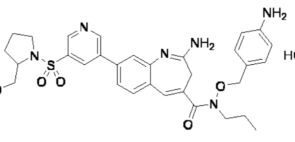
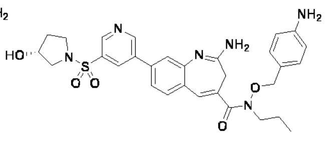

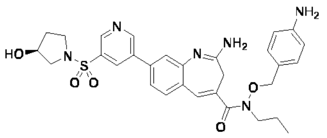
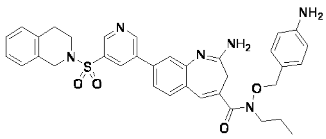

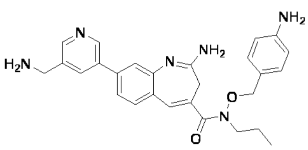
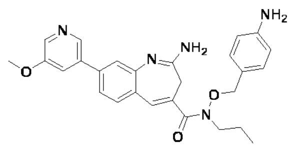
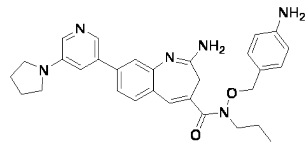
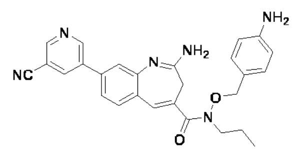

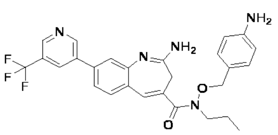
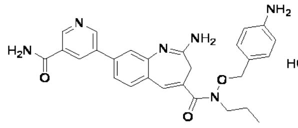
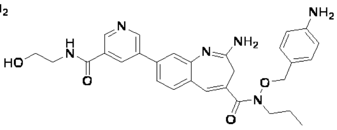
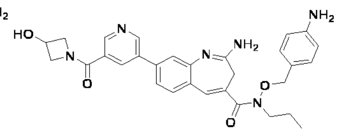

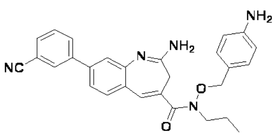
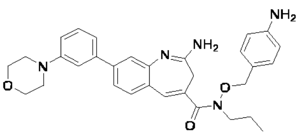
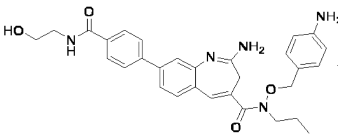
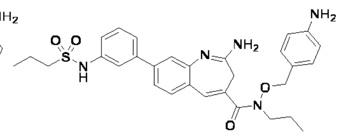

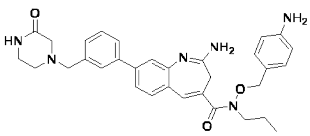
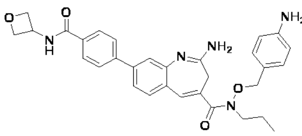
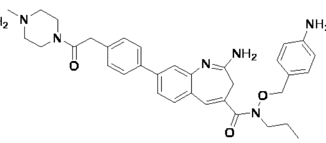
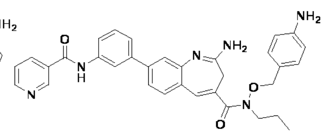

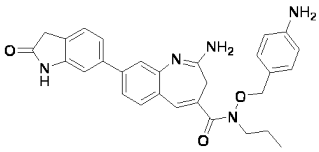
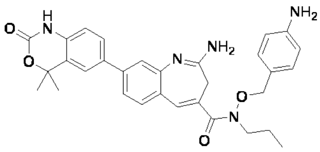
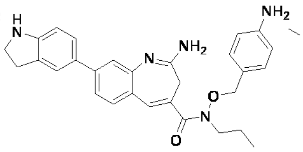
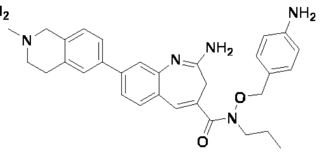

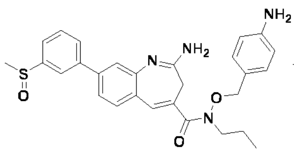
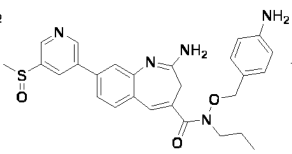
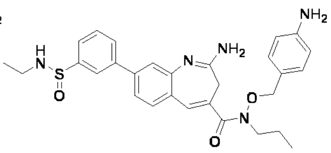
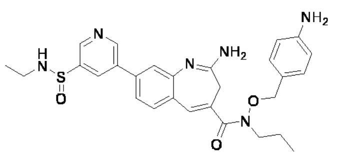

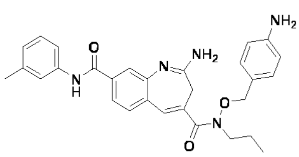
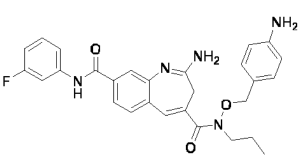
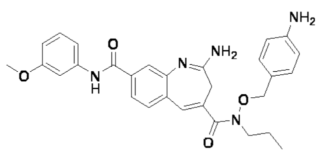
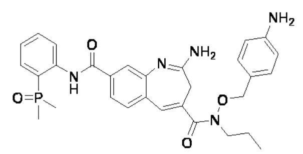

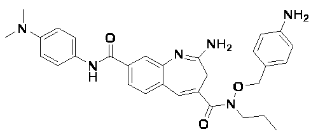
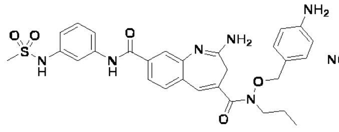
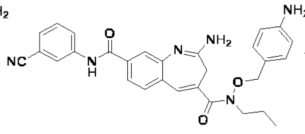
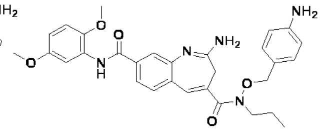

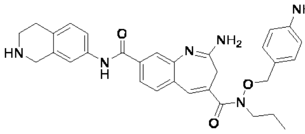
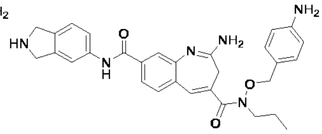
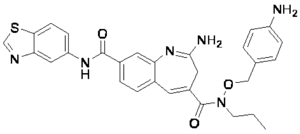
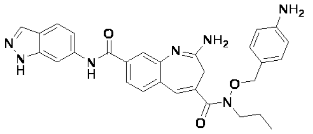

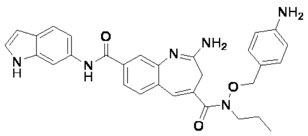
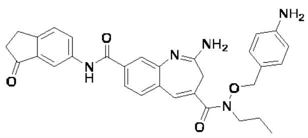
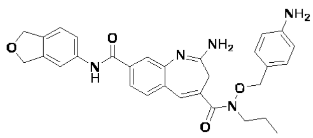
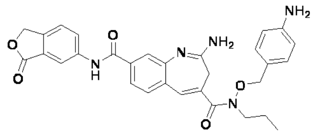

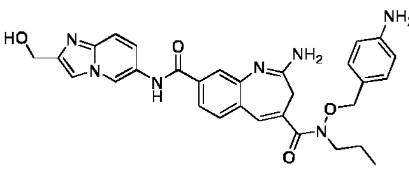
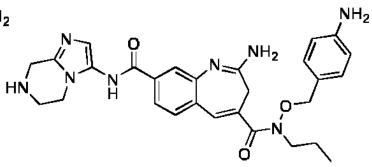
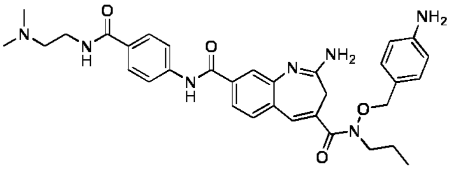

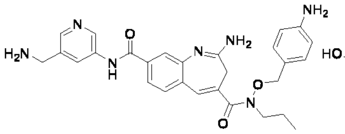
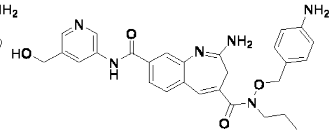
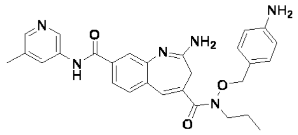
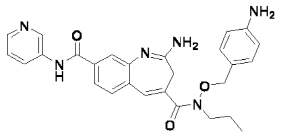

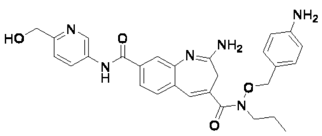
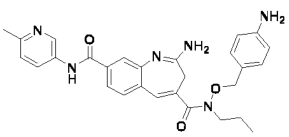
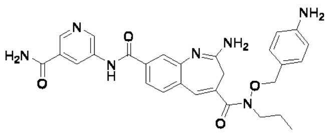
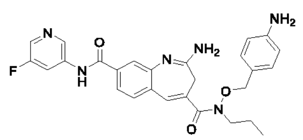

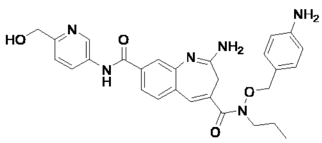
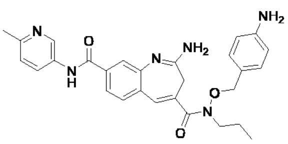
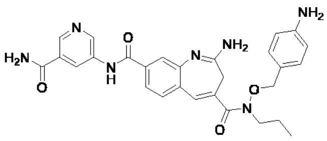
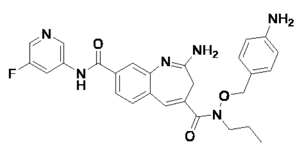

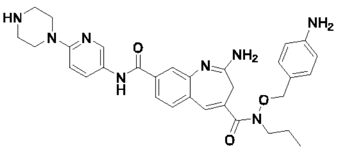
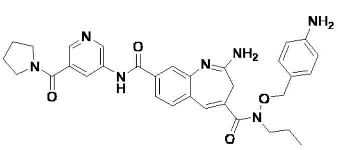

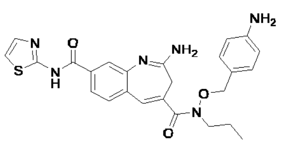
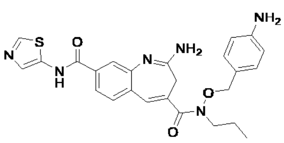
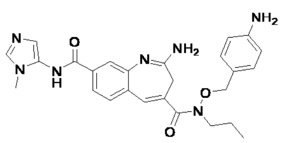
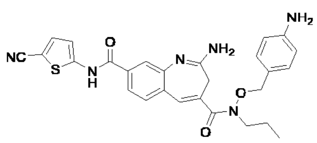

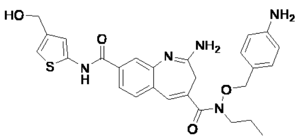
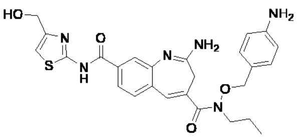
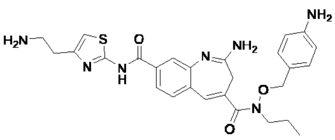
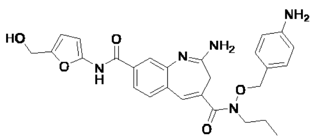

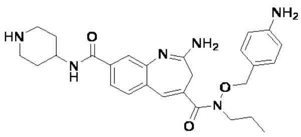
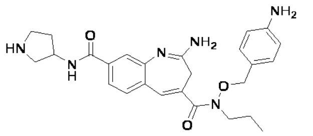
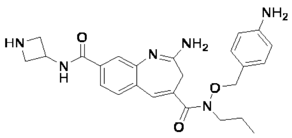
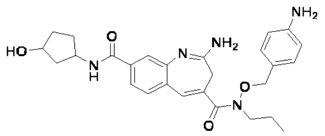

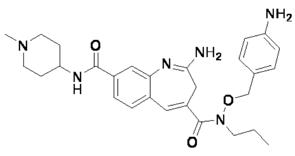
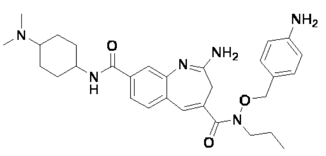
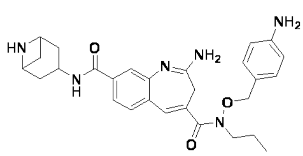
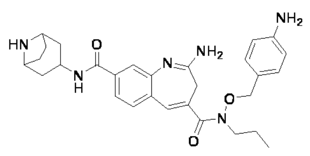

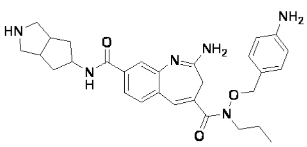
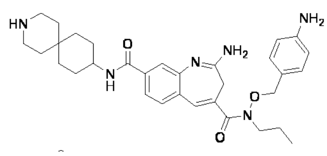
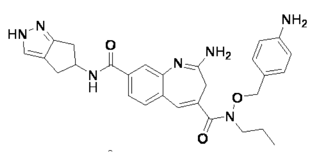
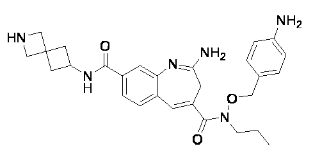

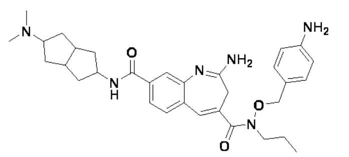
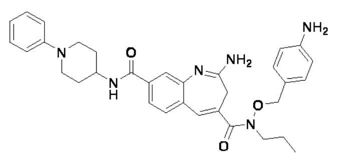
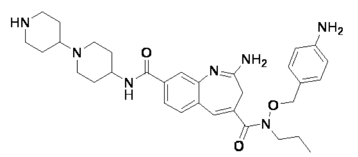

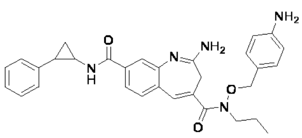
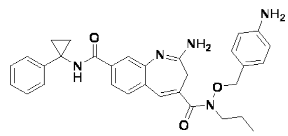
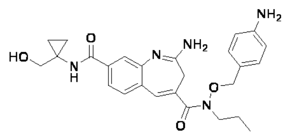
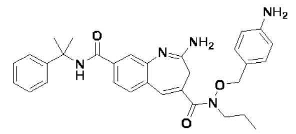

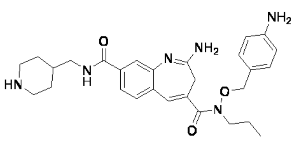
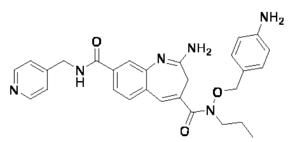
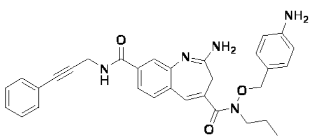
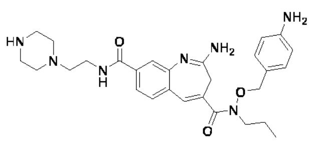

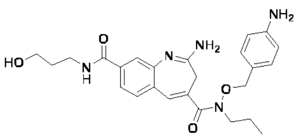
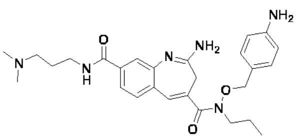
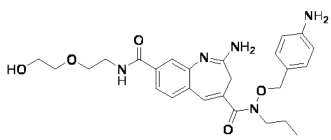
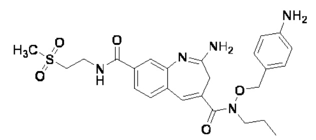

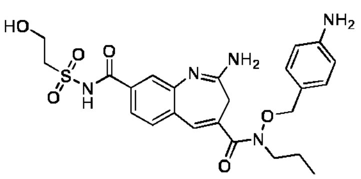
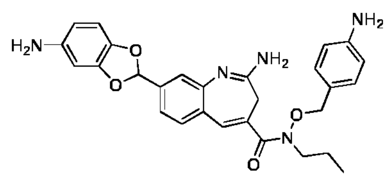
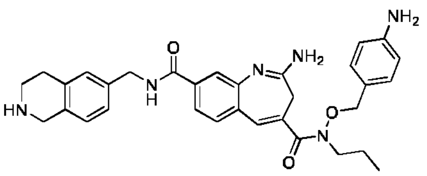

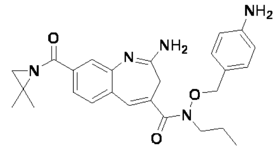 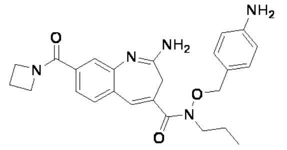 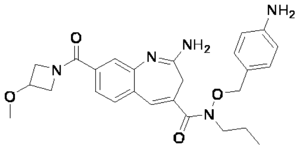 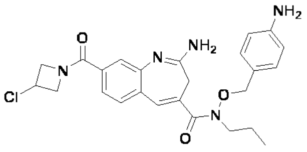

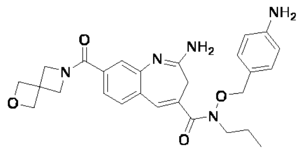 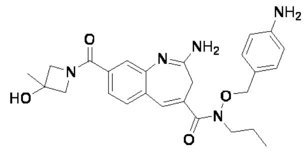 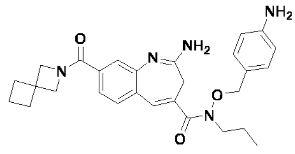 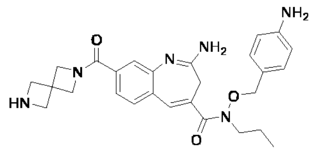

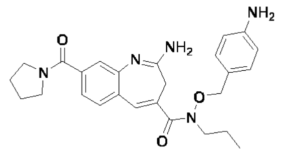 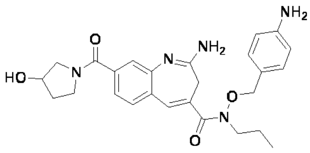 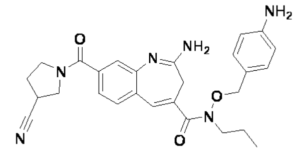 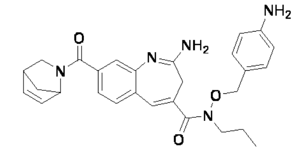

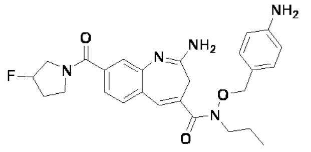 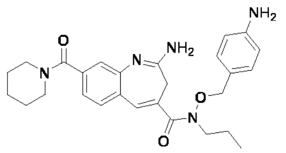 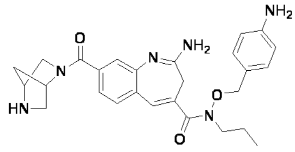 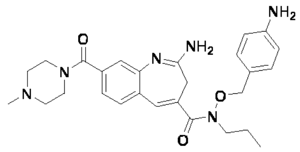

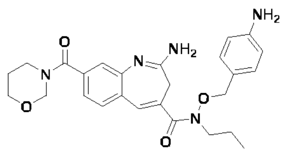 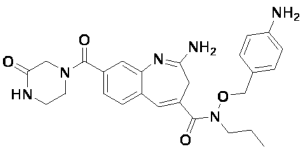 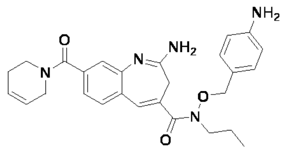 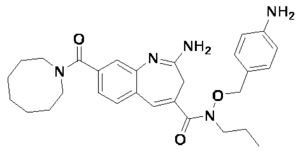

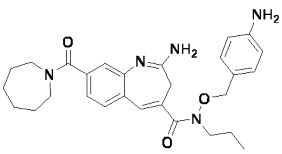 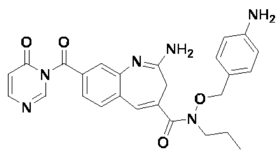 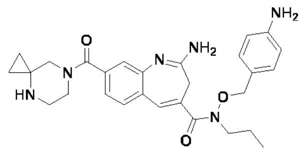 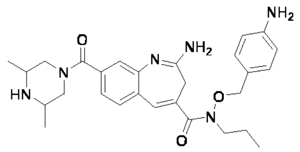

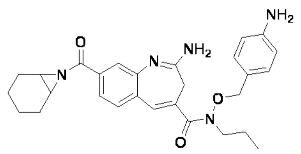 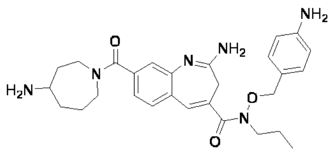 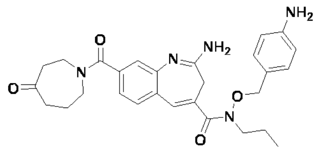 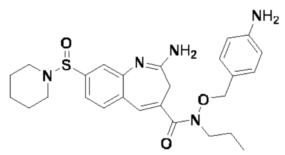

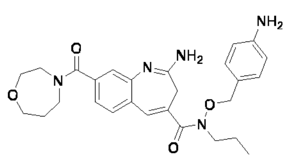 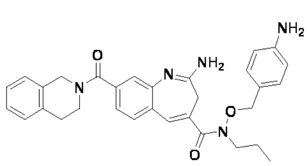 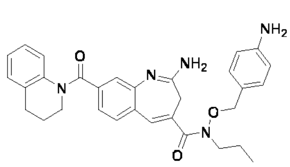 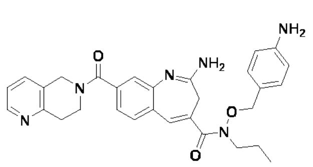

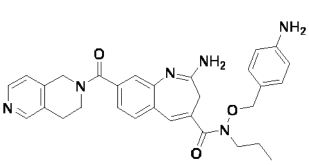 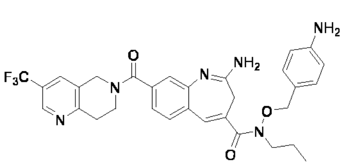 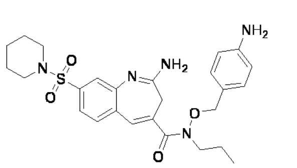

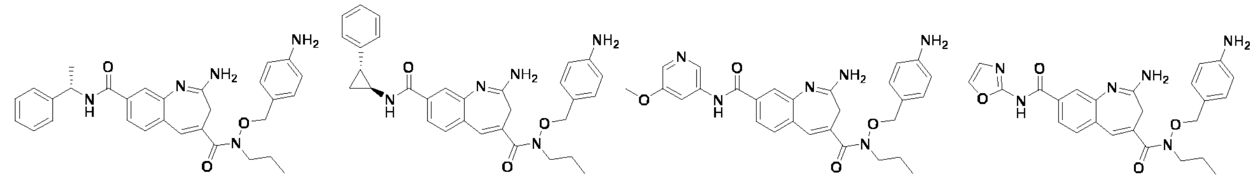

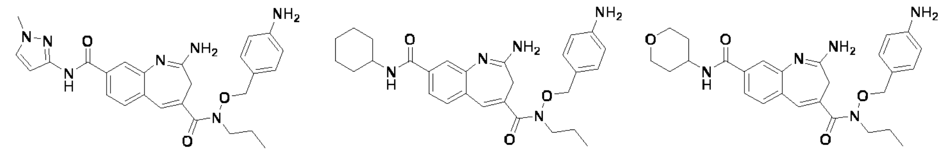

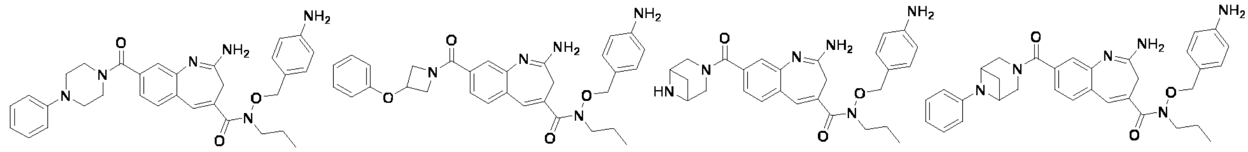

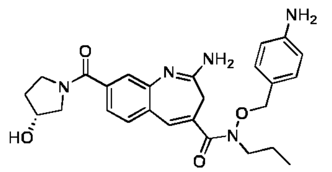

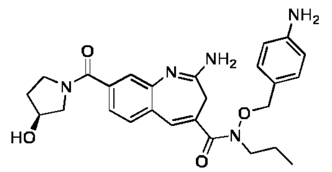

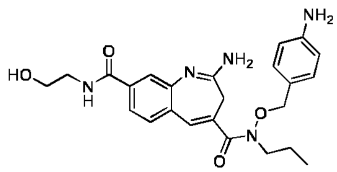

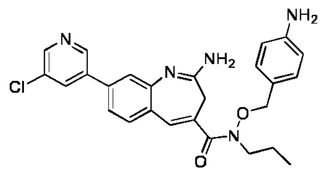

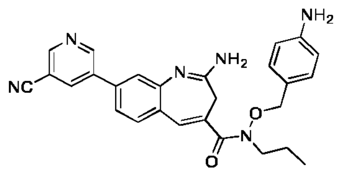

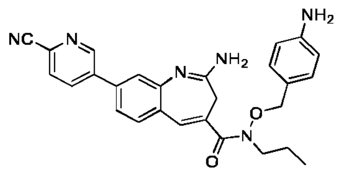

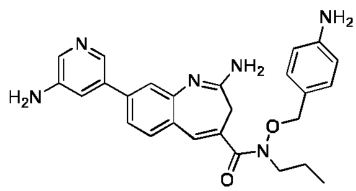

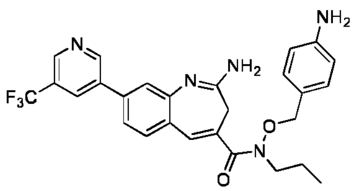

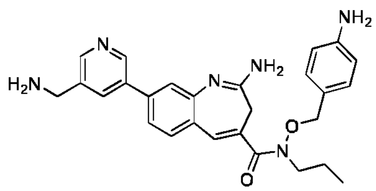

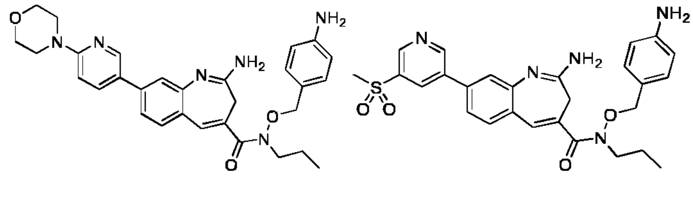.

In one aspect, the present application provides a compound of formula (C) or a tautomer, an enantiomer or a diastereoisomer, or a mixture of isomers, or a pharmaceutically acceptable salt or solvate thereof,

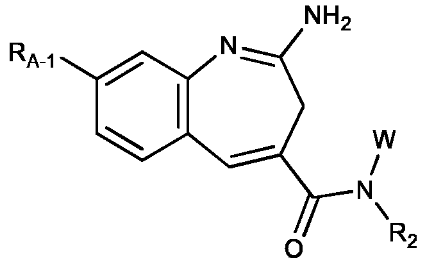

(C)

wherein:

$R_{A-1}$ is selected from $R_{A2-1}$ or $N(R_{AN-1})(R_{AN-2})C(O)$-;

$R_{A2-1}$ is phenyl, 5- to 6-membered heteroaryl, or isobenzofuranonyl, preferably phenyl, pyridinyl, or isobenzofuranonyl, and $R_{A2-1}$ is optionally substituted with one or more substituents $R_{A2-2}$, wherein $R_{A2-2}$ is each independently selected from $C_1$-$C_6$ alkyl, cyano, amino, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, -$C_1$-$C_6$ alkylhydroxy, -$C_1$-$C_6$ alkylphenyl, -phenyl, -O-phenyl, $C_{3-6}$ cycloalkyl, -$C_1$-$C_6$ alkyl-$C_1$-$C_6$ alkoxy, halogen, halogenated $C_1$-$C_6$ alkyl, halogenated $C_1$-$C_6$ alkoxy, -S(O)$C_1$-$C_6$ alkyl, -S(O)$_2$$C_1$-$C_6$ alkyl, - S(O)$_2$N$R_e$$R_f$, -C(O)N$R_e$$R_f$, or -N$R_e$$R_f$, wherein $R_e$ and $R_f$ are each independently selected from H, $C_1$-$C_6$ alkyl or -$C_1$-$C_6$ alkylhydroxy, or $R_e$ and $R_f$, together with the nitrogen atom to which they are connected, form a 4- to 8-membered nitrogen-containing heterocyclyl, e.g., tetrahydropyrrolyl, piperidinyl, morpholinyl, piperazinyl, and azetidinyl, the 4- to 8-membered nitrogen-containing heterocycle being optionally substituted with one or more substituents independently selected from hydrogen, halogen, $C_1$-$C_6$ alkyl, $C_1$-$C_6$ alkoxy, -$C_1$-$C_6$ alkylhydroxy, hydroxy, oxo, amino, -NH($C_1$-$C_6$ alkyl), -N($C_1$-$C_6$ alkyl)$_2$, -O-phenyl, and phenyl;

$R_{AN-1}$ and $R_{AN-2}$ are each independently selected from H, $C_1$-$C_6$ alkyl, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, -$C_1$-$C_6$ alkylhydroxy, halogenated $C_1$-$C_6$ alkyl, -$C_1$-$C_6$ alkyl-$C_1$-$C_6$ alkoxy, -$C_1$-$C_6$ alkyl-phenyl, -phenyl, 5- to 10-membered heteroaryl, $C_{3-6}$ cycloalkyl, -$C_{3-6}$ cycloalkyl-phenyl, 7- to 10-membered bicyclic heterocyclyl, and 3- to 8-membered heterocyclyl, wherein the phenyl, 5- to 10-membered heteroaryl, $C_{3-6}$ cycloalkyl, 7- to 10-membered bicyclic heterocyclyl, and 3- to 8-membered heterocyclyl are each independently optionally substituted with one or more substituents selected from hydrogen, $C_1$-$C_6$ alkyl, cyano, amino, - NH($C_1$-$C_6$ alkyl), -N($C_1$-$C_6$ alkyl)$_2$, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, -$C_1$-$C_6$ alkylhydroxy, -$C_1$-$C_6$ alkyl-$C_1$-$C_6$ alkoxy, halogen, halogenated $C_1$-$C_6$ alkyl, halogenated $C_1$-$C_6$ alkoxy, -S(O)$C_1$-$C_6$ alkyl, -S(O)$_2$$C_1$-$C_6$ alkyl, - S(O)$_2$N$R_e$$R_f$, or -C(O)N$R_e$$R_f$, $R_e$ and $R_f$ being each independently selected from H, $C_1$-$C_6$ alkyl, or - $C_1$-$C_6$ alkylhydroxy; or

$R_{AN-1}$ and $R_{AN-2}$, together with the nitrogen atom to which they are connected, form a 4- to 10-membered nitrogen-containing heterocycle, e.g., tetrahydropyrrolyl, piperidinyl, morpholinyl, piperazinyl, azetidinyl,

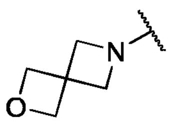, 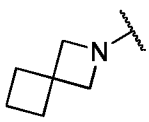, 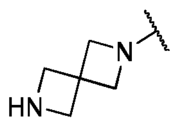, 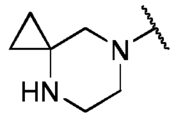, 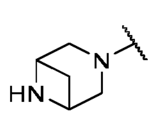,

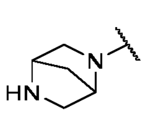, 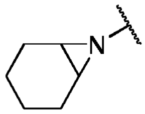, 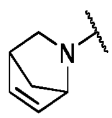, 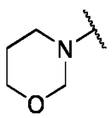, 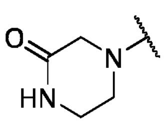, 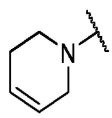, 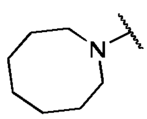, 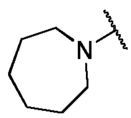,

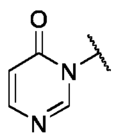, 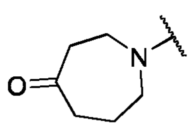, 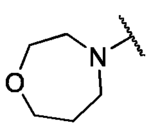, 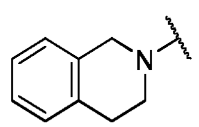, 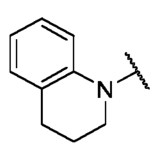, 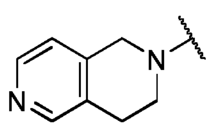

and

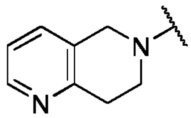,

wherein the 4- to 10-membered nitrogen-containing heterocycle is optionally substituted with one or more substituents independently selected from hydrogen, halogen, $C_1$-$C_6$ alkyl, $C_1$-$C_6$ alkoxy, -$C_1$-$C_6$ alkylhydroxy, hydroxy, oxo, amino, -NH($C_1$-$C_6$ alkyl), -N($C_1$-$C_6$ alkyl)$_2$, -O-phenyl, and phenyl;
$R_2$ is selected from -$C_1$-$C_6$ alkyl, -$C_1$-$C_6$ alkoxy, -$C_1$-$C_6$ alkylenehydroxy, -$C_1$-$C_6$ alkyl-cyclopropyl, or halogenated $C_1$-$C_6$ alkyl; preferably, $R_2$ is selected from

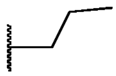, 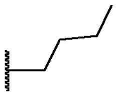, 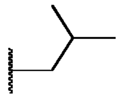, 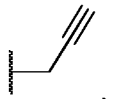,

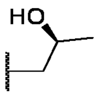, 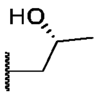, 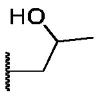, 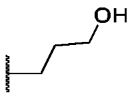, 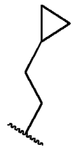, 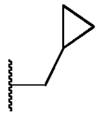, and 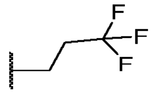;

more preferably, $R_2$ is selected from

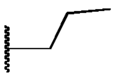 and 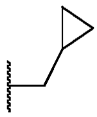;

W is selected from -OH.

In one particularly preferred embodiment, the present invention provides a compound or a tautomer, an enantiomer or a diastereoisomer, or a mixture of isomers, or a pharmaceutically acceptable salt or solvate thereof selected from:

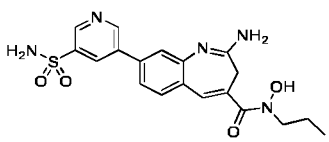 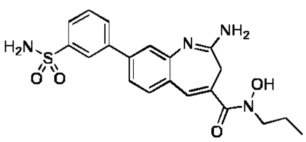 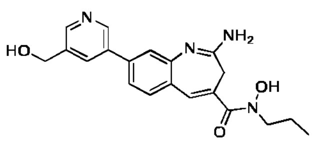 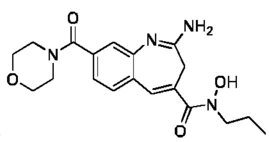

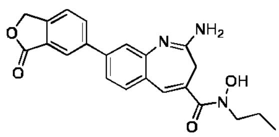 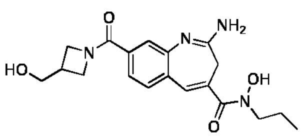 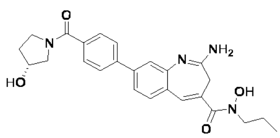 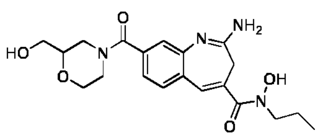

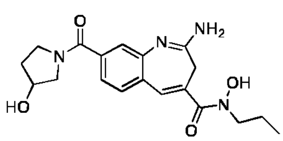 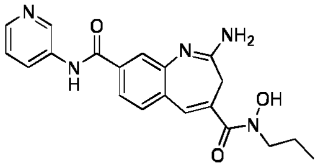 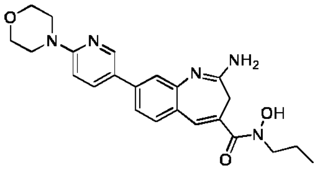 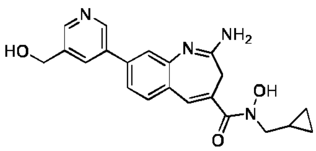

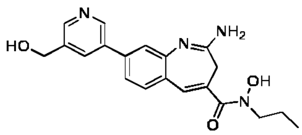
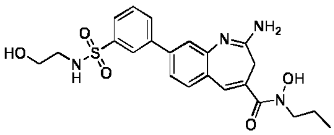
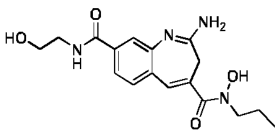
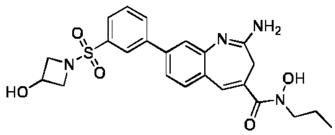

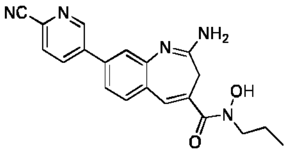
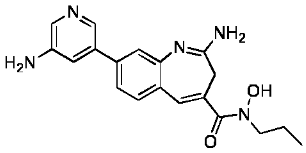
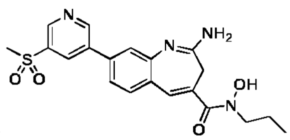
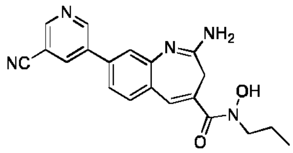

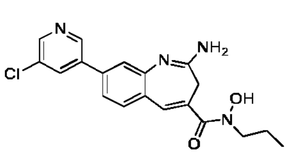
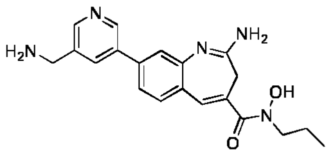
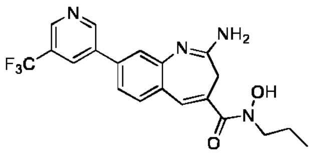.

In one aspect, the present application provides a compound or a conjugate thereof, or an isomer thereof, or a mixture thereof, or a pharmaceutically acceptable salt thereof, wherein the compound has a structure of formula (II-a):

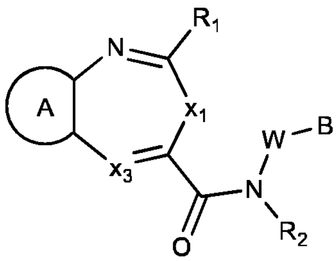
(II-a),

wherein,

$R_1$ is an optionally substituted amino;
$X_1$ is selected from optionally substituted $-CH_2-$;
$X_3$ is selected from optionally substituted -CH= or -N=;
$R_2$ is selected from hydrogen and optionally substituted $C_1$-$C_6$ alkyl; when $R_2$ comprises methylene units, the methylene units of $R_2$ are each independently unreplaced, or are each independently replaced by any structure;
W is absent, or is selected from optionally substituted $C_1$-$C_6$ alkyl; when W comprises methylene units, the methylene units of W are each independently unreplaced, or are each independently replaced by any structure;
B is selected from the group consisting of: optionally substituted aryl and optionally substituted heteroaryl;
B is substituted with one or more optionally substituted amino groups, wherein the one or more amino groups are each independently substituted with hydrogen or optionally substituted $C_1$-$C_6$ alkyl;
ring A is selected from: optionally substituted alcyl, optionally substituted aliphatic heterocyclyl, an optionally substituted aryl, and optionally substituted heteroaryl;
ring A may be substituted with m $R_{A-1}$, wherein $R_{A-1}$ is each independently selected from any group; m is selected from 1, 2, 3, 4, or 5.

In some embodiments, in the structure of formula (II-a), $R_1$ is $-NH_2$.

In some embodiments, in the structure of formula (II-a), $X_1$ is selected from $-CH_2-$ or

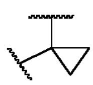.

In some embodiments, in the structure of formula (II-a), $X_3$ is selected from -CH= or $-C(CH_3)=$.

In some embodiments, in the structure of formula (II-a), $R_2$ is selected from $C_1$-$C_6$ alkyl optionally substituted

with 1, 2, or 3 $R_{2-1}$; $R_{2-1}$ is each independently selected from: hydrogen, halogen, hydroxy, amino, $C_1$-$C_6$ alkyl, and $C_3$-$C_6$ cycloalkyl, wherein $C_1$-$C_6$ alkyl and $C_3$-$C_6$ cycloalkyl are each independently optionally substituted with 1, 2, or 3 $R_{2-2}$, $R_{2-2}$ being each independently selected from: hydrogen, halogen, hydroxy, or amino; wherein the methylene unit of $R_2$ is each independently unreplaced, replaced by -O-, or replaced by $-N(R_{2-3})-$, and $R_{2-3}$ is selected from: hydrogen or $C_1$-$C_3$ alkyl.

In some embodiments, in the structure of formula (II-a), $R_{2-1}$ is each independently selected from: hydrogen, fluorine, chlorine, bromine, iodine, hydroxy, amino, -CN, $-CH_3$, $-CH_2F$, $-CHF_2$, $-CF_3$, - $CH_2CH_3$, or

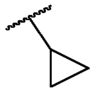.

In some embodiments, in the structure of formula (II-a), $R_2$ is selected from

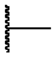, 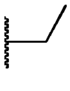, 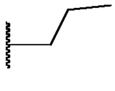,

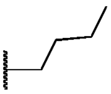, 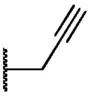, 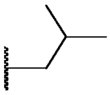, 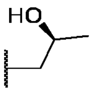, 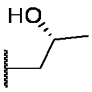, 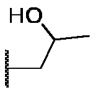, 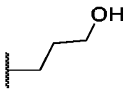, 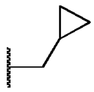, and 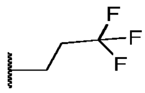,

wherein the methylene unit of $R_2$ is each independently unreplaced, replaced by -O-, or replaced by -NH-.

In some embodiments, in the structure of formula (II-a), $R_2$ is selected from:

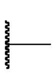, 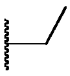, 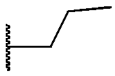,

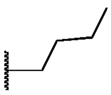, 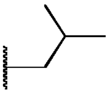, 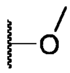, 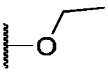, 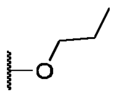, 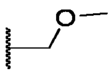, 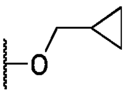, 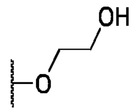, 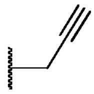,

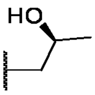, 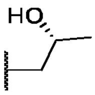, 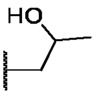, 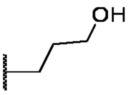, 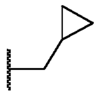, and 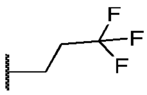.

For example, $R_2$ is selected from

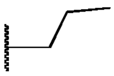.

In some embodiments, in the structure of formula (II-a), W is selected from $C_1$-$C_6$ alkyl optionally substituted with 1, 2, or 3 $R_{w-1}$; $R_{w-1}$ is each independently selected from: hydrogen, halogen, hydroxy, amino, $C_1$-$C_6$ alkyl, and $C_3$-$C_6$ cycloalkyl, wherein $C_1$-$C_6$ alkyl and $C_3$-$C_6$ cycloalkyl are each independently optionally substituted with 1, 2, or 3 $R_{w-2}$, $R_{w-2}$ being each independently selected from: hydrogen, halogen, hydroxy, or amino;

the methylene unit of W is each independently unreplaced, replaced by -O-, or replaced by $-N(R_{w-3})-$, wherein $R_{w-3}$ is selected from: hydrogen or $C_1$-$C_3$ alkyl.

In some embodiments, in the structure of formula (II-a), $R_{w-1}$ is each independently selected from: hydrogen, fluorine, chlorine, bromine, iodine, hydroxy, amino, -CN, $-CH_3$, $-CH_2F$, $-CHF_2$, $-CF_3$, - $CH_2CH_3$, or

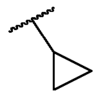.

In some embodiments, in the structure of formula (II-a), W is selected from

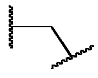, 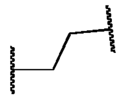,

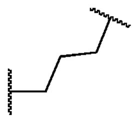, 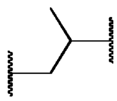, and 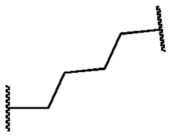,

wherein the methylene unit of W is each independently unreplaced, replaced by -O-, or replaced by -NH-.

In some embodiments, in the structure of formula (II-a), W is selected from

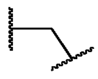, 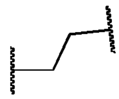,

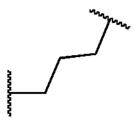, 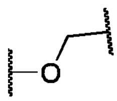, and 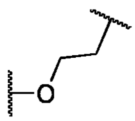.

For example, W is selected from

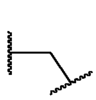 or 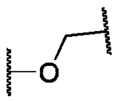.

In some embodiments, in the structure of formula (II-a), B is selected from phenyl and pyridinyl, wherein the phenyl and pyridinyl are each independently optionally substituted with 1, 2, or 3 $R_{B-1}$, $R_{B-1}$ being each independently selected from hydrogen, halogen, hydroxy, amino, $C_1$-$C_3$ alkyl, and $C_1$-$C_3$ alkoxy.

In some embodiments, in the structure of formula (II-a), B is selected from

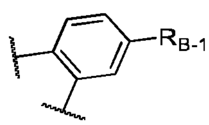,

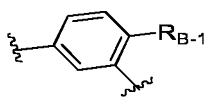, and 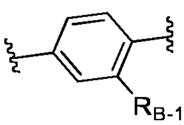;

preferably, B is selected from

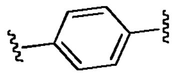, 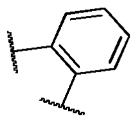,

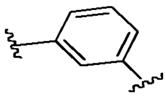, 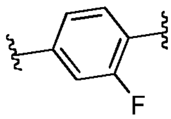, 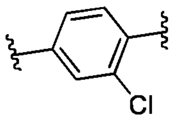, and 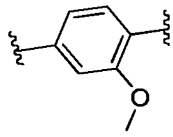.

For example, B is selected from

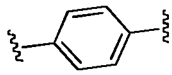.

In some embodiments, in the structure of formula (II-a), A is selected from: phenyl, pyridinyl, pyrrolyl, thienyl, furanyl, pyridazinyl, pyrimidinyl, and pyrazinyl.

In some embodiments, in the structure of formula (II-a), A is selected from phenyl and pyridinyl. For example, ring A is selected from phenyl. For example, ring A is selected from pyridinyl.

In some embodiments, in the structure of formula (II-a),

$R_{A-1}$ is $R_{A2-1}$-Lx-;

Lx is selected from: $-(CH_2)_t-$, $-(CH_2)_tO-$, $-O(CH_2)_t-$, $-(CH_2)_tN(R_{L-1})-$, $-(CH_2)_tS-$, $-(CH_2)_tC(=O)-$, $-(CH_2)_tN(R_{L-1})C(=O)-$,

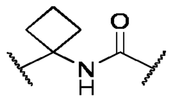, and 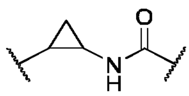,

wherein $R_{L-1}$ is selected from H or $C_1$-$C_3$ alkyl, and t is selected from 0, 1, 2, or 3;

$R_{A2-1}$ is selected from hydrogen, halogen, hydroxy, amino, cyano, $C_1$-$C_6$ alkyl, alcyl, aliphatic heterocyclyl, aryl, and heteroaryl, wherein the $C_1$-$C_6$ alkyl, alcyl, aliphatic heterocyclyl, aryl, and heteroaryl are each independently optionally substituted with 1, 2, or 3 $R_{A2-2}$; $R_{A2-2}$ is selected from hydrogen, halogen, hydroxy, amino, and $C_1$-$C_6$ alkyl optionally substituted with 1, 2, or 3 $R_{A2-3}$; $R_{A2-3}$ is each independently selected from: hydrogen, halogen, hydroxy, amino, and $C_1$-$C_3$ alkyl;

wherein any methylene unit of $R_{A2-1}$ may be replaced by the following structure: -O-, -S-, $-S(=O)_2-$, -NH-, -CO-,

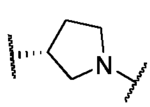, 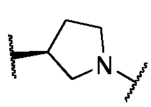, and 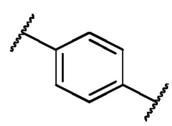;

m is selected from 1, 2, or 3.

In some embodiments, in the structure of formula (II-a), $R_{A-1}$ is $R_{A2-1}$-Lx-; Lx is single bond or - NHC(=O)-.

In some embodiments, in the structure of formula (II-a), $R_{A-1}$ is $R_{A2-1}$-Lx-; $R_{A2-1}$ is each independently selected from hydrogen, halogen, hydroxy, amino, cyano, or $C_1$-$C_6$ alkyl,

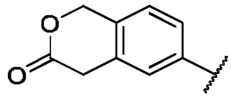,

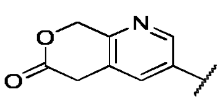, 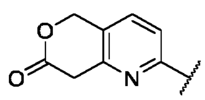, 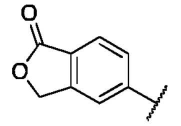, 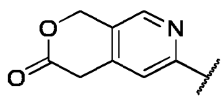, 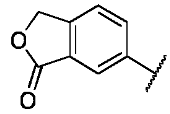,

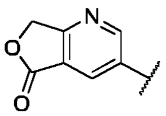, 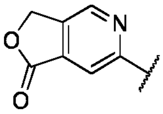, 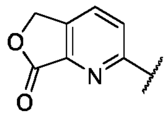, 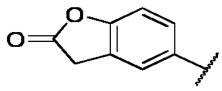, 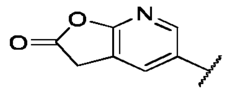,

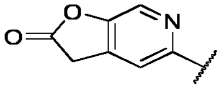, 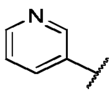, 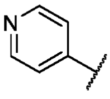, 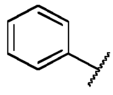, 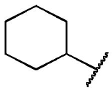, 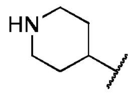, 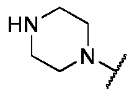, 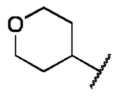,

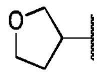, 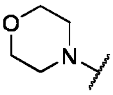, 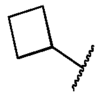, 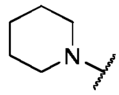, 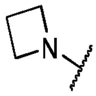 or 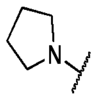

optionally substituted with 1, 2, or 3 $R_{A2-2}$, wherein any methylene unit of $R_{A2-1}$ may be replaced by the following structure: -O-, -S-, - S(=O)-, $-S(=O)_2-$, -NH-, -C(O)-,

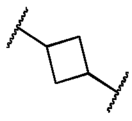, 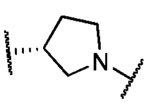, 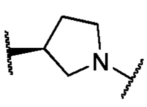, or 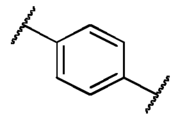.

In some embodiments, in the structure of formula (II-a), $R_{A-1}$ is selected from: hydrogen, fluorine, chlorine, bromine, iodine, hydroxy, amino, cyano, sulfhydryl, $-CH_3$, -CH(=O), -C(=O)OH, - OCH(=O), -SH(=O), $-SH(=O)_2$, $-CH_2CH_3$, $-CH_2OH$, $-OCH_3$, $-NHCH_3$, $-CH_2NH_2$, $-S(=O)_2NH_2$, - $NHSH(=O)_2$, $-SCH_3$, $-CH_2SH$, $-S(=O)CH_3$, $-CH_2SH(=O)$, $-S(=O)_2CH_3$, $-CH_2SH(=O)_2$, -NHCH(=O), $-C(=O)NH_2$, $-CH_2CH(=O)$, $-CH_2NHCH(=O)$, $-C(=O)NHCH_3$,

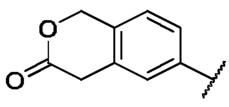,

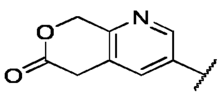, 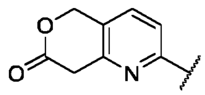, 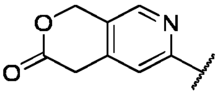, 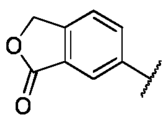, 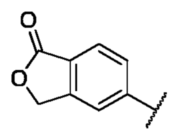,

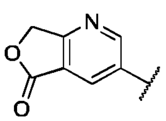, 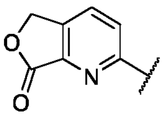, 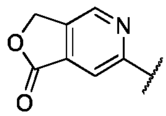, 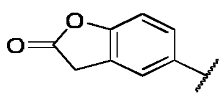, 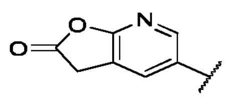,

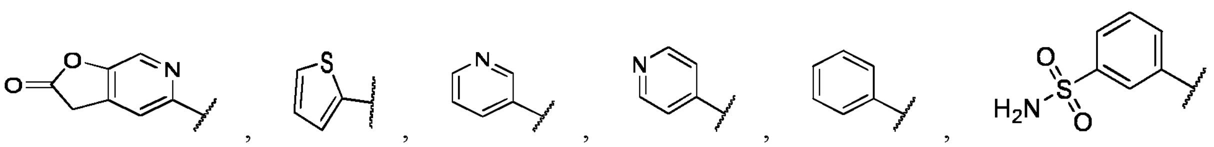, , , , , ,

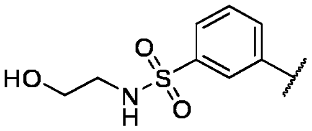 , 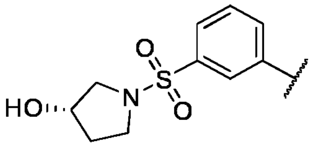 , 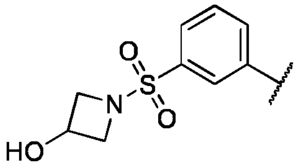 ,

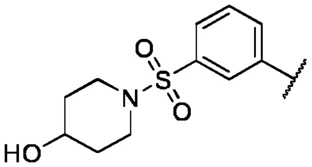 , 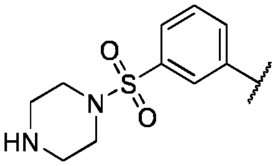 , 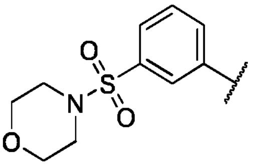 , 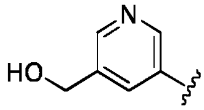 ,

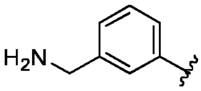 , 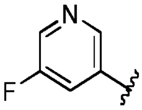 , 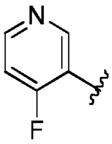 , 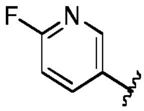 , 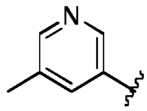 , 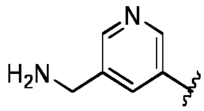 ,

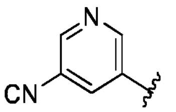 , 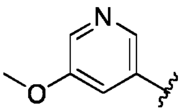 , 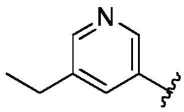 , 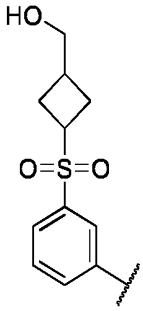 , 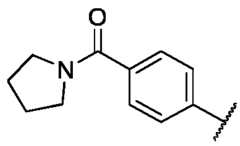 ,

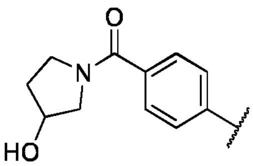 , 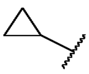 , 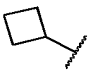 , 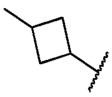 , 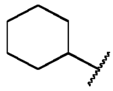 , 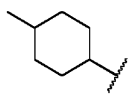 , 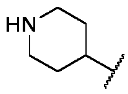 ,

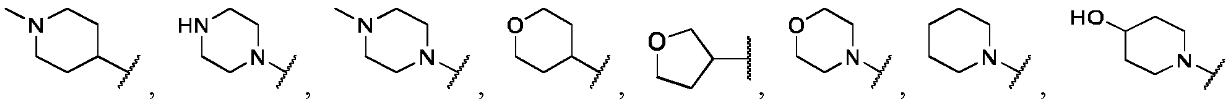 , , , , , , , ,

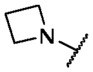 , 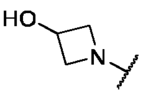 , 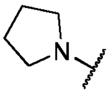 , 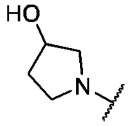 , 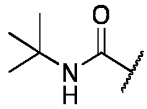 , 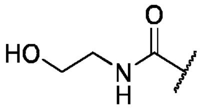 , 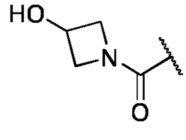 ,

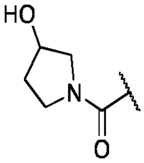 , 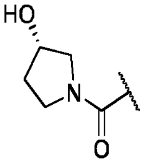 , 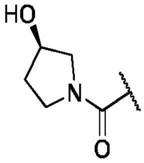 , 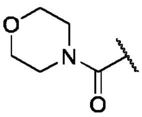 , 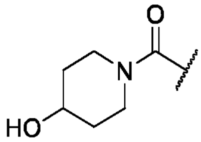 , 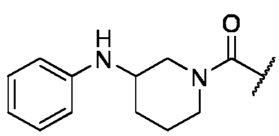 ,

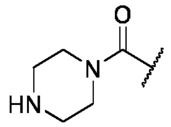, 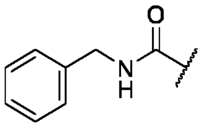, 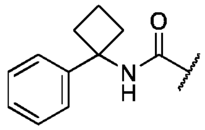, 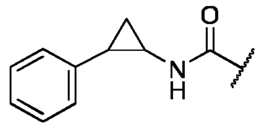, 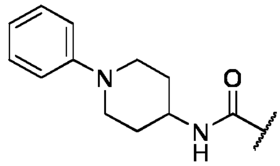,

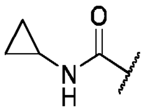, 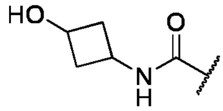, 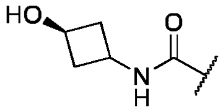, 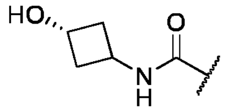, 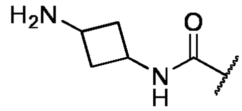,

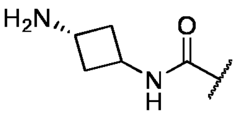, 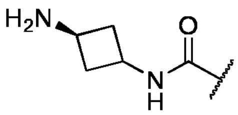, 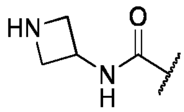, 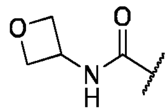, 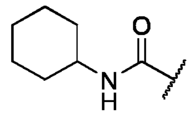,

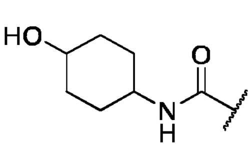, 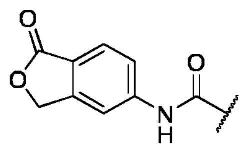, 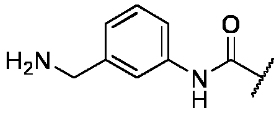, 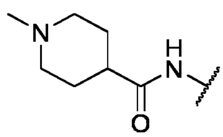,

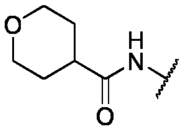, 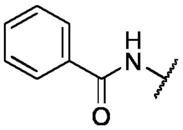, 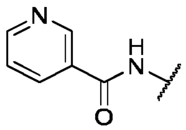, 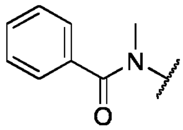, 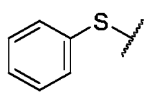, 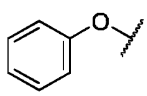,

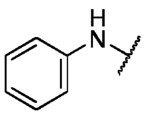, 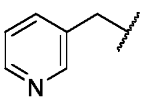, 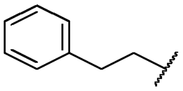, 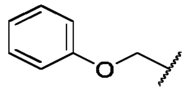, 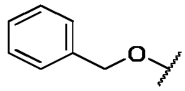, 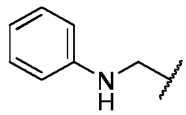,

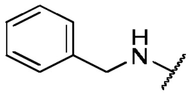, 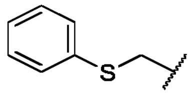, and 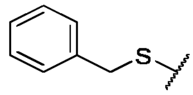.

In some embodiments, in the structure of formula (II-a), $R_{A-1}$ is $R_{A2-1}$-Lx-, wherein Lx is single bond, and $R_{A2-1}$ is each independently selected from phenyl and pyridinyl optionally substituted with 1, 2, or 3 $R_{A2-2}$; $R_{A2-2}$ is selected from hydrogen, halogen, hydroxy, amino, and $C_1$-$C_6$ alkyl optionally substituted with 1, 2, or 3 $R_{A2-3}$; $R_{A2-3}$ is each independently selected from: hydrogen, halogen, hydroxy, and amino; preferably, $R_{2A-1}$ is selected from:

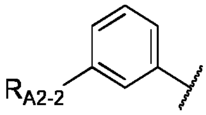, 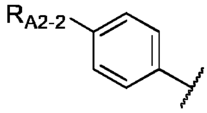, 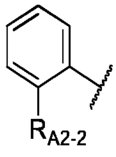, 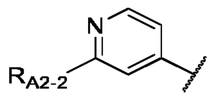,

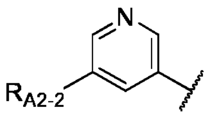, 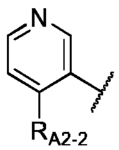, and 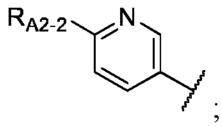;

more preferably, $R_{A-1}$ is selected from:

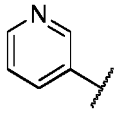, 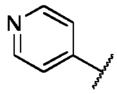, 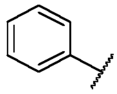, 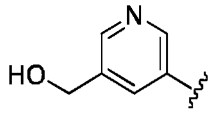,

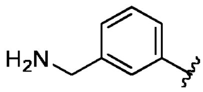, 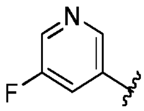, 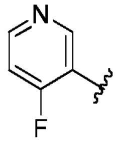, 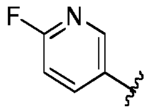, 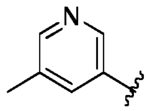, 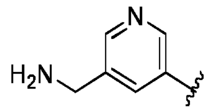,

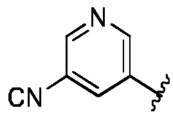, 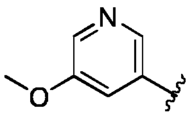, and 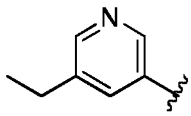.

In some embodiments, in the structure of formula (II-a), $R_{A-1}$ is $R_{A2-1}$-Lx-, wherein Lx is single bond, and $R_{A2-1}$ is selected from is $-CH_2OH$.

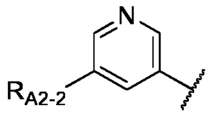;

preferably, $R_{A2-2}$

In some embodiments, in the structure of formula (II-a), $R_{A-1}$ is $R_{A2-1}$-Lx-, wherein Lx is -NHC(=O)-, and $R_{A2-1}$ is each independently selected from phenyl and pyridinyl optionally substituted with 1, 2, or 3 $R_{A2-2}$; $R_{A2-2}$ is selected from hydrogen, halogen, hydroxy, amino, and $C_1$-$C_6$ alkyl optionally substituted with 1, 2, or 3 $R_{A2-3}$; $R_{A2-3}$ is each independently selected from: hydrogen, halogen, hydroxy, and amino; preferably, $R_{2A-1}$ is selected from:

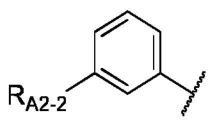, 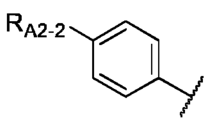, 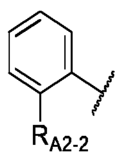, 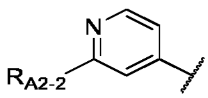,

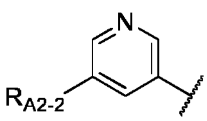, 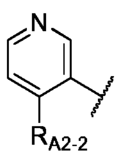, and 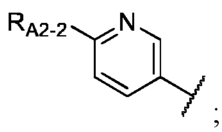;

more preferably, $R_{2A-1}$ is selected from:

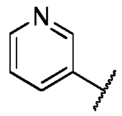, 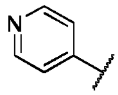, 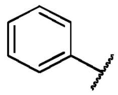, 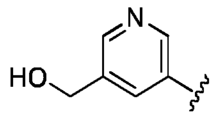,

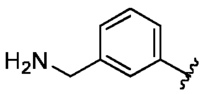, 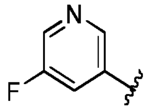, 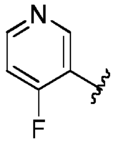, 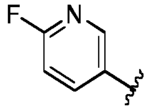, 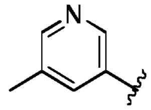, 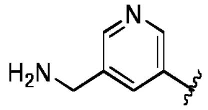,

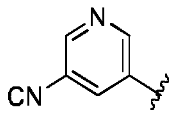, 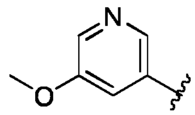, and 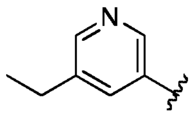.

In one aspect, the present application provides a compound of formula (A) or a tautomer, an enantiomer or a diastereoisomer, or a mixture of isomers, or a pharmaceutically acceptable salt or solvate thereof,

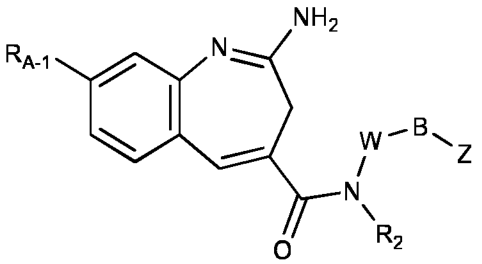

(A)

wherein:

$R_{A-1}$ is selected from $R_{A2-1}$-Lx-;

Lx is selected from single bond, $C_1$-$C_6$ alkylene, -O-, -S-, -C(O)-, -NH-, -C(O)NH-, -NHC(O)-, - C(O)-N($C_1$-$C_6$ alkyl)-, or -N($C_1$-$C_6$ alkylene)-C(O)-, and preferably, Lx is selected from single bond, -C(O)NH-, or -NHC(O)-;

$R_{A2-1}$ is aryl, heteroaryl, or heterocyclyl, preferably phenyl, pyridinyl, or isobenzofuranonyl, and $R_{A2-1}$ is optionally substituted with one or more substituents $R_{A2-2}$,

wherein $R_{A2-2}$ are each independently selected from $C_1$-$C_6$ alkyl, amino, -NH($C_1$-$C_6$ alkyl), -N($C_1$-$C_6$ alkyl)$_2$, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, - $C_1$-$C_6$ alkoxy, -$C_1$-$C_6$ alkylhydroxy, -$C_1$-$C_6$ alkyl-$C_1$-$C_6$ alkoxy, halogen, halogenated $C_1$-$C_6$ alkyl, halogenated $C_1$-$C_6$ alkoxy, or -C(O)N$R_e$$R_f$, and wherein $R_e$ and $R_f$ are each independently selected from H or $C_1$-$C_6$ alkyl, or $R_e$ and $R_f$, together with the nitrogen atom to which they are connected, form a 5- or 6-membered nitrogen-containing heterocyclyl, such as tetrahydropyrrolyl or piperidinyl, the 5- or 6-membered nitrogen-containing heterocyclyl being optionally substituted with one or more substituents independently selected from $C_1$-$C_6$ alkyl, $C_1$-$C_6$ alkoxy, hydroxy, oxo, amino, -NH($C_1$-$C_6$ alkyl), or -N($C_1$-$C_6$ alkyl)$_2$,

$R_2$ is selected from -$C_1$-$C_6$ alkyl, -$C_1$-$C_6$ alkoxy, -$C_1$-$C_6$ alkylenehydroxy, or halogenated $C_1$-$C_6$ alkyl; W is selected from -$C_1$-$C_6$ alkylene- and -O-$C_1$-$C_6$ alkylene-;

B is phenyl, and is optionally substituted with one or more substituents selected from: halogen, $C_1$-$C_6$ alkyl, halogenated $C_1$-$C_6$ alkyl, and $C_1$-$C_6$ alkoxy; and

Z is -NH($R_{Z-1}$), and $R_{Z-1}$ is selected from H, -$C_1$-$C_6$ alkyl, and halogenated $C_1$-$C_6$ alkyl.

In some embodiments, the compound or the conjugate thereof, or the isomer thereof, or the mixture thereof, or the pharmaceutically acceptable salt thereof, is selected from:

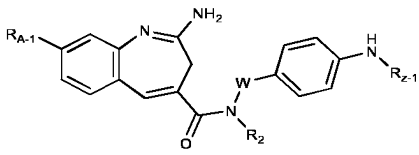,
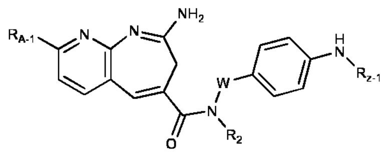,
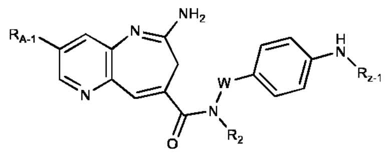,

(II-1A-1) (II-1A-2) (II-1A-3)

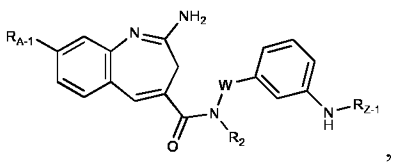,
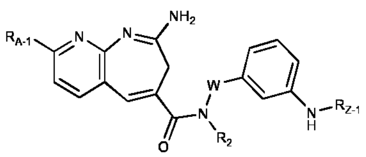, and
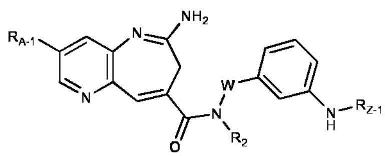,

(II-1A-4) (II-1A-5) (II-1A-6)

wherein,

$R_{Z-1}$ is selected from H or $C_1$-$C_6$ alkyl optionally substituted with 1, 2, or 3 fluorine, chlorine, bromine, iodine, hydroxy, or amino;
$R_2$ is selected from $C_1$-$C_6$ alkyl optionally substituted with 1, 2, or 3 $R_{2-1}$; $R_{2-1}$ are each independently selected from: hydrogen, halogen,
hydroxy, amino, $C_1$-$C_6$ alkyl, and $C_3$-$C_6$ cycloalkyl, wherein the $C_1$-$C_6$ alkyl and the $C_3$-$C_6$ cycloalkyl are each independently optionally substituted with 1, 2, or 3 $R_{2-2}$; $R_{2-2}$ are each independently selected from: hydrogen, halogen, hydroxy, or amino; the methylene units in $R_2$
are each independently unreplaced, replaced by -O-, or replaced by -N($R_{2-3}$)-; $R_{2-3}$ is selected from: hydrogen or $C_1$-$C_3$
alkyl;
W is selected from $C_1$-$C_6$ alkyl optionally substituted with 1, 2, or 3 $R_{w-1}$; $R_{w-1}$ are each independently selected from hydrogen, halogen, hydroxy,
amino, $C_1$-$C_6$ alkyl, and $C_3$-$C_6$ cycloalkyl, wherein the $C_1$-$C_6$ alkyl and $C_3$-$C_6$ cycloalkyl are each independently optionally substituted with 1, 2, or 3 $R_{w-2}$; $R_{w-2}$ are each independently selected from:
hydrogen, halogen, hydroxy, or amino; the methylene units in W are each independently unreplaced, replaced by -O-, or replaced by -N($R_{w-3}$)-; $R_{w-3}$ is selected from: hydrogen or $C_1$-$C_3$ alkyl;
$R_{A-1}$ is selected from $R_{A2-1}$-Lx-;
Lx is selected from single bond, $C_1$-$C_6$ alkylene, -O-, -S-, -C(O)-, -NH-, -C(O)NH-, -NHC(O)-, - C(O)-N($C_1$-$C_6$ alkyl) -, or -N($C_1$-$C_6$ alkylene)-C(O)-,
$R_{A2-1}$ is aryl, heteroaryl, or heterocyclyl, preferably phenyl, pyridinyl, or isobenzofuranonyl, and $R_{A2-1}$ is optionally substituted with one or more substituent $R_{A2-2}$,
wherein $R_{A2-2}$ are each independently selected from -$C_1$-$C_6$ alkyl, amino, -NH($C_1$-$C_6$ alkyl), -N($C_1$-$C_6$ alkyl)$_2$, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, - $C_1$-$C_6$ alkylhydroxy, -$C_1$-$C_6$ alkyl-$C_1$-$C_6$ alkoxy, halogen, halogenated $C_1$-$C_6$ alkyl, halogenated $C_1$-$C_6$ alkoxy, or -C(O)N$R_e$$R_f$, and wherein $R_e$ and $R_f$ are each independently selected from H or $C_1$-$C_6$ alkyl, or $R_e$ and $R_f$, together with the nitrogen atom to which they are connected, form a 5- or 6-membered nitrogen-containing heterocyclyl, such as tetrahydropyrrolyl or piperidinyl, the 5- or 6-membered nitrogen-containing heterocyclyl being optionally substituted with one or more substituents independently selected from $C_1$-$C_6$ alkyl, $C_1$-$C_6$ alkoxy, hydroxy, oxo, amino, -NH($C_1$-$C_6$ alkyl), or -N($C_1$-$C_6$ alkyl)$_2$.

In some certain preferred embodiments of the present invention, in the structures of formula (II-1A-1), (II-1A-2), (II-1A-3), (II-1A-4), (II-1A-5),
and (II-1A-6), wherein $R_{Z-1}$, $R_2$, W, and $R_{A-1}$ are as defined in any embodiment of formula (A) or (II-a) of the present application.

In one particularly preferred embodiment, the present invention provides a compound of formula (A), or a mixture of a tautomer,
enantiomer, diastereoisomer or isomer thereof, or a pharmaceutically acceptable salt or solvate thereof selected from:

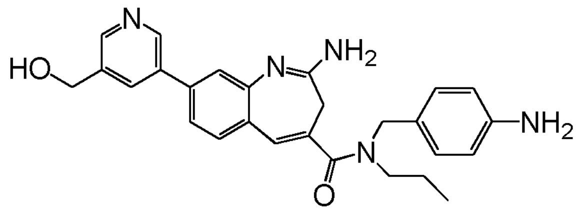

(Ex-10),

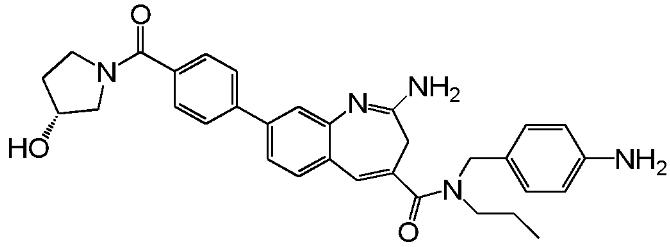

(Ex-11),

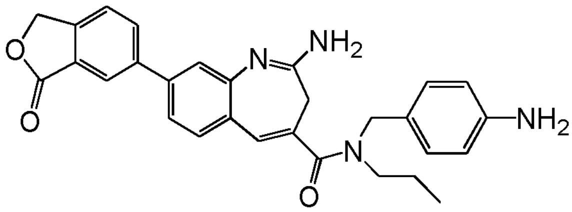

(Ex-12),

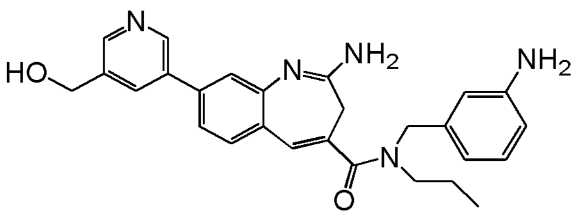

(Ex-13),

and

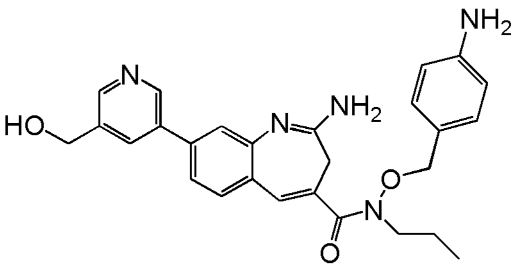

(Ex-15).

In one aspect, the present application provides a compound or a tautomer, a mesomer, a racemate, an enantiomer or a diastereoisomer thereof, or a mixture thereof, or a pharmaceutically acceptable salt thereof, wherein the compound may comprise the structure of formula (III):

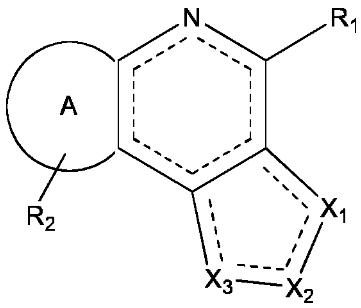

(III),

wherein $R_1$ and $R_2$ may each independently be any group, $X_1$, $X_2$, and $X_3$ may each independently be any atom optionally substituted by any group, A may be an optionally substituted cyclic structure, $\overline{---}$ represents a single bond or double bond,

wherein $X_3$ may not be N, and $R_2$ may be substituted with one or more optionally substituted amino groups.

For example, in a compound comprising the structure of formula (III), $R_1$ may be an optionally substituted amino group.

For example, in a compound comprising the structure of formula (III), $X_1$ may be selected from: -N= and -O-.

For example, in a compound comprising the structure of formula (III), $X_2$ may be selected from the group consisting of: optionally substituted -CH= and optionally substituted -NH-.

For example, in a compound comprising the structure of formula (III), $X_2$ may be substituted with $R_{X2-1}$, wherein $R_{X2-1}$ may be independently selected from any group.

For example, in a compound comprising the structure of formula (III), $R_{X2-1}$ may be selected from hydrogen and optionally substituted $C_1$-$C_6$ alkyl, such as optionally substituted methyl, optionally substituted ethyl, or optionally substituted propyl; when $R_{X2-1}$ may comprise methylene units, the methylene units of $R_{X2-1}$ may each independently be unreplaced, or each independently be replaced by any structure.

For example, in a compound comprising the structure of formula (III), $R_{X2-1}$ may comprise an optionally substituted butyl.

For example, in a compound comprising the structure of formula (III), the methylene unit that $R_{X2-1}$ may comprise may be unreplaced, or may be replaced by -O-.

For example, in a compound comprising the structure of formula (III), $R_{X2-1}$ may comprise optionally substituted $-(CH_2)_3-CH_3$ or optionally substituted $-CH_2-O-CH_2-CH_3$.

For example, in a compound comprising the structure of formula (III), $X_3$ may be selected from the group consisting of: -O-, -S-, and optionally substituted -CH=.

For example, in a compound comprising the structure of formula (III), A may comprise an optionally substituted aromatic ring.

For example, in a compound comprising the structure of formula (III), A may comprise an optionally substituted benzene ring.

For example, in a compound comprising the structure of formula (III), $R_2$ may be selected from hydrogen and optionally substituted $C_1$-$C_{10}$ alkyl; when $R_2$ may comprise methylene units, the methylene units of $R_2$ may each independently be unreplaced, or each independently be replaced by any structure.

For example, in a compound comprising the structure of formula (III), $R_2$ may comprise optionally substituted $C_1$-$C_{10}$ alkyl-$NH_2$; when $R_2$ may comprise methylene units, the methylene units of $R_2$ may each independently be unreplaced, or each independently be replaced by any structure.

For example, in a compound comprising the structure of formula (III), $R_2$ may comprise optionally substituted propyl, optionally substituted butyl, optionally substituted pentyl, or optionally substituted hexyl.

For example, in a compound comprising the structure of formula (III), the methylene unit that $R_2$ may comprise may be unreplaced, or may be replaced by a group that may be selected from the group consisting of: -O-, optionally substituted -NH-, optionally substituted alcylene, and optionally substituted arylene.

For example, in a compound comprising the structure of formula (III), $R_2$ may be selected from the group consisting of: optionally substituted

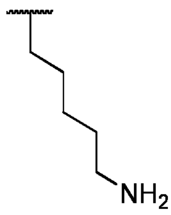,

optionally substituted

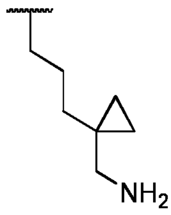,

optionally substituted

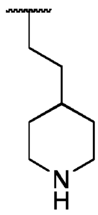,

optionally substituted

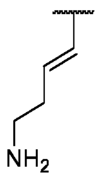,

optionally substituted

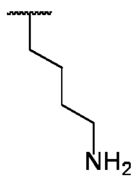,

optionally substituted

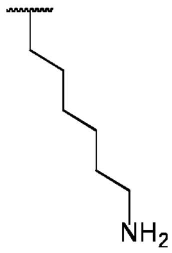,

optionally substituted

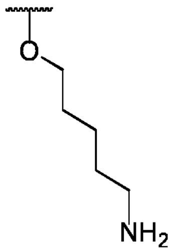,

optionally substituted

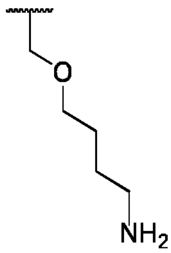,

optionally substituted

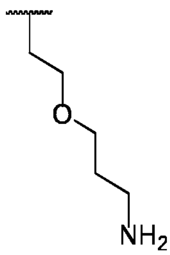,

optionally substituted

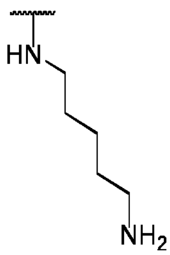,

optionally substituted

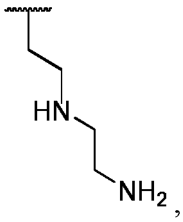

optionally substituted

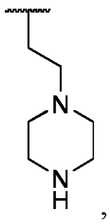

optionally substituted

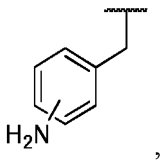

and optionally substituted

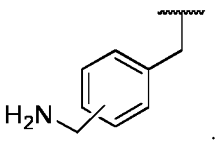

For example, in a compound comprising the structure of formula (III), $R_2$ may be selected from the group consisting of: optionally substituted

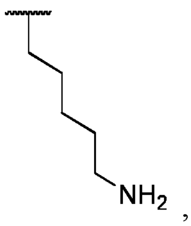

optionally substituted

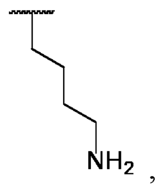

optionally substituted

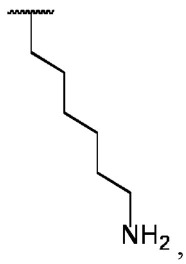

and optionally substituted

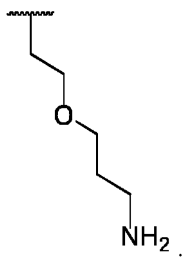

For example, in a compound comprising the structure of formula (III), $R_2$ may be selected from the group consisting of: optionally substituted

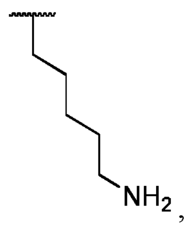

optionally substituted

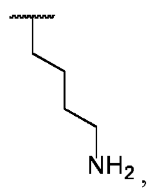

and optionally substituted

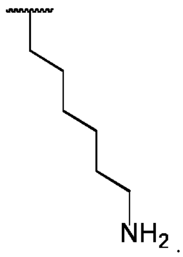

For example, in a compound comprising the structure of formula (III), $R_2$ may be substituted with one or more $R_{2-1}$, wherein the one or more $R_{2-1}$ may each independently be selected from any group. For example, $R_2$ may be substituted with 1, 2, 3, 4, or 5 $R_{2-1}$.

For example, in a compound comprising the structure of formula (III), the one or more $R_{2-1}$ may each independently be selected from the group consisting of: hydrogen and optionally substituted $C_1$-$C_6$ alkyl, such as optionally substituted methyl, optionally substituted ethyl, or optionally substituted propyl; when $R_{2-1}$ may comprise methylene units, the methylene units of $R_{2-1}$ may each independently be unreplaced, or each independently be replaced by any structure. For example, the methylene unit of the 1, 2, 3, 4, or 5 $R_{2-1}$ may each independently be replaced by any structure.

For example, in a compound comprising the structure of formula (III), the one or more $R_{2-1}$ may each independently be selected from the group consisting of: hydrogen, optionally substituted ethyl, and optionally substituted acetyl.

For example, in a compound comprising the structure of formula (III), A may also be substituted with one or more $R_{A-1}$, wherein the one or more $R_{A-1}$ may each independently be selected from any group. For example, A may be substituted with 1, 2, 3, 4, or 5 $R_{A-1}$.

For example, in a compound comprising the structure of formula (III), $R_{A-1}$ may be selected from the group consisting of: hydrogen, halogen, optionally substituted amino, optionally substituted hydroxy, optionally substituted alkyl, optionally substituted aryl, and optionally substituted heteroaryl; when $R_{A-1}$ may comprise methylene units, the methylene units of $R_{2-1}$ may each independently be unreplaced, or each independently be replaced by any structure.

For example, in a compound comprising the structure of formula (III), $R_{A-1}$ may be selected from the group consisting of: optionally substituted $NH_2C(=O)$-, optionally substituted $HC(=O)NH$-, optionally substituted

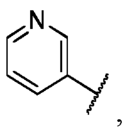

optionally substituted

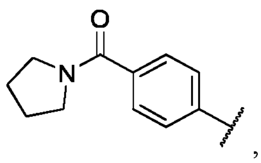

optionally substituted

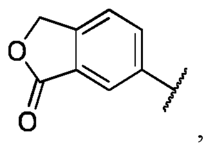

optionally substituted

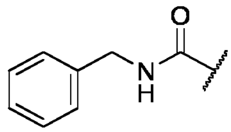

,

optionally substituted

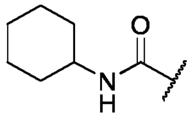

,

optionally substituted

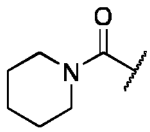

,

optionally substituted

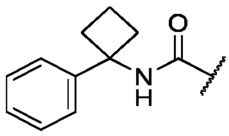

,

optionally substituted

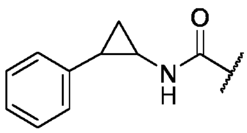

,

optionally substituted

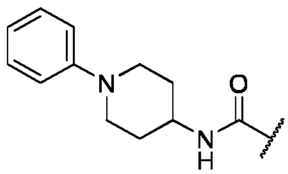

,

and optionally substituted

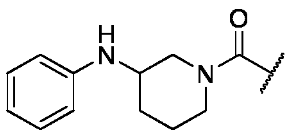

.

For example, in a compound comprising the structure of formula (III), $R_{A-1}$ comprises hydrogen.

For example, in a compound comprising the structure of formula (III), the one or more $R_{A-1}$ may each independently be substituted with one or more $R_{A-2}$, wherein the one or more $R_{A-2}$ may each independently be selected from

any group. For example, $R_{A-1}$ may be substituted with 1, 2, 3, 4, or 5 $R_{A-2}$.

For example, in a compound comprising the structure of formula (III), $R_{A-2}$ may be absent or may be selected from optionally substituted $C_1$-$C_6$ alkyl, such as optionally substituted methyl, optionally substituted ethyl, or optionally substituted propyl; when $R_{A-2}$ may comprise methylene units, the methylene units of $R_{A-2}$ may each independently be unreplaced, or each independently be replaced by any structure.

For example, in a compound comprising the structure of formula (III), $R_{A-2}$ may comprise hydrogen, halogen, optionally substituted methyl, and/or optionally substituted hydroxy.

For example, in a compound comprising the structure of formula (III), the one or more $R_{A-2}$ may each independently be substituted with one or more $R_{A-3}$, wherein the one or more $R_{A-2}$ may each independently be selected from any group. For example, $X_1$ may be substituted with 1, 2, 3, 4, or 5 $R_{A-3}$.

For example, in a compound comprising the structure of formula (III), $R_{A-3}$ may comprise optionally substituted methyl and/or optionally substituted hydroxy.

In one aspect, the present application provides a compound or a tautomer, a mesomer, a racemate, an enantiomer or a diastereoisomer thereof, or a mixture thereof, or a pharmaceutically acceptable salt thereof, wherein the compound may comprise the structure of formula (III):

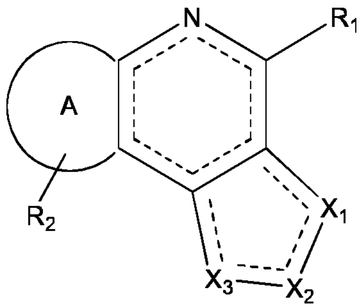

(III),

$R_1$ may be optionally substituted amino; $X_1$ may be selected from the group consisting of: -N= and - O-; $X_2$ may be selected from the group consisting of: optionally substituted -CH= and optionally substituted -NH-; $X_2$ may be substituted by $R_{X2-1}$, wherein $R_{X2-1}$ may comprise optionally substituted $-(CH_2)_3-CH_3$ or optionally substituted $-CH_2-O-CH_2-CH_3$; $X_3$ may be selected from the group consisting of: -O-, -S-, and optionally substituted -CH=;

A may comprise an optionally substituted benzene ring; $R_2$ may be selected from the group consisting of: optionally substituted

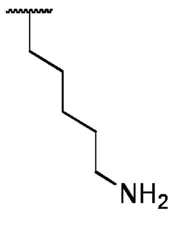

,

optionally substituted

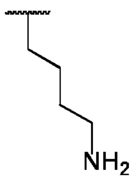

,

optionally substituted

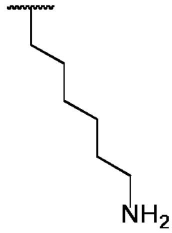

,

and optionally substituted

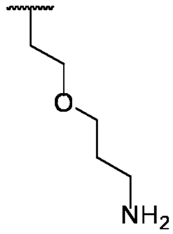

;

A may also be substituted with one or more $R_{A-1}$, wherein $R_{A-1}$ may comprise: optionally substituted $NH_2C(=O)$-, optionally substituted HC(=O)NH-, optionally substituted

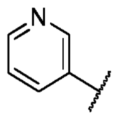

,

optionally substituted

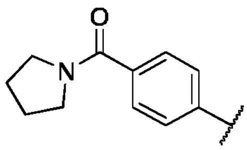

,

optionally substituted

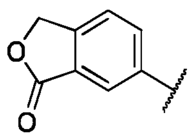

,

optionally substituted

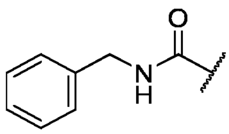

,

optionally substituted

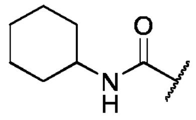,

optionally substituted

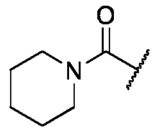,

optionally substituted

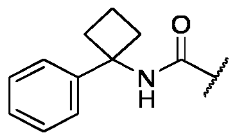,

optionally substituted

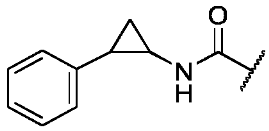,

optionally substituted

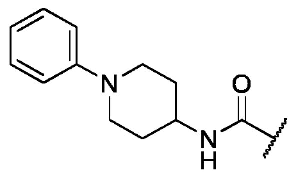,

and/or optionally substituted

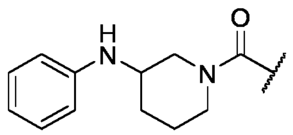;

the one or more $R_{A-1}$ are each independently substituted with one or more $R_{A-2}$, wherein $R_{A-2}$ may comprise hydrogen, halogen, optionally substituted methyl, and/or optionally substituted hydroxy; the one or more $R_{A-2}$ are each independently substituted with one or more $R_{A-3}$, wherein $R_{A-3}$ may comprise optionally substituted methyl and/or optionally substituted hydroxy;

$R_2$ may be substituted with one or more $R_{2-1}$, wherein the one or more $R_{2-1}$ may each independently be selected from the group consisting of: hydrogen, optionally substituted ethyl, and optionally substituted acetyl.

The present application provides a compound or a tautomer, a mesomer, a racemate, an enantiomer or a diastereoisomer thereof, or a mixture thereof, or a pharmaceutically acceptable salt thereof, wherein the compound comprises a structure that may be selected from the group consisting of:

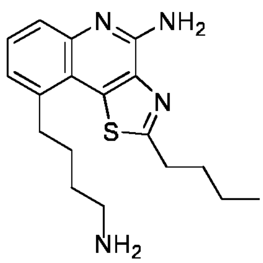
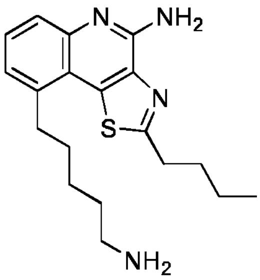
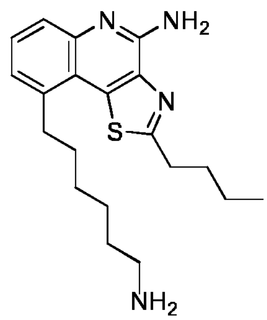
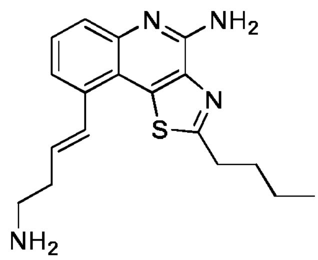

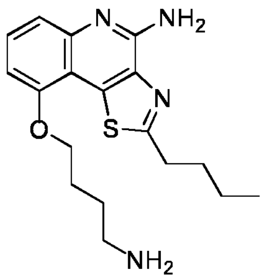
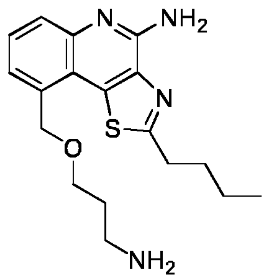
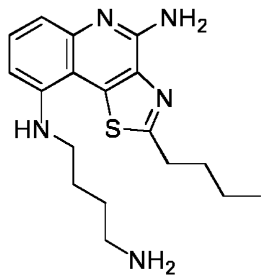
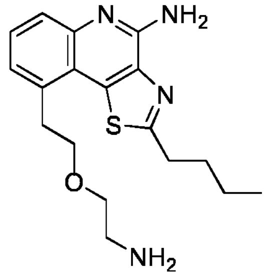

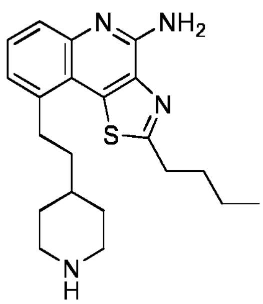
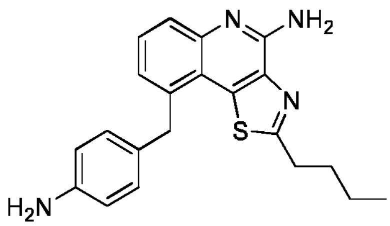
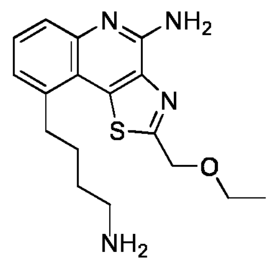
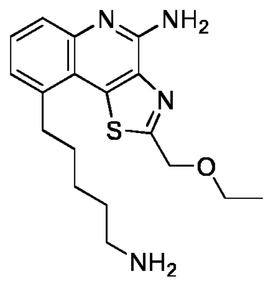

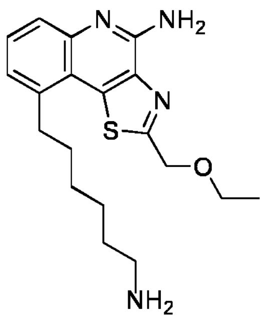
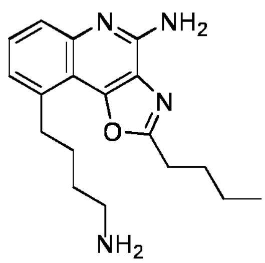
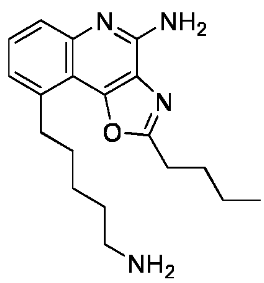
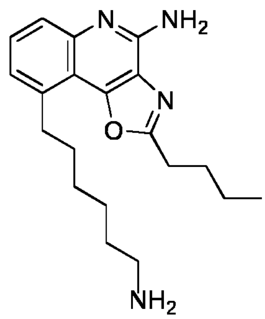

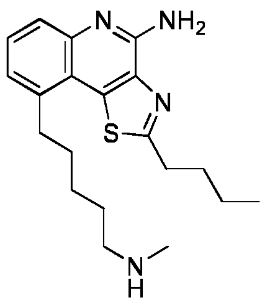
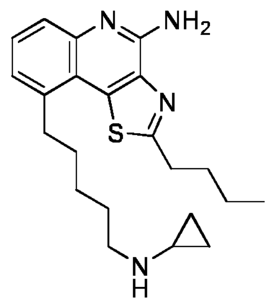
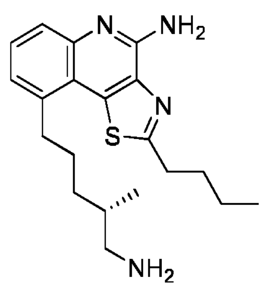
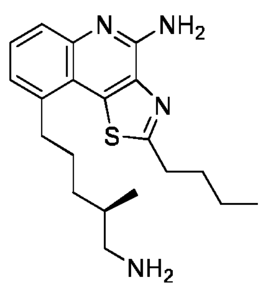

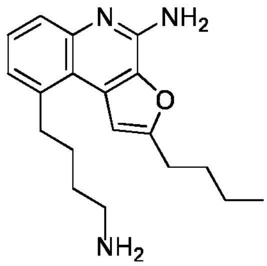 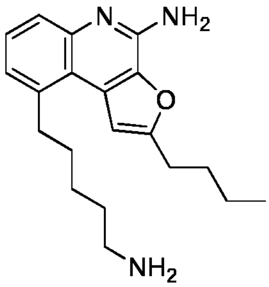 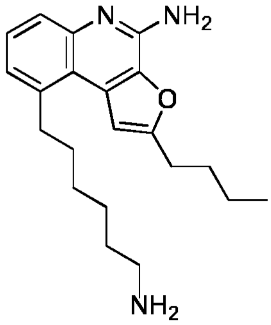

In one aspect, the present application provides a conjugate that may comprise the compound or the tautomer, the mesomer, the racemate, the enantiomer or the diastereoisomer thereof, or the mixture thereof, or the pharmaceutically acceptable salt thereof described herein. For example, the conjugate of the present application includes antibody-drug conjugates.

In one aspect, the present application provides a pharmaceutical composition that may comprise the compound or the tautomer, the mesomer, the racemate, the enantiomer or the diastereoisomer thereof, or the mixture thereof, or the pharmaceutically acceptable salt thereof described herein, and/or the conjugate described herein, and optionally a pharmaceutically acceptable carrier.

In one aspect, the present application provides a kit that may comprise the compound or the tautomer, the mesomer, the racemate, the enantiomer or the diastereoisomer thereof, or the mixture thereof, or the pharmaceutically acceptable salt thereof described herein, the conjugate described herein, and/or the pharmaceutical composition described herein.

In one aspect, the present application provides a method for influencing Toll-like receptor (TLR) functionality, comprising administering the compound or the tautomer, the mesomer, the racemate, the enantiomer or the diastereoisomer thereof, or the mixture thereof, or the pharmaceutically acceptable salt thereof described herein, the conjugate described herein, the pharmaceutical composition described herein, and/or the kit described herein.

For example, TLR may include TLR7 and/or TLR8.

In one aspect, the present application provides a method for modulating immune system functionality, comprising administering the compound or the tautomer, the mesomer, the racemate, the enantiomer or the diastereoisomer thereof, or the mixture thereof, or the pharmaceutically acceptable salt thereof described herein, the conjugate described herein, the pharmaceutical composition described herein, and/or the kit described herein.

In one aspect, the present application provides a method for preventing and/or treating a disease and/or a symptom, comprising administering the compound or the tautomer, the mesomer, the racemate, the enantiomer or the diastereoisomer thereof, or the mixture thereof, or the pharmaceutically acceptable salt thereof described herein, the conjugate described herein, the pharmaceutical composition described herein, and/or the kit described herein. For example, the disease is sensitive to TLR7 and/or TLR8-induced inflammatory responses.

In one aspect, the present application provides use of the compound or the tautomer, the mesomer, the racemate, the enantiomer or the diastereoisomer thereof, or the mixture thereof, or the pharmaceutically acceptable salt thereof described herein, the conjugate described herein, the pharmaceutical composition described herein, and/or the kit described herein in preparing a medicament for preventing and/or treating a disease and/or a symptom. For example, the disease is sensitive to TLR7 and/or TLR8-induced inflammatory responses.

In one aspect, the present application provides the compound or the tautomer, the mesomer, the racemate, the enantiomer or the diastereoisomer thereof, or the mixture thereof, or the pharmaceutically acceptable salt thereof described herein, the conjugate described herein, the pharmaceutical composition described herein, and/or the kit described herein for use in preventing and/or treating a disease and/or a symptom. For example, the disease is sensitive to TLR7 and/or TLR8-induced inflammatory responses.

For example, the disease and/or the symptom may include a disease and/or a symptom associated with Toll-like receptor (TLR) signaling.

For example, the disease and/or the symptom may be selected from the group consisting of: tumor, autoimmune disease, inflammation, septicemia, allergy, asthma, transplant rejection, graft-versus-host disease, immunodeficiency, and viral infection.

For example, the disease and/or the symptom is selected from the group consisting of: melanoma, lung tumor, liver tumor, basal cell tumor, kidney tumor, myeloma, biliary tract tumor, brain tumor, breast tumor, cervical tumor, chorionic tumor, colonic tumor, rectal tumor, head and neck tumor, peritoneal tumor, fallopian tube tumor, endometrial tumor, esophageal tumor, gastric tumor, leukemia, lymphoma, sarcoma, neuroblastoma, oral tumor, ovarian tumor, pancreatic tumor, prostate tumor, testicular tumor, skin tumor, and thyroid tumor.

For example, the disease and/or symptom is a viral infection selected from the group consisting of: dengue virus, yellow fever virus, West Nile virus, Japanese encephalitis virus, tick-borne encephalitis virus, Kunjin virus, Murray Valley encephalitis virus, St. Louis encephalitis virus, Omsk hemorrhagic fever virus, bovine viral diarrhea virus, Zika virus, HIV (human immunodeficiency virus), HBV (hepatitis B virus), HCV (hepatitis C virus), HPV (human papillomavirus), RSV (respiratory syncytial virus), SARS-CoV (severe acute respiratory syndrome coronavirus), SARS-CoV-2 (severe acute respiratory syndrome coronavirus 2), MERS-CoV (middle east respiratory syndrome coronavirus), and influenza virus.

The compound described herein may influence the activity of TLR. The influence on the activity may be a 1% or greater, 2% or greater, 4% or greater, 5% or greater, 8% or greater, 10% or greater, 15% or greater, 18% or greater, 20% or greater, 25% or greater, 40% or greater, 50% or greater, 60% or greater, 70% or greater, 80% or greater, 90% or greater, or 95% or greater increase in TLR activity when the compound of the present application is added to a culture medium, as compared with the addition of a negative control or a reference drug. For example, the influence on the activity may be an $EC_{50}$ value (nM) of 10000 or less, 5000 or less, 4000 or less, 3000 or less, 2000 or less, 1000 or less, 500 or less, 400 or less, 300 or less, 200 or less, 150 or less, 120 or less, 110 or less, 100 or less, 99 or less, 98 or less, 97 or less, 95 or less, 90 or less, 80 or less, 75 or less, 70 or less, 65 or less, 62 or less, 60 or less, 50 or less, 40 or less, 30 or less, 25 or less, 23 or less, 22 or less, 20 or less, 19 or less, 18 or less, 18.5 or less, 17 or less, 15 or less, 12 or less, 10 or less, 9 or less, 8.5 or less, 7 or less, 6.7 or less, 6 or less, 5.9 or less, 5.5 or less, 5.0 or less, 4.8 or less, 4.5 or less, 4.4 or less, 4 or less, 3.5 or less, 3 or less, 2.5 or less, 2 or less, 1.5 or less, 1.0 or less, 0.5 or less, 0.3 or less, 0.29 or less, 0.25 or less, 0.21 or less, 0.20 or less, 0.18 or less, 0.17 or less, 0.15 or less, 0.12 or less, 0.10 or less, 0.09 or less, 0.08 or less, 0.07 or less, 0.06 or less, 0.05 or less, 0.04 or less, 0.03 or less, 0.02 or less, or 0.01 or less for TLR. For example, the immune cell may include, but is not limited to, granulocytes and/or agranulocytes. For example, the immune cell includes, but is not limited to, B cells, T cells, natural killer cells, monocytes, macrophages, mast cells, and/or dendritic cells. For example, the immune cell includes PBMCs. For example, TLR includes human TLR. For example, TLR includes TLR7 and/or TLR8.

The compounds described herein may influence the ability of immune cells to express and/or release cytokines. The influence on the activity may be a 1% or greater, 2% or greater, 4% or greater, 5% or greater, 8% or greater, 10% or greater, 15% or greater, 18% or greater, 20% or greater, 25% or greater, 40% or greater, 50% or greater, 60% or greater, 70% or greater, 80% or greater, 90% or greater, or 95% or greater increase in the ability of immune cells to express and/or release cytokines when the compound of the present application is added to a culture medium containing immune cells or administered to a subject, as compared with the addition or administration of a negative control or a reference drug. For example, the immune cell may include, but is not limited to, granulocytes and/or agranulocytes. For example, the immune cell includes, but is not limited to, B cells, T cells, natural killer cells, monocytes, macrophages, mast cells, and/or dendritic cells. For example, the immune cell includes PBMCs. For example, the cytokine may be an immune cell cytokine. For example, the cytokine may be TNF-$\alpha$ and/or IFN-$\alpha$

The compounds described herein may influence the activity of immune cells. The influence on the activity may be a 1% or greater, 2% or greater, 4% or greater, 5% or greater, 8% or greater, 10% or greater, 15% or greater, 18% or greater, 20% or greater, 25% or greater, 40% or greater, 50% or greater, 60% or greater, 70% or greater, 80% or greater, 90% or greater, or 95% or greater increase in immune cell activity when the compound of the present application is added to a culture medium containing immune cells, as compared with the addition of a negative control or a reference drug. For example, the influence on the activity may be an $EC_{50}$ value (nM) of 10000 or less, 5000 or less, 4000 or less, 3000 or less, 2000 or less, 1000 or less, 500 or less, 400 or less, 300 or less, 200 or less, 150 or less, 120 or less, 110 or less, 100 or less, 99 or less, 98 or less, 97 or less, 95 or less, 90 or less, 80 or less, 75 or less, 70 or less, 65 or less, 62 or less, 60 or less, 50 or less, 40 or less, 30 or less, 25 or less, 23 or less, 22 or less, 20 or less, 19 or less, 18 or less, 18.5 or less, 17 or less, 15 or less, 12 or less, 10 or less, 9 or less, 8.5 or less, 7 or less, 6.7 or less, 6 or less, 5.9 or less, 5.5 or less, 5.0 or less, 4.8 or less, 4.5 or less, 4.4 or less, 4 or less, 3.5 or less, 3 or less, 2.5 or less, 2 or less, 1.5 or less, 1.0 or less, 0.5 or less, 0.3 or less, 0.29 or less, 0.25 or less, 0.21 or less, 0.20 or less, 0.18 or less, 0.17 or less, 0.15 or less, 0.12 or less, 0.10 or less, 0.09 or less, 0.08 or less, 0.07 or less, 0.06 or less, 0.05 or less, 0.04 or less, 0.03 or less, 0.02 or less, or 0.01 or less for immune cells. For example, the immune cell may include, but is not limited to, granulocytes and/or agranulocytes. For example, the immune cell includes, but is not limited to, B cells, T cells, natural killer cells, monocytes, macrophages, mast cells, and/or dendritic cells. For example, the immune cell includes PBMCs.

Pharmaceutical Composition

The pharmaceutical composition described herein may comprise, in addition to the active compound, one or more adjuvants, which may be selected from the group consisting of the following ingredients: a filler (diluent), a binder, a wetting agent, a disintegrant, an excipient, and the like. Depending on the method of administration, the composition may comprise 0.1 wt.% to 99% wt.% of the active compound.

The pharmaceutical composition comprising the active ingredient may be in a form suitable for oral administration, such as tablet, troche, lozenge, aqueous or oil suspension, dispersible powder or granule, emulsion, hard or soft capsule, or syrup. Oral compositions may be prepared according to any method for preparing pharmaceutical compositions known in the art, and the compositions may comprise a binder, a filler, a lubricant, a disintegrant, a pharmaceutically acceptable wetting agent, and the like, and may further comprise one or more ingredients that are selected from the group consisting of: a sweetening agent, a flavoring agent, a coloring agent, and a preservative.

Aqueous suspensions may comprise the active substance in admixture with an excipient suitable for the formulation of aqueous suspensions. Aqueous suspensions may also comprise one or more preservatives, for example, one or more coloring agents, one or more flavoring agents, and one or more sweetening agents. Oil suspensions may be formulated by suspending the active ingredient in a vegetable oil. Oil suspensions may comprise a thickening agent. The sweetening agents and the flavoring agents described above may also be added.

The pharmaceutical compositions may also be prepared from a dispersible powder or granule that provides the active ingredient for preparing the aqueous suspension by adding water to mix the active ingredient with one or more of a dispersing agent, a wetting agent, a suspending agent, or a preservative. Other excipients, such as sweetening agents, flavoring agents and coloring agents, may also be added. These compositions are well preserved by the addition of antioxidants such as ascorbic acid. The pharmaceutical composition of the present application may also be in the form of an oil-in-water emulsion.

The pharmaceutical composition may be in the form of a sterile aqueous solution for injection. Available and acceptable vehicles or solvents include water, Ringer's solution, and isotonic sodium chloride solution. The sterile formulation for injection may be a sterile oil-in-water microemulsion for injection in which the active ingredient is dissolved in the oil phase. For example, the active ingredient is dissolved in a mixture of soybean oil and lecithin. The oil solution may then be added to a mixture of water and glycerol and treated to form a microemulsion. The injection or microemulsion can be locally injected into the blood of a patient in large quantities. Alternatively, it may be desirable to administer the solution and microemulsion in such a way that the compound of the present application is kept at a constant circulating concentration. To keep such a constant concentration, a continuous intravenous delivery device may be used. For example, the device may be a Deltec CADD-PLUS. TM. 5400 intravenous injection pump.

The pharmaceutical composition may be in the form of a sterile aqueous or oily suspension for injection for intramuscular and subcutaneous administration. The suspension may be prepared according to existing techniques using suitable dispersing agents or wetting agents and suspending agents described above. The sterile formulation for injection may also be a sterile injection or suspension prepared in a parenterally acceptable non-toxic diluent or solvent. Alternatively, a sterile fixing oil may be conveniently used as a solvent or a suspending medium.

The compound of the present application may be administered in the form of a suppository for rectal administration. Such a pharmaceutical composition can be prepared by mixing a drug with a suitable non-irritative excipient, which is a solid at ambient temperature but a liquid in the rectum and therefore will melt in the rectum to release the drug. Such materials include cocoa butter, glycerinated gelatin, hydrogenated vegetable oils, and mixtures of polyethylene glycols of various molecular weights and fatty acid esters of polyethylene glycol.

As is well known to those skilled in the art, the dose of the drug administered depends on a variety of factors, including but not limited to, the activity of the particular compound employed, the age of the patient, the weight of the patient, the health condition of the patient, the behavior of the patient, the diet of the patient, the time of administration, the mode of administration, the rate of excretion, the combination of drugs, and the like. In addition, the optimal treatment regimen, such as the mode of treatment, the compound or the tautomer, the mesomer, the racemate, the enantiomer or the diastereoisomer thereof, or the mixture thereof, or the pharmaceutically acceptable salt thereof described herein, and/or the daily amount of the compound or the tautomer, the mesomer, the racemate, the enantiomer or the diastereoisomer thereof, or the mixture thereof, or the pharmaceutically acceptable salt thereof, or the identity of the pharmaceutically acceptable salt, can be verified according to conventional treatment regimens.

Prophylactic and/or Therapeutic Methods

The present application provides use of the compound or the tautomer, the mesomer, the racemate, the enantiomer or the diastereoisomer thereof, or the mixture thereof, or the pharmaceutically acceptable salt thereof described herein, and/or the pharmaceutical composition described herein in preparing a medicament for preventing and/or treating a disease and/or a symptom.

The present application provides the compound or the tautomer, the mesomer, the racemate, the enantiomer or the diastereoisomer thereof, or the mixture thereof, or the pharmaceutically acceptable salt thereof described herein, and/or the pharmaceutical composition described herein for use in preventing and/or treating a disease and/or a symptom.

The present application provides a method for preventing and/or treating a disease and/or a symptom, which may comprise administering to a subject the compound or the tautomer, the mesomer, the racemate, the enantiomer or the diastereoisomer thereof, or the mixture thereof, or the pharmaceutically acceptable salt thereof described herein,

and/or the pharmaceutical composition described herein.

Synthesis Embodiments

In order to complete the synthesis purpose of the present application, the compound provided by the present application can be prepared by the following embodiments.

Reagents that provide bromination conditions include, but are not limited to, aqueous bromine, N-bromosuccinimide, dibromohydantoin, phosphorus tribromide, liquid bromine, liquid bromine/triphenylphosphine, hydrobromic acid, and carbon tetrabromide;
Titanium catalysts include, but are not limited to, tetraisopropyl titanate, triisopropoxytitanium chloride, titanium tetrachloride, and triisopropoxytitanium methyl;
Palladium catalysts include, but are not limited to, tetrakis(triphenylphosphine)palladium, palladium acetate, palladium chloride, bis(triphenylphosphine)palladium(II) dichloride, tris(dibenzylideneacetone)dipalladium, bis(dibenzylideneacetone)dipalladium, bis(acetonitrile)palladium(II) dichloride, [1,1'-bis(diphenylphosphino)ferrocene]palladium(II) dichloride, [1,1'-bis(diphenylphosphino)ferrocene]palladium(II) dichloride dichloromethane complex, bis(benzonitrile)palladium chloride, 1,4-butylenebis(diphenylphosphine)-palladium dichloride, allylpalladium chloride dimer, and allyl(cyclopentadienyl)palladium(II);
Borate dimers include, but are not limited to, bis(pinacolato)diboron, bis(neopentyl glycolato)diboron, bis(hexylene glycolato)diboron, bis(catecholato)diboron, bis(diisopropyl-L-tartrate glycolato)diboron, bis[(-)pinanediol]diborate, bis[(1*S*,2*S*,3*R*,5*S*)-pinanediolato]diboron, tetramethyidiborane, bis(*N,N,N',N'*-tetramethyl-D-tartramidate)diboron, tetrahydroxydiboron, bis(*N,N,N',N'*-tetramethyl-L-tartramidate)diboron, bis(diisopropyl-D-tartrate glycolato)diboron, bis(diethyl-D-tartrate glycolato)diboron, bis(2,4-dimethyl-2,4-pentanediol)borate, bis(diethyl-L-tartrate glycolato)diboron, and 4,4,5,5-tetramethyl-1,3,2-dioxaborolan-2-ylboronic acid;
Metallic copper salts include, but are not limited to, copper sulfate, copper sulfate pentahydrate, cuprous sulfate, cupric chloride, cuprous chloride, cupric carbonate, cupric phosphate, cupric acetate and hydrates thereof, cupric oxalate, cupric fluoroborate and hydrates thereof, cupric methoxide, cupric tartrate, copper formate, cuprous iodide, copper(II) trifluoroacetate, copper(II) trifluoromethanesulfonate, copper carbonate, cupric bromide, cuprous bromide, and cuprous oxide; Ligands may be selected from any ligand commonly used in the Ullmann reaction, including but not limited to, L-proline, tyrosine, phenylalanine, 1,10-phenanthroline, *N,N'*-dimethylethylenediamine, ethylene glycol, 1,1'-binaphthyl-2,2'-diol, ethyl 2-cyclohexanonecarboxylate, and salicylaldehyde hydrazone;
Condensing agents may be selected from 4-(4,6-dimethoxy-1,3,5-triazin-2-yl)-4-methylmorpholinium chloride, 1-hydroxybenzotriazole and 1-(3-dimethylaminopropyl)-3-ethylcarbodiimide hydrochloride, *N,N'*-dicyclohexylcarbodiimide, *N,N'*-diisopropylcarbodiimide, O-benzotriazol-*N,N,N',N'*-tetramethyluronium tetrafluoroborate, 1-hydroxybenzotriazole, 1-hydroxy-7-azobenzotriazol, O-benzotriazol-*N,N,N',N'*-tetramethyluronium hexafluorophosphate, 2-(7-azobenzotriazol)-*N,N,N',N'*-tetramethyluronium hexafluorophosphate, benzotriazol-1-yloxytris(dimethylamino)phosphonium hexafluorophosphate or benzotriazol-1-yl-oxytripyrrolidinophosphonium hexafluorophosphate, preferably 4-(4,6-dimethoxy-1,3,5-triazin-2-yl)-4-methylmorpholinium chloride, or 1-hydroxybenzotriazole and 1-(3-dimethylaminopropyl)-3-ethylcarbodiimide hydrochloride;
Reagents that provide basic conditions include organic bases and inorganic bases, wherein the organic bases include, but are not limited to, triethylamine, diethylamine, *N*-methylmorpholine, pyridine, piperidine, *N,N*-diisopropylethylamine, n-butyllithium, lithium diisopropylamide, potassium acetate, sodium *tert*-butoxide, potassium *tert*-butoxide, and the like, and the inorganic bases include, but are not limited to, sodium hydride, potassium carbonate, sodium carbonate, cesium carbonate, sodium hydroxide, lithium hydroxide, sodium phosphate, and potassium phosphate;
Reagents that provide acidic conditions include protic acids and Lewis acids, wherein the protic acids include, but are not limited to, hydrochloric acid, sulfuric acid, nitric acid, nitrous acid, sulfurous acid, phosphoric acid, phosphorous acid, formic acid, acetic acid, propionic acid, butyric acid, citric acid, benzoic acid, p-toluenesulfonic acid, p-nitrobenzoic acid, methanesulfonic acid, trifluoromethanesulfonic acid and trifluoroacetic acid, and the Lewis acids include, but are not limited to, boron trifluoride, zinc chloride, magnesium chloride, aluminum chloride, stannic chloride and ferric chloride;
Hydrogenation conditions include, but are not limited to: Pb/C/hydrogen, Pt/C/hydrogen, palladium chloride/hydrogen, Raney nickel/hydrogen, palladium hydroxide carbon/hydrogen, and palladium hydroxide/hydrogen;
Reagents that provide oxidation conditions include, but are not limited to, Dess-Martin periodinane, hydrogen peroxide, sodium chlorite, sodium hypochlorite, and potassium perchlorate;
Reagents that provide reduction conditions include, but are not limited to, sodium hydride, calcium hydride, lithium hydride, lithium aluminum hydride, sodium borohydride, lithium borohydride, sodium triethylborohydride, sodium triacetoxyborohydride, and sodium cyanoborohydride; Reagents that provide oxidation conditions include, but are

not limited to, Dess-Martin periodinane, hydrogen peroxide, sodium chlorite, sodium hypochlorite, and potassium perchlorate;

Reagents that provide nitration conditions include, but are not limited to, dilute nitric acid, concentrated nitric acid, concentrated sulfuric acid/nitric acid, and nitric/acetic anhydride; Reagents that provide hydroboration include, but are not limited to, borane-tetrahydrofuran, borane-dimethylsulfide, catecholborane, pinacolborane, 9-borabicyclo[3.3.1]nonane, diisoamylborane, dicyclohexylborane, 1,1,2-trimethylpropylborane, monochloroborane, dichloroborane, monobromoborane, and dibromoborane; Reagents that provide oxidation conditions include, but are not limited to, Dess-Martin periodinane, hydrogen peroxide, sodium chlorite, sodium hypochlorite, and potassium perchlorate;

Basic buffers are selected from the following buffers with pH 7 to 11: citric acid-sodium citrate buffer, phosphoric acid-sodium phosphate buffer, phosphoric acid-potassium phosphate buffer, sodium dihydrogen phosphate-disodium hydrogen phosphate buffer, potassium dihydrogen phosphate-dipotassium hydrogen phosphate buffer, succinic acid-sodium succinate buffer, acetic acid-sodium acetate buffer, boric acid-borax buffer, boric acid-potassium borate buffer, borax-sodium hydroxide buffer, histidine-hydrochloric acid buffer, glycine-sodium hydroxide buffer, arginine-hydrochloric acid buffer, sodium bicarbonate-sodium carbonate buffer, potassium bicarbonate-potassium carbonate buffer, Tris-hydrochloric acid buffer, aqueous ammonia-ammonium chloride buffer, barbiturate sodium-hydrochloric acid buffer, borax-sodium carbonate buffer, boric acid-potassium chloride buffer, and a combination of two or more of the above.

In addition, the structure of the compounds in the present application is determined by nuclear magnetic resonance (NMR) or mass spectrometry (MS). NMR is performed using a Quan tum-INMR spectrometer with deuterated dimethyl sulfoxide (DMSO-D), deuterated chloroform (CDC13), and deuterated methanol (CD3OD) as the solvents and tetramethylsilane (TMS) as the internal standard, and chemical shifts are given in units of 10_6 (ppm).

MS is performed using an Agilent 6230 ESI-TOF mass spectrometer (manufacturer: Agilent, type c: 6230).

UPLC is performed using a Waters AcquityUPLCSQD liquid chromatograph-mass spectrometer (Poroshell 120 EC-C18, 2.1 mm × 50 mm, 1.9 μm column).

HPLC is performed using an Agilent 1260 high-performance liquid chromatograph (TOSOH G3000 SW SEC column).

UV detections are performed using a Thermo Nanodrop 2000 spectrophotometer.

Enzyme-linked immunoassays are performed using an EnVision microplate reader (PerkinElmer). HSGF254 or GF254 silica gel plate is adopted as a thin-layer chromatography (TLC) silica gel plate. The specification adopted by the TLC is 0.15-0.2 mm, and the specification adopted by the thin-layer chromatography for the separation and purification of products is 0.4-0.5 mm.

Yantai Yellow Sea silica gel of 200-300 mesh is generally utilized as a carrier in column chromatography.

Known starting materials of the present application can be synthesized using or according to methods known in the art, or can be purchased from companies such as ABCR GmbH & Co. KG, Acros Organics, Aldrich Chemical Company, Accela ChemBio Inc., and Darui Chemicals.

In the examples, all reactions are conducted in an argon atmosphere or a nitrogen atmosphere unless otherwise stated. The argon atmosphere or nitrogen atmosphere means that the reaction flask is connected to a balloon containing about 1 L of argon or nitrogen. The hydrogen atmosphere means that the reaction flask is connected to a balloon containing about 1 L of hydrogen.

In the examples, the solution in the reaction is an aqueous solution unless otherwise stated.

In the examples, the reaction temperature is room temperature unless otherwise stated. The room temperature is the optimum reaction temperature, which ranges from 20 °C to 30 °C.

The system of eluents for column chromatography and the system of developing agents for thin-layer chromatography used for purifying compounds include: A: dichloromethane and isopropanol system, B: dichloromethane and methanol system, and C: petroleum ether and ethyl acetate system. The volume ratio of solvents is regulated according to the different polarities of the compounds, and can also be regulated by adding a small amount of triethylamine and acidic or alkaline reagent.

Some of the compounds of the present disclosure are characterized by TOF-LC/MS. TOF-LC/MS analysis is performed using an Agilent 6230 time-of-flight mass spectrometer and an Agilent 1290-Infinity ultra-high performance liquid chromatograph.

An exemplary preparation route for the present application is as follows:

Embodiment 1

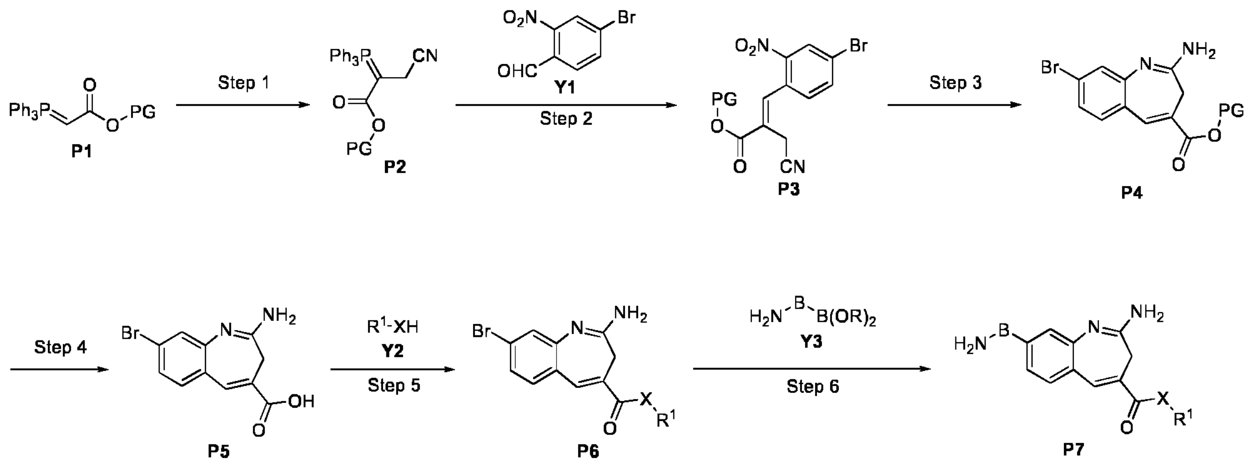


Step I: reacting a compound of general formula (P1) with bromoacetonitrile in a heating condition to give a compound of general formula (P2);

Step II: reacting the compound of general formula (P2) with a compound of general formula (Y1) at room temperature to give a compound of general formula (P3);

Step III: reacting the compound of general formula (P3) in a reducing condition to give a compound of general formula (P4);

Step IV: removing protecting group PG from the compound of general formula (P4) to give a compound of general formula (P5);

Step V: reacting the compound of general formula (P5) with a compound of general formula (Y2), optionally in the presence of a condensing agent and optionally in a basic condition, to give a compound of general formula (P6); and

Step VI: reacting the compound of general formula (P6) with a compound of general formula (Y3), optionally with the catalysis of a palladium-based reagent, to give a compound of general formula (P7);

wherein:

PG may be a common carboxy protecting group;

X is selected from: -O-, -S-, and -NR-;

ring B is an optionally substituted aryl or heteroaryl;

$-B(OR)_2$ is a boronate monomer, wherein the two R may be connected to form a heterocycle, bridged heterocycle or spiro heterocycle that may be optionally substituted with $C_1$-$C_6$ alkyl, aryl, heteroaryl, carboxy, or acyloxy $C_1$-$C_6$ alkyl;

wherein each R is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;

$R^1$ is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy.

Embodiment 2

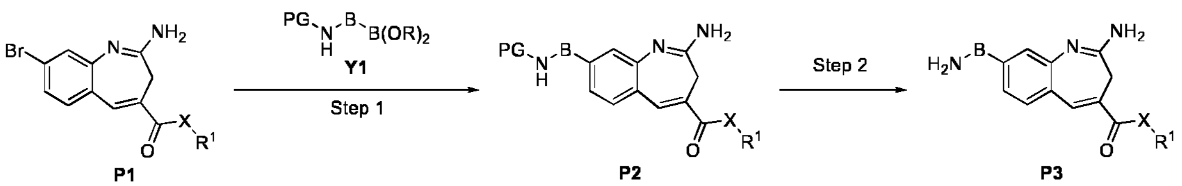


Step I: reacting a compound of general formula (P1) with a compound of general formula (Y1), optionally with the catalysis of a palladium-based reagent, to give a compound of general formula (P2); and

Step II: removing protecting group PG from the compound of general formula (P2) to give a compound of general formula (P3);

wherein:

PG may be a common amino protecting group;
X is selected from: -O-, -S-, and -NR-;
ring B is an optionally substituted aryl or heteroaryl;
$-B(OR)_2$ is a boronate monomer, wherein the two R may be connected to form a heterocycle, bridged heterocycle or spiro heterocycle that may be optionally substituted with $C_1$-$C_6$ alkyl, aryl, heteroaryl, carboxy, or acyloxy $C_1$-$C_6$ alkyl;
wherein each R is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;
$R^1$ is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy.

Embodiment 3

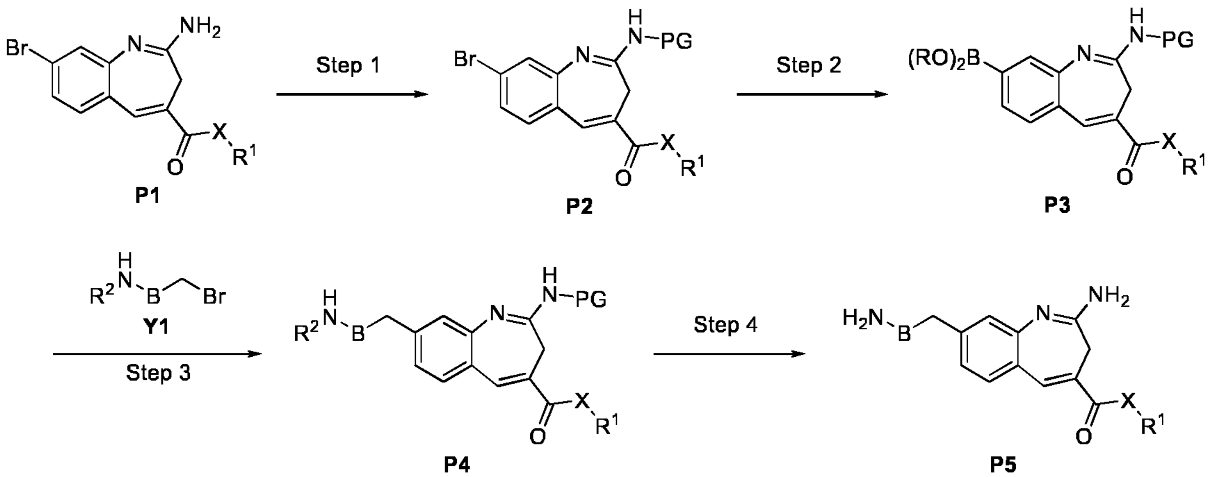


P1 P2 P3 Y1 P4 P5

Step I: adding protecting group PG to a compound of general formula (P1), optionally in an acidic or basic condition, to give a compound of general formula (P2);
Step II: reacting the compound of general formula (P2) with a borate dimer, optionally with the catalysis of a palladium-based reagent, to give a compound of general formula (P3);
Step III: reacting the compound of general formula (P3) with a compound of general formula (Y1), optionally with the catalysis of a palladium-based reagent, to give a compound of general formula (P4); and
Step IV: removing protecting group PG from the compound of general formula (P4) to give a compound of general formula (P5);
wherein:

PG may be a common amino protecting group;
$R^2$ may be hydrogen or a common amino protecting group;
X is selected from: -O-, -S-, and -NR-;
ring B is an optionally substituted aryl or heteroaryl;
$-B(OR)_2$ is a boronate monomer, wherein the two R may be connected to form a heterocycle, bridged heterocycle or spiro heterocycle that may be optionally substituted with $C_1$-$C_6$ alkyl, aryl, heteroaryl, carboxy, or acyloxy $C_1$-$C_6$ alkyl;
wherein each R is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;
$R^1$ is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$

alkoxy.

Embodiment 4

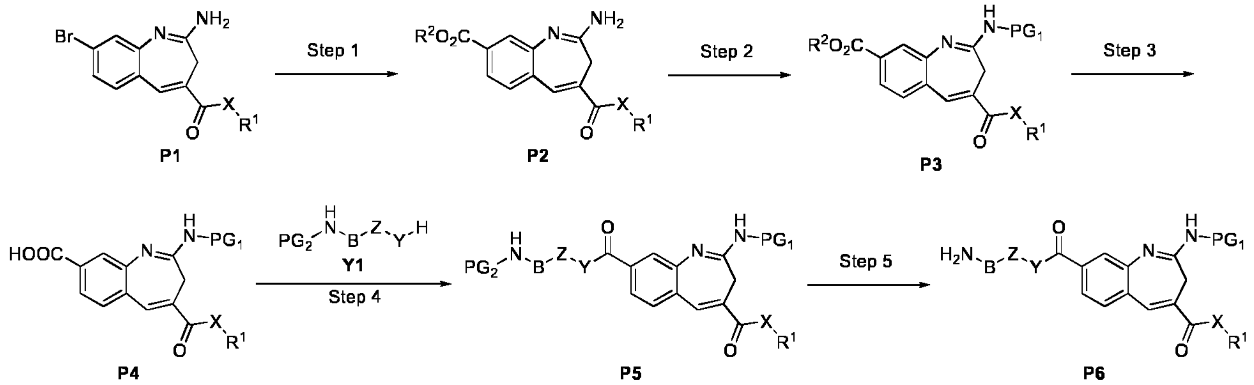


Step I: subjecting a compound of general formula (P1) to a carbonyl insertion reaction, optionally with the catalysis of a palladium-based reagent, to give a compound of general formula (P2);
Step II: adding protecting group $PG_1$ to the compound of general formula (P2), optionally in an acidic or basic condition, to give a compound of general formula (P3);
Step III: hydrolyzing the ester group of the compound of general formula (P3), optionally in a basic condition, to give a compound of general formula (P4);
Step IV: reacting the compound of general formula (P4) with a compound of general formula (Y1), optionally in the presence of a condensing agent and optionally in a basic condition, to give a compound of general formula (P5); and
Step IV: removing protecting groups $PG_1$ and $PG_2$ from the compound of general formula (P5) to give a compound of general formula (P6);
wherein:

$PG_1$ and $PG_2$ may be common amino protecting groups;
X is selected from: -O-, -S-, and -NR-;
Y is selected from: -O-, -S-, and -NR-;
R is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;
$R^1$ is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;
$R^2$ is optionally selected from $C_1$-$C_6$ alkyl;
ring B is independently selected from optionally substituted aryl or heteroaryl;
Z is absent or selected from $C_1$-$C_6$ alkylene, arylene, heteroarylene, cycloalkylene, heterocycloalkylene, and halogenated $C_1$-$C_6$ alkylene.

Embodiment 5

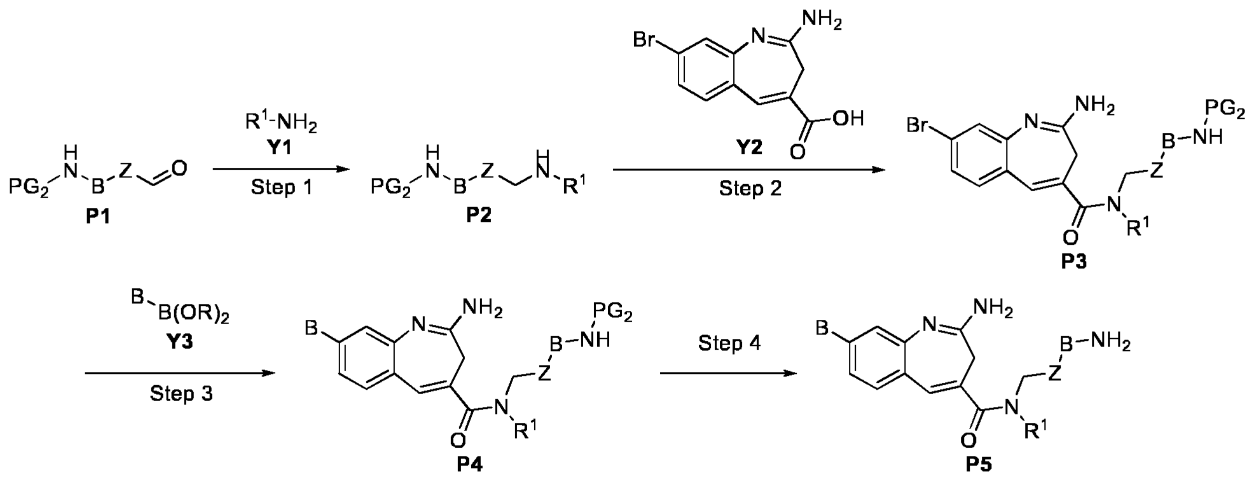


Step I: subjecting a compound of general formula (P1) and a compound of general formula (Y1) to the reductive amination reaction, optionally in a reducing condition, to give a compound of general formula (P2);
Step II: reacting the compound of general formula (P2) with a compound of general formula (Y2), optionally in the presence of a condensing agent and optionally in a basic condition, to give a compound of general formula (P3);
Step III: reacting the compound of general formula (P3) with a compound of general formula (Y3), optionally with the catalysis of a palladium-based reagent, to give a compound of general formula (P4); and
Step IV: removing protecting group PG from the compound of general formula (P4) to give a compound of general formula (P5);
wherein:

PG may be a common amino protecting group;
ring B is an optionally substituted aryl or heteroaryl;
$-B(OR)_2$ is a boronate monomer, wherein the two R may be connected to form a heterocycle, bridged heterocycle or spiro heterocycle that may be optionally substituted with $C_1$-$C_6$ alkyl, aryl, heteroaryl, carboxy, or acyloxy $C_1$-$C_6$ alkyl;
wherein each R is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;
$R^1$ is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;
Z is absent or selected from $C_1$-$C_6$ alkylene, arylene, heteroarylene, cycloalkylene, heterocycloalkylene, and halogenated $C_1$-$C_6$ alkylene.

Embodiment 6

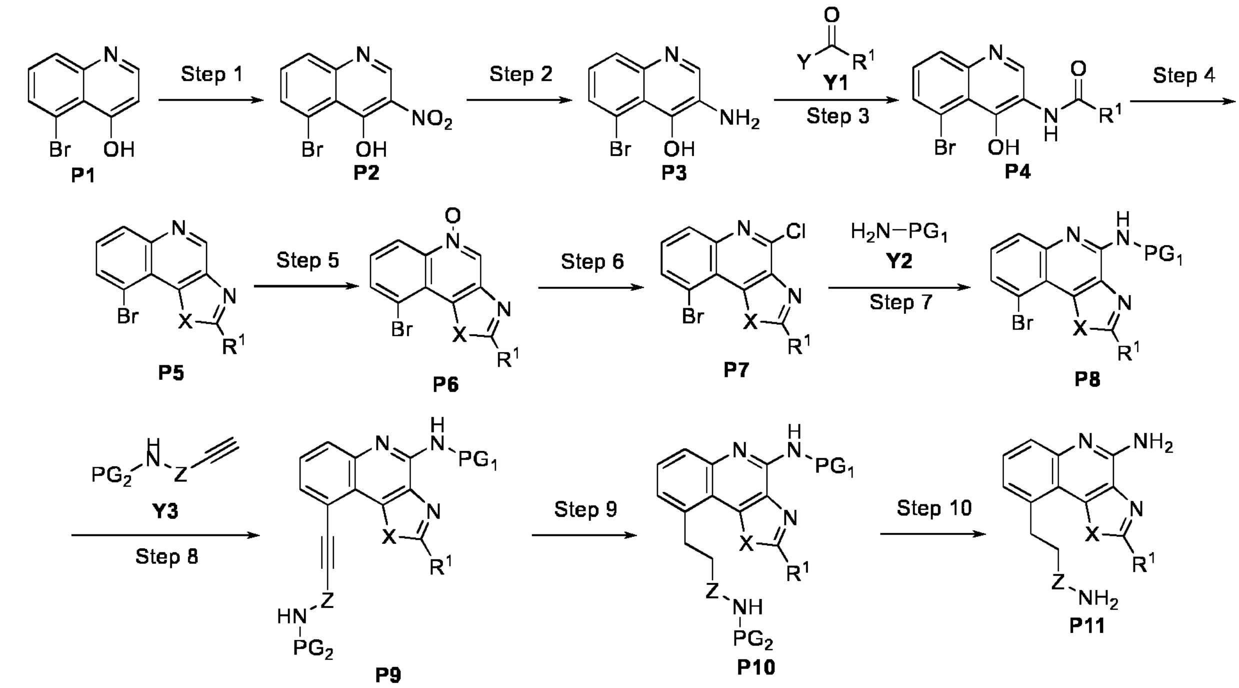


Step I: subjecting a compound of general formula (P1) to a nitration condition to give a compound of general formula (P2);

Step II: subjecting the compound of general formula (P2) to a reducing condition to give a compound of general formula (P3);

Step III: reacting the compound of general formula (P3) with a compound of general formula (Y1), optionally in the presence of a condensing agent and optionally in a basic condition, to give a compound of general formula (P4);

Step IV: subjecting the compound of general formula (P4) to cyclization reaction in a heating condition to give a compound of general formula (P5);

Step V: subjecting the compound of general formula (P5) to an oxidation condition to give a compound of general formula (P6);

Step VI: reacting the compound of general formula (P6) in the presence of phosphorus oxychloride to give a compound of general formula (P7);

Step VII: reacting the compound of general formula (P7) with a compound of general formula (Y2) in a heating condition to give a compound of general formula (P8);

Step VIII: reacting the compound of general formula (P8) with a compound of general formula (Y3), optionally with the catalysis of a palladium-based reagent, to give a compound of general formula (P9);

Step IX: reacting the compound of general formula (P9) in a reducing condition to give a compound of general formula (P10); and

Step X: removing protecting groups $PG_1$ and $PG_2$ from the compound of general formula (P10) to give a compound of general formula (P11);

wherein:

$PG_1$ and $PG_2$ may be common amino protecting groups;

X is selected from: -O- and -S-;

Z is absent or selected from $C_1$-$C_6$ alkylene, arylene, heteroarylene, cycloalkylene, heterocycloalkylene, and halogenated $C_1$-$C_6$ alkylene;

$R^1$ is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy; wherein, when $R^1$ comprises methylene units, the methylene units of $R^1$ are each independently unreplaced, or the methylene units of $R^1$ are each independently replaced by a group selected from the group consisting of: -O-, -S-, and optionally substituted -NH-.

Embodiment 7

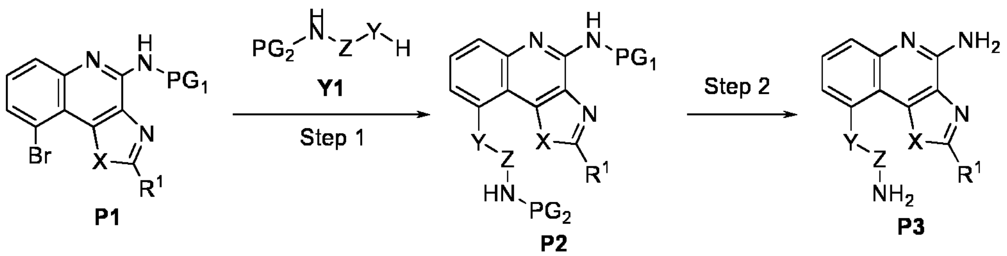


Step I: reacting a compound of general formula (P1) with a compound of general formula (Y1), optionally with the catalysis of a palladium-based reagent, to give a compound of general formula (P2); and

Step II: removing protecting groups $PG_1$ and $PG_2$ from the compound of general formula (P2) to give a compound of general formula (P3); wherein:

- $PG_1$ and $PG_2$ may be common amino protecting groups;
- X is selected from: -O- and -S-;
- Y is selected from: -O- and -NH-;
- Z is absent or selected from $C_1$-$C_6$ alkylene, arylene, heteroarylene, cycloalkylene, heterocycloalkylene, and halogenated $C_1$-$C_6$ alkylene;
- $R^1$ is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy; wherein, when $R^1$ comprises methylene units, the methylene units of $R^1$ are each independently unreplaced, or the methylene units of $R^1$ are each independently replaced by a group selected from the group consisting of: -O-, -S-, and optionally substituted -NH-.

Embodiment 8

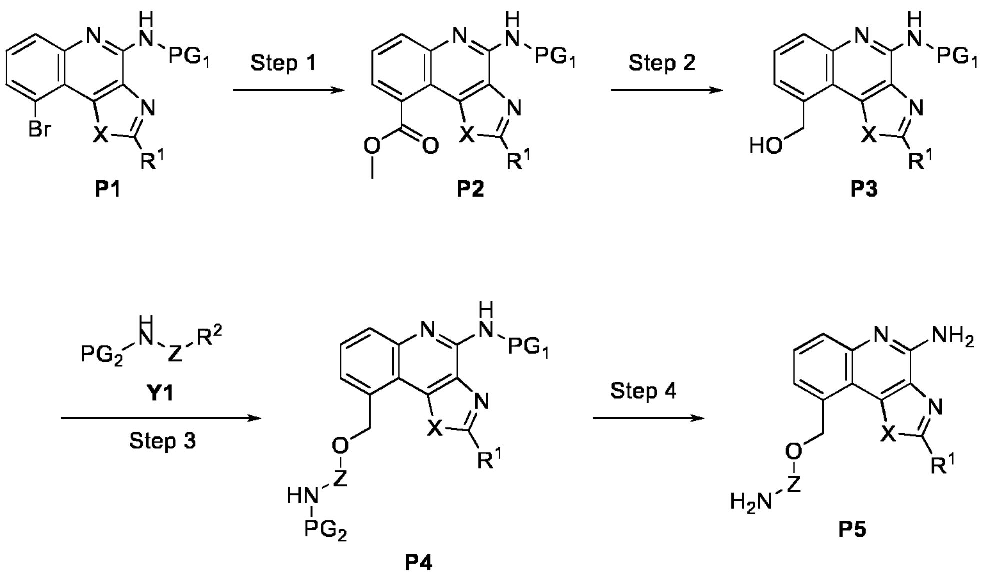


Step I: subjecting a compound of general formula (P1) to a carbonyl insertion reaction, optionally with the catalysis of a palladium-based reagent, to give a compound of general formula (P2);

Step II: subjecting the compound of general formula (P2) to a reducing condition to give a compound of general formula (P3);

Step III: subjecting the compound of general formula (P3) and a compound of general formula (Y1) to substitution reaction in a basic condition to give a compound of general formula (P4); and

Step IV: removing protecting groups $PG_1$ and $PG_2$ from the compound of general formula (P4) to give a compound of general formula (P5);

wherein:

PG$_1$ and PG$_2$ may be common amino protecting groups;
X is selected from: -O- and -S-;
$R^2$ is selected from: halogen, methanesulfonyloxy, and p-toluenesulfonyloxy;
Z is absent or selected from $C_1$-$C_6$ alkylene, arylene, heteroarylene, cycloalkylene, heterocycloalkylene, and halogenated $C_1$-$C_6$ alkylene;
$R^1$ is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy; wherein, when $R^1$ comprises methylene units, the methylene units of $R^1$ are each independently unreplaced, or the methylene units of $R^1$ are each independently replaced by a group selected from the group consisting of: -O-, -S-, and optionally substituted -NH-.

Embodiment 9

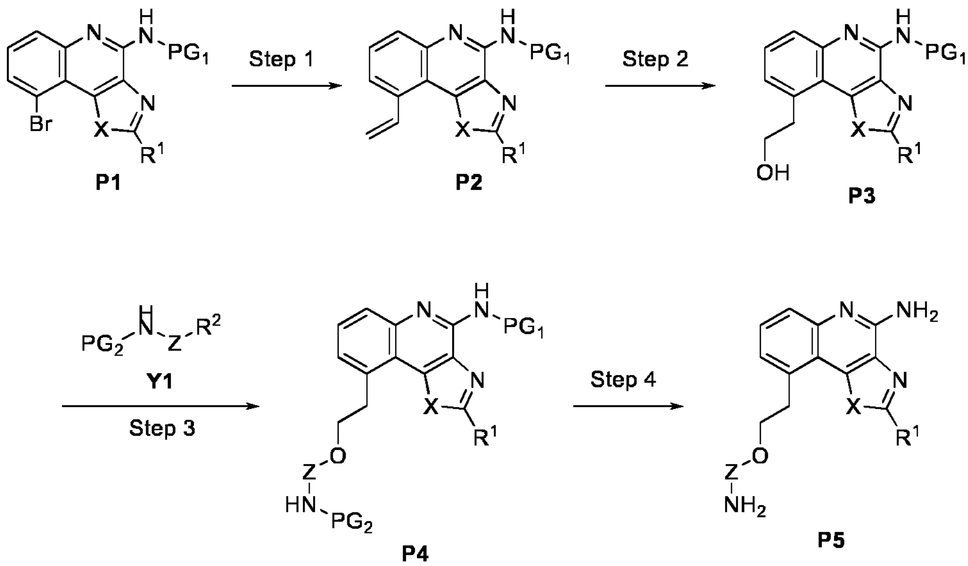


Step I: subjecting a compound of general formula (P1) to a Heck reaction, optionally with the catalysis of a palladium-based reagent, to give a compound of general formula (P2);
Step II: subjecting the compound of general formula (P2) to hydroboration and oxidation conditions to give a compound of general formula (P3);
Step III: subjecting the compound of general formula (P3) and a compound of general formula (Y1) to substitution reaction in a basic condition to give a compound of general formula (P4); and
Step IV: removing protecting groups PG$_1$ and PG$_2$ from the compound of general formula (P4) to give a compound of general formula (P5);
wherein:

PG$_1$ and PG$_2$ may be common amino protecting groups;
X is selected from: -O- and -S-;
$R^2$ is selected from: halogen, methanesulfonyloxy, and p-toluenesulfonyloxy;
Z is absent or selected from $C_1$-$C_6$ alkylene, arylene, heteroarylene, cycloalkylene, heterocycloalkylene, and halogenated $C_1$-$C_6$ alkylene;
$R^1$ is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy; wherein, when $R^1$ comprises methylene units, the methylene units of $R^1$ are each independently unreplaced, or the methylene units of $R^1$ are each independently replaced by a group selected from the group consisting of: -O-, -S-, and optionally substituted -NH-.

Embodiment 10

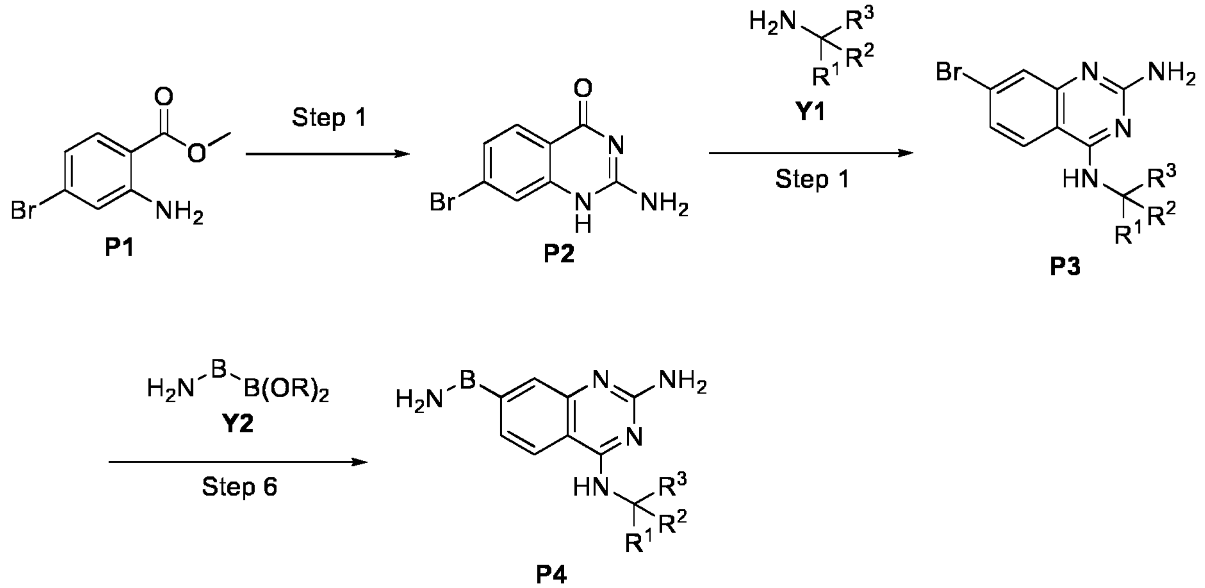


P1 P2 P3 P4

Step I: reacting a compound of general formula (P1) with aminoacetonitrile in a heating condition to give a compound of general formula (P2);
Step II: reacting the compound of general formula (P2) with a compound of general formula (Y 1), optionally in the presence of a condensing agent and optionally in a basic condition, to give a compound of general formula (P3); and
Step VI: reacting the compound of general formula (P3) with a compound of general formula (Y2), optionally with the catalysis of a palladium-based reagent, to give a compound of general formula (P4);
wherein:

ring B is an optionally substituted aryl or heteroaryl;
-B(OR)$_2$ is a boronate monomer, wherein the two R may be connected to form a heterocycle, bridged heterocycle or spiro heterocycle that may be optionally substituted with $C_1$-$C_6$ alkyl, aryl, heteroaryl, carboxy, or acyloxy $C_1$-$C_6$ alkyl;
wherein each R is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;
$R^1$, $R^2$, and $R^3$ are independently selected from hydrogen, protium, deuterium, tritium, optionally substituted $C_1$-$C_6$ alkyl, optionally substituted $C_1$-$C_6$ alkoxy, optionally substituted aryl, optionally substituted heteroaryl, optionally substituted cycloalkyl, and optionally substituted heterocycloalkyl.

Embodiment 11

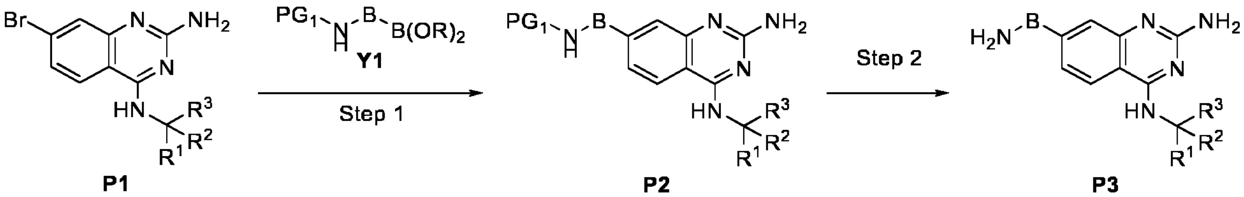


P1 P2 P3

Step I: reacting a compound of general formula (P1) with a compound of general formula (Y1), optionally with the catalysis of a palladium-based reagent, to give a compound of general formula (P2); and
Step II: removing protecting groups $PG_1$ and $PG_2$ from the compound of general formula (P2) to give a compound

of general formula (P3);
wherein:

- $PG_1$ may be a common amino protecting group;
- ring B is an optionally substituted aryl or heteroaryl;
- $-B(OR)_2$ is a boronate monomer, wherein the two R may be connected to form a heterocycle, bridged heterocycle or spiro heterocycle that may be optionally substituted with $C_1$-$C_6$ alkyl, aryl, heteroaryl, carboxy, or acyloxy $C_1$-$C_6$ alkyl;
- wherein each R is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;
- $R^1$, $R^2$, and $R^3$ are independently selected from hydrogen, protium, deuterium, tritium, optionally substituted $C_1$-$C_6$ alkyl, optionally substituted $C_1$-$C_6$ alkoxy, optionally substituted aryl, optionally substituted heteroaryl, optionally substituted cycloalkyl, and optionally substituted heterocycloalkyl.

Embodiment 12

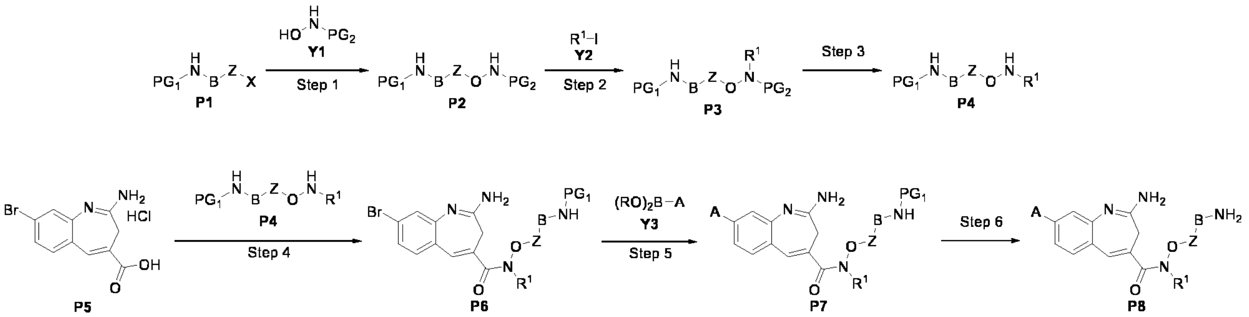


Step I: reacting a compound of general formula (P1) with a compound of general formula (Y1), optionally in a basic condition, to give a compound of general formula (P2);
Step II: reacting the compound of general formula (P2) with a compound of general formula (Y2), optionally in a basic condition, to give a compound of general formula (P3);
Step III: removing protecting group $PG_2$ from the compound of general formula (P3) to give a compound of general formula (P4);
Step IV: reacting a compound of general formula (P5) with the compound of general formula (P4), optionally in the presence of a condensing agent and optionally in a basic condition, to give a compound of general formula (P6);
Step V: reacting the compound of general formula (P6) with a compound of general formula (Y3), optionally with the catalysis of a palladium-based reagent, to give a compound of general formula (P7); and
Step VI: removing protecting group $PG_1$ from the compound of general formula (P7) to give a compound of general formula (P8);
wherein:

- $PG_1$ and $PG_2$ may be common amino protecting groups;
- X is independently selected from: halogen, methanesulfonyloxy, and p-toluenesulfonyloxy;
- ring A and ring B are independently selected from optionally substituted aryl or heteroaryl;
- $-B(OR)_2$ is a boronate monomer, wherein the two R may be connected to form a heterocycle, bridged heterocycle or spiro heterocycle that may be optionally substituted with $C_1$-$C_6$ alkyl, aryl, heteroaryl, carboxy, or acyloxy $C_1$-$C_6$ alkyl;
- wherein each R is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;
- $R^1$ is optionally substituted $C_1$-$C_6$ alkyl;
- Z is selected from optionally substituted $C_1$-$C_6$ alkylene, optionally substituted cycloalkylene, and optionally substituted heterocycloalkylene.

Embodiment 13

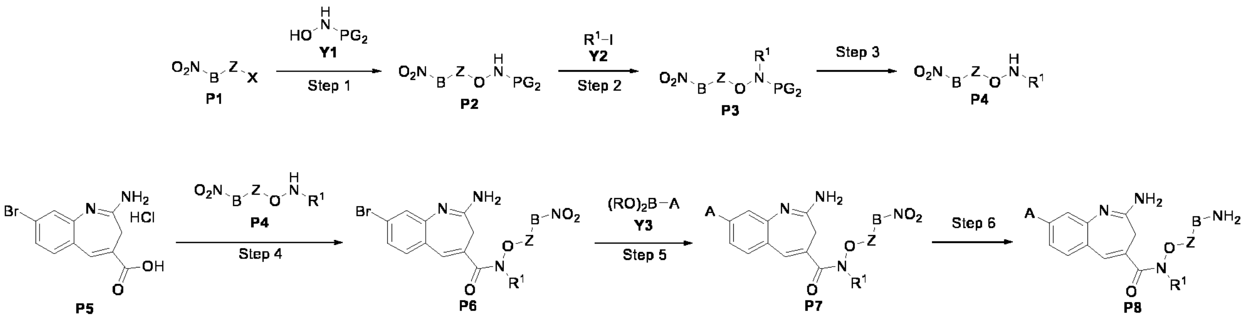


Step I: reacting a compound of general formula (P1) with a compound of general formula (Y1), optionally in a basic condition, to give a compound of general formula (P2);

Step II: reacting the compound of general formula (P2) with a compound of general formula (Y2), optionally in a basic condition, to give a compound of general formula (P3);

Step III: removing protecting group $PG_2$ from the compound of general formula (P3) to give a compound of general formula (P4);

Step IV: reacting a compound of general formula (P5) with the compound of general formula (P4), optionally in the presence of a condensing agent and optionally in a basic condition, to give a compound of general formula (P6);

Step V: reacting the compound of general formula (P6) with a compound of general formula (Y3), optionally with the catalysis of a palladium-based reagent, to give a compound of general formula (P6); and

Step VI: removing protecting group $PG_1$ from the compound of general formula (P4) to give a compound of general formula (P7);

wherein:

$PG_2$ may be a common amino protecting group;

X is independently selected from: halogen, methanesulfonyloxy, and p-toluenesulfonyloxy;

ring A and ring B are independently selected from optionally substituted aryl or heteroaryl; $-B(OR)_2$ is a boronate monomer, wherein the two R may be connected to form a heterocycle, bridged heterocycle or spiro heterocycle that may be optionally substituted with $C_1$-$C_6$ alkyl, aryl, heteroaryl, carboxy, or acyloxy $C_1$-$C_6$ alkyl;

wherein each R is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;

$R^1$ is optionally substituted $C_1$-$C_6$ alkyl;

Z is selected from optionally substituted $C_1$-$C_6$ alkylene, optionally substituted cycloalkylene, and optionally substituted heterocycloalkylene.

Embodiment 14

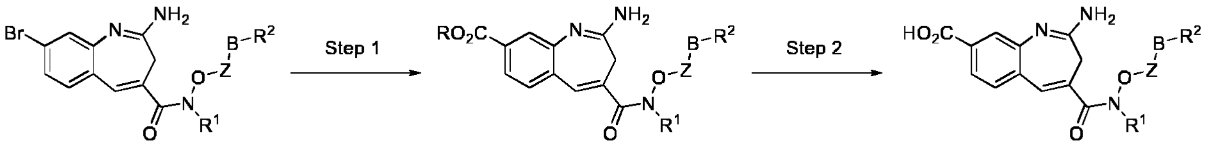


P1 P2 P3

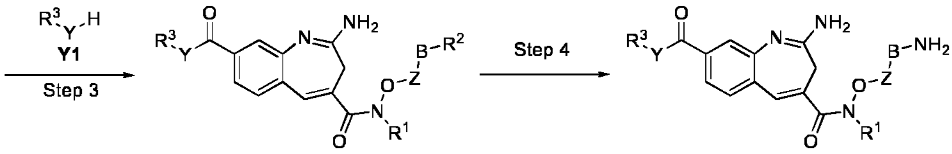


P4 P5

Step I: subjecting a compound of general formula (P1) to a carbonyl insertion reaction, optionally with the catalysis of a palladium-based reagent, to give a compound of general formula (P2);
Step II: hydrolyzing the ester group of the compound of general formula (P2), optionally in a basic condition, to give a compound of general formula (P3);
Step III: reacting the compound of general formula (P3) with a compound of general formula (Y1), optionally in the presence of a condensing agent and optionally in a basic condition, to give a compound of general formula (P4); and
Step IV: reducing the nitro group of the compound of the general formula (P4) in a reducing condition, or removing protecting group PG optionally in a basic or acidic condition, to give a compound of the general formula (P5);
wherein:

- ring B is an optionally substituted aryl or heteroaryl;
- Z is selected from optionally substituted $C_1$-$C_6$ alkylene, optionally substituted cycloalkylene, and optionally substituted heterocycloalkylene;
- $R^1$ is independently selected from hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;
- $R^2$ is optionally selected from nitro or -NH-PG, wherein PG may be a common amino protecting group;
- R is optionally selected from $C_1$-$C_6$ alkyl;
- Y is selected from: -O-, -S-, and -NR-;
- $R^3$ is optionally selected from $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy.

Without being bound by any theory, the following examples are intended only to illustrate the compounds, preparation methods, use, etc., of the present application, and are not intended to limit the scope of the present application.

## Examples

### Example 1. Preparation of compounds

## Compound 1

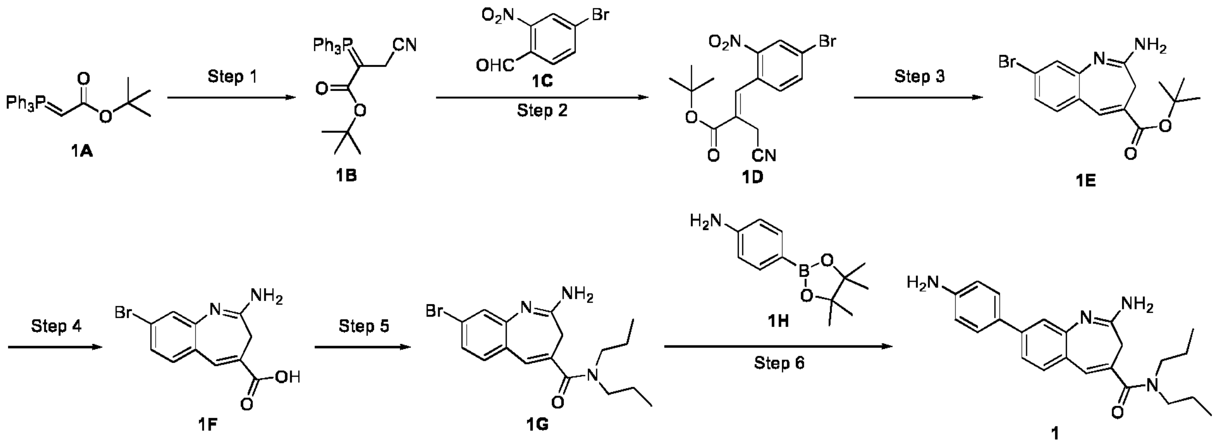


**Step** 1 EA (180 mL) and 1A (30 g, 79.7 mmol, 1 eq) were added to a 500-mL three-necked flask. Bromoacetonitrile (5.73 g, 47.7 mmol, 0.6 eq) was added dropwise at room temperature. After the addition, the temperature was raised to 85 °C for reaction overnight. The reaction solution was cooled and directly filtered. The filter cake was washed with 50 mL of EA, and the filtrate was concentrated by rotary evaporation to give a crude dark red solid product **1B** (9 g), which was directly used in the next step without calculating the yield.

**Step 2 1B** (9 g, 21.74 mmol, 1 eq) from the previous step was dissolved (incompletely) in toluene (90 mL). **1C** (5 g, 21.74 mmol, 1 eq) was added at room temperature. After the addition, the system was stirred at room temperature overnight. Toluene was removed by rotary evaporation. The residue was subjected to column chromatography (PE:EA = 20:1 to 10:1), concentrated by rotary evaporation at reduced pressure, triturated in PE (50 mL), and filtered to give a pale yellow flaky crystal **1D** (5 g, 62.5% yield).

**Step 3** In a 250-mL three-necked flask, **1D** (5 g, 13.6 mmol, 1 eq) was added to glacial acetic acid (50 mL). The internal temperature was raised to about 60-65 °C, then iron powder (3.8 g, 68 mmol, 5 eq) was slowly added in portions. After the addition, the internal temperature was raised to 85 °C for reaction for 2 hours. The reaction solution was cooled to around 60 °C, diluted by adding DCM (50 mL), and filtered through celite before cooling. The filter cake was washed with DCM (100 mL), and the filtrate was concentrated by rotary evaporation at reduced pressure to give a dark red oily substance. The oil was diluted with DCM (50 mL), poured into saturated aqueous sodium bicarbonate (200 mL), and extracted with DCM (100 mL × 4). The organic phases were combined, dried over sodium sulfate, and concentrated at reduced pressure. After rotary evaporation, the mixture was triturated in PE:EA = 2:1 (30 mL) and filtered to give an off-white powder **1E** (4.5 g, 97% yield). MS-ESI: m/z 337.1 $[M+H]^+$.

**Step 4** 4 N HCl/dioxane (40 mL) was added to a 100-mL single neck flask, then **1E** (4.5 g, 13.4 mmol, 1 eq) was added. The system was stirred at room temperature overnight. When LCMS indicated the depletion of the starting materials, the mixture was concentrated by rotary evaporation to give an off-white powder **1F** (4.2 g, 99% yield). MS-ESI: m/z 281.0 $[M+H]^+$.

**Step 5** DMF (60 mL) and **1F** (3 g, 9.45 mmol, 1 eq) were added into a 250-mL three-necked flask, then HBTU (4.3 g, 11.34 mmol, 1.2 eq) and DIEA (4.88 g, 37.79 mmol, 4 eq) were added at 0 °C. After the addition, the system was stirred for 10 min, dipropylamine (1.86 g, 18.42 mmol, 1.95 eq) was added dropwise, and the system was warmed to room temperature after the addition and stirred overnight. The reaction solution was poured into water (240 mL), and extracted with EA (100 mL × 5). The organic phase was washed with saturated brine (50 mL × 2), dried over sodium sulfate, concentrated by rotary evaporation, and purified on a silica gel column to give a dark red solid **1G** (3 g, 87.5% yield). MS-ESI: m/z 364.2 $[M+H]^+$.

**Step 6** In a 25-mL three-necked flask, **1G** (200 mg, 0.55 mmol, 1 eq), **1H** (121 mg, 0.55 mmol, 1 eq) and anhydrous potassium phosphate (350 mg, 1.65 mmol, 3 eq) were dissolved in a mixed solvent of dioxane (5 mL) and water (0.5 mL). The system was purged with argon twice before $Pd(PPh_3)_4$ (50 mg) was added, then purged with argon twice again, heated to 85 °C, and reacted for 3 hours. After cooling, the reaction solution was dried over sodium sulfate to remove water and directly purified by thin-layer chromatography to give a dark yellow powder **1** (25 mg, 12% yield).

MS-ESI: m/z 377.3 $[M+H]^+$.

$^1H$ NMR (400 MHz, DMSO-$d_6$) δ 7.36 (d, *J*= 8.6 Hz, 2H), 7.25 (d, *J*= 8.2 Hz, 1H), 7.15 (d, *J*= 1.9 Hz, 1H), 7.11 (dd, *J* = 8.2, 2.0 Hz, 1H), 6.79 - 6.67 (m, 3H), 6.62 (d, *J* = 8.6 Hz, 2H), 5.22 (s, 2H), 3.30 - 3.25 (m, 4H), 2.70 (s, 2H), 1.63 - 1.48 (m, 4H), 0.82 (brs, 6H).

Compound **2**

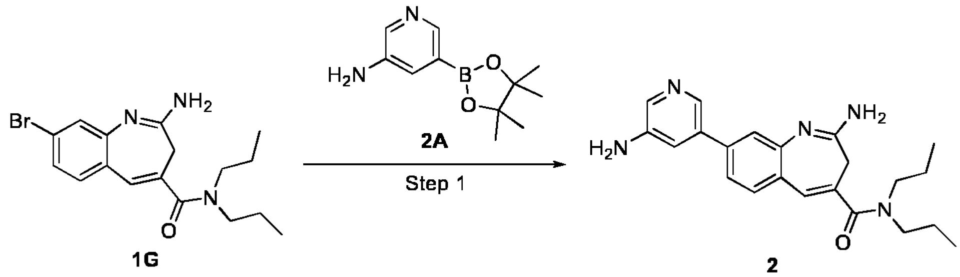


1G 2

**Step 1** In a 25-mL three-necked flask, **1G** (200 mg, 0.55 mmol, 1 eq), **2A** (121 mg, 0.55 mmol, 1 eq) and anhydrous potassium phosphate (350 mg, 1.65 mmol, 3 eq) were dissolved in a mixed solvent of dioxane (5 mL) and water (0.5 mL). The system was purged with argon twice before $Pd(PPh_3)_4$ (50 mg) was added, then purged with argon twice again, heated to 85 °C, and reacted for 3 hours. After cooling, the reaction solution was dried over sodium sulfate to remove water and purified by column chromatography (DCM:MeOH = 100:0 to 10: 1) to give a pale yellow powder **2** (23 mg, 11% yield).

MS-ESI: m/z 378.3 $[M+H]^+$.

$^1H$ NMR (400 MHz, DMSO-$d_6$) δ 8.04 (d, *J* = 2.0 Hz, 1H), 7.93 (d, *J* = 2.5 Hz, 1H), 7.44 (d, *J* = 8.0 Hz, 1H), 7.34 - 7.23 (m, 2H), 7.16 (t, *J* = 2.3 Hz, 1H), 6.82 (s, 1H), 5.43 (s, 2H), 3.31 - 3.26 (m, 4H), 2.89 (s, 2H), 1.61 - 1.51 (m, 4H), 0.83 (brs, 6H).

Compound **3**

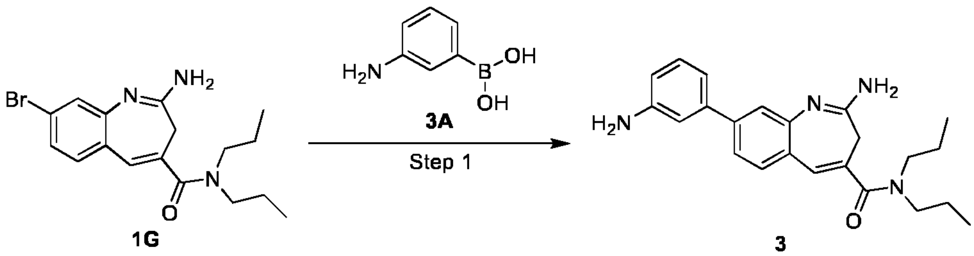


1G 3

**Step 1** In a 25-mL three-necked flask, **1G** (400 mg, 1.1 mmol, 1 eq), **3A** (151 mg, 1.1 mmol, 1 eq) and anhydrous potassium phosphate (700 mg, 3.29 mmol, 3 eq) were dissolved in a mixed solvent of dioxane (10 mL) and water (1 mL). The system was purged with argon twice before $Pd(PPh_3)_4$ (100 mg) was added, then purged with argon twice again, heated to 85 °C, and reacted for 3 hours. After cooling, the reaction solution was dried over 5 g of sodium sulfate, mixed with silica gel, and subjected to column chromatography to give a crude product. The crude product was then purified to give a pale yellow powder **3** (45 mg, 11% yield).

MS-ESI: m/z 377.2 $[M+H]^+$.

$^1H$ NMR (400 MHz, Methanol-$d_4$) δ 7.72 - 7.60 (m, 3H), 7.59 - 7.37 (m, 3H), 7.37 - 7.12 (m, 1H), 7.08 (s, 1H), 3.47 (brs, 4H), 3.36 (s, 2H), 1.77 - 1.63 (m, 4H), 0.95 (brs, 6H).

Compound **4**

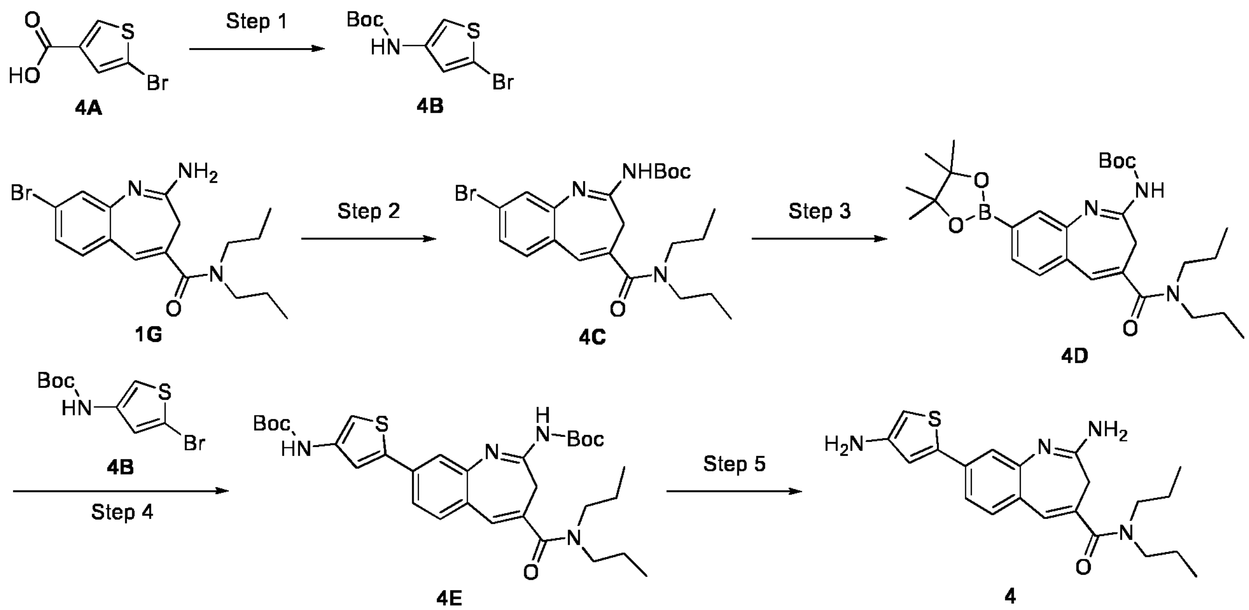

4A 4B 1G 4C 4D 4B 4E 4

**Step 1 4A** (2 g, 9.66 mmol, 1 eq) was dissolved in toluene (40 mL), then triethylamine (2.44 g, 24.15 mmol, 2.5 eq), DPPA (3.19 g, 11.59 mmol, 1.2 eq), and tert-butanol (7.16 g, 96.6 mmol, 10 eq) were added at room temperature. After the addition, the system was heated to 100 °C and stirred overnight. The reaction solution was poured into 50 mL of water and extracted with EA (50 mL × 3). The organic phase was dried over sodium sulfate, concentrated at reduced pressure, mixed with silica gel, and subjected to column chromatography (PE:EA = 5:1) to give an off-white solid **4B** (0.6 g, 22.3% yield).

**Step 2 1G** (2 g, 5.49 mmol, 1 eq) was dissolved in DCM (40 mL), then triethylamine (1.67 g, 16.47 mmol, 3 eq) was added at room temperature. $Boc_2O$ (1.8 g, 8.24 mmol, 1.5 eq) was then added dropwise, and the system was stirred at room temperature overnight after the addition. The reaction solution was poured into 50 mL of water and extracted with DCM (50 mL × 3). The organic phase was dried over sodium sulfate, concentrated at reduced pressure, mixed with silica gel, and subjected to column chromatography (PE:EA= 5:1) to give a pale yellow solid **4C** (1.2 g, 49% yield).

**Step 3 4C** (1.2 g, 2.58 mmol, 1 eq) was dissolved in dioxane (20 mL), then bis(pinacolato)diboron (722 mg, 2.84 mmol, 1.1 eq) and AcOK (507 mg, 5.17 mmol, 2 eq) were added. The system was purged with argon twice before Pd(dppf)$Cl_2$ (0.2 g) was added in one portion, then purged with argon twice again, heated to 85 °C, and reacted for 3 hours. When LCMS indicated the depletion of the starting materials, the reaction solution was cooled to room temperature and directly poured into a silica gel column. The product was eluted with PE:EA = 2:1 to give a yellow solid **4D** (600 mg, 45% yield). MS-ESI: m/z 512.3 $[M+H]^+$.

**Step 4 4B** (359 mg, 1.29 mmol, 1.2 eq) and **4D** (550 mg, 1.08 mmol, 1 eq) were added to a mixed solvent of dioxane (10 mL) and water (1 mL), then sodium carbonate (285 mg, 2.69 mmol, 2.5 eq) was added. The system was purged with argon twice before Pd(dppf)$Cl_2$ (100 mg) was added in one portion, then purged with argon twice again, heated to 85 °C, and reacted for 3 hours. When LCMS indicated the depletion of the starting materials, the reaction solution was cooled to room temperature, dried over sodium sulfate, directly mixed with silica gel, and purified by column chromatography to give a yellow solid **4E** (100 mg, 15.9% yield). MS-ESI: m/z 583.3 $[M+H]^+$.

**Step 5 4E** (100 mg, 0.17 mmol, 1 eq) was dissolved in EA (5 mL), then 4 N hydrochloride in dioxane (1 mL) was added in an ice-water bath. The system was stirred at room temperature overnight. After concentration at reduced pressure, the reaction mixture was subjected to preparative chromatography and lyophilized to give a yellow solid **4** (22 mg, 27% yield).

MS-ESI: m/z 383.2 $[M+H]^+$.

$^1H$ NMR (400 MHz, Methanol-$d_4$) δ 7.35 (d, *J*= 1.7 Hz, 1H), 7.30 (d, *J*= 8.1 Hz, 1H), 7.26 (dd, J = 8.2, 1.8 Hz, 1H), 7.05 (d, *J*= 1.5 Hz, 1H), 6.81 (s, 1H), 6.25 (d, *J*= 1.6 Hz, 1H), 4.52 - 4.38 (m, 2H), 3.46 - 3.37 (m, 4H), 1.73 - 1.60 (m, 4H), 0.91 (brs, 6H).

## Compound 5

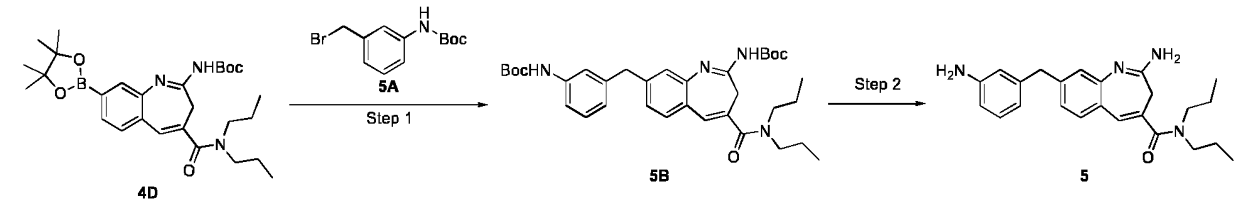


**Step 1** **5A** (280 mg, 0.98 mmol, 1 eq) and **4D** (500 mg, 0.98 mmol, 1 eq) were added to a mixed solvent of dioxane (10 mL) and water (2 mL), then sodium carbonate (259 mg, 2.44 mmol, 2.5 eq) was added. The system was purged with argon twice before Pd(dppf)$Cl_2$ (100 mg) was added in one portion, then purged with argon twice again, heated to 95 °C, and reacted for 3 hours. When LCMS indicated the depletion of the starting materials, the reaction solution was cooled to room temperature, dried over sodium sulfate, and directly purified by thin-layer chromatography to give a yellow solid **5B** (200 mg, 35% yield). MS-ESI: m/z 591.4 $[M+H]^+$.

**Step 2** **5B** (200 mg, 0.33 mmol, 1 eq) was dissolved in EA (5 mL), then 4 N hydrochloride in dioxane (0.5 mL) was added in an ice-water bath. The reaction system was stirred at room temperature overnight. When LCMS indicated the depletion of the starting materials, the mixture was concentrated at reduced pressure, subjected to preparative chromatography, and lyophilized to give a yellow solid **5** (25 mg, 19% yield).

MS-ESI: m/z 391.3 $[M+H]^+$.

$^1$H NMR (400 MHz, Methanol-$d_4$) δ 7.20 (d, *J*= 7.9 Hz, 1H), 7.05 - 6.96 (m, 2H), 6.90 (dd, *J*= 8.0, 1.9 Hz, 1H), 6.78 (s, 1H), 6.63 - 6.53 (m, 3H), 4.69 - 4.48 (m, 2H), 3.85 (s, 2H), 3.45 - 3.36 (m, 4H), 1.75 - 1.57 (m, 4H), 0.90 (brs, 6H).

## Compound 6

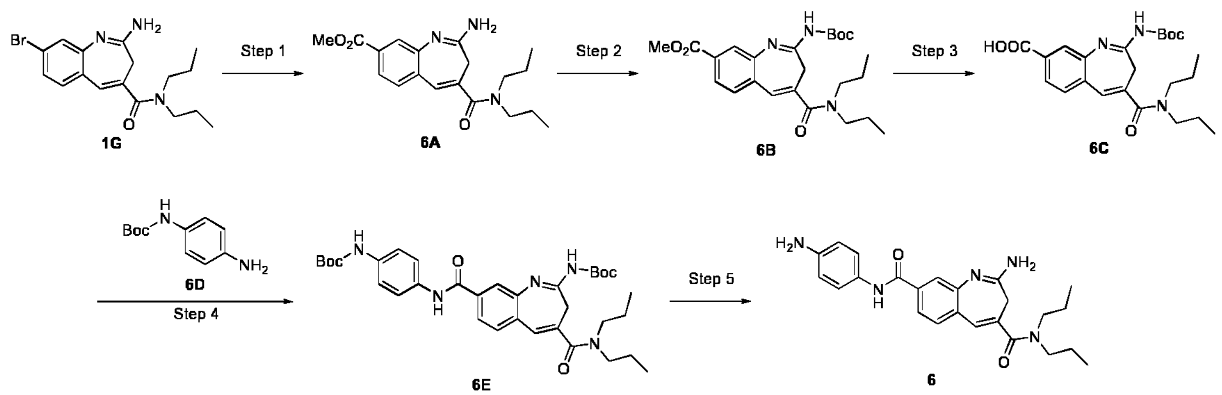


**Step 1** Methanol (20 mL), **1G** (1.2 g, 3.29 mmol, 1 eq), and triethylamine (1 g, 9.88 mmol, 3 eq) were added to a 50-mL autoclave. The system was purged with argon before Pd(dppf)$Cl_2$ (0.2 g) was added, then sealed soon after the addition, purged twice with argon before CO (60 psi) was introduced, heated to 80 °C, and reacted for 40 hours. When LCMS indicated a small amount of remaining starting materials, the mixture was filtered and concentrated at reduced pressure to give a crude dark red oil product **6A** (1.5 g), which was directly used in the next step without calculating the yield. MS-ESI: m/z 344.2 $[M+H]^+$.

**Step 2** The crude product **6A** (1.5 g, 4.37 mmol, 1 eq) from the previous step was dissolved in DCM (30 mL), then triethylamine (1.33 g, 13.10 mmol, 3 eq) was added at room temperature. $Boc_2O$ (1.43 g, 6.55 mmol, 1.5 eq) was then added dropwise, and the system was stirred at room temperature overnight after the addition. The reaction solution was poured into water (50 mL) and extracted with DCM (50 mL × 3). The organic phase was dried over sodium sulfate, concentrated at reduced pressure, and subjected to column chromatography (PE:EA = 5:1) to give a pale yellow solid **6B** (0.4 g, 27.6% yield over the two steps). MS-ESI: m/z 444.3 $[M+H]^+$.

**Step 3** **6B** (400 mg, 0.9 mmol, 1 eq) was added to a mixed solvent of water (5 mL) and THF (5 mL), then lithium hydroxide monohydrate (56.77 mg, 1.35 mmol, 1.5 eq) was added at room temperature. The system was stirred at room temperature overnight. When TLC indicated that the starting materials were depleted, the reaction solution was adjusted to pH 5 with saturated citric acid, and extracted with ethyl acetate (10 mL × 2). The organic phases were combined, dried over sodium sulfate, and concentrated at reduced pressure to give a yellow powder **6C** (350 mg, 90% yield).

**Step 4** **6C** (150 mg, 0.35 mmol, 1 eq) was added to DCM (5 mL), then HATU (159 mg, 0.42 mmol, 1.2 eq) was

added at room temperature. The system was stirred for 10 minutes, then NMM (89 mg, 0.873 mmol, 2 eq) and **6D** (73 mg, 0.35 mmol, 1 eq) were added. The system was then stirred overnight at room temperature. When TLC indicated that the starting materials were depleted, the reaction solution was poured into water (15 mL) and extracted with EA (30 mL × 4). The organic phases were combined, concentrated at reduced pressure, and directly purified by thin-layer chromatography to give an off-white powder **6E** (50 mg, 23% yield). MS-ESI: m/z 620.4 $[M+H]^+$. **Step 5 6E** (50 mg, 0.08 mmol, 1 eq) was dissolved in ethyl acetate (5 mL), then hydrochloride in dioxane (4 N, 0.5 mL) was added in an ice-water bath. The system was stirred at room temperature overnight. When LCMS indicated the depletion of the starting materials, the mixture was concentrated at reduced pressure and directly purified by thin-layer chromatography to give a pale yellow powder **6** (24 mg, 70.8% yield).

MS-ESI: m/z 420.6 $[M+H]^+$.

$^1$H NMR (400 MHz, Methanol-$d_4$) δ 7.66 (d, *J*= 2.0 Hz, 1H), 7.55 (dd, *J*= 8.1, 1.9 Hz, 1H), 7.43 (d, *J*= 8.2 Hz, 1H), 7.41 - 7.36 (m, 2H), 6.89 (s, 1H), 6.78 - 6.71 (m, 2H), 4.58 (s, 2H), 3.47 - 3.38 (m, 4H), 1.76 - 1.60 (m, 4H), 1.07 - 0.77 (m, 6H).

Compound 7

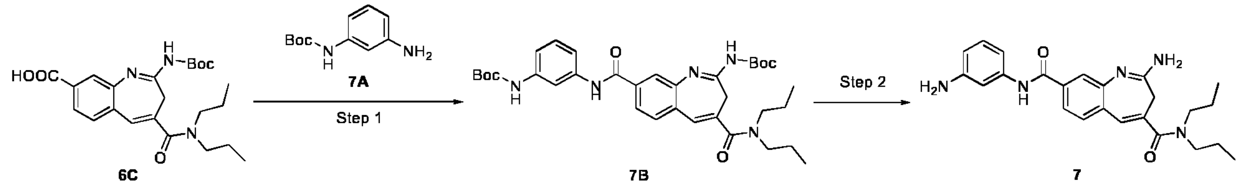

6C 7B 7

**Step 1 6C** (300 mg, 0.70 mmol) was added to a 100-mL flask, then DMF (10 mL), **7A** (174 mg, 0.84 mmol), DIEA (271 mg, 2.10 mmol), and HATU (372 mg, 0.98 mmol) were added at 0 °C. The reaction system was stirred at room temperature for 16 hours until LCMS indicated the depletion of the starting materials. Water (100 mL) was added, and the reaction mixture was extracted with EA (100 mL × 3). The organic phase was concentrated by rotary evaporation, and purified by column chromatography (EA:PE = 0 to 20%) to give a pale yellow oily liquid **7B** (300 mg, 69% yield). MS-ESI: m/z 620.4 $[M+H]^+$.

**Step 2 7B** (300 mg, 0.48 mmol) was added to a 100-mL flask, then DCM (8 mL) and TFA (2 mL) were added at 0 °C. The reaction system was stirred at room temperature for 16 hours until LCMS indicated the depletion of the starting materials. The reaction mixture was dried by rotary evaporation and purified by reversed-phase column chromatography ($NH_4HCO_3$) to give a pale yellow solid **7** (70 mg, 34% yield).

MS-ESI: m/z 420.2 $[M+H]^+$.

$^1$H NMR (400 MHz, DMSO-$d_6$) δ 9.90 (s, 1H), 7.60 (d, *J*= 1.8 Hz, 1H), 7.43 (dd, *J*= 8.1, 1.9 Hz, 1H), 7.38 (d, *J* = 8.2 Hz, 1H), 7.14 (t, *J* = 2.0 Hz, 1H), 6.94 (t, *J* = 7.9 Hz, 1H), 6.91 - 6.81 (m, 3H), 6.77 (s, 1H), 6.33 - 6.26 (m, 1H), 5.04 (s, 2H), 3.31 - 3.26 (m, 4H), 2.71 (s, 2H), 1.64 - 1.49 (m, 4H), 0.83 (brs, 6H).

Compound **8**

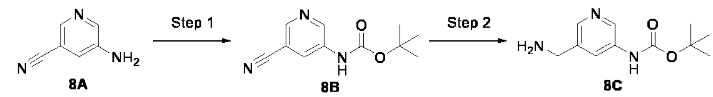

8A 8B 8C

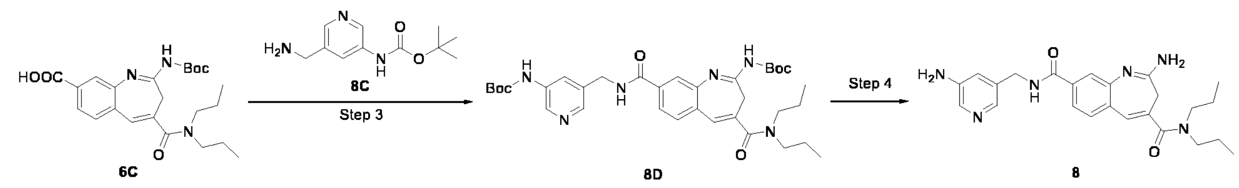

6C 8D 8

**Step 1 8A** (1 g, 8.4 mmol) was dissolved in DCM (20 mL), then DMAP (102 mg, 0.84 mmol) and $Boc_2O$ (2.2 g, 10.1 mmol) were added. The system was then stirred at room temperature overnight. After concentration, the mixture was purified by column chromatography (PE:EA = 1:1) to give a white solid **8B** (1.2 g, 65% yield, 95% purity). MS-ESI: m/z 220.0 $[M+H]^+$.

**Step 2 8B** (500 mg, 2.27 mmol) was dissolved in MeOH/$NH_3 \cdot H_2O$ (5 mL, v/v = 4/1), then Raney Ni (100 mg) was added at room temperature. The system was purged with $H_2$ thrice and stirred at room temperature overnight. The mixture was filtered and concentrated to give a green solid **8C** (450 mg, 85% yield, 84% purity). MS-ESI: m/z 224.2 $[M+H]^+$.

**Step 1 6C** (300 mg, 0.7 mmol) and **8C** (156 mg, 0.7 mmol) were dissolved in DMF (3 mL), then DIEA (271 mg,

2.1 mmol) and HATU (320 mg, 0.84 mmol) were added. The system was then stirred at room temperature overnight. The reaction solution was directly subjected to preparative HPLC (MeCN/$H_2O$) to give a pale yellow solid **8D** (200 mg, 45% yield, 85% purity). MS-ESI: m/z 635.4 $[M+H]^+$.

**Step 4 8D** (200 mg, 0.3 mmol) was dissolved in DCM (5 mL), then TFA (180 mg, 1.5 mmol) was added. The reaction system was stirred at room temperature for 3 hours. The mixture was diluted with water and adjusted to pH 8-9 with $NaHCO_3$. The aqueous phase was extracted with ethyl acetate (100 mL × 3). The organic phase was collected, washed with saturated brine (50 mL), dried over anhydrous sodium sulfate, filtered, concentrated by rotary evaporation, and subjected to preparative HPLC (MeCN/$H_2O$) to give a yellow solid **8** (52.8 mg, 35% yield, 95.23% purity).

MS-ESI: m/z 435.3 $[M+H]^+$.

$^1H$ NMR (400 MHz, DMSO-$d_6$) δ 7.90 - 7.77 (m, 2H), 7.64 - 7.58 (m, 1H), 7.52 - 7.44 (m, 1H), 7.40 (dd, *J* = 8.1, 3.3 Hz, 1H), 7.10 (s, 1H), 6.90 - 6.84 (m, 1H), 4.55 - 4.47 (m, 2H), 3.47 - 3.36 (m, 4H), 3.34 - 3.32 (m, 2H), 1.78 - 1.56 (m, 4H), 1.11 - 0.71 (m, 6H).

Compound **9**

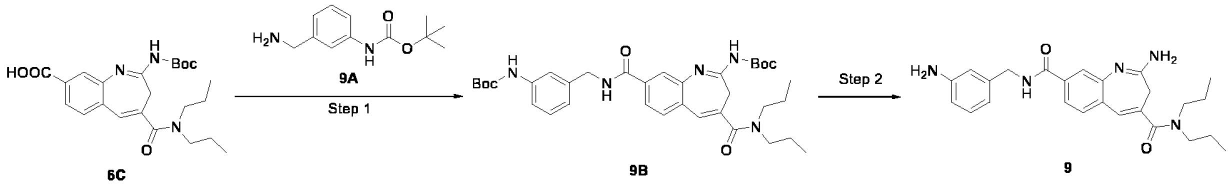


6C 9B 9

**Step 1 6C** (300 mg, 0.7 mmol) was dissolved in DMF (3 mL), then **9A** (155 mg, 0.7 mmol), DIEA (211 mg, 2.1 mmol) and HATU (320 mg, 0.84 mmol) were added. The system was then stirred at room temperature overnight. The reaction solution was directly subjected to preparative HPLC (MeCN/$H_2O$) to give a white solid **9B** (250 mg, 55% yield, 90% purity). MS-ESI: m/z 634.4$[M+H]^+$. **Step 2 9B** (250 mg, 0.4 mmol) was dissolved in DCM (5 mL), then TFA (135 mg, 1.2 mmol) was added. The reaction solution was incubated at room temperature for 3 hours. The mixture was diluted with water and adjusted to pH 8-9 with $NaHCO_3$. The aqueous phase was extracted with ethyl acetate (100 mL × 3). The organic phase was collected, washed with saturated brine (50 mL), dried over anhydrous sodium sulfate, filtered, concentrated by rotary evaporation, and subjected to preparative HPLC (MeCN/$H_2O$) to give a pale yellow solid **9** (26.3 mg, 15% yield, 99.59% purity).

MS-ESI: m/z 434.3 $[M+H]^+$.

$^1H$ NMR(400 MHz, Methanol-$d_4$) δ 7.61 (d, *J* = 1.8 Hz, 1H), 7.48 (dd, *J* = 8.1, 1.9 Hz, 1H), 7.39 (d, *J* = 8.2 Hz, 1H), 7.06 (t, *J* = 7.7 Hz, 1H), 6.87 (s, 1H), 6.74 (t, *J* = 2.0 Hz, 1H), 6.71 - 6.66 (m, 1H), 6.62 (ddd, *J*= 8.0, 2.4, 1.0 Hz, 1H), 4.49 (s, 2H), 3.46 - 3.37 (m, 4H), 1.76 - 1.57 (m, 4H), 1.11 - 0.72 (m, 6H).

Compound **10**

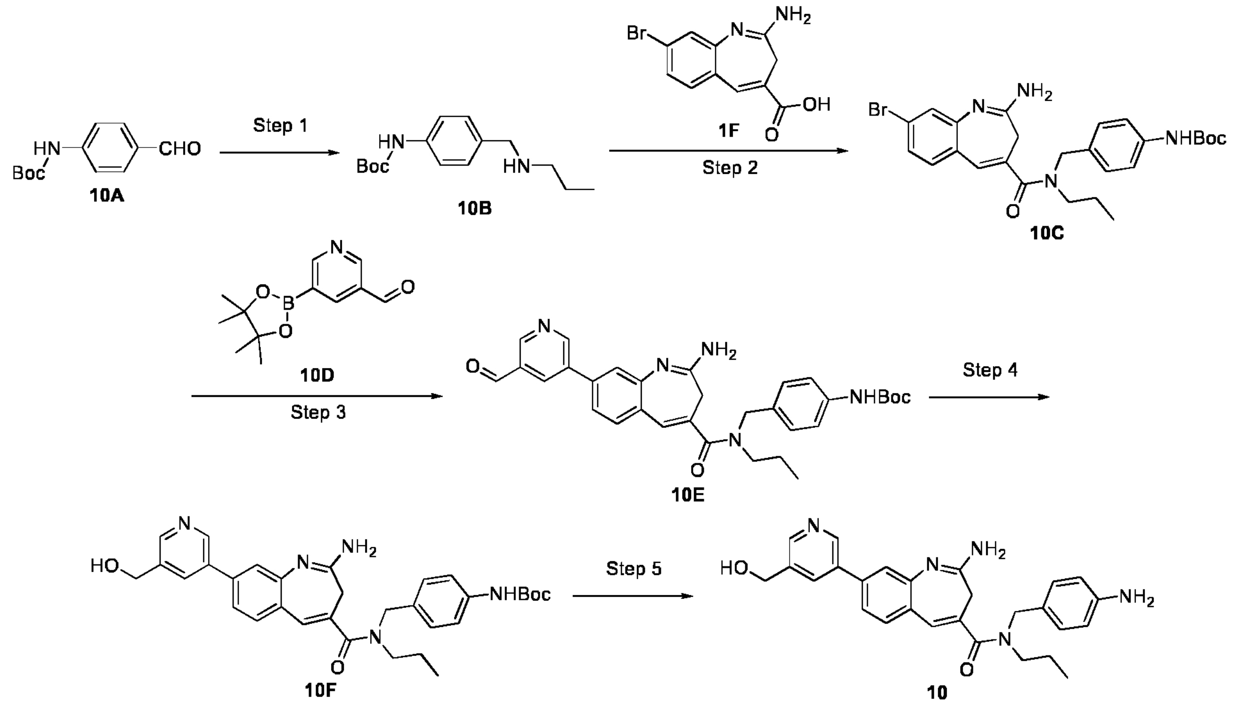


**Step 1 10A** (5 g, 22.60 mmol, 1 eq) was added to DCM (100 mL), then n-propylamine (4.01 g, 67.80 mmol, 3 eq) was added at room temperature. After the addition, the system was stirred at room temperature overnight. At room temperature, sodium cyanoborohydride (2.84 g, 45.20 mmol, 2 eq) was added slowly, and after the addition, the system was stirred at room temperature overnight. The reaction solution was poured into 100 mL of water and the phases were separated. The aqueous phase was extracted with DCM (100 mL × 2). The organic phases were combined, dried over sodium sulfate, concentrated at reduced pressure, mixed with silica gel, and subjected to column chromatography (DCM:MeOH = 50:1 to 10:1) to give a white solid **10B** (3 g, 50.3% yield).

**Step 2 1F** (1 g, 3.15 mmol, 1 eq) was dissolved in DMF (20 mL). The temperature was reduced to about 0 °C, then HBTU (1.43 g, 3.78 mmol, 1.2 eq) and DIEA (1.63 g, 12.60 mmol, 4 eq) were added. The system was stirred for 10 min at 0 °C, then **10B** (1 g, 3.78 mmol, 1.2 eq) was added. After the addition, the system was stirred at room temperature overnight. The reaction solution was poured into water (60 mL) and extracted with EA (50 mL × 5). The organic phases were combined, washed with saturated brine (50 mL × 2), dried over sodium sulfate, concentrated at reduced pressure, mixed with silica gel, and subjected to column chromatography (DCM:MeOH = 20:1 to 10:1). The product was then concentrated at reduced pressure to give a yellow solid **10C** (550 mg, 30% yield). MS-ESI: m/z 527.2 $[M+H]^+$.

**Step 3 10C** (550 mg, 1.04 mmol, 1 eq) was added to a mixed solvent of dioxane (20 mL) and water (2 mL), then **10D** (243 mg, 1.04 mmol, 1 eq) and anhydrous potassium phosphate (664 mg, 3.13 mmol, 3 eq) were added. The system was purged with argon twice before $Pd(PPh_3)_4$ (110 mg) was added, then purged with argon twice again, heated to 90 °C, and reacted for 3 hours. When LCMS indicated the depletion of the starting materials, the reaction solution was poured into 50 mL of water and extracted with EA (50 mL × 3). The organic phases were combined, dried over sodium sulfate, concentrated at reduced pressure, and subjected to column chromatography (PE:EA = 2: 1 to 0:1) to give a white solid **10E** (200 mg, 34.7% yield). MS-ESI: m/z 554.4 $[M+H]^+$.

**Step 4 10E** (200 mg, 0.36 mmol, 1 eq) was added to methanol (10 mL), then sodium borohydride (41 mg, 1.08 mmol, 3 eq) was added slowly in portions at room temperature. After the addition, the system was reacted at room temperature for 2 hours. When LCMS indicated the depletion of the starting materials, the reaction was quenched with saturated aqueous ammonium chloride (5 mL), and the mixture was concentrated at reduced pressure to remove the solvent and directly purified by thin-layer chromatography to give a white solid **10F** (160 mg, 80% yield). MS-ESI: m/z 556.3 $[M+H]^+$. **Step 5 10F** (160 mg, 0.28 mmol, 1 eq) was dissolved in EA (5 mL), then 4 N hydrochloride in dioxane (0.5 mL) was added in an ice-water bath. The system was stirred at room temperature overnight. When LCMS indicated the depletion of the starting materials, the mixture was concentrated at reduced pressure, subjected to preparative chromatography, and lyophilized to give a white powder **10** (30 mg, 23% yield).

MS-ESI: m/z 456.3 $[M+H]^+$.

$^1H$ NMR (400 MHz, Methanol-$d_4$) $\delta$ 8.88 (d, *J* = 2.2 Hz, 1H), 8.66 (d, *J* = 1.9 Hz, 1H), 8.34 (s, 1H), 7.84 - 7.56 (m, 3H), 7.56 - 7.18 (m, 4H), 7.16 (s, 1H), 4.81 (s, 2H), 4.78 (s, 2H), 3.55 - 3.45 (m, 2H), 3.45 - 3.33 (m, 2H), 1.76 - 1.63 (m, 2H), 0.92 (s, 3H).

Compound **11**

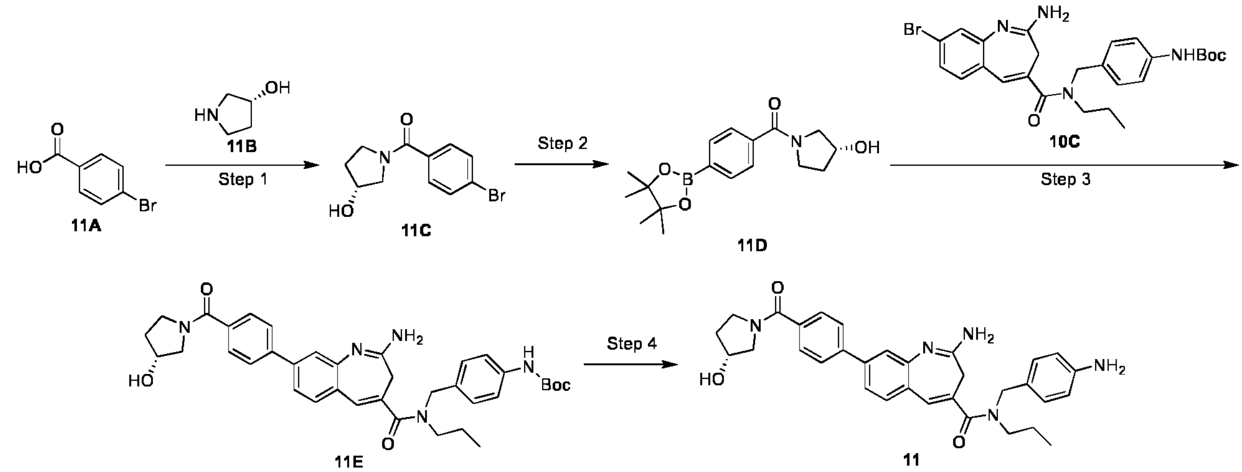


**Step 1 11A** (1 g, 4.97 mmol, 1 eq) was dissolved in DMF (20 mL), then DIEA (1.93 g, 14.92 mmol, 3 eq) and HATU (2.08 g, 5.47 mmol, 1.1 eq) were added at room temperature. After the addition, the system was stirred at room temperature for 5 min, and **11B** (0.434 g, 4.97 mmol, 1 eq) was added. The system was then stirred at room temperature overnight after the addition. The reaction solution was poured into 60 mL of water and extracted with EA (50 mL × 3). The organic phase was dried over sodium sulfate, concentrated at reduced pressure, mixed with silica gel, and subjected to column chromatography (PE:EA = 5:1) to give a white solid **11C** (1 g, 74.6% yield). MS-ESI: m/z 270.0

[M+H]$^+$.

**Step 2 11C** (0.9 g, 3.33 mmol, 1 eq) was dissolved in dioxane (20 mL), then bis(pinacolato)diboron (931 mg, 3.67 mmol, 1.1 eq) and AcOK (654 mg, 6.66 mmol, 2 eq) were added. The system was purged with argon twice before Pd(dppf)$Cl_2$ (0.2 g) was added in one portion, then purged with argon twice again, heated to 85 °C, and reacted for 3 hours. When LCMS indicated the depletion of the starting materials, the reaction solution was cooled to room temperature and directly loaded on a silica gel column (PE:EA = 2:1) for purification to give a pale yellow solid **11D** (300 mg, 28.3% yield). MS-ESI: m/z 318.3 [M+H]$^+$.

**Step 3 10C** (300 mg, 0.569 mmol, 1 eq) and **11D** (180.4 mg, 0.569 mmol, 1 eq) were added to a mixed solvent of dioxane (10 mL) and water (1 mL), then sodium carbonate (150.71 mg, 1.42 mmol, 2.5 eq) was added. The system was purged with argon twice before Pd(dppf)$Cl_2$ (50 mg) was added in one portion, then purged with argon twice again, heated to 85 °C, and reacted for 3 hours. When LCMS indicated the depletion of the starting materials, the reaction solution was cooled to room temperature, dried over sodium sulfate, directly mixed with silica gel, and purified by column chromatography to give a yellow solid **11E** (100 mg, 27.6% yield). MS-ESI: m/z 638.4 [M+H]$^+$. **Step 4 11E** (100 mg, 0.157 mmol, 1 eq) was dissolved in EA (5 mL), then 4 N hydrochloride in dioxane (1 mL) was added in an ice-water bath. The system was stirred at room temperature overnight. When LCMS indicated the depletion of the starting materials, the mixture was concentrated at reduced pressure, subjected to preparative chromatography, and lyophilized to give a yellow solid **11** (30 mg, 35.7% yield).

MS-ESI: m/z 538.3 [M+H]$^+$.

$^1H$ NMR (400 MHz, Methanol-$d_4$) $\delta$ 7.80 (d, *J* = 7.9 Hz, 2H), 7.76 - 7.66 (m, 4H), 7.66 - 7.30 (m, 3H), 7.25 (s, 2H), 7.14 (s, 1H), 4.78 (s, 2H), 4.45 (d, *J* = 47.8 Hz, 1H), 3.84 - 3.58 (m, 3H), 3.58 - 3.32 (m, 5H), 2.19 - 1.90 (m, 2H), 1.75 - 1.63 (m, 2H), 0.91 (s, 3H).

Compound **12**

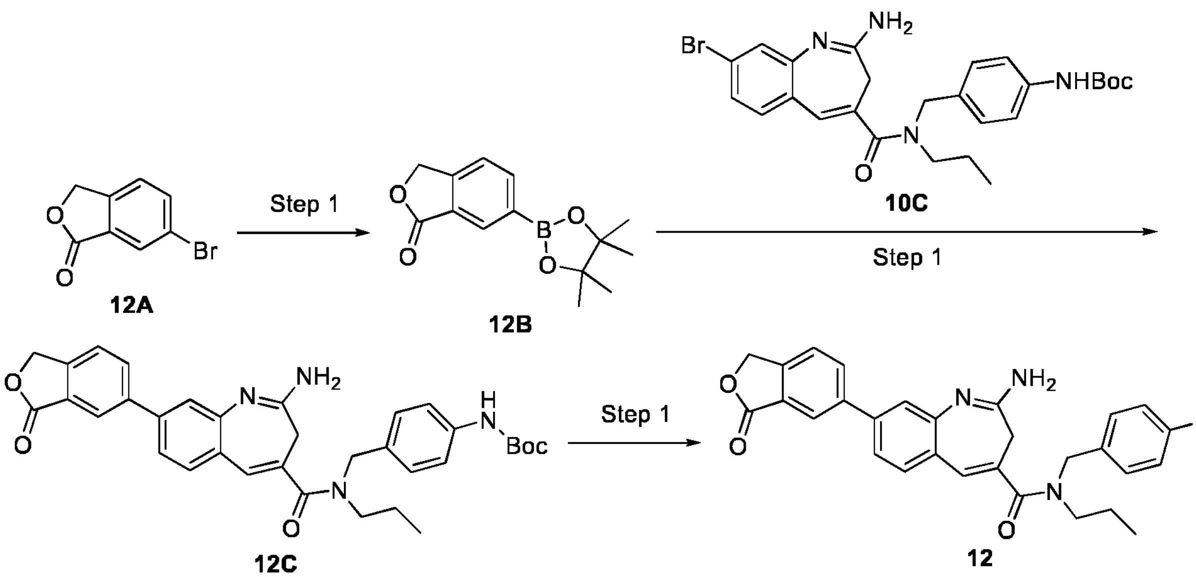

12A 12B 10C 12C 12

**Step 1 12A** (1 g, 4.69 mmol, 1 eq) was dissolved in dioxane (20 mL), then bis(pinacolato)diboron (1.31 g, 5.16 mmol, 1.1 eq) and AcOK (921.4 mg, 9.39 mmol, 2 eq) were added. The system was purged with argon twice before Pd(dppf)$Cl_2$ (0.1 g) was added in one portion, then purged with argon twice again, heated to 85 °C, and reacted for 3 hours. When LCMS indicated the depletion of the starting materials, the reaction solution was cooled to room temperature and directly loaded on a silica gel column (PE:EA = 10:1) for purification to give a white solid **12B** (300 mg, 24.6% yield). MS-ESI: m/z 261.2 $[M+H]^+$.

**Step 2 10C** (250 mg, 0.474 mmol, 1 eq) and **12B** (247 mg, 0.948 mmol, 2 eq) were added to a mixed solvent of dioxane (10 mL) and water (1 mL), then potassium phosphate (302 mg, 1.42 mmol, 3 eq) was added. The system was purged with argon twice before Pd(dppf)$Cl_2$ (50 mg) was added in one portion, then purged with argon twice again, heated to 85 °C, and reacted for 3 hours. When LCMS indicated the depletion of the starting materials, the reaction solution was cooled to room temperature, dried over sodium sulfate, directly mixed with silica gel, and purified by column chromatography to give a yellow solid **12C** (100 mg, 36.4% yield). MS-ESI: m/z 581.2 $[M+H]^+$.

**Step 3 12C** (100 mg, 0.172 mmol, 1 eq) was dissolved in EA (5 mL), then 4 N hydrochloride in dioxane (1 mL) was added in an ice-water bath. The system was stirred at room temperature overnight. When LCMS indicated the depletion of the starting materials, the mixture was concentrated at reduced pressure, subjected to preparative chromatography, and lyophilized to give a yellow solid **12** (23 mg, 28% yield).

MS-ESI: m/z 481.3 $[M+H]^+$.

$^1$H NMR (400 MHz, Methanol-$d_4$) δ 8.17 (s, 1H), 8.10 (d, $J$ = 8.1 Hz, 1H), 7.84 - 7.70 (m, 3H), 7.70 - 7.27 (m, 3H), 7.27 - 7.06 (m, 3H), 5.47 (s, 2H), 4.77 (s, 2H), 3.54 - 3.44 (m, 2H), 3.44 - 3.32 (m, 2H), 1.77 - 1.64 (m, 2H), 0.92 (s, 3H).

## Compound **13**

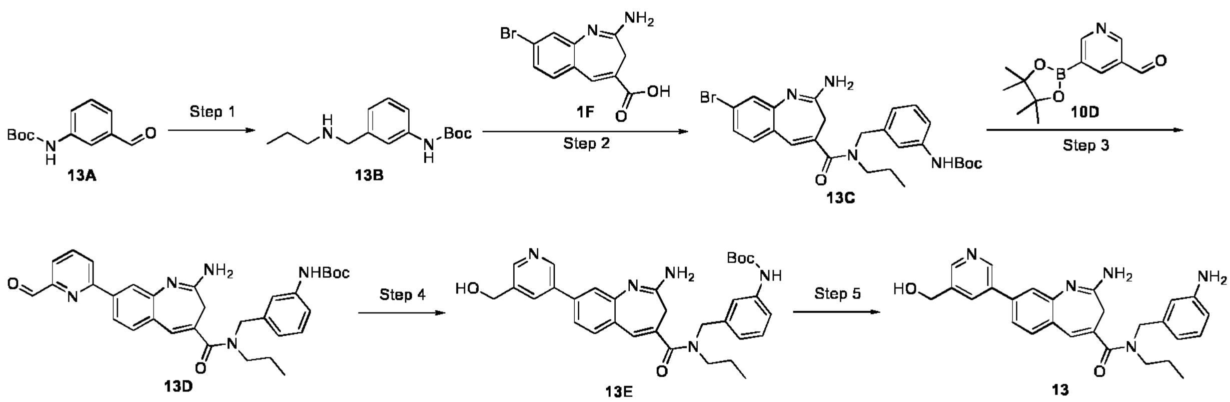

13A 13B 1F 13C 10D 13D 13E 13

**Step 1 13A** (5 g, 22.60 mmol, 1 eq) was added to DCM (100 mL), then n-propylamine (4.01 g, 67.80 mmol, 3

eq) was added at room temperature. After the addition, the system was stirred at room temperature overnight. At room temperature, sodium cyanoborohydride (2.84 g, 45.20 mmol, 2 eq) was added slowly, and after the addition, the system was stirred at room temperature overnight. The reaction solution was poured into 100 mL of water and the phases were separated. The aqueous phase was extracted with DCM (100 mL × 2). The organic phases were combined, dried over sodium sulfate, concentrated at reduced pressure, mixed with silica gel, and subjected to column chromatography (DCM:MeOH = 50:1 to 10:1) to give a white solid **13B** (1.5 g, 25.1% yield). MS-ESI: m/z 265.2 $[M+H]^+$.

**Step 2 1F** (2 g, 6.30 mmol, 1 eq) was dissolved in DMF (40 mL). The temperature was reduced to about 0 °C, then HBTU (2.86 g, 7.56 mmol, 1.2 eq) and DIEA (3.26 g, 25.20 mmol, 4 eq) were added. The system was stirred for 10 min at 0 °C, then **13B** (2 g, 7.56 mmol, 1.2 eq) was added. After the addition, the system was stirred at room temperature overnight. When LCMS indicated the depletion of the starting material **1F**, the reaction solution was poured into water (120 mL) and extracted with EA (100 mL × 5). The organic phases were combined, washed with saturated brine (100 mL × 2), dried over sodium sulfate, concentrated at reduced pressure, mixed with silica gel, and subjected to column chromatography (DCM:MeOH = 20:1 to 10:1). The product was then concentrated at reduced pressure to give a yellow solid **13C** (1.2 g, 36.1% yield). MS-ESI: m/z 527.2 $[M+H]^+$.

**Step 3 13C** (1.2 g, 2.28 mmol, 1 eq) was added to a mixed solvent of dioxane (40 mL) and water (4 mL), then **10D** (530.3 mg, 2.28 mmol, 1 eq) and anhydrous potassium phosphate (1.45 g, 6.83 mmol, 3 eq) were added. The system was purged with argon twice before $Pd(PPh_3)_4$ (220 mg) was added, then purged with argon twice again, heated to 90 °C, and reacted for 3 hours. When LCMS indicated the depletion of the starting materials, the reaction solution was poured into 100 mL of water and extracted with EA (100 mL × 3). The organic phases were combined, dried over sodium sulfate, concentrated at reduced pressure, and subjected to column chromatography (PE:EA =2:1 to 0:1) to give a white solid **13D** (250 mg, 19.9% yield). MS-ESI: m/z 554.3 $[M+H]^+$.

**Step 4 13D** (250 mg, 0.45 mmol, 1 eq) was added to methanol (10 mL), then sodium borohydride (51.3 mg, 1.35 mmol, 3 eq) was added slowly in portions at room temperature. After the addition, the system was reacted at room temperature for 2 hours. The reaction was quenched with 5 mL of saturated aqueous ammonium chloride, and the mixture was concentrated at reduced pressure to remove the solvent and directly purified by thin-layer chromatography to give a white solid **13E** (124 mg, 49.4% yield). MS-ESI: m/z 556.2 $[M+H]^+$.

**Step 5 13E** (124 mg, 0.22 mmol, 1 eq) was dissolved in EA (5 mL), then 4 N hydrochloride in dioxane (0.5 mL) was added in an ice-water bath. The system was stirred at room temperature overnight. When LCMS indicated the depletion of the starting materials, the mixture was concentrated at reduced pressure, adjusted to pH 8 with saturated $NaHCO_3$ solution, extracted with EA (10 mL × 3), concentrated, mixed with silica gel, and subjected to column chromatography (DCM:MeOH = 30:1 to 10:1) and lyophilized to give a white powder **13** (25 mg, 24.6% yield).

MS-ESI: m/z 456.2 $[M+H]^+$.

$^1H$ NMR (400 MHz, DMSO-$d_6$) δ 8.76 (d, *J* = 2.0 Hz, 1H), 8.50 (s, 1H), 7.99 (s, 1H), 7.32 (s, 1H), 7.28 - 7.26 (d, *J* = 7.7 Hz, 1H), 6.99 - 6.97 (d, *J* = 7.6 Hz, 1H), 6.86 (s, 2H), 6.47 - 6.45 (d, *J* = 8.1 Hz, 3H), 5.08 (s, 2H), 4.61 (s, 2H), 4.48 (s, 2H), 3.32 - 3.21 (m, 2H), 2.79 (s, 2H), 1.56 - 1.52 (m, 2H), 1.23 (s, 1H), 0.85 - 0.81 (m, 3H).

Compound **14**

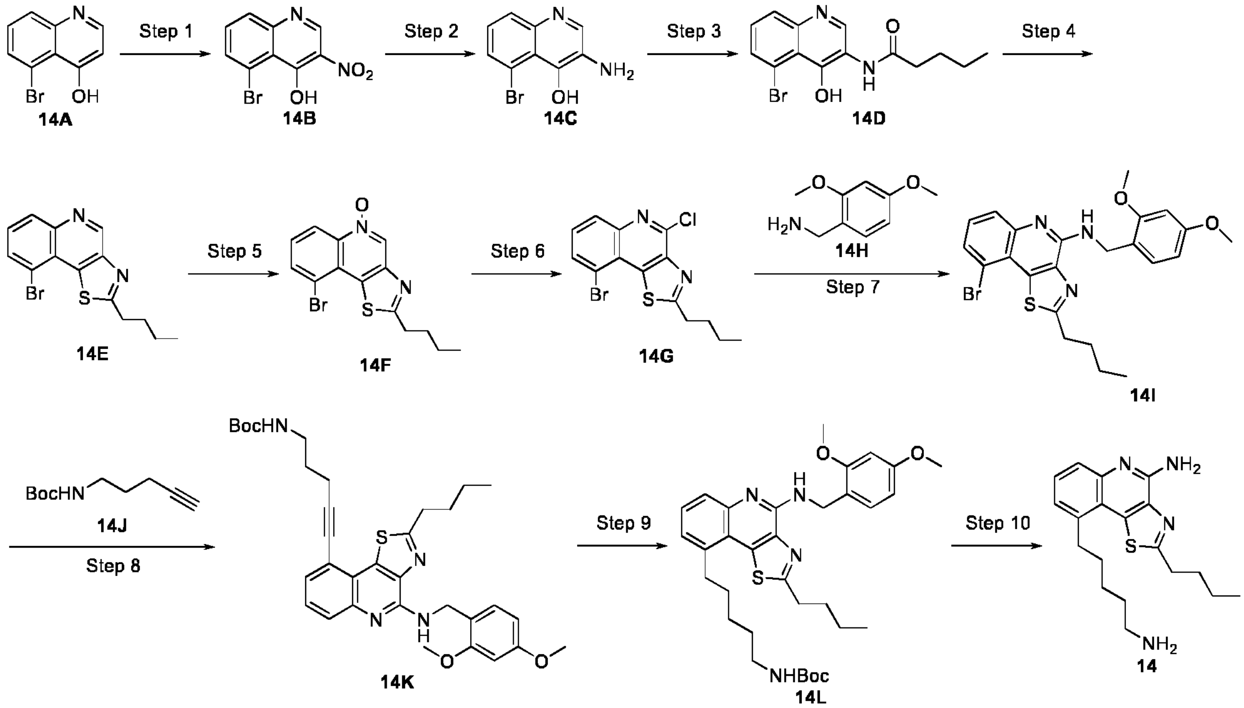


**14A** **14B** **14C** **14D** **14E** **14F** **14G** **14H** **14I** **14J** **14K** **14L** **14**

**Step 1** At room temperature, **14A** (4.5 g, 20.2 mmol) and propionic acid (100 mL) were added to a 250-mL three-necked flask. The reaction system was heated to 125 °C, and $HNO_3$ (3.2 g, 50.8 mmol) was added dropwise with the internal temperature controlled at 120-130 °C. The reaction system was stirred at 125 °C for 1 hour until LCMS indicated the completion of the reaction. The reaction solution was cooled to room temperature, slowly poured into ethanol (500 mL) and filtered. The filter cake was rinsed sequentially with ethanol (100 mL), water (100 mL), and ethanol (100 mL). The filter cake was collected and dried to give a yellow solid **14B** (3 g, 55.6% yield). MS-ESI: m/z 269.0[M+H]$^+$.

**Step 2** At room temperature, **14B** (3 g, 11.2 mmol) was added to a 250-mL single-neck flask and dissolved in EtOH (50 mL) and $H_2O$ (10 mL), then $NH_4Cl$ (1.8 g, 33.6 mmol) and iron powder (3.1 g, 56 mmol) were added. The reaction system was stirred at 80 °C for 2 hours until LCMS indicated the completion of the reaction. The reaction solution was filtered before cooling. Ethyl acetate (200 mL) and water (100 mL) were added to the filtrate, and the organic phase was separated. The aqueous phase was extracted with ethyl acetate (100 mL × 3), and the organic phase was collected, washed with saturated brine (100 mL), dried over anhydrous sodium sulfate, filtered, and concentrated by rotary evaporation. The crude product was purified by column chromatography (PE/EA = 10:1 to 3:1) to give a yellow solid **14C** (2 g, 75% yield). MS-ESI: m/z 241.0 [M+H]$^+$.

**Step 3** **14C** (2 g, 8.4 mmol), DCM (20 mL), and TEA (1.3 g, 12.6 mmol) were added into a 100-mL three-necked flask. *n*-Valeryl chloride (1.2 g, 10.1 mmol) was added slowly in an ice bath and the reaction system was stirred for 1 hour at 25 °C. When LCMS indicated the completion of the reaction, the reaction solution was poured into water (20 mL) and extracted with EA (50 mL × 3). The organic phase was collected, washed with saturated brine (20 mL), and dried over anhydrous sodium sulfate. After filtration and concentration, the crude product was purified by column chromatography (PE/EA = 3/1) to isolate a yellow oily liquid **14D** (1.5 g, 55.6% yield). MS-ESI: m/z 323.0 [M+H]$^+$.

**Step 4** At room temperature, **14D** (1.5 g, 4.7 mmol) and pyridine (50 mL) were added into a 250-mL single-neck flask, then $P_2S_5$ (9 g, 47 mmol) was added. The reaction system was stirred at 120 °C overnight. The reaction solution was concentrated by rotary evaporation, poured into water (100 mL), and extracted with EA (3 × 100 mL). The organic phase was collected, washed with saturated brine (100 mL), and dried over anhydrous sodium sulfate. After filtration and concentration, the crude product was purified by column chromatography (PE/EA = 10/1) to give a yellow solid **14E** (1 g, 66.7% yield). MS-ESI: m/z 321.0 [M+H]$^+$.

**Step 5** At room temperature, **14E** (2.6 g, 8.1 mmol) and $CHCl_3$ (50 mL) were added into a 250-mL single-neck flask, then m-CPBA (3.3 g, 16.2 mmol) was added slowly in an ice bath. The reaction system was stirred at 25 °C overnight. The reaction mixture was poured into DCM (200 mL). The mixture was sequentially washed with 5% sodium thiosulfate (50 mL), saturated sodium bicarbonate (50 mL), and saturated brine (50 mL), and dried over anhydrous sodium sulfate. After filtration and concentration, the crude product was purified by column chromatography (PE/EA = 3/1) to give a yellow oily liquid **14F** (2 g, 73.5% yield). MS-ESI: m/z 337.0 [M+H]$^+$.

**Step 6** **14F** (2 g, 5.95 mmol) was added to a 100-mL three-necked flask, then $POCl_3$ (27 g, 178.5 mmol) was

added slowly in an ice bath. DIEA (2.3 g, 17.85 mmol) was slowly and dropwise added with the internal temperature kept at 15 °C, and the reaction system was stirred at 100 °C overnight. When LCMS indicated the completion of the reaction, the reaction solution was cooled to room temperature, concentrated by rotary evaporation at reduced pressure to remove the solvent, and poured slowly into ice water (100 mL). The mixture was adjusted to pH 9 with solid potassium carbonate and the aqueous phase was extracted with ethyl acetate (100 mL × 3). The organic phase was collected, washed with saturated brine (50 mL), and dried over anhydrous sodium sulfate. After filtration and rotary evaporation, the crude product was purified by column chromatography (PE/EA = 10/1) to give a yellow oily liquid **14G** (1.8 g, 85.7% yield). MS-ESI: m/z 355.0 $[M+H]^+$.

**Step 7** At room temperature, **14G** (1.8 g, 5.1 mmol) and toluene (20 mL) were added into a 100-mL single-neck flask, then **14H** (8.5 g, 51 mmol) and 2-chlorobenzoic acid (799 mg, 5.1 mmol) were sequentially added at room temperature. The reaction system was stirred at 120 °C overnight. When LCMS indicated the completion of the reaction, the solvent was removed by rotary evaporation at reduced pressure. 100 mL of water was added, and the mixture was extracted with EA (3 × 100 mL). The organic phase was collected, washed with saturated brine (50 mL), and dried over anhydrous sodium sulfate. After filtration and concentration, the crude product was purified by reversed-phase column chromatography (neutral system) to give a yellow solid **14I** (2 g, 80% yield). MS-ESI: m/z 486.1 $[M+H]^+$.

**Step 8** At room temperature, **14I** (0.5 g, 1.03 mmol) and DMF (20 mL) were added into a 100-mL single-neck flask, then **14J** (377 mg, 2.06 mmol), $Pd(PPh_3)_2Cl_2$ (75 mg, 0.103 mmol) and $Cu_2O$ (442 mg, 3.09 mmol) were sequentially added at room temperature. The reaction system was stirred overnight at 110 °C under $N_2$ atmosphere. When LCMS indicated the completion of the reaction, 100 mL of water was added to the reaction solution and the mixture was extracted with EA (100 mL × 3). The organic phase was collected, washed with saturated brine (50 mL), and dried over anhydrous sodium sulfate. After filtration and concentration, the crude product was separated by column chromatography (PE:EA = 3:1) to give a yellow oily liquid **14K** (0.3 g, 49.5% yield). MS-ESI: m/z 589.3 $[M+H]^+$.

**Step 9** At room temperature, **14K** (0.3 g, 0.51 mmol) and THF (20 mL) were added into a 100-mL single-neck flask, then Pd/C (50 mg) was added at room temperature. The system was purged with $H_2$ thrice and stirred at 25 °C overnight. When LCMS indicated the completion of the reaction, the reaction solution was filtered through celite and rinsed with THF (3 × 20 mL). The filtrate was concentrated by rotary evaporation at reduced pressure, and the crude product was separated by column chromatography (PE/EA = 5/1) to give a yellow oily liquid **14L** (0.2 g, 66.7% yield). MS-ESI: m/z 593.3 $[M+H]^+$.

**Step 10** At room temperature, **14L** (200 mg, 0.34 mmol) was added into a 100-mL single-neck flask, then TFA (5 mL) was slowly added in an ice bath. The reaction system was stirred at 80 °C for 15 min. When LCMS indicated the completion of the reaction, the solvent was removed by rotary evaporation at reduced pressure, and the mixture was purified by reversed-phase column chromatography (neutral system) to give a white solid **14** (77.6 mg, 66.9% yield).

MS-ESI: m/z 343.2 $[M+H]^+$.

$^1H$ NMR (400 MHz, Methanol-$d_4$) δ 7.57 (d, *J* = 8.3 Hz, 1H), 7.49 - 7.42 (m, 1H), 7.18 (d, *J* = 7.2 Hz, 1H), 3.23 - 3.15 (m, 2H), 3.11 - 3.02 (m, 2H), 2.72 (t, *J* = 6.7 Hz, 2H), 1.97 - 1.85 (m, 2H), 1.76 - 1.65 (m, 2H), 1.64 - 1.46 (m, 6H), 1.02 (t, *J* = 7.4 Hz, 3H).

## Compound 15

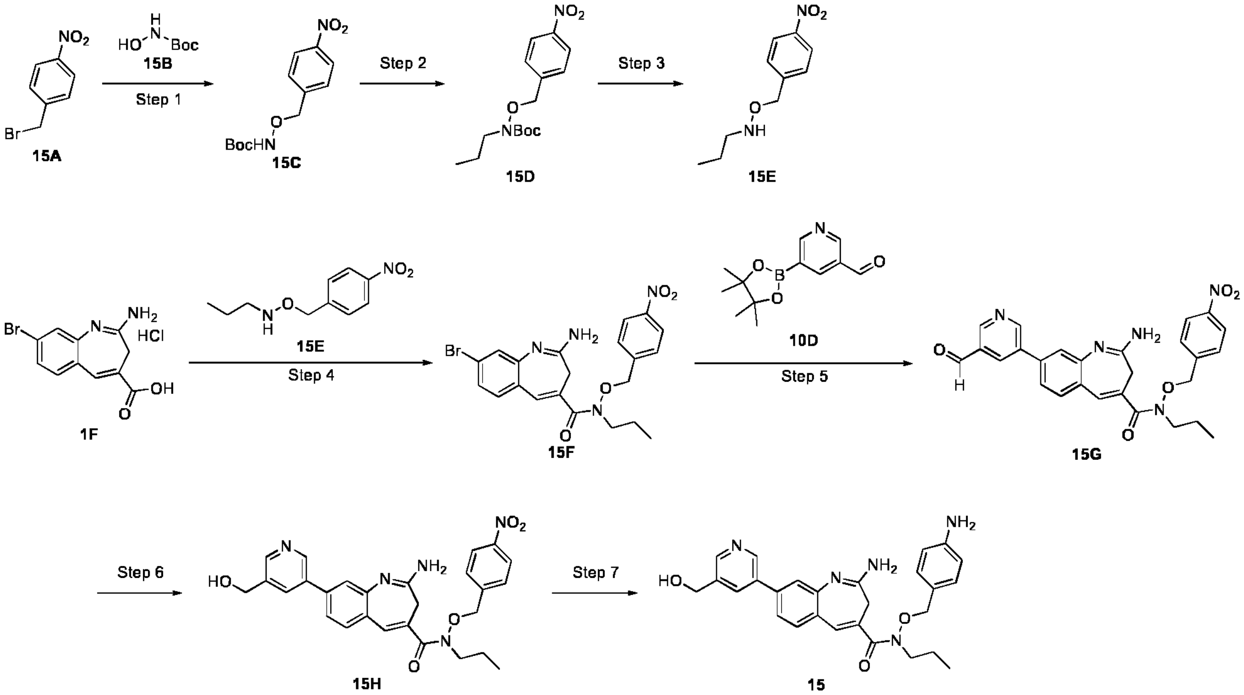


**Step 1 15A** (5 g, 23.14 mmol, 1 eq) was dissolved in acetonitrile (100 mL), then tert-butyl *N*-hydroxycarbamate **15B** (4.01 g, 30.09 mmol, 1.3 eq) was added at 0 °C. DBU (3.52 g, 23.14 mmol, 1 eq) was then slowly and dropwise added, and the system was warmed to room temperature and stirred for 3 hours after the addition. When LCMS indicated the depletion of **15A**, the reaction solution was concentrated at reduced pressure, then saturated potassium carbonate solution (100 mL) was added. The mixture was extracted with DCM (50 mL × 3). The organic phases were combined, washed with saturated brine (50 mL), dried over sodium sulfate, concentrated at reduced pressure, mixed with silica gel, and subjected to column chromatography (PE:EA = 5:1) to give a white solid **15C** (4.5 g, 72.5% yield). MS-ESI: m/z 291.0 $[M+Na]^+$.

**Step 2 15C** (4.5 g, 16.77 mmol, 1 eq) was dissolved in DMF (50 mL), and the mixture was cooled to 0 °C. NaH (671 mg, 16.77 mmol, 1 eq, 60%) was added in one portion, and the mixture was stirred at 0 °C for 10 min before iodopropane (3.14 g, 18.45 mmol, 1.1 eq) was added dropwise. After the addition, the reaction system was warmed to room temperature and reacted overnight. When LCMS indicated the depletion of the starting material **15C**, the reaction solution was poured into ice water (150 mL) and extracted with EA (100 mL × 3). The organic phases were combined, sequentially washed with 10% aqueous citric acid (100 mL), saturated sodium bicarbonate (100 mL) and saturated brine (100 mL), dried over sodium sulfate, concentrated at reduced pressure, mixed with silica gel, and subjected to column chromatography (PE:EA = 5:1) to give a yellow solid **15D** (4.5 g, 86.4% yield). MS-ESI: m/z 333.2 $[M+Na]^+$.

**Step 3 15D** (4.5 g, 14.52 mmol, 1 eq) was dissolved in EA (50 mL), then 4 N hydrochloride in dioxane (50 mL) was added in an ice-water bath. The system was stirred at room temperature overnight. When LCMS indicated the depletion of **15D**, the reaction mixture was directly filtered. The filter cake was rinsed with EA (100 mL), quickly transferred to a flask, and dried under vacuum to give a yellow solid **15E** (2.5 g, 83.3% yield). MS-ESI: m/z 211.2 $[M+H]^+$.

**Step 4 1F** (3 g, 9.45 mmol, 1 eq) and **15E** (2.5 g, 10.39 mmol, 1.1 eq) were added to a mixed solvent of DMA (30 mL) and DCM (30 mL). The system was purged with argon twice before EDCI (7.24 g, 37.79 mmol, 4 eq) was added in one portion, then purged with argon twice again, warmed to room temperature, and reacted overnight. When LCMS indicated the completion of the reaction, the reaction solution was poured into 100 mL of water and extracted with DCM (100 mL × 3). The organic phases were combined, washed with saturated brine (100 mL), dried over sodium sulfate, concentrated by rotary evaporation, mixed with silica gel, and subjected to column chromatography to give a yellow solid **15F** (1.9 g, 42.5% yield). MS-ESI: m/z 473.1 $[M+H]^+$.

**Step 5 15F** (1.9 g, 4.01 mmol, 1 eq) was added to a mixed solvent of dioxane (50 mL) and water (5 mL), then **10D** (936 mg, 4.01 mmol, 1 eq) and anhydrous potassium phosphate (2.56 g, 12.04 mmol, 3 eq) were added. The system was purged with argon twice before $Pd(PPh_3)_4$ (200 mg) was added, then purged with argon twice again, heated to 85 °C, and reacted for 3 hours. When LCMS indicated the depletion of the starting materials, the reaction solution was poured into water (150 mL) and extracted with EA (150 mL × 3). The organic phases were combined, dried over sodium sulfate, concentrated at reduced pressure, and subjected to column chromatography (DCM:MeOH = 20:1) to give a dark red solid **15G** (900 mg, 45% yield). MS-ESI: m/z 500.2 $[M+H]^+$.

**Step 6 15G** (700 mg, 1.4 mmol, 1 eq) was added to methanol (20 mL), then sodium borohydride (32 mg, 0.84 mmol, 0.6 eq) was added slowly in portions at room temperature. After the addition, the system was reacted at room temperature for 2 hours. The reaction was quenched with saturated aqueous ammonium chloride (50 mL), and the reaction solution was extracted with DCM (50 mL × 3). The organic phases were combined, dried over sodium sulfate, concentrated at reduced pressure, and subjected to column chromatography (DCM:MeOH = 20:1) to give a pale yellow solid **15H** (200 mg, 22.2% yield). MS-ESI: m/z 502.2 $[M+H]^+$.

**Step 7 15H** (200 mg, 0.31 mmol, 1 eq) was dissolved in methanol (10 mL), then Raney Ni (0.1 g) was added. The reaction system was purged with hydrogen twice and stirred under hydrogen atmosphere for 3 hours at room temperature. When LCMS indicated the depletion of **15H**, the reaction solution was filtered through celite, concentrated by rotary evaporation, triturated in petroleum ether (10 mL) and methanol (2 mL), and filtered to give a yellow solid **15** (28 mg, 14.9% yield).

MS-ESI: m/z 472.2 $[M+H]^+$.

$^1$H NMR (400 MHz, DMSO-$d_6$) δ 8.80 (d, *J* = 2.2 Hz, 1H), 8.52 (d, *J* = 2.0 Hz, 1H), 8.02 (s, 1H), 7.41 (d, *J* = 8.2 Hz, 1H), 7.36 (d, *J* = 1.9 Hz, 1H), 7.29 (dd, *J* = 8.0, 2.0 Hz, 1H), 7.09 (s, 1H), 6.89 (d, *J* = 8.1 Hz, 2H), 6.83 (s, 2H), 6.41 (d, *J* = 8.2 Hz, 2H), 5.37 (t, *J* = 5.8 Hz, 1H), 5.16 (s, 2H), 4.69 - 4.52 (m, 4H), 3.63 (t, *J* = 7.0 Hz, 2H), 2.77 (s, 2H), 1.71 - 1.58 (m, 2H), 0.90 (t, *J* = 7.4 Hz, 3H).

## Compound **16**

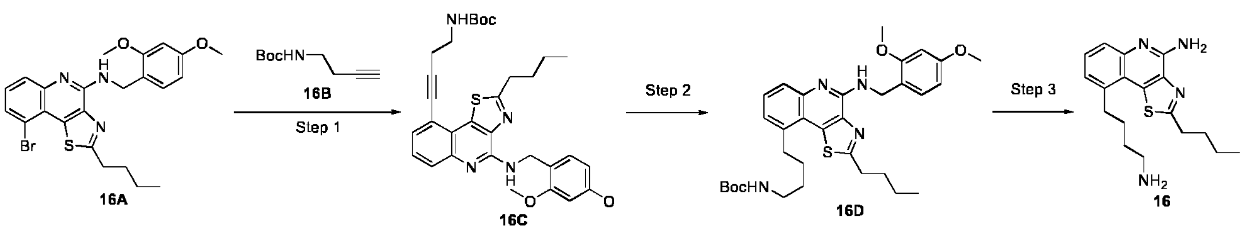


**Step 1** At room temperature, **16A** (0.5 g, 1.03 mmol) and DMF (20 mL) were added into a 100-mL single-neck flask, then **16B** (348 mg, 2.06 mmol), $Pd(PPh_3)_2Cl_2$ (75 mg, 0.103 mmol) and $Cu_2O$ (442 mg, 3.09 mmol) were sequentially added at room temperature. The system was stirred at 110 °C under $N_2$ atmosphere overnight until LCMS indicated the completion of the reaction. 100 mL of water was added to the reaction solution and the mixture was extracted with EA (3 × 100 mL). The organic phase was collected, washed with saturated brine (50 mL), dried over anhydrous sodium sulfate, filtered and concentrated. The crude product was separated by column chromatography (PE/EA = 3/1) to give a yellow oily liquid **16C** (0.3 g, 50.8% yield). MS-ESI: m/z 575.3 $[M+H]^+$.

**Step 2** At room temperature, **16C** (0.3 g, 0.52 mmol) and THF (20 mL) were added into a 100-mL single-neck flask, then Pd/C (50 mg) was added at room temperature. The system was purged with $H_2$ thrice and stirred overnight at 25 °C until LCMS indicated the completion of the reaction. The reaction solution was filtered through celite and rinsed with THF (3 × 20 mL). The filtrate was concentrated by rotary evaporation at reduced pressure, and the crude product was separated by column chromatography (PE/EA = 5/1) to give a yellow oily liquid **16D** (0.2 g, 66.2% yield). MS-ESI: m/z 579.4 $[M+H]^+$.

**Step 3** At room temperature, **16D** (200 mg, 0.35 mmol) was added into a 100-mL single-neck flask, then TFA (5 mL) was slowly added in an ice bath. The system was stirred at 80 °C for 15 min until LCMS indicated the completion of the reaction. The solvent was removed by rotary evaporation at reduced pressure, and the mixture was purified by reversed-phase column chromatography (neutral system) to give a white solid **16** (59.4 mg, 52.6% yield). MS-ESI: m/z 329.2$[M+H]^+$.

$^1$H NMR (400 MHz, Methanol-$d_4$) δ 7.58 (dd, *J* = 8.4, 1.3 Hz, 1H), 7.47 (dd, *J* = 8.4, 7.2 Hz, 1H), 7.22 (dd, *J* = 7.3, 1.3 Hz, 1H), 3.24 - 3.17 (m, 2H), 3.17 - 3.10 (m, 2H), 2.88 - 2.81 (m, 2H), 1.97 - 1.87 (m, 2H), 1.83 - 1.67 (m, 4H), 1.58 - 1.46 (m, 2H), 1.02 (t, *J* = 7.4 Hz, 3H).

Compound **17**

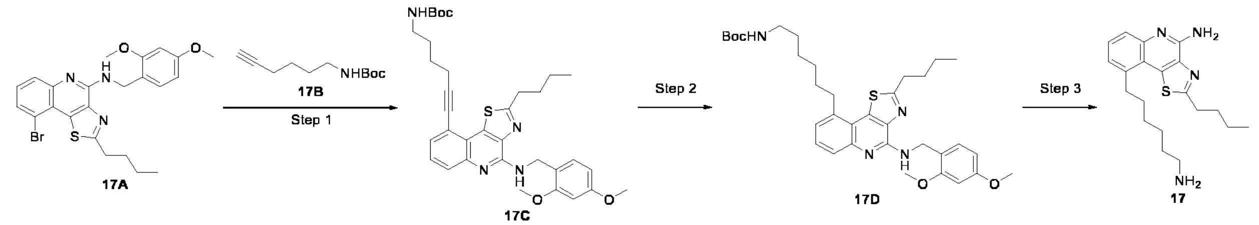


**Step 1** At room temperature, **17A** (0.5 g, 1.03 mmol) and DMF (20 mL) were added into a 100-mL single-neck flask, then **17B** (203 mg, 2.06 mmol), $Pd(PPh_3)_2Cl_2$ (75 mg, 0.103 mmol) and $Cu_2O$ (442 mg, 3.09 mmol) were sequentially added at room temperature. The system was stirred at 110 °C under $N_2$ atmosphere overnight until LCMS indicated the completion of the reaction. 100 mL of water was added to the reaction solution and the mixture was extracted with EA (3 × 100 mL). The organic phase was collected, washed with saturated brine (50 mL), dried over anhydrous sodium sulfate, filtered and concentrated. The crude product was separated by column chromatography (PE/EA = 3/1) to give a yellow oily liquid **17C** (0.3 g, 48.4% yield). MS-ESI: m/z 603.4 $[M+H]^+$.

**Step 2** At room temperature, **17C** (0.3 g, 0.50 mmol) and THF (20 mL) were added into a 100-mL single-neck flask, then Pd/C (50 mg) was added at room temperature. The system was purged with $H_2$ thrice and stirred overnight at 25 °C until LCMS indicated the completion of the reaction. The reaction solution was filtered through celite and rinsed with THF (3 × 20 mL). The filtrate was concentrated by rotary evaporation at reduced pressure, and the crude product was separated by column chromatography (PE/EA = 5/1) to give a yellow oily liquid **17D** (0.2 g, 66.2% yield). MS-ESI: m/z 607.4 $[M+H]^+$.

**Step 3** At room temperature, **17D** (200 mg, 0.33 mmol) was added into a 100-mL single-neck flask, then TFA (5 mL) was slowly added in an ice bath. The system was stirred at 80 °C for 15 min until LCMS indicated the completion of the reaction. The reaction mixture was concentrated by rotary evaporation at reduced pressure and purified by reversed-phase column chromatography (neutral system) to give a white solid **17** (51.4 mg, 43.7% yield). MS-ESI: m/z 357.2$[M+H]^+$.

$^1H$ NMR (400 MHz, Methanol-$d_4$) δ 7.58 (dd, *J* = 8.3, 1.3 Hz, 1H), 7.46 (dd, *J* = 8.4, 7.2 Hz, 1H), 7.20 (dd, *J* = 7.3, 1.3 Hz, 1H), 3.25 - 3.18 (m, 2H), 3.13 - 3.07 (m, 2H), 2.78 - 2.71 (m, 2H), 1.98 - 1.87 (m, 2H), 1.78 - 1.67 (m, 2H), 1.62 - 1.39 (m, 8H), 1.02 (t, *J* = 7.4 Hz, 3H).

Compound **18**

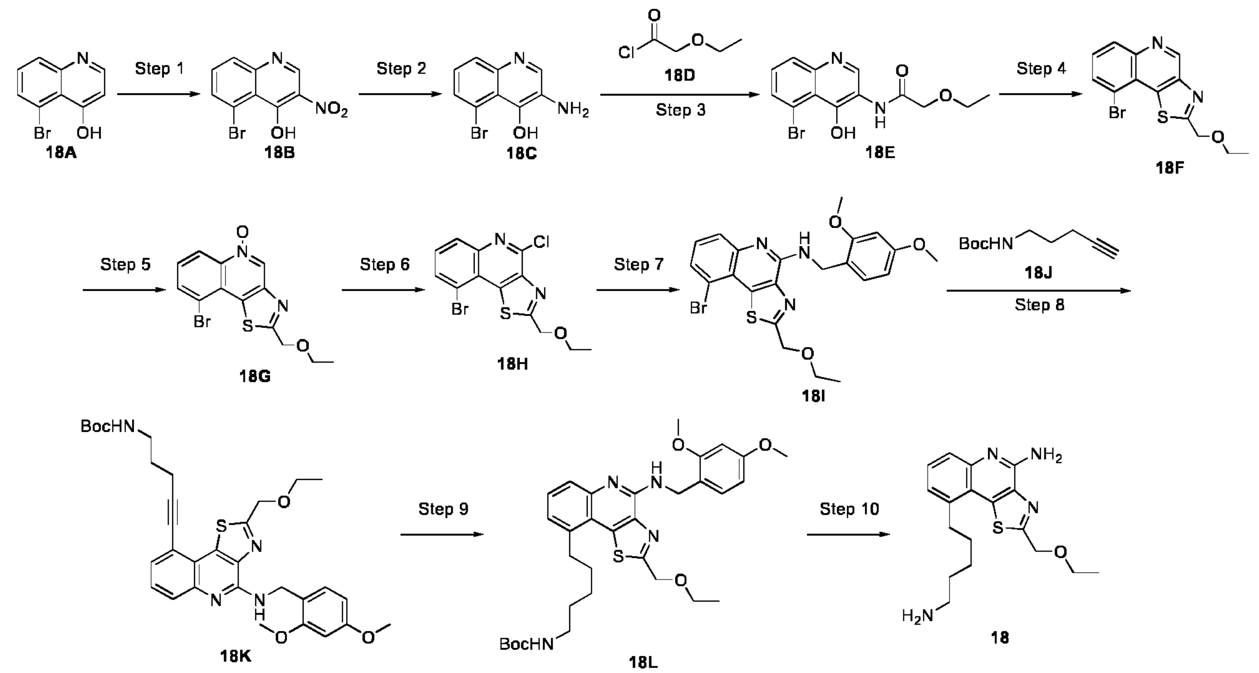


**Step 1** At room temperature, **18A** (10 g, 44.8 mmol) and propionic acid (200 mL) were added to a 250-mL three-necked flask. The reaction system was heated to 125 °C, then $HNO_3$ (8.6 g, 136.6 mmol) was added dropwise with the internal temperature controlled at 120-130 °C. The reaction system was stirred at 125 °C for 1 hour until LCMS

indicated the completion of the reaction. The reaction solution was cooled to room temperature, slowly poured into ethanol (200 mL) and filtered. The filter cake was rinsed sequentially with ethanol (100 mL), water (100 mL), and ethanol (100 mL). The filter cake was collected and dried to give a yellow solid **18B** (7 g, 58% yield). MS-ESI: m/z 269.0[M+H]$^+$.

**Step 2** At room temperature, **18B** (7 g, 26.1 mmol) was added to a 250-mL single-neck flask and dissolved in EtOH (100 mL) and $H_2O$ (20 mL), then $NH_4Cl$ (4.23 g, 78.3 mmol) and Fe powder (7.31 g, 130.5 mmol) were added. The reaction system was stirred at 80 °C for 2 hours until LCMS indicated the completion of the reaction. The reaction solution was filtered before cooling. Ethyl acetate (200 mL) and water (100 mL) were added to the filtrate, and the organic phase was separated. The aqueous phase was extracted with ethyl acetate (3 × 100 mL), and the organic phase was collected, washed with saturated brine (100 mL), dried over anhydrous sodium sulfate, filtered, and concentrated by rotary evaporation. The crude product was purified by column chromatography (DCM/MeOH = 10:1) to give a yellow solid **18C** (4.5 g, 72% yield). MS-ESI: m/z 239.0[M+H]$^+$. **Step 3 18C** (2.2 g, 9.2 mmol) and DCM (100 mL) were added into a 250-mL three-necked flask, then TEA (1.4 g, 13.8 mmol) was added. **18D** (1.35 g, 11.04 mmol) was added slowly in an ice bath and the reaction system was stirred for 1 hour at 25 °C until LCMS indicated the completion of the reaction. The reaction solution was poured into water (20 mL) and extracted with EA (3 × 50 mL). The organic phase was collected, washed with saturated brine (20 mL), dried over anhydrous sodium sulfate, filtered, and concentrated. The crude product was separated by column chromatography (PE/EA = 3/1) to give a yellow solid **18E** (1.6 g, 53.5% yield). MS-ESI: m/z 325.0[M+H]$^+$.

**Step 4** At room temperature, **18E** (2.8 g, 8.6 mmol) and pyridine (120 mL) were added into a 250-mL single-neck flask, then $P_2S_5$ (16.4 g, 86 mmol) was added. The reaction system was stirred at 120 °C overnight until LCMS indicated the completion of the reaction. The reaction solution was concentrated by rotary evaporation to remove the solvent, poured into water (100 mL), and extracted with EA (3 × 100 mL). The organic phase was collected, washed with saturated brine (100 mL), dried over anhydrous sodium sulfate, filtered, and concentrated. The crude product was separated by chromatography (PE/EA = 10/1) to give a yellow solid **18F** (0.5 g, 18.5% yield). MS-ESI: m/z 323.0[M+H]$^+$.

**Step 5** At room temperature, **18F** (0.5 g, 1.55 mmol) and $CHCl_3$ (10 mL) were added into a 250-mL single-neck flask, then m-CPBA (0.53 g, 3.1 mmol) was added slowly in an ice bath. The reaction system was stirred at 25 °C for 2 hours until LCMS indicated the completion of the reaction. The reaction mixture was poured into DCM (50 mL). The mixture was sequentially washed with 5% sodium thiosulfate (50 mL), saturated sodium bicarbonate (50 mL), and saturated brine (50 mL), dried over anhydrous sodium sulfate, filtered, and concentrated. The crude product was separated by column chromatography (PE/EA = 3/1) to give a yellow oily liquid **18G** (0.43 g, 82.6% yield). MS-ESI: m/z 339.0[M+H]$^+$.

**Step 6 18G** (0.43 g, 1.27 mmol) was added to a 50-mL single-neck flask, then $POCl_3$ (5.1 g, 33.1 mmol) was added slowly in an ice bath. DIPEA (0.43 g, 3.31 mmol) was slowly and dropwise added with the internal temperature controlled at 15 °C. The system was stirred at 100 °C for 2 hours until LCMS indicated the completion of the reaction. The reaction solution was cooled to room temperature, concentrated by rotary evaporation at reduced pressure to remove the solvent, and poured slowly into ice water (50 mL). The mixture was adjusted to pH 9 with solid potassium carbonate and the aqueous phase was extracted with ethyl acetate (3 × 30 mL). The organic phase was collected, washed with saturated brine (50 mL), dried over anhydrous sodium sulfate, filtered, concentrated by rotary evaporation, and purified by column chromatography (PE/EA = 10: 1) to give a yellow oil **18H** (0.32 g, 71.1% yield). MS-ESI: m/z 357.0[M+H]$^+$.

**Step 7** At room temperature, **18H** (0.32 g, 0.9 mmol) and toluene (5 mL) were added into a 50-mL single-neck flask, then **2,4-dimethoxybenzylamine** (1.5 g, 9 mmol) and 2-chlorobenzoic acid (0.14 g, 0.9 mmol) were added. The reaction system was stirred at 120 °C overnight until LCMS indicated the completion of the reaction. The solvent was removed by rotary evaporation at reduced pressure. 10 mL of water was added, and the mixture was extracted with EA (3 × 30 mL). The organic phase was collected, washed with saturated brine (30 mL), dried over anhydrous sodium sulfate, filtered, and concentrated. The crude product was purified by reversed-phase column chromatography (neutral system) to give a yellow solid **18I** (0.14 g, 32.5% yield). MS-ESI: m/z 488.0[M+H]$^+$.

**Step 8** At room temperature, **18I** (0.14 g, 0.28 mmol) and DMF (5 mL) were added into a 50-mL single-neck flask, then **18J** (0.102 g, 0.56 mmol), $Pd(PPh_3)_2Cl_2$ (0.02 g, 0.028 mmol), and $Cu_2O$ (0.12 g, 0.84 mmol) were sequentially added at room temperature. The system was stirred at 110 °C under $N_2$ atmosphere overnight until LCMS indicated the completion of the reaction. 20 mL of water was added to the reaction solution and the mixture was extracted with EA (3 × 30 mL). The organic phase was collected, washed with saturated brine (20 mL), dried over anhydrous sodium sulfate, filtered and concentrated. The crude product was separated by column chromatography (PE/EA = 3/1) to give a yellow oily liquid **18K** (0.12 g, 70.5% yield). MS-ESI: m/z 591.2[M+H]$^+$.

**Step 9** At room temperature, **18K** (0.12 g, 0.2 mmol) and THF (10 mL) were added into a 50-mL single-neck flask, then Pd/C (50 mg) was added at room temperature. The system was purged with $H_2$ thrice and stirred at 25 °C overnight until LCMS indicated the completion of the reaction. The reaction solution was filtered through celite and rinsed with THF (3 × 20 mL). The filtrate was concentrated by rotary evaporation at reduced pressure, and the crude product was separated by column chromatography (PE/EA = 5/1) to give a yellow oily liquid **18L** (0.1 g, 83.3% yield). MS-ESI: m/z 595.3[M+H]$^+$.

**Step 10** At room temperature, **18L** (0.1 g, 0.17 mmol) was added into a 50-mL single-neck flask, then TFA (3 mL) was slowly added in an ice bath. The system was stirred at 80 °C for 15 min until LCMS indicated the completion of the reaction. The solvent was removed by rotary evaporation at reduced pressure, and the mixture was purified by reversed-phase column chromatography (neutral system) to give a white solid **18** (18 mg, 31.1% yield). MS-ESI: m/z 345.2[M+H]+.

$^1$H NMR (400 MHz, Methanol-$d_4$) δ 7.59 (dd, *J* = 8.3, 1.3 Hz, 1H), 7.49 (dd, *J* = 8.4, 7.2 Hz, 1H), 7.23 (dd, *J* = 7.2, 1.3 Hz, 1H), 4.98 (s, 2H), 3.77 (q, *J* = 7.0 Hz, 2H), 3.19 - 3.11 (m, 2H), 2.78 (t, *J* = 6.9 Hz, 2H), 1.82 - 1.70 (m, 2H), 1.69 - 1.55 (m, 4H), 1.33 (t, *J* = 7.0 Hz, 3H).

## Compound **19**

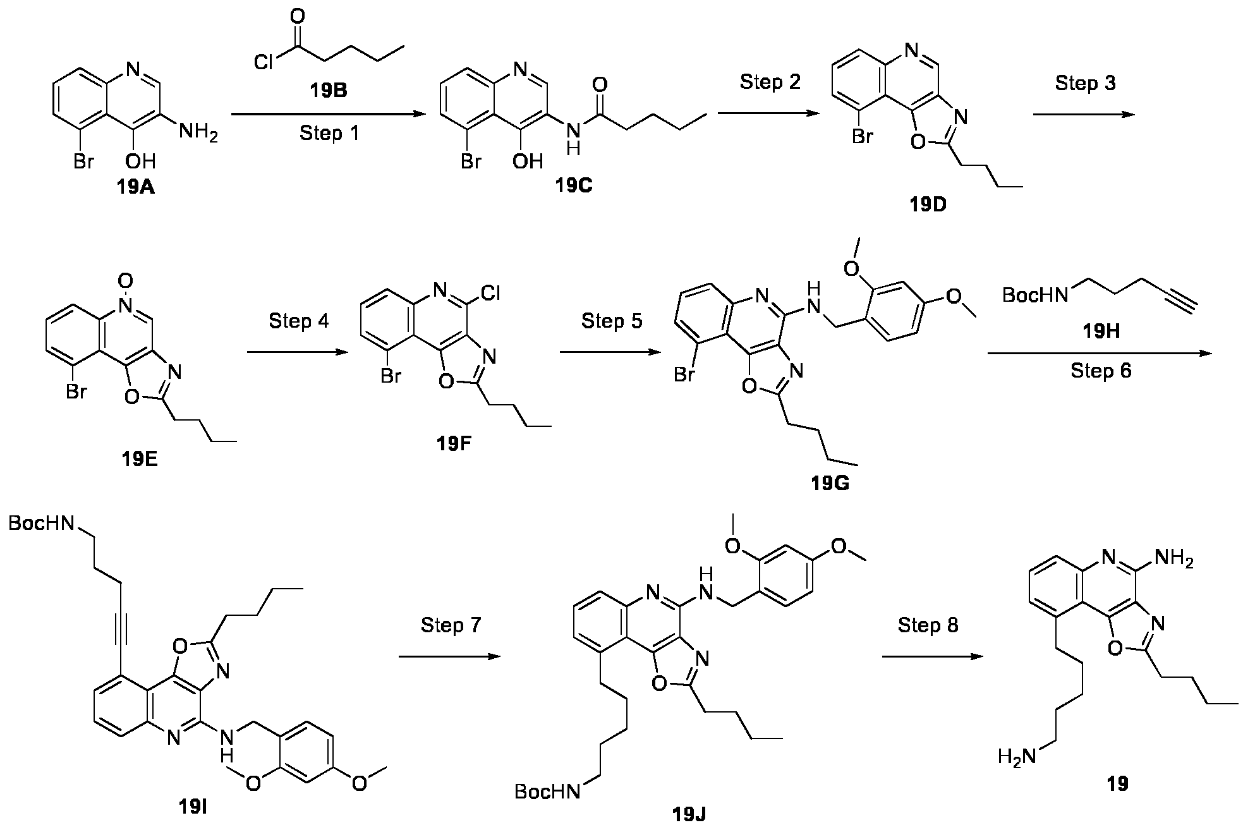


**Step 1** **19A** (2 g, 8.4 mmol) and DCM (100 mL) were added into a 250-mL three-necked flask, then TEA (1.27 g, 12.6 mmol) was added. **19B** (1.21 g, 10.08 mmol) was added slowly in an ice bath and the reaction system was stirred for 1 hour at 25 °C until LCMS indicated the completion of the reaction. The reaction solution was poured into water (20 mL) and extracted with EA (3 × 50 mL). The organic phase was collected, washed with saturated brine (20 mL), dried over anhydrous sodium sulfate, filtered, and concentrated. The crude product was separated by column chromatography (PE/EA= 3/1) to give a yellow solid **19C** (1.4 g, 51.8% yield). MS-ESI: m/z 323.0[M+H]+.

**Step 2** At room temperature, **19C** (1.4 g, 4.3 mmol) and pyridine (60 mL) were added into a 100-mL single-neck flask, then $P_2O_5$ (6.1 g, 43 mmol) was added. The reaction system was stirred at 120 °C overnight until LCMS indicated the completion of the reaction. The reaction solution was concentrated by rotary evaporation to remove the solvent, poured into water (100 mL), and extracted with EA (3 × 50 mL). The organic phase was collected, washed with saturated brine (50 mL), dried over anhydrous sodium sulfate, filtered, and concentrated. The crude product was separated by chromatography (PE/EA = 10/1) to give a yellow solid **19D** (0.5 g, 37.8% yield). MS-ESI: m/z 305.1[M+H]+.

**Step 3** At room temperature, **19D** (0.5 g, 1.6 mmol) and $CHCl_3$ (10 mL) were added into a 50-mL single-neck flask, then **m-CPBA** (0.65 g, 3.2 mmol) was added slowly in an ice bath. The reaction system was stirred at 25 °C for 2 hours until LCMS indicated the completion of the reaction. The reaction mixture was poured into DCM (50 mL). The mixture was sequentially washed with 5% sodium thiosulfate (50 mL), saturated sodium bicarbonate (50 mL), and saturated brine (50 mL), dried over anhydrous sodium sulfate, filtered, and concentrated. The crude product was separated by column chromatography (PE/EA = 3/1) to give a yellow oily liquid **19E** (0.4 g, 76% yield). MS-ESI: m/z 321.0[M+H]+.

**Step 4 19E** (0.4 g, 1.25 mmol) was added to a 250-mL three-necked flask, then $POCl_3$ (5.75 g, 37.5 mmol) was added slowly in an ice bath. DIPEA (0.48 g, 3.75 mmol) was slowly and dropwise added with the internal temperature controlled at 15 °C. The system was stirred at 100 °C for 2 hours until LCMS indicated the completion of the reaction. The reaction solution was cooled to room temperature, concentrated by rotary evaporation at reduced pressure to remove the solvent, and poured slowly into ice water (50 mL). The mixture was adjusted to pH 9 with solid potassium carbonate and the aqueous phase was extracted with ethyl acetate (3 × 30 mL). The organic phase was collected, washed with saturated brine (50 mL), dried over anhydrous sodium sulfate, filtered, concentrated by rotary evaporation, and purified by column chromatography (PE/EA = 10:1) to give a yellow oily liquid **19F** (0.35 g, 83.3% yield). MS-ESI: m/z 339.0$[M+H]^+$.

**Step 5** At room temperature, **19F** (0.35 g, 1.04 mmol) and toluene (5 mL) were added into a 50-mL single-neck flask, then **2,4-dimethoxybenzylamine** (1.74 g, 10.4 mmol) and 2-chlorobenzoic acid (0.16 g, 1.04 mmol) were added. The reaction system was stirred at 120 °C overnight until LCMS indicated the completion of the reaction. The solvent was removed by rotary evaporation at reduced pressure. 10 mL of water was added, and the mixture was extracted with EA (3 × 30 mL). The organic phase was collected, washed with saturated brine (30 mL), dried over anhydrous sodium sulfate, filtered, and concentrated. The crude product was purified by reversed-phase column chromatography (neutral system) to give a yellow solid **19G** (0.2 g, 41.1% yield). MS-ESI: m/z 470.0$[M+H]^+$.

**Step 6** At room temperature, **19G** (0.2 g, 0.43 mmol) and DMF (5 mL) were added into a 50-mL single-neck flask, then **19H** (0.16 g, 0.86 mmol), $Pd(PPh_3)_2Cl_2$ (0.03 g, 0.043 mmol), and $Cu_2O$ (0.18 g, 1.29 mmol) were sequentially added at room temperature. The system was stirred at 110 °C under $N_2$ atmosphere overnight until LCMS indicated the completion of the reaction. 20 mL of water was added to the reaction solution and the mixture was extracted with EA (3 × 30 mL). The organic phase was collected, washed with saturated brine (20 mL), dried over anhydrous sodium sulfate, filtered and concentrated. The crude product was separated by column chromatography (PE/EA = 3/1) to give a yellow oily liquid **19I** (0.12 g, 49.4% yield). MS-ESI: m/z 573.4$[M+H]^+$.

**Step 7** At room temperature, **19I** (0.12 g, 0.21 mmol) and THF (10 mL) were added into a 50-mL single-neck flask, then Pd/C (50 mg) was added at room temperature. The system was purged with $H_2$ thrice and stirred at 25 °C overnight until LCMS indicated the completion of the reaction. The reaction solution was filtered through celite and rinsed with THF (3 × 20 mL). The filtrate was concentrated by rotary evaporation at reduced pressure, and the crude product was separated by column chromatography (PE/EA = 5/1) to give a yellow oily liquid **19J** (96 mg, 80% yield). MS-ESI: m/z 577.4$[M+H]^+$.

**Step 8** At room temperature, **19J** (0.096 g, 0.17 mmol) was added into a 50-mL single-neck flask, then TFA (3 mL) was slowly added in an ice bath. The system was stirred at 80 °C for 15 min until LCMS indicated the completion of the reaction. The solvent was removed by rotary evaporation at reduced pressure, and the mixture was purified by reversed-phase column chromatography (neutral system) to give a white solid **19** (22 mg, 40.5% yield). MS-ESI: m/z 327.3 $[M+H]^+$.

$^1$H NMR (400 MHz, Methanol-$d_4$) δ 7.54 (dd, *J* = 8.5, 1.2 Hz, 1H), 7.45 (dd, *J* = 8.5, 7.1 Hz, 1H), 7.16 (d, *J* = 7.1 Hz, 1H), 3.25 - 3.17 (m, 2H), 3.08 (t, *J* = 7.4 Hz, 2H), 2.69 (t, *J* = 7.0 Hz, 2H), 1.99 - 1.88 (m, 2H), 1.79 - 1.68 (m, 2H), 1.64 - 1.44 (m, 6H), 1.02 (t, *J* = 7.4 Hz, 3H).

Compound **20**

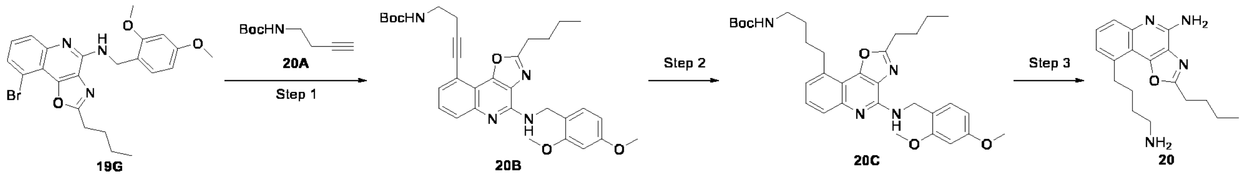


**Step 1** At room temperature, **19G** (0.28 g, 0.6 mmol) and DMF (6 mL) were added into a 50-mL single-neck flask, then **20A** (0.254 g, 1.5 mmol), $Pd(PPh_3)_2Cl_2$ (0.042 g, 0.06 mmol), and $Cu_2O$ (0.257 g, 1.8 mmol) were sequentially added at room temperature. The system was stirred at 110 °C under $N_2$ atmosphere overnight until LCMS indicated the completion of the reaction. 20 mL of water was added to the reaction solution and the mixture was extracted with EA (3 × 30 mL). The organic phase was collected, washed with saturated brine (20 mL), dried over anhydrous sodium sulfate, filtered and concentrated. The crude product was separated by column chromatography (PE/EA = 3/1) to give a yellow oily liquid **20B** (140 mg, 42% yield). MS-ESI: m/z 559.2$[M+H]^+$.

**Step 2** At room temperature, **20B** (0.14 g, 0.25 mmol) and THF (10 mL) were added into a 50-mL single-neck flask, then Pd/C (50 mg) was added at room temperature. The system was purged with $H_2$ thrice and stirred at 25 °C overnight until LCMS indicated the depletion of the starting materials. The reaction solution was filtered through celite and rinsed with THF (3 × 20 mL). The filtrate was concentrated by rotary evaporation at reduced pressure, and the crude product was separated by column chromatography (PE/EA = 5/1) to give a yellow oily liquid **20C** (120 mg, 85%

yield). MS-ESI: m/z 563.4[M+H]+.

**Step 3** At room temperature, **20C** (0.12 g, 0.21 mmol) was added into a 50-mL single-neck flask, then TFA (4 mL) was slowly added in an ice bath. The system was stirred at 80 °C for 15 min until LCMS indicated the completion of the reaction. The reaction solution was concentrated by rotary evaporation at reduced pressure and subjected to preparative reversed-phase chromatography (TFA system) to give a white solid **20** (4.2 mg, 6.3% yield). MS-ESI: m/z 313.2[M+H]+.

$^1$H NMR (400 MHz, Methanol-$d_4$) δ 7.76 - 7.66 (m, 2H), 7.47 (dd, *J* = 6.9, 1.5 Hz, 1H), 3.36 - 3.32 (m, 2H), 3.15 (t, *J* = 7.5 Hz, 2H), 3.04 - 2.95 (m, 2H), 2.02 - 1.92 (m, 2H), 1.90 - 1.77 (m, 4H), 1.61 - 1.49 (m, 2H), 1.03 (t, *J* = 7.4 Hz, 3H).

## Compound 30

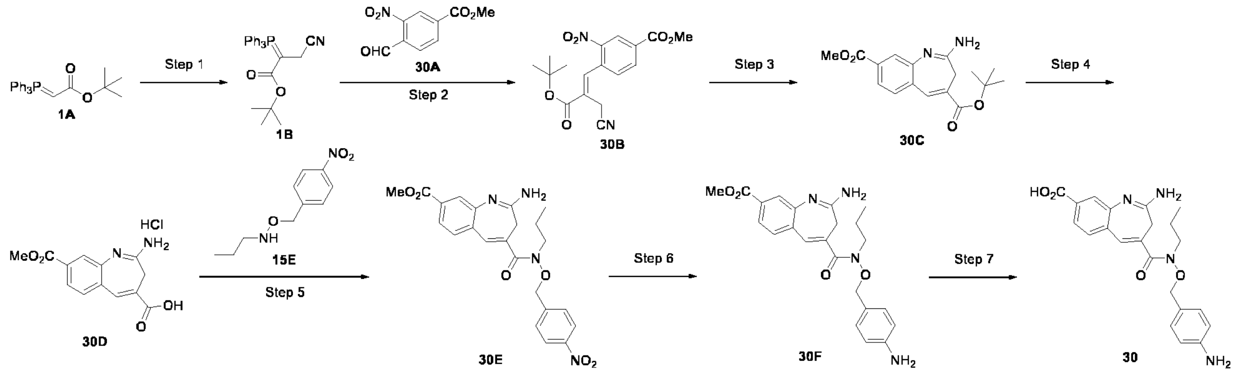


**Step 1** Compound 1A (533.5 g, 1.42 mol) was added to a 5-L three-necked flask at 25 °C, then EA (1500 mL) was added. Bromoacetonitrile (10 g, 833.3 mmol) was added with mechanical stirring, and the mixture was heated to 77 °C and reacted overnight. When LCMS indicated 40% of bromoacetonitrile was remained, the reaction solution was cooled to 25 °C and filtered. The filter cake was washed with EA (500 mL), and the filtrate was concentrated at reduced pressure to give a yellow solid product, compound **1B** (148 g, 45.8% yield). MS-ESI: m/z 416.02 [M+H]+.

**Step 2** Compound **1B** (148 g, 356.25 mmol) was added to a 3-L three-necked flask at 25 °C, then toluene (800 mL) was added. Compound 30A (80 g, 347.8 mmol) was added with stirring, and the mixture was reacted at room temperature overnight. When TLC indicated the depletion of compound **30A**, the reaction solution was concentrated at reduced pressure, mixed with silica gel, and purified by column chromatography (PE/EA = 5/1) to give a yellow solid compound **30B** (98 g, 72.5% yield). MS-ESI: m/z 347.0 [M+H]+.

**Step 3** Compound **30C** (98 g, 266.88 mmol) was added into a 3-L three-necked flask at 25 °C, then AcOH (800 mL) was added. The internal temperature was raised to 60 °C, then Fe (74.52 g, 1334.4 mmol) was slowly added in portions with stirring. After the addition, the system was heated to 85 °C and reacted for 3 hours. When TLC indicated the depletion of the starting materials, the reaction solution was cooled to about 60 °C, diluted with DCM (1000 mL), and filtered before cooling. The filter cake was washed with DCM (1000 mL), and the filtrate was concentrated at reduced pressure. EA (800 mL) was added, and the mixture was adjusted to pH 9 with saturated sodium bicarbonate solution and extracted with EA (800 mL × 3). The organic phase was concentrated at reduced pressure, and purified by column chromatography (PE/EA = 3/1) to give a pale yellow solid compound **30C** (68 g, 79.9% yield).

**Step 4** 4 N HCl/dioxane (700 mL) was added to a 2-L single-neck flask at 25 °C, then compound **30C** (68 g, 132.28 mmol) was added at 0 °C. The system was reacted overnight at room temperature. When LCMS indicated the depletion of the starting materials, the reaction solution was concentrated at reduced pressure to give an off-white solid compound **30D** (59 g, 92% yield). MS-ESI: m/z 261.0 [M+H]+.

$^1$H NMR (400 MHz, DMSO) δ 13.99 - 12.29 (m, 2H), 10.03 (s, 1H), 9.09 (s, 1H), 7.97 (s, 1H), 7.91 (s, 1H), 7.90 - 7.85 (m, 1H), 7.82 - 7.80 (d, J = 8.1 Hz, 1H), 3.91 (s, 3H), 3.49 (s, 2H).

**Step 5** Compound **30D** (3 g, 10.11 mmol, 1 eq) and compound **15E** (2.62 g, 10.62 mmol, 1.05 eq) were added to a mixed solvent of DMA (30 mL) and DCM (30 mL). The system was purged with argon twice before EDCI (7.75 g, 40.44 mmol, 4 eq) was added in one portion, then purged with argon twice again, warmed to room temperature, and reacted overnight. When LCMS indicated the depletion of the starting materials, the reaction solution was poured into 100 mL of water and extracted thrice with DCM (100 mL × 3). The organic phases were combined, washed with saturated brine (100 mL), and dried over anhydrous sodium sulfate. After filtration, the filtrate was concentrated, mixed with silica gel, and purified by column chromatography to give a yellow solid **30E** (1.7 g, 38% yield). MS-ESI: m/z 453.2 [M+H]+.

**Step 6** Compound **30E** (700 mg, 1.55 mmol, 1 eq) was dissolved in methanol (10 mL), then Raney nickel (0.1 g) was added. The system was purged twice with hydrogen and stirred at room temperature for 3 hours. When LCMS demonstrated remarkable related peaks of compound 30F, the mixture was filtered through celite, and the filtrate was

directly used in the next reaction. MS-ESI: m/z 423.2 [M+H]+.

**Step 7** THF (10 mL) and water (10 mL) were added to the methanol reaction solution from the previous step, then lithium hydroxide monohydrate (208 mg, 4.97 mmol, 3 eq) was added. The system was stirred at room temperature for 3 hours. When LCMS indicated the depletion of the starting materials, the mixture was concentrated at reduced pressure and adjusted to pH 5-6 with 1 N hydrochloric acid. The resultant mixture was filtered, and the yellow filter cake was subjected to preparative HPLC to give a white solid **30** (8 mg, 1.3% yield over the two steps). MS-ESI: m/z 409.2 [M+H]+.

$^1$H NMR (400 MHz, DMSO) δ 7.69 (s, 1H), 7.52 (s, 1H), 7.42 - 7.40 (d, J = 7.4 Hz, 1H), 7.09 (s, 1H), 6.90 - 6.88 (d, J = 8.0 Hz, 2H), 6.42 - 6.40 (d, J = 8.2 Hz, 2H), 5.19 (s, 2H), 4.61 (s, 2H), 3.68 - 3.64 (t, J = 6.8 Hz, 2H), 2.82 (s, 2H), 1.69 - 1.64 (dd, J = 14.6, 7.6 Hz, 2H), 0.94 - 0.90 (t, J = 7.3 Hz, 3H).

## Compound 31

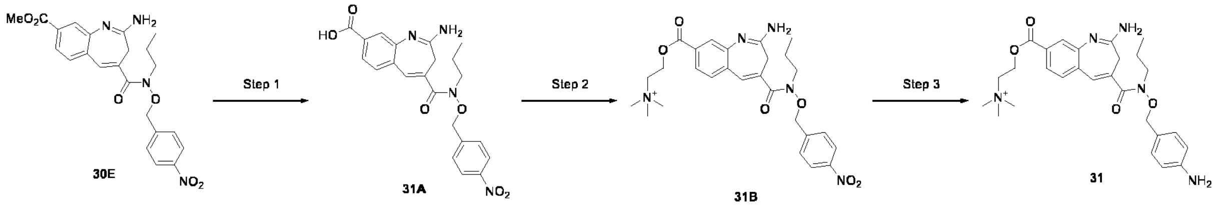


**Step 1** Compound **30E** (2.4 g, 5.68 mmol, 1 eq) was added to a mixed solution of methanol (20 mL), THF (20 mL) and water (20 mL). The temperature was controlled at 0 °C in an ice-water bath, and lithium hydroxide monohydrate (715 mg, 17.04 mmol, 3 eq) was added. The system was reacted for 3 hours in the ice-water bath. When LCMS indicated the depletion of the starting materials, the reaction solution was poured into 50 mL of water, adjusted to pH 5-6 with 1 N hydrochloric acid, and extracted thrice with ethyl acetate (50 mL × 3). The organic phases were combined and dried over anhydrous sodium sulfate. After concentration at reduced pressure, the mixture was triturated in acetonitrile (10 mL) and filtered to give a yellow solid **31A** (1 g, 43% yield). MS-ESI: m/z 439.1 [M+H]+.

**Step 2** Compound **31A** (400 mg, 0.912 mmol, 1 eq) was dissolved in DMF (8 mL), then HATU (382 mg, 1.00 mmol, 1.1 eq) and DIEA (484 mg, 3.74 mmol, 4.1 eq) were added while controlling the temperature at 0 °C. The system was stirred for 10 min while maintaining the temperature at 0 °C before choline chloride (140 mg, 1.00 mmol) was added in one portion. The system was then warmed to room temperature and stirred overnight. When LCMS indicated the production of product **31B**, the mixture was directly used in the next reaction without treatment. MS-ESI: m/z 524.3 [M]+.

**Step 3** Raney nickel (100 mg) was added to the reaction solution from the previous step. The system was purged with hydrogen twice and stirred at room temperature for 3 hours. When LCMS indicated the depletion of the starting materials and the production of the target product, the mixture was filtered, and the filtrate was subjected to preparative HPLC to give a white solid **31** (6 mg, 1.3% yield for the two steps). MS-ESI: m/z 494.1 [M]+.

$^1$H NMR (400 MHz, DMSO) δ 7.66 (d, J = 1.6 Hz, 1H), 7.51 - 7.49 (dd, J = 8.1, 1.7 Hz, 1H), 7.45 - 7.42 (d, J = 8.3 Hz, 1H), 7.08 (s, 1H), 6.94 (s, 1H), 6.86 - 6.84 (d, J = 8.2 Hz, 2H), 6.40 - 6.38 (d, J = 8.2 Hz, 2H), 5.17 (s, 2H), 4.70 (s, 2H), 4.58 (s, 2H), 3.85 - 3.78 (m, 2H), 3.65 - 3.61 (d, J = 7.1 Hz, 2H), 3.20 (s, 9H), 3.10 (s, 1H), 2.73 (s, 2H), 1.67 - 1.61 (dd, J = 14.0, 7.0 Hz, 2H), 0.92 - 0.88 (t, J = 7.4 Hz, 4H).

## Compound 32

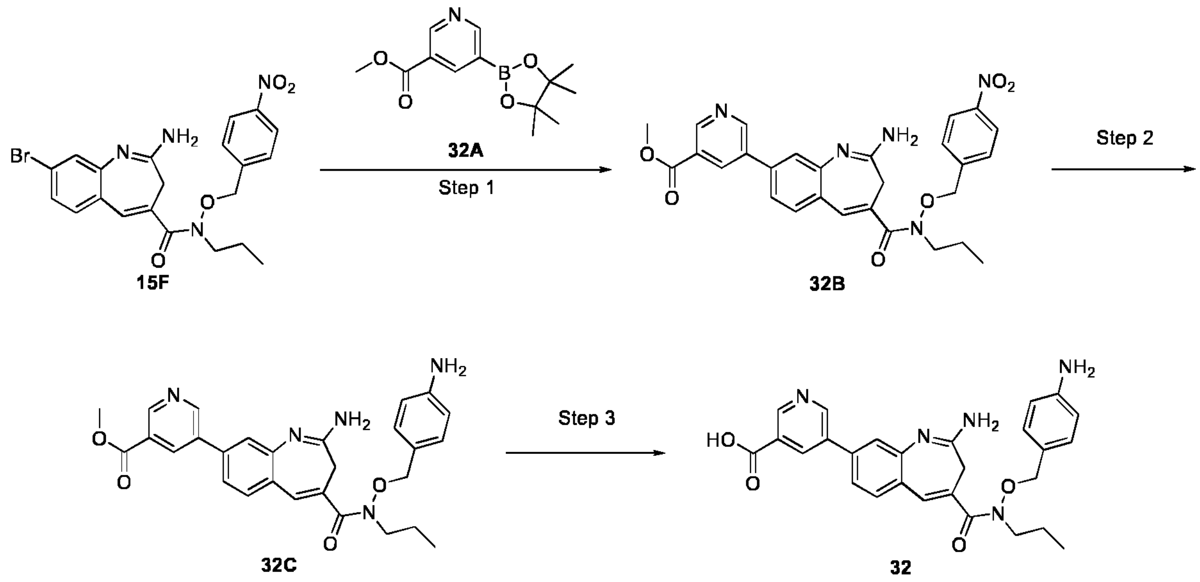


15F 32B 32C 32

**Step 1** Compound **15F** (1.0 g, 2.11 mmol, 1 eq) was added to a mixed solvent of 1,4-dioxane (20 mL) and water (2 mL), then compound **32A** (556 mg, 2.11 mmol, 1 eq) and anhydrous potassium phosphate (1.35 g, 6.34 mmol, 3 eq) were added. The system was purged with argon twice before $Pd(PPh_3)_4$ (100 mg) was added, then purged with argon twice again, heated to 90 °C, and reacted for 2 hours. When LCMS indicated the depletion of the starting materials, the reaction solution was poured into 100 mL of water and extracted thrice with ethyl acetate (100 mL × 3). The organic phases were combined and dried over anhydrous sodium sulfate. The mixture was concentrated at reduced pressure and purified by column chromatography (DCM:MeOH = 20:1) to give a yellow solid **32B** (400 mg, 36% yield). MS-ESI: m/z 530.2 $[M+H]^+$.

**Step 2** Compound **32B** (400 mg, 0.756 mmol, 1 eq) was dissolved in methanol (10 mL), then Raney nickel (0.1 g) was added. The system was purged twice with hydrogen and stirred at room temperature for 3 hours. When LCMS demonstrated remarkable product peaks, the mixture was filtered through celite, and the filtrate was directly used in the next reaction. MS-ESI: m/z 500.2 $[M+H]^+$.

**Step 3** THF (10 mL) and water (10 mL) were added to the reaction solution from the previous step, then lithium hydroxide monohydrate (91 mg, 2.27 mmol, 3 eq) was added. The system was stirred at room temperature for 3 hours. When LCMS indicated the depletion of the starting materials, the mixture was concentrated at reduced pressure and adjusted to pH 5-6 with 1 N hydrochloric acid. The resultant mixture was filtered, and the yellow filter cake was subjected to preparative HPLC to give a white solid **32** (21 mg, 5.7% yield over the two steps). MS-ESI: m/z 486.2 $[M+H]^+$.

$^1H$ NMR (400 MHz, DMSO) δ 12.87 - 11.32 (m, 1H), 9.87 (s, 1H), 9.26 (s, 1H), 9.20 (d, J = 2.2 Hz, 1H), 9.13 - 9.12 (d, J = 1.8 Hz, 1H), 8.55 - 8.54 (t, J = 2.0 Hz, 1H), 7.88 - 7.86 (d, J = 8.2 Hz, 1H), 7.79 (s, 1H), 7.70 - 7.68 (d, J = 8.3 Hz, 1H), 7.22 (s, 1H), 7.01 - 6.99 (d, J = 8.2 Hz, 2H), 6.59 - 6.57 (d, J = 8.1 Hz, 2H), 4.70 (s, 2H), 3.70 (s, 2H), 3.26 (s, 2H), 1.73 - 1.60 (m, 2H), 0.94 - 0.91 (t, J = 7.4 Hz, 3H).

## Compound 33

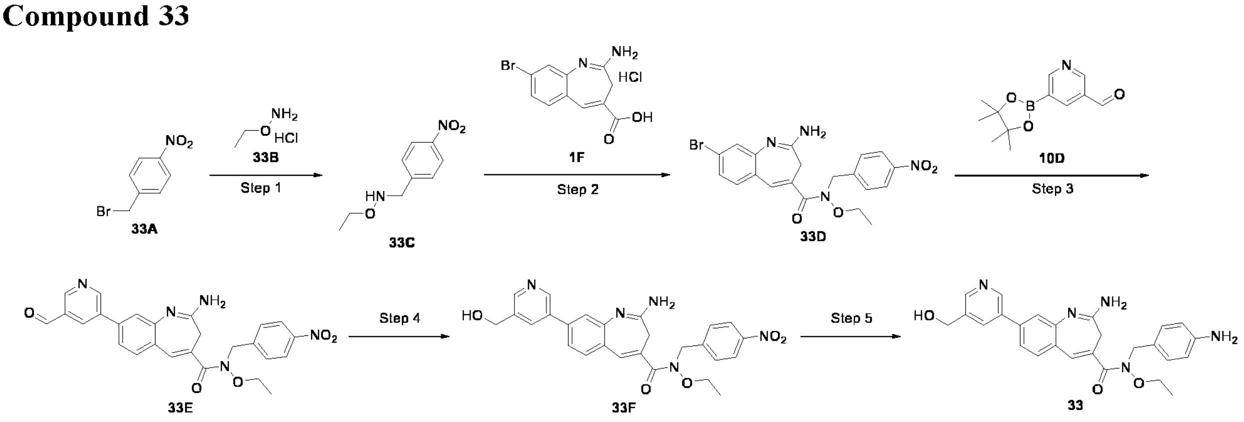


33A 33C 33D 33E 33F 33

**Step** 1 Compound **33A** (5 g, 23.14 mmol, 1 eq) was added to DMF (100 mL), then potassium carbonate (11.20 g, 81.01 mmol, 3.5 eq) and compound **33B** (2.71 g, 27.77 mmol, 1.2 eq) were sequentially added. After the addition, the system was stirred at room temperature for 16 h. When LCMS indicated the depletion of the starting material 33A, the reaction solution was poured into 300 mL of water and extracted twice with ethyl acetate (300 mL × 2). The organic phases were combined, washed with saturated brine (300 mL), and dried over anhydrous sodium sulfate. After concentration at reduced pressure, the mixture was mixed with silica gel and purified by column chromatography (PE:EA = 2:1) to give a white solid **33C** (2 g, 44% yield). MS-ESI: m/z 197.0 $[M+H]^+$.

**Step 2** Compound **1F** (3.24 g, 10.20 mmol, 1 eq) and compound **33C** (2 g, 10.20 mmol, 1 eq) were added to a mixed solution of DMA (60 mL) and DCM (60 mL). The system was purged with argon twice before EDCI (7.82 g, 40.81 mmol, 4 eq) was added in one portion, then purged with argon twice again, warmed to room temperature, and reacted overnight. When LCMS indicated the depletion of the starting materials, the reaction solution was poured into 180 mL of water and extracted thrice with DCM (100 mL × 3). The organic phases were combined, washed with saturated brine (100 mL), and dried over anhydrous sodium sulfate. The mixture was concentrated at reduced pressure, mixed with silica gel, and purified by column chromatography to give a yellow solid **33D** (1.9 g, 40.5% yield). MS-ESI: m/z 459.2 $[M+H]^+$.

**Step 3** Compound **33D** (1.9 g, 4.14 mmol, 1 eq) was added to a mixed solution of 1,4-dioxane (40 mL) and water (4 mL), then compound 10D (964 mg, 4.14 mmol, 1 eq) and anhydrous potassium phosphate (2.63 g, 12.41 mmol, 3 eq) were added. The system was purged with argon twice before $Pd(PPh_3)_4$ (200 mg) was added, then purged with argon twice again, heated to 90 °C, and reacted for 2 hours. When LCMS indicated the depletion of the starting materials, the mixture was cooled to room temperature, and the phases were separated. The organic phase was concentrated at reduced pressure to give a black crude product **33E**, which was directly used in the next reaction. MS-ESI: m/z 486.2 $[M+H]^+$.

**Step 4** The crude product of compound 33E from the previous step (theoretical amount: 2.01 g, 4.14 mmol, 1 eq) was added to methanol (50 mL), then sodium borohydride (157 mg, 4.14 mmol, 1 eq) was added slowly in portions at room temperature. After the addition, the system was reacted at room temperature for 2 hours. When LCMS indicated the depletion of the starting materials, the reaction was quenched with saturated aqueous ammonium chloride (1 mL), and the mixture was concentrated at reduced pressure, mixed with silica gel, and purified by column chromatography (MeOH:DCM = 0-8%) to give a brown solid **33F** (400 mg, 19.8% yield over the two steps). MS-ESI: m/z 488.2 $[M+H]^+$.

**Step 5** Compound **33F** (100 mg, 0.205 mmol, 1 eq) was dissolved in methanol (10 mL), then Raney nickel (0.1 g) was added in an ice-water bath. The system was purged twice with hydrogen and stirred for 3 hours in the ice-water bath. When LCMS indicated the depletion of the starting materials, the reaction solution was filtered through celite, concentrated, and purified by reversed-phase column chromatography to give an off-white solid **33** (20 mg, 21.5% yield). MS-ESI: m/z 458.2 $[M+H]^+$.

$^1H$ NMR (400 MHz, DMSO) δ 8.77 (d, J = 2.2 Hz, 1H), 8.51 (d, J = 1.9 Hz, 1H), 8.00 - 7.99 (t, J = 2.0 Hz, 1H), 7.44 - 7.42 (d, J = 8.2 Hz, 1H), 7.33 - 7.32 (d, J = 1.8 Hz, 1H), 7.28 - 7.26 (dd, J = 8.1, 1.9 Hz, 1H), 7.15 (s, 1H), 7.03 - 7.01 (d, J = 8.3 Hz, 2H), 6.84 (s, 2H), 6.55 - 6.53 (d, J = 8.4 Hz, 2H), 5.38 - 5.35 (t, J = 5.8 Hz, 1H), 5.05 (s, 2H), 4.66 (s, 2H), 4.62 - 4.60 (d, J = 5.7 Hz, 2H), 3.82 - 3.77 (q, J = 7.0 Hz, 2H), 2.82 (s, 2H), 1.01 - 0.97 (t, J = 7.0 Hz, 3H).

## Compound 34

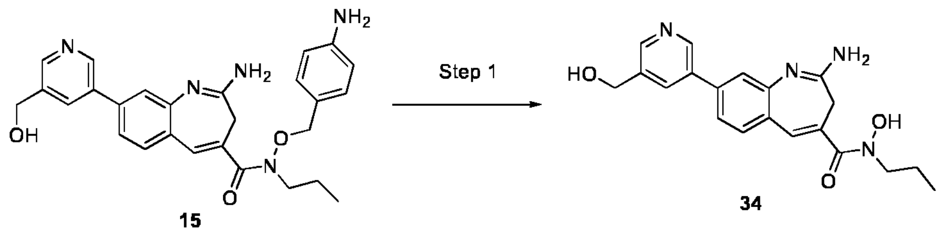


15

34

**Step 1** Compound **15** (100 mg, 0.204 mmol, 1 eq) was added to a phosphate buffer, pH 5.5 (5 mL). The mixture was let stand overnight at room temperature. When LCMS indicated the depletion of the starting materials, the reaction solution was subjected to preparative HPLC to give a yellow solid 34 (20 mg, 21.5% yield). MS-ESI: m/z 367.2 $[M+H]^+$.

$^1H$ NMR (400 MHz, DMSO) δ 12.41 (s, 1H), 10.14 (s, 1H), 9.81 (s, 1H), 9.51 (s, 1H), 8.91 - 8.90 (d, J = 2.1 Hz, 1H), 8.65 - 8.64 (d, J = 1.7 Hz, 1H), 8.20 (s, 1H), 7.87 - 7.60 (m, 3H), 7.35 (s, 1H), 4.68 (s, 2H), 3.61 - 3.57 (t, J = 7.0 Hz, 4H), 3.39 (s, 2H), 1.71 - 1.58 (m, 2H), 0.93 - 0.89 (t, J = 7.4 Hz, 3H).

## Compound 35

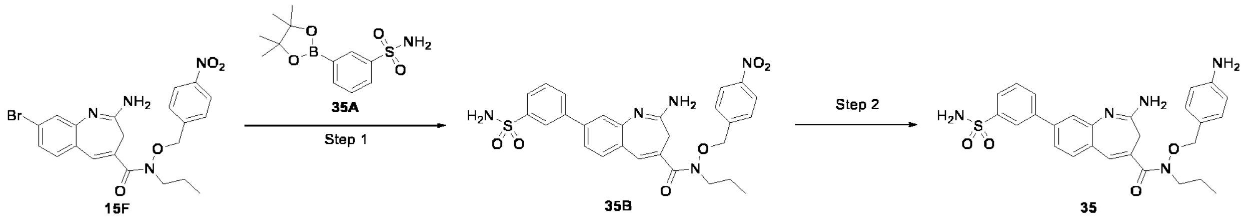


**Step 1** Compound **15F** (300 mg, 0.63 mmol, 1 eq.), 1,4-dioxane (6 mL), and water (0.6 mL) were added into a 20-mL microwave reaction tube, then compound **35A** (180 mg, 0.63 mmol, 1 eq.) and anhydrous potassium phosphate (404 mg, 1.9 mmol, 3 eq.) were added. The system was purged with argon twice before Pd(dppf)$Cl_2$ (45 mg, 15% w/w ) was added, then purged with argon twice again, and reacted in a microwave reactor at 90 °C for 2 hours. When LCMS indicated the depletion of the starting materials, the reaction solution was poured into 15 mL of water and extracted thrice with ethyl acetate (30 mL × 3). The organic phases were combined and dried over anhydrous sodium sulfate. The mixture was concentrated at reduced pressure and purified by column chromatography (DCM:MeOH = 20: 1) to give a pale yellow solid **35B** (250 mg, 72% yield).

MS-ESI: m/z 550.2 $[M+H]^+$.

**Step 2** Compound **35B** (250 mg, 0.45 mmol, 1 eq.) was dissolved in methanol (10 mL), then Raney nickel (0.1 g) was added. The system was purged twice with hydrogen and stirred at 13 to 15 °C for 36 hours. When LCMS indicated a 50% conversion of the starting materials and increased by-product content, the reaction was stopped. The reaction solution was filtered through celite, concentrated, and purified by preparative HPLC to give a yellow solid **35** (30 mg, 12.7% yield). MS-ESI: m/z 520.2 $[M+H]^+$.

$^1$H NMR (400 MHz, DMSO) δ 9.88 (S, 1H), 9.36 (s, 1H), 8.20 (s, 1H), 8.02 - 8.0 (d, J=8, 1H), 7.93 - 7.91 (d, J=8, 1H), 7.79 -7.69(m, 4H), 7.50 (s, 2H), 7.24 (s, 1H), 7.04 - 7.02 (d, J=8, 1H), 6.62 - 6.60 (d, J=8, 2H),, 4.72 (s, 2H), 3.74 - 3.71 (m, 4H), 3.29 (s, 2H), 1.73 - 1.67 (dd, J = 16, 8 Hz, 2H), 0.97 - 0.93 (t, J = 8 Hz, 3H).

## Compound 36

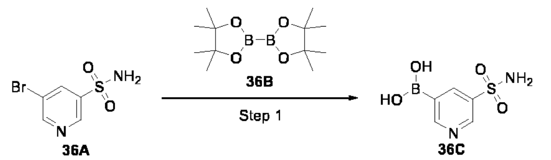


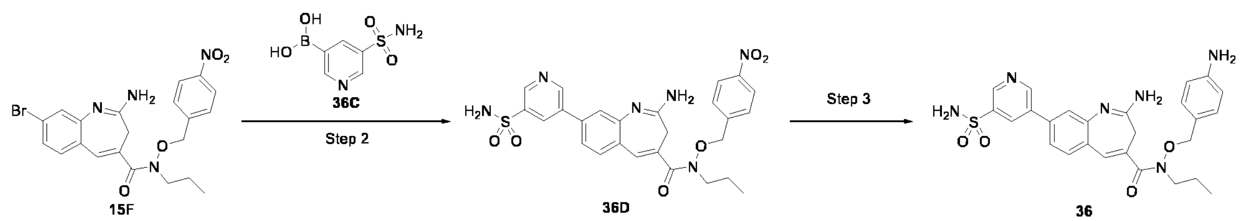


**Step 1** Compound **36A** (1 g, 4.22 mmol, 1 eq.) was dissolved in dioxane (15 mL), then compound **36B** (1.18 mg, 4.64 mmol, 1.1 eq.) and AcOK (828 mg, 8.44 mmol, 2 eq.) were added. The system was purged with argon twice before Pd(dppf)$Cl_2$ (0.2 g) was added in one portion, then purged with argon twice again, and reacted in a microwave reactor at 85 °C for 3 hours. When LCMS indicated the depletion of the starting materials, the reaction solution was cooled to room temperature before 3 mL of water was added. The mixture was poured directly into a reversed-phase column, the product was eluted with water (0.1% TFA). The resultant mixture was lyophilized to give a pale yellow solid compound **36C** (400 mg, 41% yield). MS-ESI: m/z 203.0 $[M+H]^+$.

**Step 2** Compound **15F** (0.5 g, 1.06 mmol, 1 eq.) and compound **36C** (224 mg, 1.11 mmol, 1 eq.) were added to a mixed solvent of dioxane (10 mL) and water (1 mL), then potassium phosphate (673 mg, 3.17 mmol, 3 eq.) was added. The system was purged with argon twice before Pd(dppf)Cl2 (100 mg) was added in one portion, then purged with argon twice again, and reacted in a microwave reactor at 85 °C for 2 hours. When LCMS indicated the depletion of the starting materials, the reaction solution was cooled to room temperature, dried over sodium sulfate, directly mixed with silica gel, and purified by column chromatography to give a yellow solid compound **36D** (300 mg, 51% yield). MS-ESI: m/z 551.2 $[M+H]^+$.

**Step 3** Compound **36D** (100 mg, 0.172 mmol, 1 eq) was dissolved in MeOH (5 mL) with the temperature controlled at 15 °C, then Raney nickel (20 mg) was added. The system was purged with hydrogen and stirred at 15 °C overnight. When LCMS indicated the depletion of the starting materials, the mixture was concentrated at reduced pressure, purified by preparative HPLC, and lyophilized to give an off-white solid compound **36** (28 mg, 29.6% yield). MS-ESI: m/z 521.2 $[M+H]^+$. $^1$H NMR (400 MHz, DMSO) δ 9.171 - 9.166 (d, J = 2.0 Hz 1H), 8.939 - 8.934 (d, J = 2.0 Hz 1H), 8.507 - 8.496 (t, $J_1$= 2.0 Hz, $J_2$= 4.4 Hz 1H), 7.6 (s, 2H), 7.48 -7.459 (d, J = 8.4 Hz 2H), 7.381 - 7.356 (dd, $J_1$= 2.0 Hz, $J_2$= 8.4 Hz, 1H), 7.108 (s, 1H), 6.899 - 6.878 (d, J = 8.4 Hz 3H), 6.425 - 6.404 (d, J = 8.4 Hz, 2H), 5.165 (s, 2H), 4.602 (s, 2H), 3.461 (m, 2H), 2.784 (s, 2H),1.908(S, 1H), 1.678 - 1.625 (m, 2H), 0.926 - 0.889 (t, $J_1$= 7.6 Hz, $J_2$= 14.8 Hz 3H).

## Compound 37

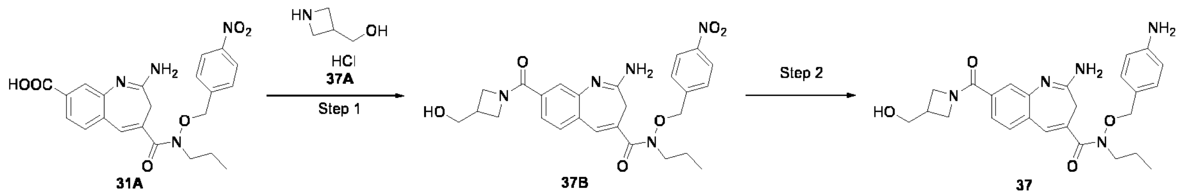


31A 37B 37

**Step 1** Compound **31A** (200 mg, 0.456 mmol, 1 eq.) was dissolved in DMF (5 mL), then HOBT (73.97 mg, 0.547 mmol, 1.2 eq.), EDCI (104.94 mg, 0.547 mmol, 1.2 eq.), and DIEA (176.87 mg, 1.37 mmol, 3 eq.) were added sequentially with the temperature controlled at 0 °C. Compound **37A** (67.65 mg, 0.547 mmol, 1.2 eq.) was added after stirring for 10 min at 0 °C, and the system was warmed to room temperature after the addition and stirred overnight. When LCMS indicated the depletion of the starting materials, the reaction solution was poured into 15 mL of water and extracted thrice with DCM (5 mL × 3). The organic phases were combined, washed with saturated brine (15 mL), and dried over anhydrous sodium sulfate. After concentration under reduced pressure, the reaction solution was mixed with silica gel, and purified by column chromatography (DCM:MeOH =10:1) to give a pale yellow solid **37B** (75 mg, 36.8% yield). MS-ESI: m/z 508.2 $[M+H]^+$.

**Step 2** Compound **37B** (75 mg, 0.140 mmol, 1 eq.) was dissolved in methanol (10 mL), then Raney nickel (0.15 g) was added with the temperature controlled at 0 °C. The system was purged twice with hydrogen and stirred at room temperature for 1 hour. When LCMS indicated the depletion of the starting materials, the reaction solution was filtered through celite, concentrated, and purified by preparative HPLC to give an off-white solid **37** in the form of hydrochloride (25.3 mg, 35.7% yield). MS-ESI: m/z 478.2 $[M+H]^+$.

$^1$H NMR (400 MHz, DMSO) δ 9.85 (s, 1H), 9.29 (s, 1H), 7.63 (s, 1H), 7.61 (d, J = 9.7 Hz, 2H), 7.17 (s, 1H), 6.97 (d, J = 8.0 Hz, 2H), 6.56 (d, J = 7.5 Hz, 2H), 4.68 (s, 2H), 4.38 (t, J = 8.5 Hz, 1H), 4.08 (t, J = 9.3 Hz, 2H), 3.82 (dd, J = 9.9, 5.4 Hz, 3H), 3.71 (s, 1H), 3.69 (s, 1H), 3.67 (s, 1H), 3.57 (s, 1H), 3.55 (s, 1H), 3.23 (s, 2H), 2.76 (s, 1H), 1.67 (dd, J = 14.3, 7.2 Hz, 2H), 0.92 (t, J = 7.4 Hz, 3H).

## Compound 38

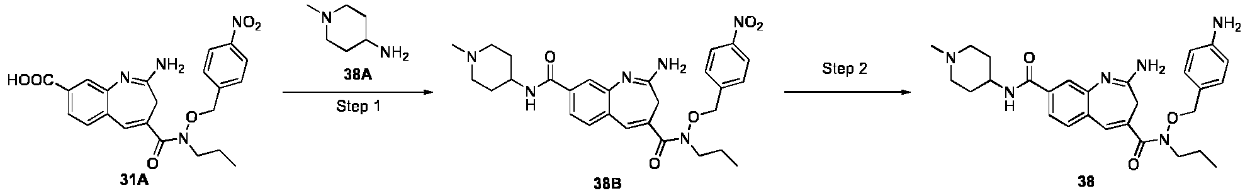


31A 38B 38

**Step 1** Compound **31A** (200 mg, 0.456 mmol, 1 eq.) was dissolved in DMF (5 mL), then HOBT (73.97 mg, 0.547 mmol, 1.2 eq.), EDCI (104.94 mg, 0.547 mmol, 1.2 eq.), and DIEA (176.87 mg, 1.37 mmol, 3 eq.) were added sequentially at 0 °C. Compound **38A** (62.51 mg, 0.547 mmol, 1.2 eq.) was added after stirring for 10 min at 0 °C, and the system was warmed to room temperature after the addition and stirred overnight. When LCMS indicated the depletion of the starting materials, the reaction solution was poured into 15 mL of water and extracted thrice with DCM (5 mL × 3). The organic phases were combined, washed with saturated brine (15 mL), and dried over anhydrous sodium sulfate. The resultant mixture was concentrated at reduced pressure to give a pale yellow solid **38B** (crude, 180 mg). MS-ESI: m/z 535.3 $[M+H]^+$.

**Step 2** Compound **38B** (150 mg, 0.280 mmol, 1 eq.) was dissolved in methanol (10 mL), then Raney nickel (0.15 g) was added with the temperature controlled at 0 °C. The system was purged twice with hydrogen and stirred at room temperature for 1 hour. The reaction solution was filtered through celite, concentrated, and purified by reversed-

phase column chromatography ($H_2O$:ACN = 3:2) to give an off-white solid **38** (14.9 mg, 10.6% yield over the two steps). MS-ESI: m/z 505.3 [M+H]$^+$.

$^1$H NMR (400 MHz, DMSO) δ 8.26 (d, J = 7.6 Hz, 1H), 7.56 (s, 1H), 7.36 (d, J = 8.4 Hz, 2H), 7.07 (s, 1H), 6.92 - 6.79 (m, 3H), 6.41 (s, 1H), 5.18 (s, 1H), 4.59 (s, 1H), 3.65 (t, J = 6.9 Hz, 3H), 2.89 - 2.63 (m, 3H), 2.18 (s, 2H), 1.95 (s, 2H), 1.74 (s, 2H), 1.63 (dd, J = 16.6, 9.3 Hz, 3H), 0.91 (t, J = 7.4 Hz, 2H).

## Compound 39

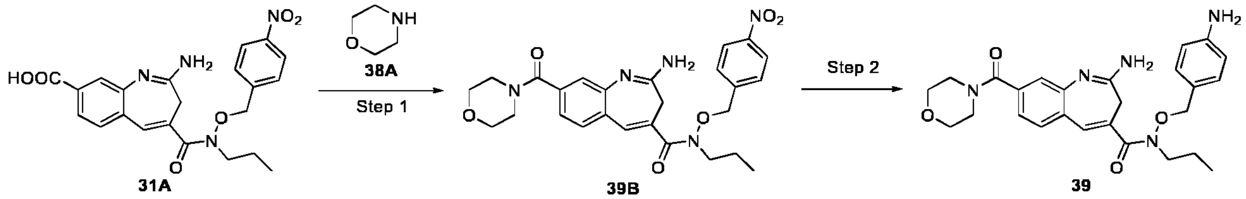

31A 39B 39

**Step 1** Compound **31A** (200 mg, 0.456 mmol, 1 eq.) was dissolved in DMF (5 mL), then HOBT (73.97 mg, 0.547 mmol, 1.2 eq.), EDCI (104.94 mg, 0.547 mmol, 1.2 eq.), and DIEA (176.87 mg, 1.37 mmol, 3 eq.) were added sequentially at 0 °C. Compound **39A** (47.69 mg, 0.547 mmol, 1.2 eq.) was added after stirring for 10 min at 0 °C, and the system was warmed to room temperature after the addition and stirred overnight. When LCMS indicated the depletion of the starting materials, the reaction solution was poured into 15 mL of water and extracted thrice with DCM (5 mL × 3). The organic phases were combined, washed with saturated brine (15 mL), and dried over anhydrous sodium sulfate. After concentration under reduced pressure, the reaction solution was mixed with silica gel, and purified by column chromatography (DCM:MeOH =20:1) to give a pale yellow solid **39B** (175 mg, 72.5% yield). MS-ESI: m/z 508.2 [M+H]$^+$.

**Step 2** Compound **39B** (150 mg, 0.295 mmol, 1 eq.) was dissolved in methanol (10 mL), then Raney nickel (0.15 g) was added with the temperature controlled at 0 °C. The system was purged twice with hydrogen and stirred at room temperature for 1 hour. When LCMS indicated the depletion of the starting materials, the reaction solution was filtered through celite, concentrated, and purified by reversed-phase column chromatography ($H_2O$:CAN = 3:2) to give an off-white solid 39 (22 mg, 15.7% yield). MS-ESI: m/z 478.0 [M+H]$^+$.

$^1$H NMR (400 MHz, DMSO) δ 7.36 (d, J = 8.0 Hz, 1H), 7.07 (s, 1H), 7.02 (s, 1H), 6.97 - 6.85 (m, 5H), 6.42 (d, J = 8.3 Hz, 2H), 5.19 (s, 2H), 4.60 (s, 2H), 3.88 - 3.41 (m, 10H), 2.76 (s, 2H), 1.65 (d, J = 7.1 Hz, 2H), 0.91 (t, J = 7.4 Hz, 3H).

## Compound 40

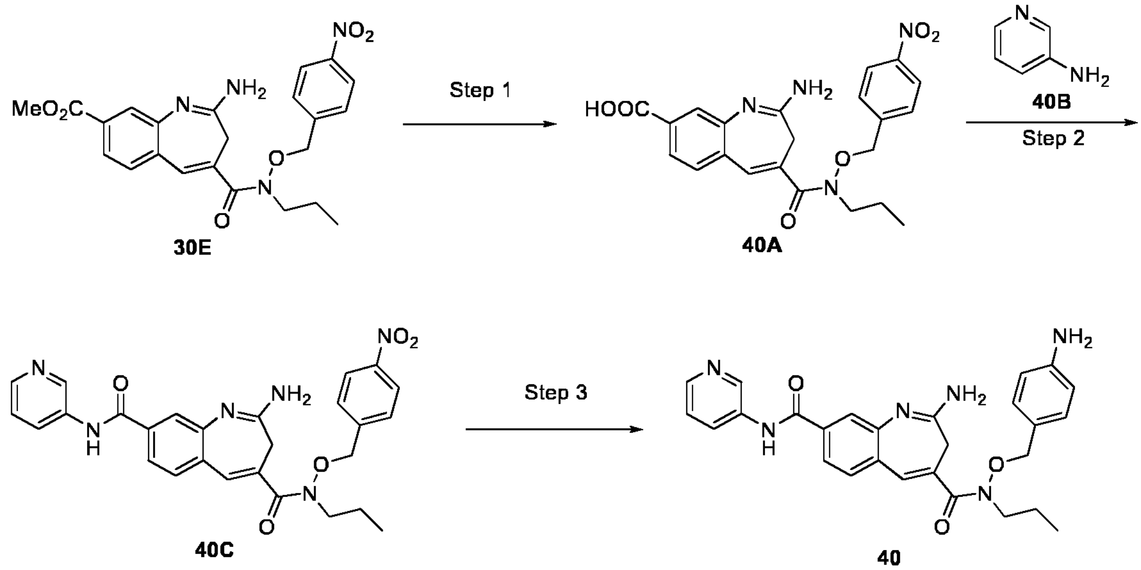

30E 40A 40C 40

**Step 1** Compound **30E** (1.5 g, 3.32 mmol, 1 eq.) was dissolved in a mixed solvent of MeOH (10 mL), THF (10 mL), and $H_2O$ (10 mL). The system was cooled to 0 °C before LiOH($H_2O$) (417.35 mg, 9.95 mmol, 3 eq) was added in one portion, and reacted at 0 °C to 10 °C for 3 hours. When LCMS indicated the depletion of the starting materials, the reaction solution was poured into 50 mL of water, and adjusted to pH 5-6 with 1 N hydrochloric acid. The mixture was filtered, and the filter cake was rinsed with water (10 mL) and dried to give an off-white solid **40A** (0.5 g, 45% yield). MS-ESI: m/z 439.2 [M+H]$^+$.

**Step 2** Compound **40A** (100 mg, 0.228 mmol, 1 eq.) was dissolved in DMF (5 mL), then TCFH (95.99 mg, 0.342 mmol, 1.5 eq.) and NMI (74.91 mg, 0.912 mmol, 4 eq.) were added sequentially with the temperature controlled at 0 °C. Compound **40B** (25.76 mg, 0.274 mmol, 1.2 eq.) was added after stirring for 10 min at 0 °C, and the system was warmed to room temperature after the addition and stirred overnight. When LCMS indicated the depletion of the starting materials, the reaction solution was poured into 15 mL of water and extracted with DCM (5 mL × 3). The organic phases were combined, washed with saturated brine (15 mL), and dried over anhydrous sodium sulfate. After concentration at reduced pressure, the mixture was triturated in MeOH. The mother liquor was concentrated at reduced pressure to give a pale yellow solid **40C** (65 mg, 40% yield).

**Step 3** Compound **40C** (50 mg, 0.097 mmol, 1 eq) was dissolved in methanol (5 mL), then Raney nickel (5 mg) was added. The system was purged twice with hydrogen and stirred at room temperature for 3 hours. When LCMS indicated the depletion of the starting materials, the reaction solution was filtered through celite, concentrated by rotary evaporation, and purified by preparative HPLC to give a yellow solid **40** (3 mg, 13% yield). MS-ESI: m/z 485.3 $[M+H]^+$.

$^1$H NMR (400 MHz, DMSO) δ 10.70 (s, 1H), 9.89 (s, 1H), 8.99 (s, 2H), 8.39 (d, J = 5.1 Hz, 1H), 8.25 (d, J = 6.8 Hz, 1H), 8.03 (d, J = 8.0 Hz, 1H), 7.98 (s, 1H), 7.74 (d, J = 8.1 Hz, 1H), 7.48 (dd, J = 8.4, 4.8 Hz, 1H), 7.23 (s, 1H), 6.91 (d, J = 7.9 Hz, 2H), 6.46 (d, J = 7.9 Hz, 2H), 4.67 (s, 2H), 3.75 - 3.67 (m, 3H), 3.25 (s, 2H), 1.73 - 1.62 (m, 2H), 1.24 (s, 1H), 0.94 (t, J = 7.4 Hz, 3H).

## Compound 41

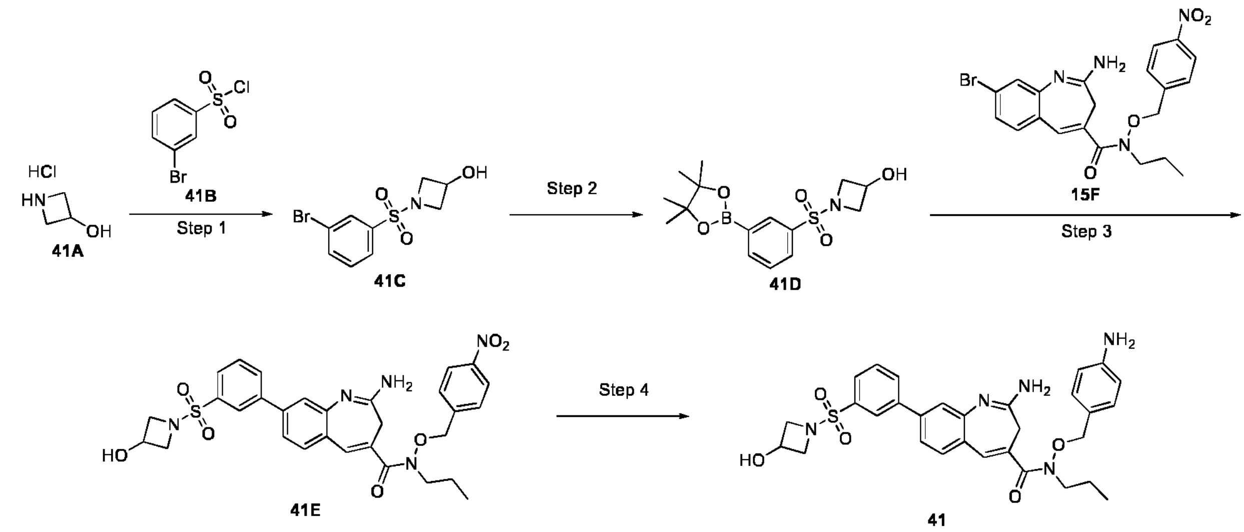


**Step 1** **41A** (2.0 g, 7.83 mmol, 1 eq.) was dissolved in DCM (20 mL). Triethylamine (3.96 g, 39.14 mmol, 5 eq.) was added at 0 °C, then **41B** (1.29 g, 11.74 mmol, 1 eq.) was slowly and dropwise added. The system was warmed to room temperature and stirred for 3 hours after the addition. When LCMS indicated the depletion of the starting materials, the reaction solution was washed with 10 mL of water. The organic phase was dried over sodium sulfate, concentrated at reduced pressure, mixed with silica gel, and purified by column chromatography (PE:EA = 3:1) to give a white solid **41C** (1.2 g, 52.5% yield). MS-ESI: m/z 292.0 $[M+H]^+$.

**Step 2** Compound **41C** (1.2 g, 4.11 mmol, 1 eq.), potassium acetate (0.8 g, 8.21 mmol, 2 eq.), $B_2Pin_2$ (1.2 g, 4.11 mmol, 1 eq.), and 1,4-dioxane (15 mL) were added to a 20-mL microwave reaction tube. The system was purged twice with argon, and reacted in a microwave reactor at 100 °C for 3 hours. When LCMS indicated the completion of the reaction, the reaction solution was poured into 15 mL of ice water and extracted with ethyl acetate (30 mL × 3). The organic phases were combined, washed with saturated brine (30 mL), dried over sodium sulfate, concentrated at reduced pressure, mixed with silica gel, and purified by column chromatography (PE:EA= 3:1) to give a yellow oily substance **41D** (0.7 g, 53% yield).

MS-ESI: m/z 340.2 $[M+H]^+$.

**Step 3** Compound **15F** (209 mg, 0.44 mmol, 1 eq.), dioxane (6 mL), and water (0.6 mL) were added into a 20-mL microwave reaction tube, then compound **41D** (150 mg, 0.44 mmol, 1 eq.) and anhydrous potassium phosphate (280 mg, 1.32 mmol, 3 eq.) were added. The system was purged with argon twice before $Pd(dppf)Cl_2$ (40 mg, 15% ) was added, then purged with argon twice again, and reacted in a microwave reactor at 90 °C for 2 hours. When LCMS indicated the depletion of the starting materials, the reaction solution was poured into 15 mL of water and extracted with ethyl acetate (30 mL × 3). The organic phases were combined, dried over sodium sulfate, concentrated at reduced pressure, and purified by column chromatography (DCM:MeOH = 20:1) to give a pale yellow solid compound **41E** (200

mg, 72% yield). MS-ESI: m/z 606.2 $[M+H]^+$.

**Step 4** Compound **41E** (200 mg, 0.33 mmol, 1 eq.) was dissolved in methanol (10 mL), then Raney nickel (0.1 g) was added. The system was purged twice with hydrogen and stirred at 13 to 15 °C for 16 hours under hydrogen atmosphere. When LCMS indicated the depletion of the starting materials, the reaction solution was filtered through celite, concentrated by rotary evaporation, purified by preparative HPLC, and lyophilized to give an off-white solid **41** (27 mg, 10.7% yield). MS-ESI: m/z 576.2 $[M+H]^+$.

$^1$H NMR (400 MHz, DMSO) δ 8.149-8.123 (m, 1H), 7.988 (s, 1H), 7.812-7.799 (d, J=5.2, 2H), 7.461 - 7.350 (m, 3H), 7.112 (s, 1H), 6.908 -6.888(d, J=8, 1H), 6.435-6.414 (d, J=8.4, 2H), 5.755-5.741 (d, J=5.6, 1H), 5.17 (s, 2H), 4.608 (s, 2H), 4.316-4.302 (m, 1H), 3.977-3.939 (dd, $J_1$=7.2, $J_2$=8.4, 2H), 3.663-3.628 (t, $J_1$=6.8, $J_2$=14, 2H), 2.806 (s, 2H), 1.683 - 1.629 (dd, $J_1$= 7.2, $J_2$=14.4 Hz, 2H), 0.931 - 0.894 (t, $J_1$ = 7.2, $J_2$=14.8 Hz, 3H).

## Compound 42

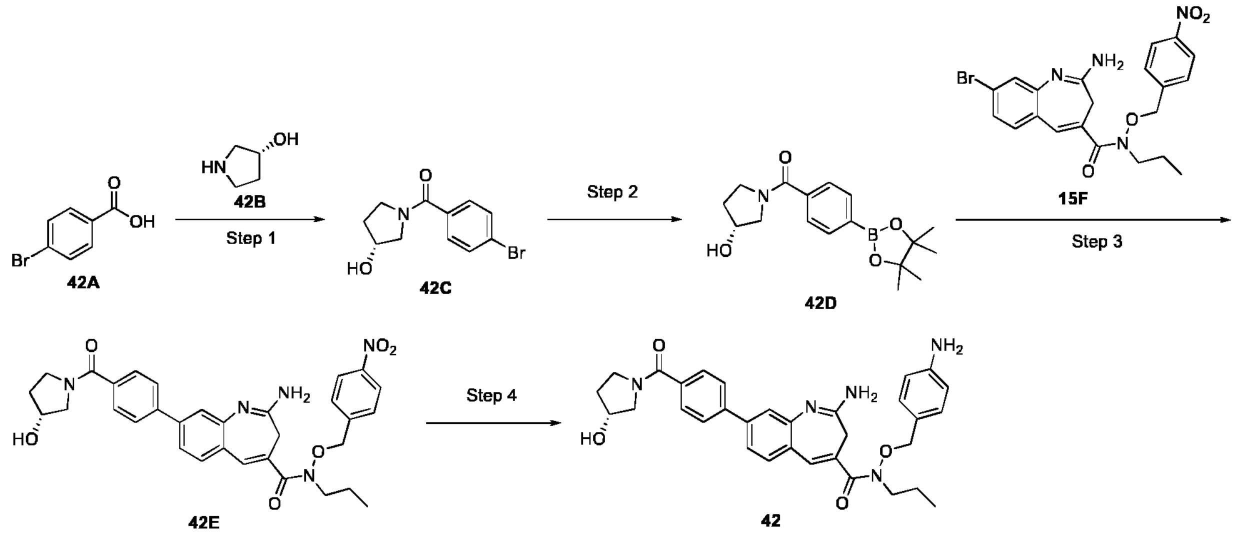


**Step 1** Compound **42A** (0.5 g, 2.49 mmol, 1 eq.) was dissolved in DMF (10 mL), then DIEA (0.96 g, 7.46 mmol, 3 eq.) and HATU (1.04 g, 2.87 mmol, 1.1 eq.) were added at room temperature. After the addition, the system was stirred at room temperature for 5 min, and compound **42B** (0.217 g, 2.49 mmol, 1 eq.) was added. The system was then stirred at room temperature overnight after the addition. When LCMS indicated the completion of the reaction, the reaction solution was poured into 30 mL of water and extracted with ethyl acetate (30 mL × 3). The organic phase was dried over sodium sulfate, concentrated at reduced pressure, mixed with silica gel, and purified by column chromatography (PE:EA= 5:1) to give a white solid compound **42C** (0.5 g, 74% yield). MS-ESI: m/z 270.0 $[M+H]^+$.

**Step 2** Compound **42C** (0.3 g, 1.11 mmol, 1 eq.) was dissolved in dioxane (20 mL), then HY-515_12 (310 mg, 1.22 mmol, 1.1 eq.) and AcOK (218 mg, 2.22 mmol, 2 eq.) were added. The system was purged with argon twice before Pd(dppf)Cl2 (45 mg) was added in one portion, then purged with argon twice again, and reacted in a microwave reactor at 100 °C for 3 hours. When LCMS indicated the depletion of the starting materials, the reaction solution was cooled to room temperature and directly poured into a silica gel column. The product was eluted with PE:EA = 2:1 to give a pale yellow solid compound **42D** (150 mg, 42.3% yield). MS-ESI: m/z 318.2 $[M+H]^+$.

**Step 3** Compound **42D** (223 mg, 0.49 mmol, 1.05 eq.) and compound **15F** (150.4 mg, 0.47 mmol, 1 eq.) were added to a mixed solvent of dioxane (10 mL) and water (1 mL), then potassium phosphate (296 mg, 1.4 mmol, 3 eq.) was added. The system was purged with argon twice before Pd(dppf)Cl2 (40 mg) was added in one portion, then purged with argon twice again, and reacted in a microwave reactor at 90 °C for 3 hours. When LCMS indicated the depletion of the starting materials, the reaction solution was cooled to room temperature, added to 15 mL of water, and extracted with ethyl acetate (30 mL × 3). The organic phases were combined, dried over sodium sulfate, concentrated at reduced pressure, and purified by column chromatography (DCM:MeOH = 20:1) to give a yellow solid compound **42E** (100 mg, 36% yield). MS-ESI: m/z 584.2 $[M+H]^+$.

**Step 4** Compound **42E** (100 mg, 0.172 mmol, 1 eq) was dissolved in MeOH (5 mL) with the temperature controlled at 15 °C, then Raney nickel (20 mg) was added. The system was purged with hydrogen and stirred at 15 °C overnight. When LCMS indicated the depletion of the starting materials, the mixture was concentrated at reduced pressure, purified by preparative HPLC, and lyophilized to give an off-white solid compound **42** (27 mg, 28.4% yield). MS-ESI: m/z 554.3 $[M+H]^+$.

$^1$H NMR (400 MHz, DMSO) δ 7.802 - 7.782 (d, J = 8.0 Hz, 2H), 7.664 - 7.633 (t, J 1= 4.4 Hz, J 2= 12.4 Hz 2H),

7.568 - 7.526 (t, J 1= 8 Hz, J 2= 16.8 Hz 2H), 7.157 (s, 1H), 6.91 - 6.89 (d, J = 8.0 Hz, 1H), 6.433 - 6.412 (d, J = 8.4 Hz, 1H), 5.20 (s, 1H), 5.044 - 4.964 (d, J = 32 Hz, 1H), 4.627 (s, 1H), 4.348 - 4.256 (d, J = 36.8 Hz, 1H), 3.681 - 3.277 (m, 8H), 3.065 (s, 2H), 2.017 - 1.811 (m, 3H), 1.689 - 1.635 (dd, J 1= 7.2 Hz, J 2= 14.4 Hz 2H), 0.935 - 0.898 (t, J 1= 7.6 Hz, J 2= 14.8 Hz 3H).

## Compound 43

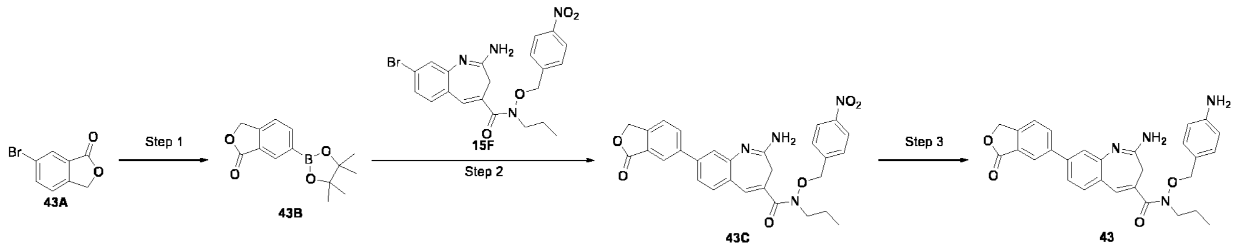


**Step 1** Compound **43A** (0.5 g, 2.35 mmol, 1 eq.) was dissolved in dioxane (10 mL), then $B_2Pin_2$ (0.65 mg, 2.58 mmol, 1.1 eq.) and AcOK (460 mg, 4.69 mmol, 2 eq.) were added. The system was purged with argon twice before Pd(dppf)Cl2 (45 mg, 15% ) was added in one portion, then purged with argon twice again, and reacted in a microwave reactor at 85 °C for 3 hours. When LCMS indicated the depletion of the starting materials, the reaction solution was directly poured into a silica gel column. The product was eluted with PE:EA = 2:1 to give a pale yellow solid compound **43B** (300 mg, 49.1% yield). MS-ESI: m/z 261.1 $[M+H]^+$.

**Step 2** Compound **15F** (300 mg, 0.57 mmol, 1.0 eq.), dioxane (8 mL), and water (0.8 mL) were added into a 20-mL microwave reaction tube, then compound **43B** (148 mg, 0.57 mmol, 1 eq.) and anhydrous potassium phosphate (360 mg, 1.7 mmol, 3 eq.) were added. The system was purged with argon twice before Pd(dppf)$Cl_2$ (45 mg, 15% ) was added, then purged with argon twice again, and reacted in a microwave reactor at 90 °C for 2 hours. When LCMS indicated the depletion of the starting materials, the reaction solution was poured into 15 mL of water and extracted with ethyl acetate (30 mL × 3). The organic phases were combined, dried over sodium sulfate, concentrated at reduced pressure, and purified by column chromatography (DCM:MeOH = 20:1) to give a pale yellow solid compound **43C** (200 mg, 60.5% yield). MS-ESI: m/z 527.2 $[M+H]^+$.

**Step 3** Compound **43C** (200 mg, 0.172 mmol, 1 eq) was dissolved in MeOH (5 mL) with the temperature controlled at 15 °C, then Raney nickel (20 mg) was added. The system was purged with hydrogen and stirred at 15 °C overnight. When LCMS indicated the depletion of the starting materials, the mixture was concentrated at reduced pressure, purified by preparative HPLC, and lyophilized to give an off-white solid compound **43** (36 mg, 19.7% yield). MS-ESI: m/z 554.2 $[M+H]^+$.

$^1H$ NMR (400 MHz, DMSO) $\delta$ 8.14 - 8.116 (dd, $J_1$= 1.6 Hz $J_2$ = 8 Hz, 1H), 8.066 (s, 1H), 7.782 - 7.762 (d, J = 8 Hz, 1H), 7.423 - 7.347 (m, 3H), 7.098 (s, 1H), 6.905 - 6.884 (d, J = 8.4 Hz, 1H), 6.430 - 6.409 (d, J = 8.4 Hz, 1H), 5.473 (s, 2H), 5.170 (s, 1H), 4.601 (s, 2H), 3.649-3.614 (t, $J_1$= 7.2 Hz $J_2$ = 14 Hz 2H), 2.782 (s, 2H), 1.875 (s, 6H), 1.674 - 1.620 (dd, $J_1$= 7.2 Hz $J_2$ = 14.4 Hz, 2H), 0.91 (t, $J_1$= 7.6 Hz $J_2$ = 14.8 Hz 3H).

## Compound 44

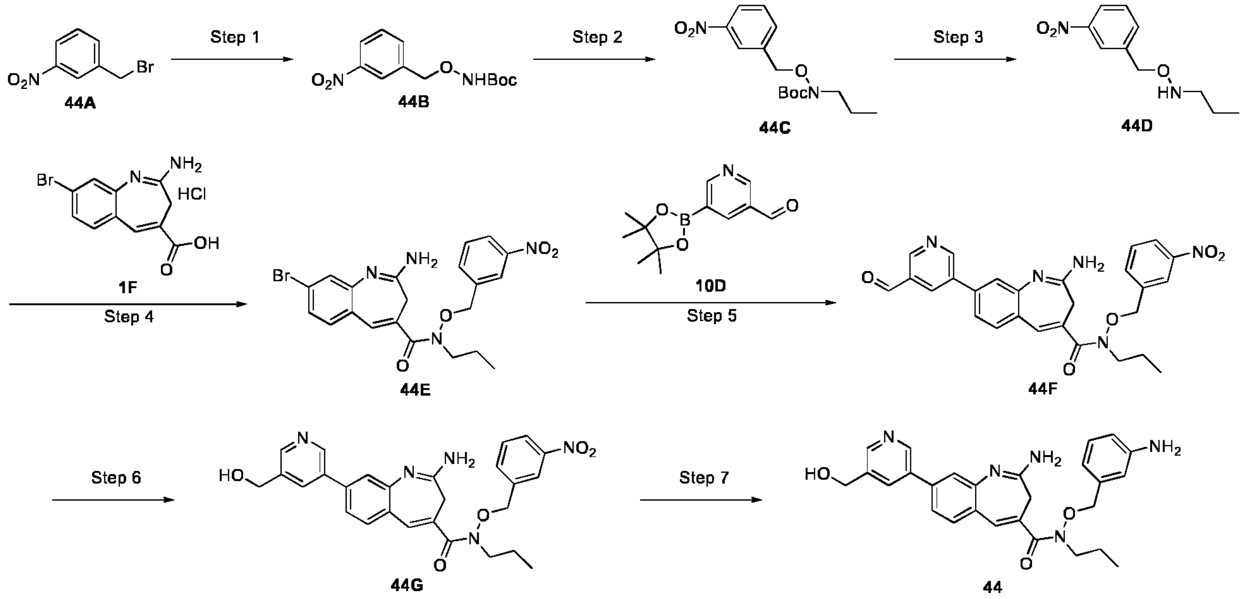


44A, 44B, 44C, 44D, 1F, 44E, 10D, 44F, 44G, 44

**Step 1** Compound **44A** (5 g, 23.14 mmol, 1 eq.) was dissolved in acetonitrile (100 mL), then BocNHOH (4.01 g, 30.09 mmol, 1.3 eq.) was added at 0 °C. DBU (3.52 g, 23.14 mmol, 1 eq) was then slowly and dropwise added, and the system was warmed to room temperature and stirred for 3 hours after the addition. When LCMS indicated the depletion of starting materials, the reaction solution was concentrated at reduced pressure, then 100 mL of saturated potassium carbonate solution was added. The mixture was extracted with DCM (50 mL × 3). The organic phases were combined, washed with saturated brine (50 mL), dried over sodium sulfate, concentrated at reduced pressure, mixed with silica gel, and purified by column chromatography (PE:EA = 5:1) to give a white solid compound **44B** (4.6 g, 74% yield). MS-ESI: m/z 291.0 $[M+Na]^+$.

**Step 2** Compound **44B** (4.5 g, 16.77 mmol, 1 eq) was dissolved in DMF (50 mL), and the mixture was cooled to 0 °C. NaH (671 mg, 16.77 mmol, 1 eq., 60%) was added in one portion, and the mixture stirred at 0 °C for 10 min before iodopropane (3.14 g, 18.45 mmol, 1.1 eq) was added dropwise. After the addition, the reaction system was warmed to room temperature and reacted overnight. When LCMS indicated the depletion of starting materials, the reaction solution was poured into 150 mL of ice water and extracted with ethyl acetate (100 mL × 3). The organic phases were combined, sequentially washed with 10% aqueous citric acid (100 mL), saturated sodium bicarbonate (100 mL) and saturated brine (100 mL), dried over sodium sulfate, concentrated at reduced pressure, mixed with silica gel, and purified by column chromatography (PE:EA = 5:1) to give a yellow solid compound **44C** (3.4 g, 65% yield). MS-ESI: m/z 333.2 $[M+Na]^+$.

**Step** 3 Compound **44C** (3.4 g, 10.96 mmol, 1 eq.) was dissolved in EA (40 mL), then 4 N hydrochloride in dioxane (40 mL) was added in an ice-water bath. The system was stirred at room temperature overnight. When LCMS indicated the depletion of the starting materials, the reaction solution was concentrated by rotary evaporation, triturated in EA (100 mL), and dried at reduced pressures to give a yellow solid compound **44D** (2.0 g, 85% yield). MS-ESI: m/z 211.2 $[M+H]^+$. **Step 4** Compound **44D** (1 g, 4.05 mmol, 1.1 eq.) and compound **1F** (1.17 g, 3.69 mmol, 1 eq.) were added to a mixed solvent of DMA (10 mL) and DCM (10 mL). The system was purged with argon twice before EDCI (2.83 g, 14.74 mmol, 4 eq.) was added in one portion, then purged with argon twice again, warmed to room temperature, and reacted overnight. When LCMS indicated the completion of the reaction, the reaction solution was poured into 30 mL of water and extracted with DCM (30 mL × 3). The organic phases were combined, washed with saturated brine (50 mL), dried over sodium sulfate, concentrated by rotary evaporation, mixed with silica gel, and purified by column chromatography to give a yellow solid compound **44E** (0.7 g, 41.2% yield). MS-ESI: m/z 473.1 $[M+H]^+$.

**Step 5** Compound **44E** (0.6 g, 1.27 mmol, 1 eq.) was added to a mixed solvent of dioxane (10 mL) and water (1 mL), then compound **10D** (295.45 mg, 1.27 mmol, 1 eq.) and anhydrous potassium phosphate (807.23 mg, 3.8 mmol, 3 eq.) were added. The system was purged with argon twice before $Pd(PPh_3)_4$ (60 mg) was added, then purged with argon twice again, heated to 85 °C, and reacted for 3 hours. When LCMS indicated the depletion of the starting materials, the reaction solution was poured into 30 mL of water and extracted with ethyl acetate (30 mL × 3). The organic phases were combined, dried over sodium sulfate, concentrated at reduced pressure, and purified by column chromatography (DCM:MeOH = 20:1) to give a dark red solid **44F** (350 mg, 48% yield). MS-ESI: m/z 500.2 $[M+H]^+$.

**Step 6** Compound **44F** (300 mg, 0.6 mmol, 1 eq.) was added to methanol (10 mL), then sodium borohydride

(13.63 mg, 0.36 mmol, 0.6 eq.) was added slowly in portions at room temperature. After the addition, the system was reacted at room temperature for 2 hours. When LCMS indicated a small amount of remaining starting materials, the reaction was quenched with saturated aqueous ammonium chloride (50 mL), and the reaction solution was extracted with DCM (50 mL × 3). The organic phases were combined, dried over sodium sulfate, concentrated at reduced pressure, and purified by column chromatography (DCM:MeOH = 20:1) to give a pale yellow solid **44G** (120 mg, 38% yield). MS-ESI: m/z 502.2 $[M+H]^+$.

**Step** 7 Compound **44G** (100 mg, 0.199 mmol, 1 eq) was dissolved in methanol (5 mL), then Raney nickel (0.01 g) was added. The system was purged twice with hydrogen and stirred at room temperature for 3 hours under hydrogen atmosphere. When LCMS indicated the depletion of the starting materials, the reaction solution was filtered through celite, concentrated by rotary evaporation, and purified by preparative HPLC to give a white solid **44** (29 mg, 14.9% yield). MS-ESI: m/z 472.2 $[M+H]^+$.

$^1H$ NMR (400 MHz, DMSO) δ 9.88 (s, 1H), 9.31 (s, 1H), 8.90 (d, J = 2.0 Hz, 1H), 8.64 (s, 1H), 8.17 (s, 1H), 7.79 (dd, J = 8.1, 1.6 Hz, 1H), 7.74 (s, 1H), 7.67 (d, J = 8.3 Hz, 1H), 7.28 (s, 1H), 7.10 (t, J = 8.0 Hz, 1H), 6.79 (d, J = 6.6 Hz, 2H), 6.72 (d, J = 7.3 Hz, 1H), 4.80 (s, 2H), 4.68 (s, 2H), 3.74 (s, 2H), 3.71 (s, 2H), 3.32 (s, 2H), 1.69 (dd, J = 14.3, 7.2 Hz, 2H), 0.93 (t, J = 7.4 Hz, 2H).

## Compound 45

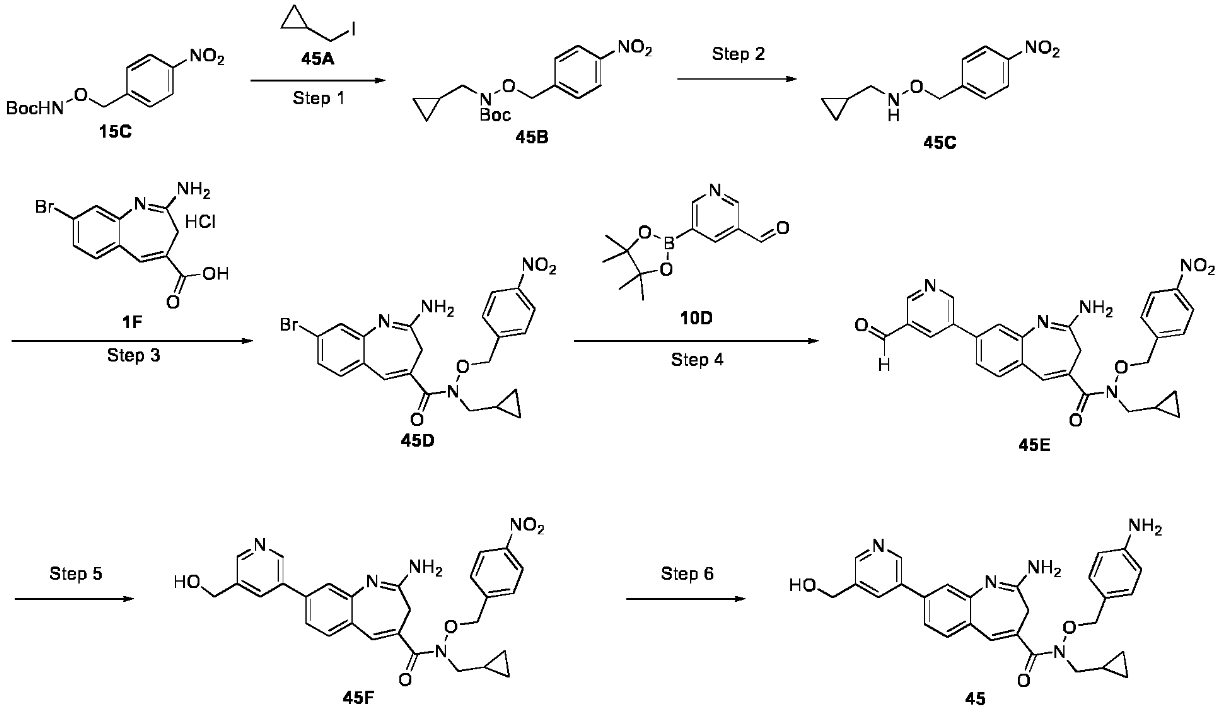


**Step 1** Compound **15C** (4.5 g, 16.77 mmol, 1 eq) was dissolved in DMF (50 mL), and the mixture was cooled to 0 °C. NaH (671 mg, 16.77 mmol, 1 eq., 60%) was added in one portion, and the mixture stirred at 0 °C for 10 min before compound **45A** (3.36 g, 18.45 mmol, 1.1 eq) was added dropwise. After the addition, the reaction system was warmed to room temperature and reacted overnight. When LCMS indicated the depletion of starting materials, the reaction solution was poured into 150 mL of ice water and extracted with ethyl acetate (100 mL × 3). The organic phases were combined, sequentially washed with 10% aqueous citric acid (100 mL), saturated sodium bicarbonate (100 mL) and saturated brine (100 mL), dried over sodium sulfate, concentrated at reduced pressure, mixed with silica gel, and purified by column chromatography (PE:EA = 5:1) to give a yellow solid compound **45B** (3.5 g, 60% yield). MS-ESI: m/z 267.1 $[M+H-C_4H_8]^+$.

**Step 2** Compound **45B** (3.4 g, 10.96 mmol, 1 eq.) was dissolved in EA (40 mL), then 4 N hydrochloride in dioxane (40 mL) was added in an ice-water bath. The system was stirred at room temperature overnight. When LCMS indicated the depletion of the starting materials, the reaction solution was concentrated by rotary evaporation, triturated in EA (100 mL), and dried at reduced pressure to give a yellow solid compound **45C** (2.9 g, 75% yield). MS-ESI: m/z 223.0 $[M+H]^+$. **Step 3** Compounds **45C** (1 g, 3.87 mmol, 1.1 eq.) and **1F** (1.12 g, 3.51 mmol, 1 eq.) were added to a mixed solvent of DMA (10 mL) and DCM (10 mL). The system was purged with argon twice before EDCI (2.69 g, 14.06 mmol, 4 eq.) was added in one portion, then purged with argon twice again, warmed to room temperature, and reacted

overnight. When LCMS indicated the completion of the reaction, the reaction solution was poured into 30 mL of water and extracted with DCM (30 mL × 3). The organic phases were combined, washed with saturated brine (50 mL), dried over sodium sulfate, concentrated by rotary evaporation, mixed with silica gel, and purified by column chromatography to give a yellow solid compound **45D** (0.71 g, 40% yield). MS-ESI: m/z 485.1 $[M+H]^+$.

**Step 4** Compound **45D** (0.8 g, 1.65 mmol, 1 eq.) was added to a mixed solvent of dioxane (10 mL) and water (1 mL), then compound **10D** (384.19 mg, 1.65 mmol, 1 eq.) and anhydrous potassium phosphate (1.05 g, 4.95 mmol, 3 eq.) were added. The system was purged with argon twice before $Pd(PPh_3)_4$ (80 mg) was added, then purged with argon twice again, heated to 85 °C, and reacted for 3 hours. When LCMS indicated the depletion of the starting materials, the reaction solution was poured into 30 mL of water and extracted with ethyl acetate (30 mL × 3). The organic phases were combined, dried over sodium sulfate, concentrated at reduced pressure, and purified by column chromatography (DCM:MeOH = 20:1) to give a dark red solid compound **45E** (400 mg, 46% yield). MS-ESI: m/z 512.2 $[M+H]^+$.

**Step 5** Compound **45E** (300 mg, 0.6 mmol, 1 eq.) was added to methanol (5 mL), then sodium borohydride (13.31 mg, 0.35 mmol, 0.6 eq.) was added slowly in portions at room temperature. After the addition, the system was reacted at room temperature for 2 hours. When LCMS indicated a small amount of remaining starting materials, the reaction was quenched with saturated aqueous ammonium chloride (15 mL), and the reaction solution was extracted with DCM (15 mL × 3). The organic phases were combined, dried over sodium sulfate, concentrated at reduced pressure, and purified by column chromatography (DCM:MeOH = 20:1) to give a pale yellow solid compound **45F** (110 mg, 25% yield). MS-ESI: m/z 514.2 $[M+H]^+$.

**Step 6** Compound **45F** (100 mg, 0.195 mmol, 1 eq) was dissolved in methanol (5 mL), then Raney nickel (0.01 g) was added. The system was purged twice with hydrogen and stirred at room temperature for 3 hours under hydrogen atmosphere. When LCMS indicated the depletion of the starting materials, the reaction solution was filtered through celite, concentrated by rotary evaporation, and purified by preparative HPLC to give a yellow solid **45** (37 mg, 35% yield). MS-ESI: m/z 484.3 $[M+H]^+$.

$^1$H NMR (400 MHz, DMSO) δ 9.91 (s, 1H), 9.56 (s, 1H), 8.96 (s, 1H), 8.69 (s, 1H), 8.27 (s, 1H), 7.83 (d, J = 8.3 Hz, 1H), 7.77 (s, 1H), 7.71 (d, J = 8.2 Hz, 1H), 7.29 (s, 1H), 7.14 (d, J = 8.0 Hz, 2H), 6.77 (d, J = 7.8 Hz, 2H), 4.81 (s, 2H), 4.71 (s, 2H), 3.65 (d, J = 6.8 Hz, 2H), 3.32 (s, 2H), 1.20 (s, 1H), 0.58 (d, J = 7.3 Hz, 2H), 0.35 (d, J = 4.4 Hz, 2H).

## Compound 46

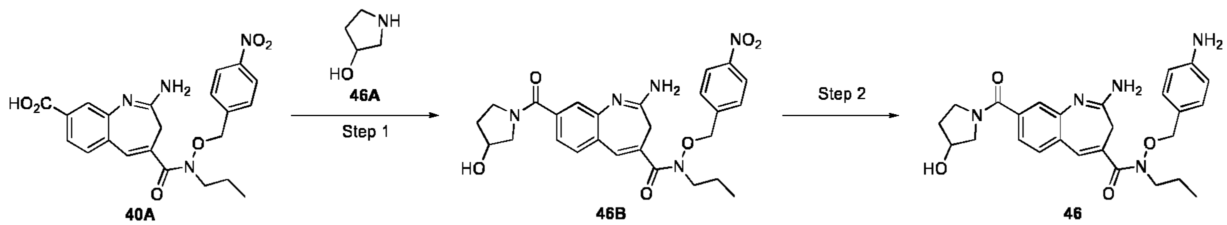


40A 46B 46

**Step 1** Compound **40A** (100 mg, 0.228 mmol, 1 eq.) and compound **46A** (19.87 mg, 0.228 mmol, 1 eq.) were dissolved in DMA (3 mL), then EDCI (174.90 g, 0.912 mmol, 4 eq.) was added in one portion. The system was stirred at room temperature overnight. When LCMS indicated the depletion of compound **40A**, the reaction solution was directly used in the next reaction without treatment. MS-ESI: m/z 508.2 $[M+H]^+$.

**Step 2** Raney nickel (0.5 g) was added to the untreated compound **46B** solution from the previous step. The system was purged twice with hydrogen and stirred overnight at room temperature under hydrogen atmosphere. When LCMS indicated the depletion of the starting materials, the reaction solution was filtered through celite and purified by preparative HPLC to give a yellow solid compound **46** (8.2 mg, 7.5% yield over the two steps). MS-ESI: m/z 478.2 $[M+H]^+$.

$^1$H NMR (400 MHz, DMSO) δ 8.14 (s, 1H), 7.38 (d, J = 6.5 Hz, 2H), 7.17 (d, J = 20.9 Hz, 2H), 7.06 (s, 1H), 6.87 (d, J = 7.4 Hz, 2H), 6.41 (d, J = 7.3 Hz, 2H), 4.59 (s, 2H), 4.28 (d, J = 37.6 Hz, 2H), 3.59 (dd, J = 23.4, 7.9 Hz, 7H), 3.25 (d, J = 10.5 Hz, 1H), 2.82 (d, J = 14.7 Hz, 1H), 1.88 (d, J = 56.6 Hz, 3H), 1.63 (d, J = 6.4 Hz, 2H), 0.89 (s, 3H).

## Compounds 46A and 46B

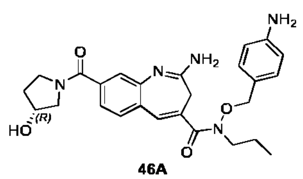

46A

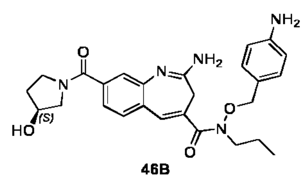

46B

Compound 46 was subjected to chiral resolution via conventional methods to give compounds 46A (1.8 mg)

and 46B (2.1 mg).

Compound 46A: MS-ESI: m/z 478.2 $[M+H]^+$.

Compound 46B: MS-ESI: m/z 478.2 $[M+H]^+$.

## Compound 47

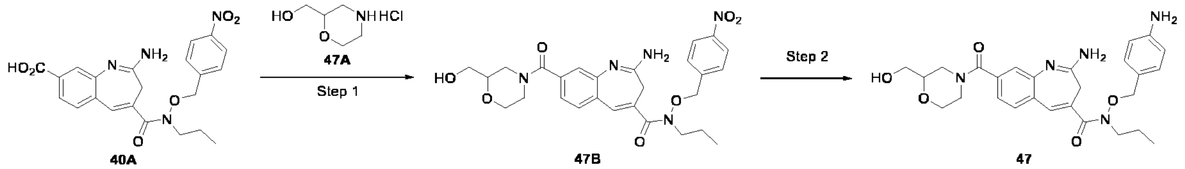


40A 47B 47

**Step 1** Compound **40A** (150 mg, 0.342 mmol, 1 eq.) was dissolved in DMF (5 mL), then HOBT (55.48 mg, 0.411 mmol, 1.2 eq.), EDCI (78.70 mg, 0.411 mmol, 1.2 eq.), and DIEA (132.65 mg, 1.03 mmol, 3 eq.) were added at 0 °C. The system was stirred for 10 min at 0 °C before compound **47A** (62.65 mg, 0.411 mmol, 1.2 eq.) was added, and warmed to room temperature and stirred overnight after the addition. When LCMS indicated the completion of the reaction, the reaction solution was poured into 15 mL of water and extracted with DCM (5 mL × 3). The organic phases were combined, washed with saturated brine (15 mL), dried over sodium sulfate, concentrated at reduced pressure, mixed with silica gel, and purified by column chromatography (DCM:MeOH = 20: 1) to give a brown solid compound **47B** (100 mg, 50% yield). MS-ESI: m/z 538.3 $[M+H]^+$.

**Step 2** Compound **47B** (100 mg, 0.186 mmol, 1 eq.) was dissolved in methanol (10 mL), then Raney nickel (0.01 g) was added with the temperature controlled at 0 °C. The system was purged twice with hydrogen and stirred at room temperature for 1 hour under hydrogen atmosphere. When LCMS indicated the depletion of the starting materials, the reaction solution was filtered through celite, concentrated by rotary evaporation, and purified by preparative HPLC to give an off-white solid **47** in the form of trifluoroacetate (18 mg, 18% yield). MS-ESI: m/z 508.3 $[M+H]^+$.

$^1H$ NMR (400 MHz, DMSO) δ 7.58 (dd, J = 20.0, 8.4 Hz, 2H), 7.43 (dd, J = 10.8, 2.7 Hz, 2H), 7.17 (s, 1H), 6.95 (d, J = 8.2 Hz, 2H), 6.57 (dd, J = 49.2, 8.4 Hz, 3H), 4.67 (s, 2H), 3.71 - 3.64 (m, 3H), 3.24 (s, 2H), 2.53 (d, J = 1.9 Hz, 10H), 2.07 (s, 1H), 1.67 (dd, J = 14.3, 7.2 Hz, 2H), 0.92 (t, J = 7.4 Hz, 3H).

## Compound 48

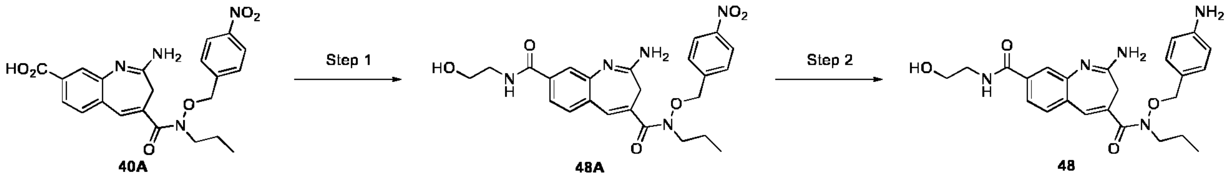


40A 48A 48

**Step 1** Compound **40A** (150 mg, 0.342 mmol, 1 eq.) was dissolved in DMF (5 mL), then HOBT (55.48 mg, 0.411 mmol, 1.2 eq.), EDCI (78.70 mg, 0.411 mmol, 1.2 eq.), and DIEA (132.65 mg, 1.03 mmol, 3 eq.) were added at 0 °C. The system was stirred for 10 min at 0 °C before ethanolamine (25.08 mg, 0.411 mmol, 1.2 eq.) was added, and warmed to room temperature and stirred overnight after the addition. When LCMS indicated the completion of the reaction, the reaction solution was poured into 15 mL of water and extracted with DCM (5 mL × 3). The organic phases were combined, washed with saturated brine (15 mL), dried over sodium sulfate, concentrated at reduced pressure, mixed with silica gel, and purified by column chromatography (DCM:MeOH = 20: 1) to give a pale yellow solid compound **48A** (90 mg, 50% yield). MS-ESI: m/z 482.2 $[M+H]^+$.

**Step 2** Compound **48A** (90 mg, 0.187 mmol, 1 eq.) was dissolved in methanol (10 mL), then Raney nickel (0.01 g) was added with the temperature controlled at 0 °C. The system was purged twice with hydrogen and stirred at room temperature for 1 hour under hydrogen atmosphere. When LCMS indicated the depletion of the starting materials, the reaction solution was filtered through celite, concentrated by rotary evaporation, and purified by preparative HPLC to give an off-white solid **48** trifluoroacetate (12 mg, 13% yield). MS-ESI: m/z 452.2 $[M+H]^+$.

$^1H$ NMR (400 MHz, DMSO) δ 7.90 - 7.83 (m, 2H), 7.59 (dd, J = 26.0, 8.4 Hz, 2H), 7.17 (s, 1H), 6.95 (d, J = 8.3 Hz, 2H), 6.63 (d, J = 8.5 Hz, 1H), 6.52 (d, J = 8.2 Hz, 2H), 4.67 (s, 2H), 3.70 (t, J = 6.9 Hz, 3H), 3.56 - 3.51 (m, 5H), 3.40 - 3.34 (m, 3H), 3.22 (s, 1H), 2.07 (s, 1H), 1.67 (dd, J = 14.3, 7.2 Hz, 2H), 0.92 (t, J = 7.4 Hz, 3H).

## Compound 49

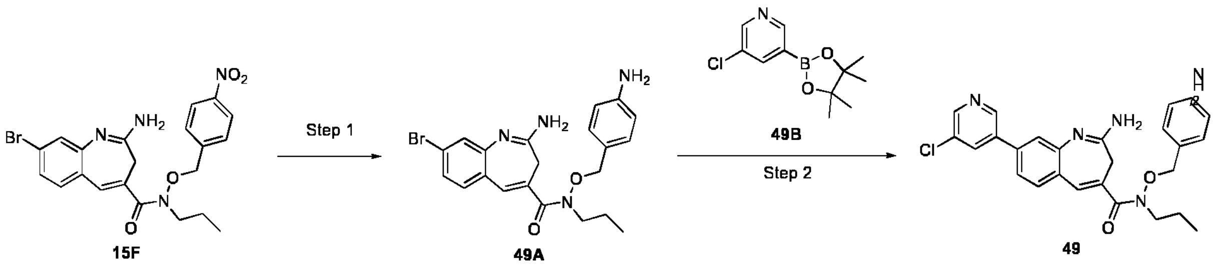


15F 49A 49

**Step 1** Compound **15F** (1.0 g, 2.11 mmol, 1 eq) was dissolved in 20 mL of methanol, then Raney nickel was added. The system was reacted for 6 hours. When LCMS indicated the depletion of the starting materials, the reaction solution was filtered through celite. The filter cake was washed with 10 mL of methanol, and the filtrate was collected and concentrated at reduced pressure to give a yellow foamy solid **49A** (0.7 g, 75% yield). MS-ESI: m/z 443.1 $[M+H]^+$.

**Step 2** Compound **49A** (100 mg, 0.22 mmol, 1 eq.), dioxane (4 mL), and water (0.4 mL) were added into a 10-mL microwave reaction tube, then compound **49B** (54 mg, 0.22 mmol, 1 eq.) and anhydrous potassium phosphate (143 mg, 0.66 mmol, 3 eq.) were added. The system was purged with argon twice before $Pd(dppf)Cl_2$ (15 mg, 15% ) was added, then purged with argon twice again, and reacted in a microwave reactor at 90 °C for 2 hours. When LCMS indicated the depletion of the starting materials, the reaction solution was poured into 15 mL of water and extracted with ethyl acetate (20 mL × 2). The organic phases were combined, dried over sodium sulfate, concentrated at reduced pressure, and purified by preparative HPLC to give a solid **49** (19 mg, 17.7% yield). MS-ESI: m/z 476.2 $[M+H]^+$.

$^1$H NMR (400 MHz, DMSO) δ 8.847-8.842 (d, J=2, 1H), 8.579-8.573 (d, J=2.4, 1H), 8.224-8.214 (t, $J_1$=2, $J_2$=4, 2H), 7.449-7.427 (dd, J=8.8, 3H), 7.063 (s, 1H), 6.83 -6.809(d, J=8.4, 1H), 6.356-6.335 (d, J=8.4, 2H), 4.543 (s, 2H), 3.6-3.565 (m, 1H), 2.838 (s, 2H), 1.614 - 1.561 (dd, $J_1$= 7.2, $J_2$=14 Hz, 2H), 0.861 - 0.824 (t, $J_1$ = 7.2, $J_2$=14.8 Hz, 3H).

## Compound 50

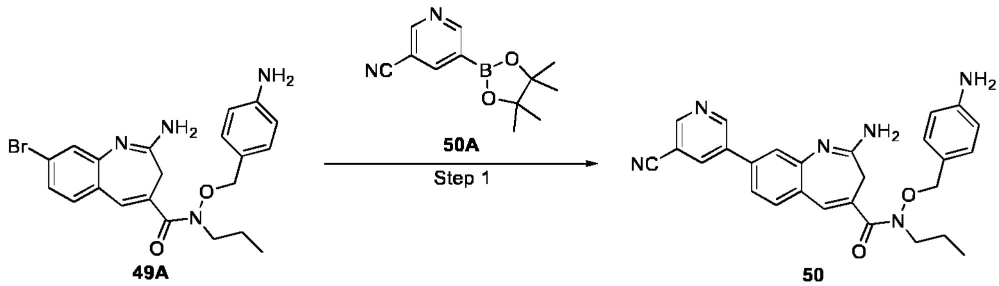


49A 50

**Step 1** Compound **49A** (100 mg, 0.22 mmol, 1 eq.), dioxane (4 mL), and water (0.4 mL) were added into a 10-mL microwave reaction tube, then compound **50A** (52 mg, 0.22 mmol, 1 eq.) and anhydrous potassium phosphate (143 mg, 0.66 mmol, 3 eq.) were added. The system was purged with argon twice before $Pd(dppf)Cl_2$ (15 mg, 15% $^w/_w$ ) was added, then purged with argon twice again, and reacted in a microwave reactor at 90 °C for 2 hours. When LCMS indicated the depletion of the starting materials, the reaction solution was poured into 15 mL of water and extracted with ethyl acetate (20 mL × 2). The organic phases were combined, dried over sodium sulfate, concentrated at reduced pressure, and purified by preparative HPLC to give a solid compound **50** (21 mg, 19.8% yield). MS-ESI: m/z 467.2 $[M+H]^+$.

$^1$H NMR (400 MHz, DMSO) δ 9.145- 9.141 (d, J=1.6, 1H), 8.385- 8.379 (d, J=2.4, 1H), 8.122- 8.101 (d, J=8.4, 1H), 7.475- 7.405 (m, 3H), 7.100 (s, 1H), 6.888 - 6.867 (d, J=8.4, 4H), 6.416 - 6.395 (d, J=8.4, 2H), 5.163 (s, 2H), 4.595 (s, 2H), 3.652- 3.617 (t, J=6.8, J=14, 4H), 2.773 (s, 2H), 1.674 - 1.620 (dd, $J_1$=7.2, $J_2$=14, 2H), 0.923 - 0.886 (t, $J_1$=7.2, $J_2$=14.8, 3H).

## Compound 51

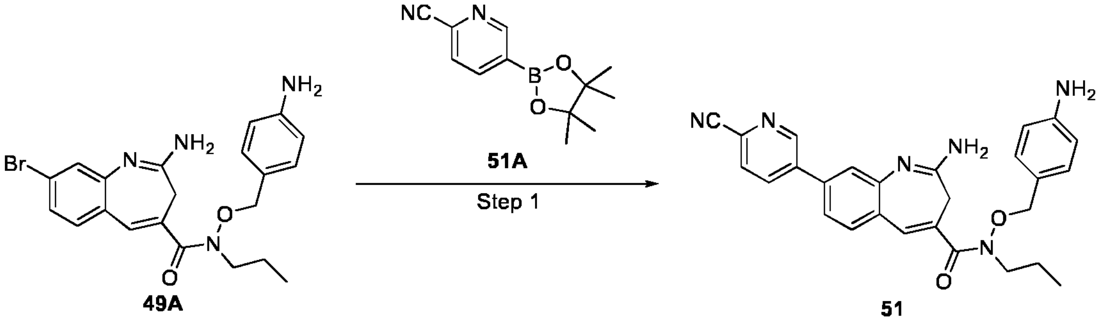


49A 51

**Step 1** Compound **49A** (100 mg, 0.22 mmol, 1 eq.), dioxane (4 mL), and water (0.4 mL) were added into a 10-mL microwave reaction tube, then compound **51A** (52 mg, 0.22 mmol, 1 eq.) and anhydrous potassium phosphate (143 mg, 0.66 mmol, 3 eq.) were added. The system was purged with argon twice before Pd(dppf)$Cl_2$ (15 mg, 15% $^{w}/_{w}$ ) was added, then purged with argon twice again, and reacted in a microwave reactor at 90 °C for 2 hours. When LCMS indicated the depletion of the starting materials, the reaction solution was poured into 15 mL of water and extracted with ethyl acetate (20 mL × 2). The organic phases were combined, dried over sodium sulfate, concentrated at reduced pressure, and purified by preparative HPLC to give a solid compound **51** (19 mg, 18.1% yield). MS-ESI: m/z 467.2 $[M+H]^+$.

$^1H$ NMR (400 MHz, DMSO) δ9.229-9.223 (d, J=2.4, 1H), 9.003-8.998 (d, J=2, 1H), 8.685- 8.675 (d, $J_1$=2, $J_2$=4, 1H), 7.469- 7.372 (m, 3H), 7.100 (s, 1H), 6.892- 6.871 (d, J=8.4, 4H), 6.419 - 6.398 (d, J=8.4, 2H), 5.164 (s, 2H), 4.597 (s, 2H), 3.652- 3.617 (t, J=6.8, J=14, 4H), 2.769 (s, 2H), 1.674 - 1.621 (dd, $J_1$=6.8, $J_2$=14, 2H), 0.923 - 0.886 (t, $J_1$=7.2, $J_2$=14.8, 3H).

## Compound 52

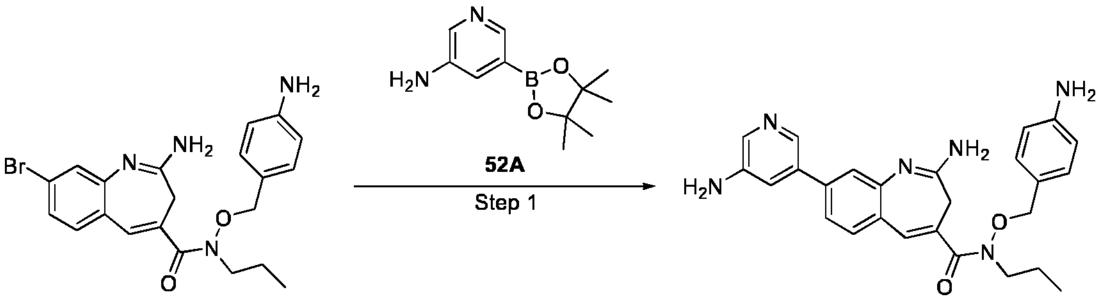


49A 52

**Step 1** Compound **49A** (100 mg, 0.22 mmol, 1 eq.), dioxane (4 mL), and water (0.4 mL) were added into a 10-mL microwave reaction tube, then compound **52A** (66 mg, 0.22 mmol, 1 eq.) and anhydrous potassium phosphate (143 mg, 0.66 mmol, 3 eq.) were added. The system was purged with argon twice before Pd(dppf)$Cl_2$ (15 mg, 15% ) was added, then purged with argon twice again, and reacted in a microwave reactor at 90 °C for 2 hours. When LCMS indicated the depletion of the starting materials, the reaction solution was poured into 15 mL of water and extracted with ethyl acetate (20 mL × 2). The organic phases were combined, dried over sodium sulfate, concentrated at reduced pressure, and purified by preparative HPLC to give a solid compound **52** (20 mg, 19.2% yield). MS-ESI: m/z 457.2 $[M+H]^+$.

$^1H$ NMR (400 MHz, DMSO) δ8.087-8.082 (d, J=2, 1H), 7.978-7.972 (d, J=2.4, 1H), 7.546-7.484 (m, 3H), 7.206-7.195 (t, $J_1$=2, $J_2$=4.4, 1H), 7.145 (s, 1H), 6.910- 6.889 (d, J=8.4, 2H), 6.435 - 6.415 (d, J=8, 2H), 5.502 (s, 2H), 4.626 (s, 2H), 3.681 - 3.646 (t, $J_1$=6.8, $J_2$=14, 2H), 3.098 (s, 2H), 1.689 - 1.635 (dd, $J_1$=7.2, $J_2$=14.4 2H), 0.933 - 0.896 (t, $J_1$=7.2, $J_2$=14.8, 3H).

## Compound 53

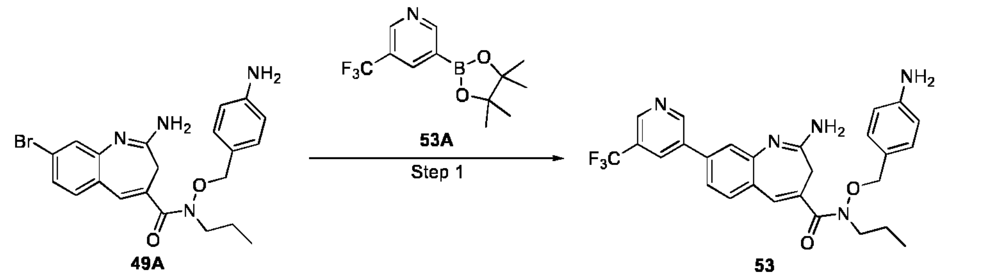


49A 53A 53

**Step 1** Compound **49A** (100 mg, 0.22 mmol, 1 eq.), dioxane (4 mL), and water (0.4 mL) were added into a 10-mL microwave reaction tube, then compound **53A** (61.6 mg, 0.22 mmol, 1 eq.) and anhydrous potassium phosphate (143 mg, 0.66 mmol, 3 eq.) were added. The system was purged with argon twice before Pd(dppf)$Cl_2$ (15 mg, 15% $^w/_w$ ) was added, then purged with argon twice again, and reacted in a microwave reactor at 90 °C for 2 hours. When LCMS indicated the depletion of the starting materials, the reaction solution was poured into 15 mL of water and extracted with ethyl acetate (20 mL × 2). The organic phases were combined, dried over sodium sulfate, concentrated at reduced pressure, and purified by preparative HPLC to give a solid compound **53** (22 mg, 19.1% yield). MS-ESI: m/z 510.2 $[M+H]^+$.

$^1$H NMR (400 MHz, DMSO) δ9.261- 9.256 (d, J=2, 1H), 8.990 (s, 1H), 8.502 (s, 1H), 7.592- 7.535 (m, 3H), 7.142 (s, 1H), 6.899- 6.878 (d, J=8.4, 2H), 6.424 - 6.403 (d, J=8.4, 2H), 4.615 (s, 2H), 3.674-3.639 (t, $J_1$=6.8, $J_2$=14, 2H), 2.920 (s, 2H), 1.685 - 1.632 (dd, $J_1$=7.2, $J_2$=21.2, 2H), 0.932 - 0.895 (t, $J_1$=7.6, $J_2$=14.8, 3H).

## Compound 54

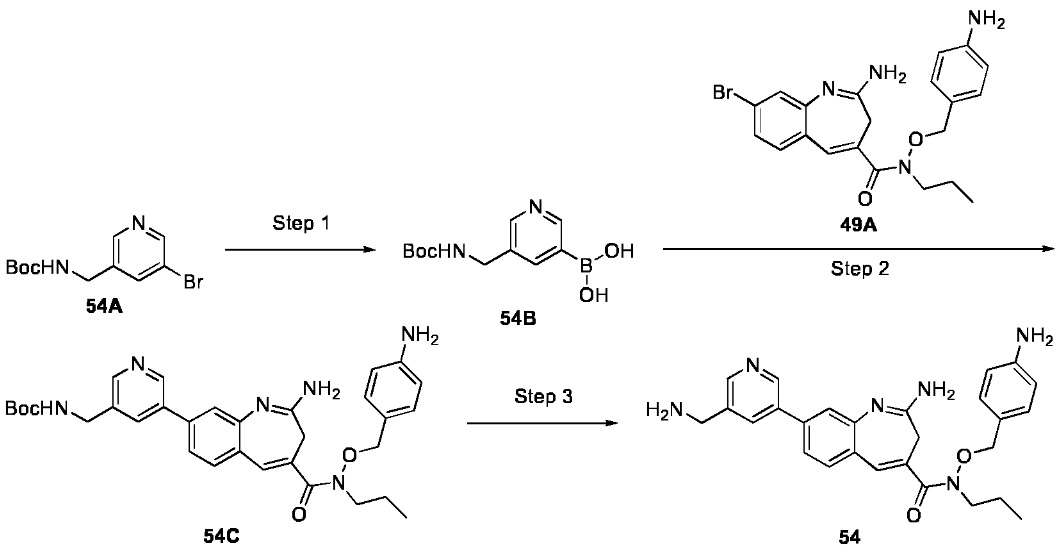


54A 54B 49A 54C 54

**Step 1** Compound **54A** (0.5 g, 1.74 mmol, 1 eq.) was dissolved in dioxane (15 mL), then $B_2Pin_2$ (530 mg, 2.09 mmol, 1.1 eq.) and AcOK (342 mg, 3.48 mmol, 2 eq.) were added. The system was purged with argon twice before Pd(dppf)Cl2 (75 mg, 15% $^w/_w$ ) was added in one portion, then purged with argon twice again, and reacted in a microwave reactor at 90 °C for 3 hours. When LCMS indicated the depletion of the starting materials, the reaction solution was cooled to room temperature, extracted thrice with water (15 mL) and EA (20 mL), dried over sodium sulfate, and concentrated to give a crude brown solid **54B** (400 mg, 60% purity, 51% yield). MS-ESI: m/z 253.2 $[M+H]^+$.

**Step 2** Compound **54B** (100 mg, 0.198 mmol, 1 eq.) was added to a mixed solvent of dioxane (5 mL) and water (0.5 mL), then compound **49A** (125.63 mg, 0.198 mmol, 1 eq.) and anhydrous potassium phosphate (126.31 mg, 0.595 mmol, 3 eq.) were added. The system was purged with argon twice before Pd(dppf)$Cl_2$ (15 mg, 15% $^w/_w$ ) was added, then purged with argon twice again, heated to 90 °C and reacted for 4 hours. When LCMS indicated the depletion of the starting materials, the reaction solution was poured into 15 mL of water and extracted with ethyl acetate (15 mL × 3). The organic phases were combined, dried over sodium sulfate, and concentrated at reduced pressure to give a crude

brown solid **54C** (90 mg, 70% purity, 40% yield). MS-ESI: m/z 571.4 $[M+H]^+$.

**Step 3** Compound **54C** (90 mg, 0.157 mmol, 1 eq.) was dissolved in DCM (3 mL), then TFA (1 mL) was added. The system was stirred at room temperature for 2 h. When LCMS indicated the depletion of the starting materials, the reaction solution was filtered through celite, concentrated by rotary evaporation, and purified by preparative HPLC to give a pale yellow solid **54** in the form of trifluoroacetate (20 mg, 14% yield). MS-ESI: m/z 471.2 $[M+H]^+$.

$^1$H NMR (400 MHz, DMSO) δ 9.91 (s, 1H), 9.61 (s, 1H), 9.00 (s, 1H), 8.74 (s, 1H), 8.34 (d, J = 26.7 Hz, 4H), 7.76 (dd, J = 17.2, 5.8 Hz, 3H), 7.26 (s, 1H), 7.04 (d, J = 8.0 Hz, 2H), 6.63 (d, J = 7.7 Hz, 2H), 4.73 (s, 2H), 4.23 (d, J = 5.3 Hz, 3H), 3.74 (s, 1H), 3.73 (s, 1H), 3.71 (s, 1H), 3.30 (s, 2H), 1.70 (dd, J = 14.2, 7.1 Hz, 2H), 0.94 (t, J = 7.3 Hz, 3H).

## Compound 55

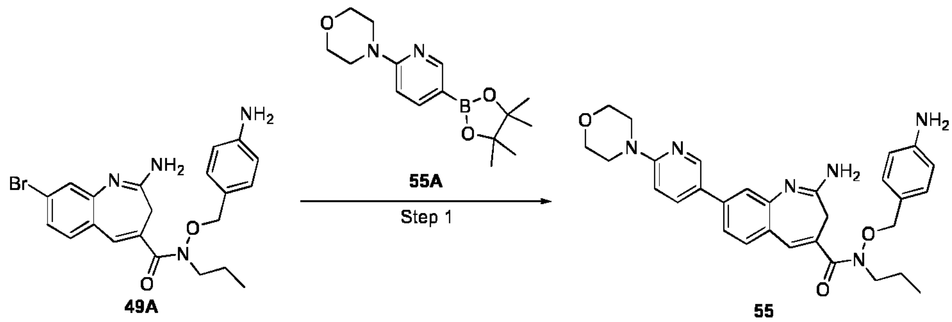


49A 55

**Step 1** Compound **49A** (100 mg, 0.22 mmol, 1 eq.), dioxane (4 mL), and water (0.4 mL) were added into a 10-mL microwave reaction tube, then compound **55A** (66 mg, 0.22 mmol, 1 eq.) and anhydrous potassium phosphate (143 mg, 0.66 mmol, 3 eq.) were added. The system was purged with argon twice before Pd(dppf)$Cl_2$ (15 mg, 15% w/w) was added, then purged with argon twice again, and reacted in a microwave reactor at 90 °C for 2 hours. When LCMS indicated the depletion of the starting materials, the reaction solution was poured into 15 mL of water and extracted with ethyl acetate (20 mL × 2). The organic phases were combined, dried over sodium sulfate, concentrated at reduced pressure, and purified by preparative HPLC to give a solid compound **55** (15 mg, 12.8% yield). MS-ESI: m/z 527.2 $[M+H]^+$.

$^1$H NMR (400 MHz, DMSO) δ 8.528-8.523 (d, J=2, 1H), 7.954-7.932 (d, J=8.8, 1H), 7.364-7.344 (d, J=8, 1H), 7.285 (s, 1H), 7.253-7.233 (d, J=8, 1H), 7.095 (s, 1H), 6.954 -6.901(m, 4H), 6.450 -6.430 (d, J=8, 2H), 5.185 (s, 2H), 4.610 (s, 2H), 3.741-3.729 (d, J=4.8, 4H), 3.645-3.628 (d, J=6.8, 2H), 3.533-3.522 (d, J=4.4, 4H), 2.787 (s, 2H), 1.688 - 1.633 (dd, $J_1$=7.6, $J_2$=14.8 2H), 0.936 - 0.899 (t, $J_1$=7.6, $J_2$=14.8, 3H).

## Compound 56

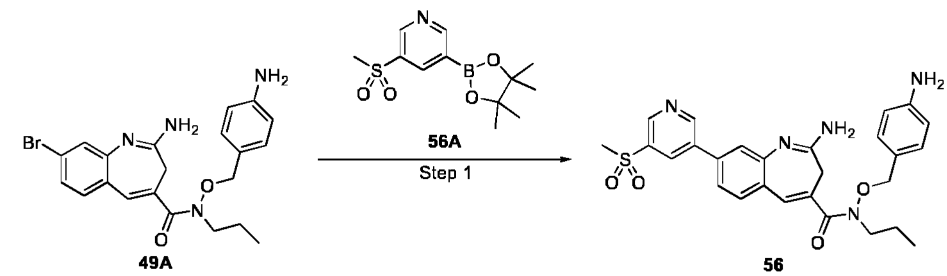


49A 56

**Step 1** Compound **49A** (100 mg, 0.22 mmol, 1 eq.), dioxane (4 mL), and water (0.4 mL) were added into a 10-mL microwave reaction tube, then compound **56A** (64 mg, 0.22 mmol, 1 eq.) and anhydrous potassium phosphate (143 mg, 0.66 mmol, 3 eq.) were added. The system was purged with argon twice before Pd(dppf)$Cl_2$ (15 mg, 15% ) was added, then purged with argon twice again, and reacted in a microwave reactor at 90 °C for 2 hours. When LCMS indicated the depletion of the starting materials, the reaction solution was poured into 15 mL of water and extracted with ethyl acetate (20 mL × 2). The organic phases were combined, dried over sodium sulfate, concentrated at reduced pressure, and purified by preparative HPLC to give a solid compound **56** (22 mg, 18.7% yield). MS-ESI: m/z 520.2 $[M+H]^+$.

$^1$H NMR (400 MHz, DMSO) δ 9.280-9274 (d, J=2.4, 1H), 9.040-9035 (d, J=2, 1H), 8.584-8.574 (t, $J_1$=2, $J_2$=4, 1H), 7.520-7.432 (m, 3H), 7.116 (s, 1H), 6.902 -6.863(m, 4H), 6.428 -6.407 (d, J=8.4, 2H), 5.168 (s, 2H), 4.607 (s, 2H), 3.664-3.629 (t, $J_1$=6.8, $J_2$=14, 2H), 3.43 (s, 3H), 2.781 (s, 2H), 1.702 - 1.612 (m, 2H), 0.932 - 0.895 (t, $J_1$=7.2, $J_2$=14.8, 3H).

**Synthesis of compounds 58-73: the following compounds were synthesized using a synthetic method similar to that of compound 45.**

| Compound No. | Structural formula | Molecular weight |
|---|---|---|
| 58 | 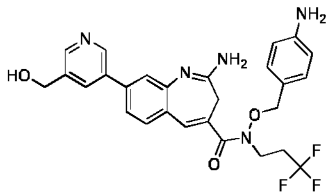 | MS-ESI: m/z 526.2 [M+H]+ |
| 59 | 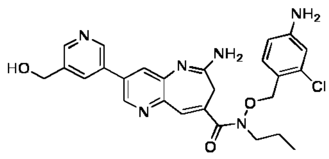 | MS-ESI: m/z 507.2 [M+H]+ |
| 60 | 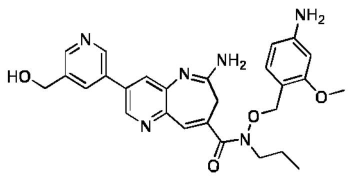 | MS-ESI: m/z 503.2 [M+H]+ |
| 61 | 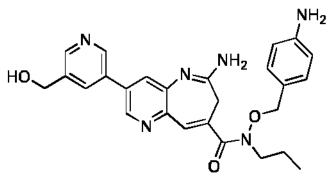 | MS-ESI: m/z 473.2 [M+H]+ |
| 62 | 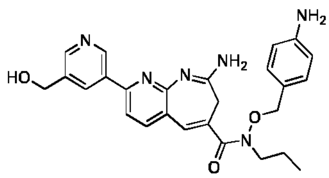 | MS-ESI: m/z 473.2 [M+H]+ |
| 63 | 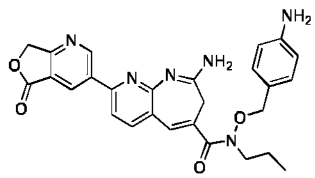 | MS-ESI: m/z 499.2 [M+H]+ |
| 64 | 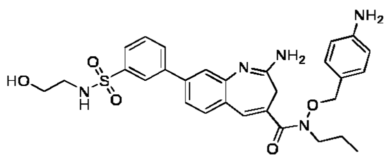 | MS-ESI: m/z 564.2 [M+H]+ |
| 65 | 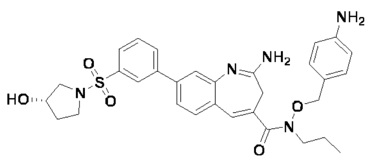 | MS-ESI: m/z 509.2 [M+H]+ |

(continued)

| Compound No. | Structural formula | Molecular weight |
|---|---|---|
| 66 | 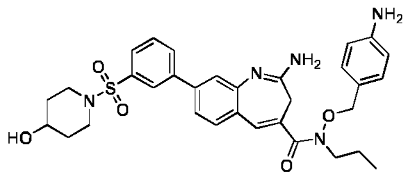 | MS-ESI: m/z 604.3 [M+H]+ |
| 67 | 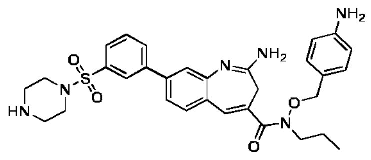 | MS-ESI: m/z 589.3 [M+H]+ |
| 68 | 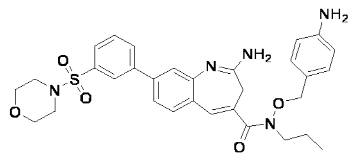 | MS-ESI: m/z 590.2 [M+H]+ |
| 69 | 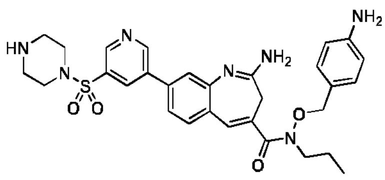 | MS-ESI: m/z 590.2 [M+H]+ |
| 70 | 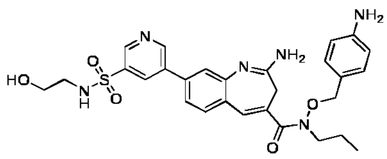 | MS-ESI: m/z 565.2 [M+H]+ |
| 71 | 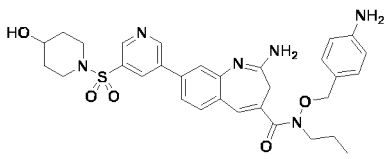 | MS-ESI: m/z 605.2 [M+H]+ |
| 72 | 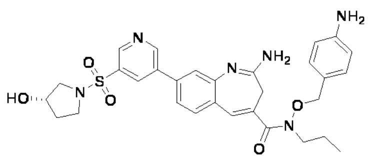 | MS-ESI: m/z 591.2 [M+H]+ |
| 73 | 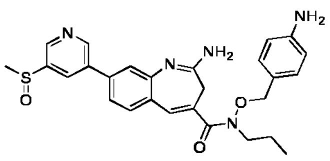 | MS-ESI: m/z 504.2 [M+H]+ |

**Synthesis of compounds 74-99: the following compounds were synthesized using a synthetic method similar to that of compound 37.**

| Compound No. | Structural formula | Molecular weight |
|---|---|---|
| 74 | 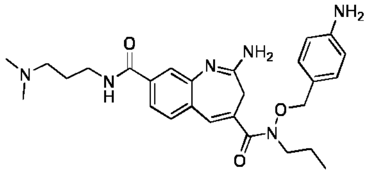 | MS-ESI: m/z 493.3 [M+H]+ |
| 75 | 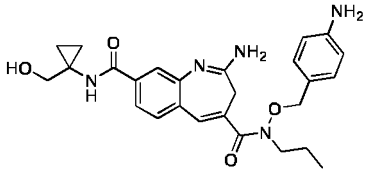 | MS-ESI: m/z 478.2 [M+H]+ |
| 76 | 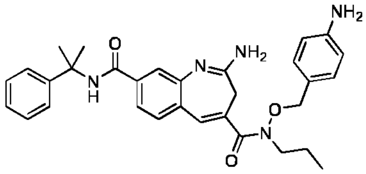 | MS-ESI: m/z 526.3 [M+H]+ |
| 77 | 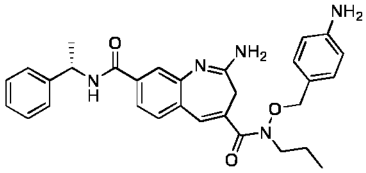 | MS-ESI: m/z 512.3 [M+H]+ |
| 78 | 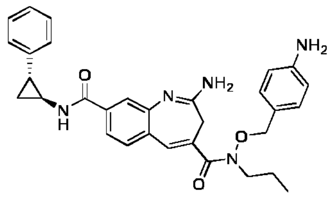 | MS-ESI: m/z 524.3 [M+H]+ |
| 79 | 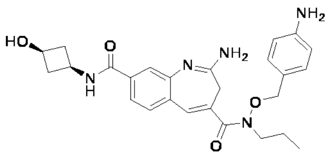 | MS-ESI: m/z 478.2 [M+H]+ |
| 80 | 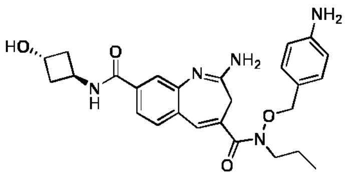 | MS-ESI: m/z 478.2 [M+H]+ |
| 81 | 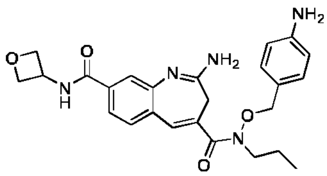 | MS-ESI: m/z 464.2 [M+H]+ |

(continued)

| Compound No. | Structural formula | Molecular weight |
|---|---|---|
| 82 | 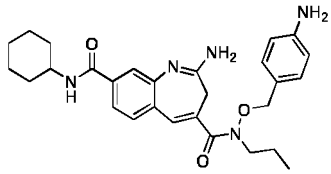 | MS-ESI: m/z 490.3 [M+H]$^+$ |
| 83 | 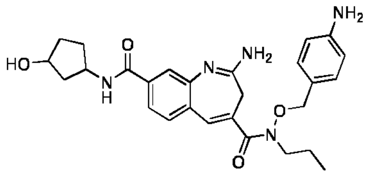 | MS-ESI: m/z 492.2 [M+H]$^+$ |
| 84 | 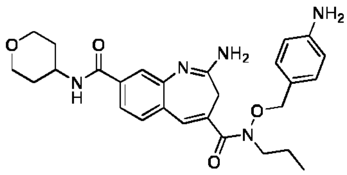 | MS-ESI: m/z 492.3 [M+H]$^+$ |
| 85 | 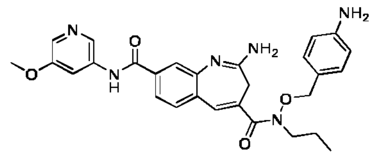 | MS-ESI: m/z 515.2 [M+H]$^+$ |
| 86 | 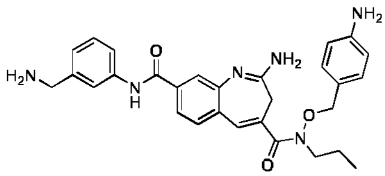 | MS-ESI: m/z 513.2 [M+H]$^+$ |
| 87 | 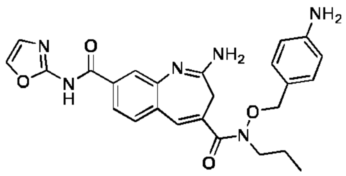 | MS-ESI: m/z 475.2 [M+H]$^+$ |
| 88 | 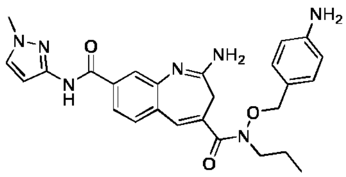 | MS-ESI: m/z 488.2 [M+H]$^+$ |
| 89 | 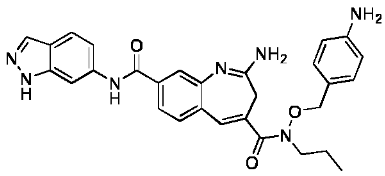 | MS-ESI: m/z 524.2 [M+H]$^+$ |

(continued)

| Compound No. | Structural formula | Molecular weight |
|---|---|---|
| 90 | 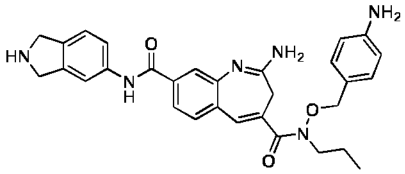 | MS-ESI: m/z 525.2 [M+H]$^+$ |
| 91 | 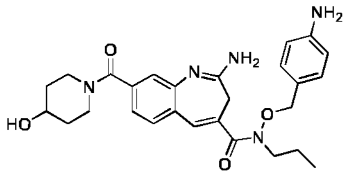 | MS-ESI: m/z 492.2 [M+H]$^+$ |
| 92 | 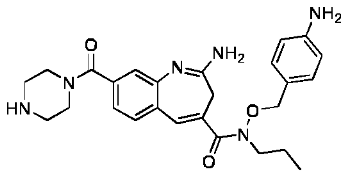 | MS-ESI: m/z 477.2 [M+H]$^+$ |
| 93 | 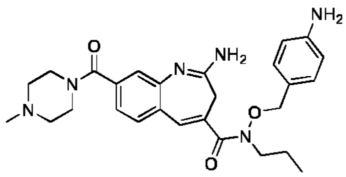 | MS-ESI: m/z 491.3 [M+H]$^+$ |
| 94 | 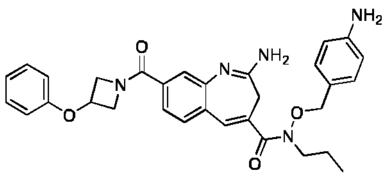 | MS-ESI: m/z 540.2 [M+H]$^+$ |
| 95 | 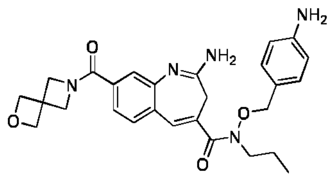 | MS-ESI: m/z 490.2 [M+H]$^+$ |
| 96 | 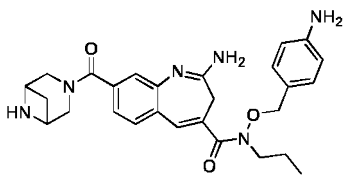 | MS-ESI: m/z 489.2 [M+H]$^+$ |
| 97 | 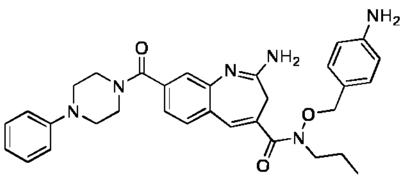 | MS-ESI: m/z 553.3 [M+H]$^+$ |

(continued)

| Compound No. | Structural formula | Molecular weight |
|---|---|---|
| 98 | 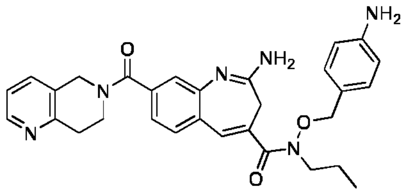 | MS-ESI: m/z 525.3 [M+H]+ |
| 99 | 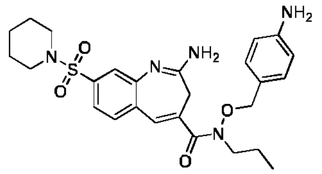 | MS-ESI: m/z 512.2 [M+H]+ |

**Synthesis of compounds 100-199: the following compounds were synthesized using a synthetic method similar to that of compound 37.**

| No. | Structure | LC-MS |
|---|---|---|
| 100 | 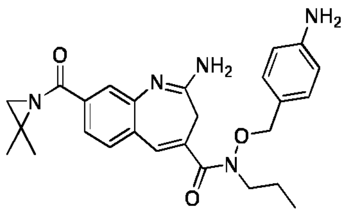 | MS-ESI: m/z 462.2 [M+H]+ |
| 101 | 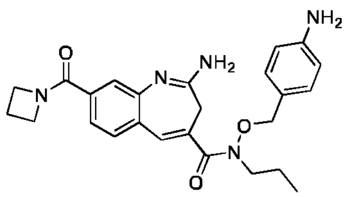 | MS-ESI: m/z 448.2 [M+H]+ |
| 102 | 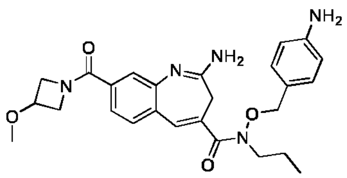 | MS-ESI: m/z 478.2 [M+H]+ |
| 103 | 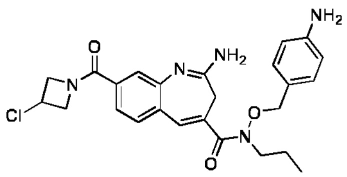 | MS-ESI: m/z 482.2 [M+H]+ |
| 104 | 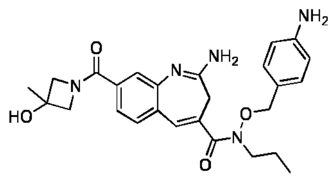 | MS-ESI: m/z 478.2 [M+H]+ |

(continued)

| No. | Structure | LC-MS |
|---|---|---|
| 105 | 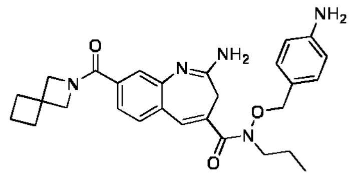 | MS-ESI: m/z 488.3 [M+H]+ |
| 106 | 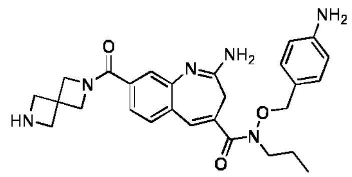 | MS-ESI: m/z 489.3 [M+H]+ |
| 107 | 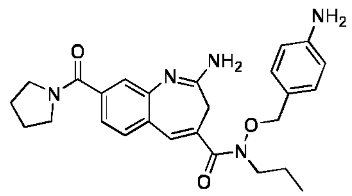 | MS-ESI: m/z 462.2 [M+H]+ |
| 108 | 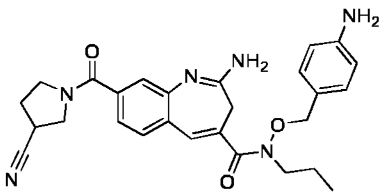 | MS-ESI: m/z 487.2 [M+H]+ |
| 109 | 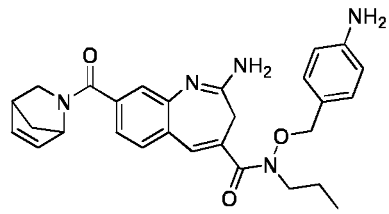 | MS-ESI: m/z 486.2 [M+H]+ |
| 110 | 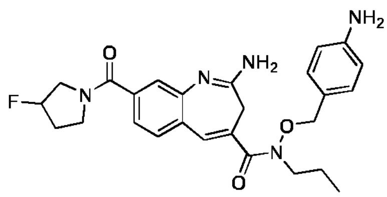 | MS-ESI: m/z 480.2 [M+H]+ |
| 111 | 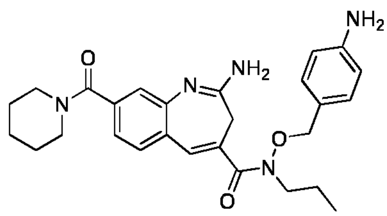 | MS-ESI: m/z 476.3 [M+H]+ |

(continued)

| No. | Structure | LC-MS |
|---|---|---|
| 112 | 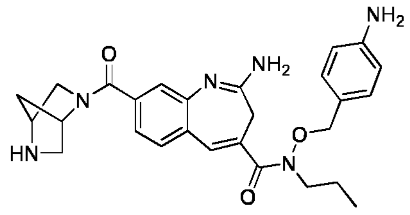 | MS-ESI: m/z 489.3 [M+H]$^+$ |
| 113 | 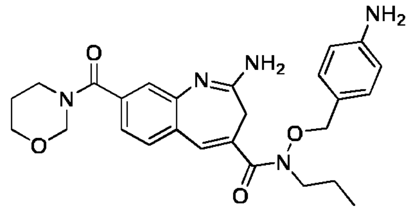 | MS-ESI: m/z 478.2 [M+H]$^+$ |
| 114 | 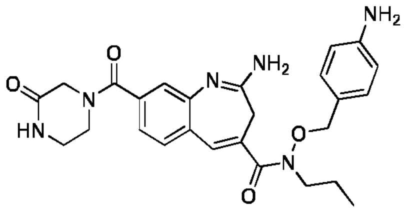 | MS-ESI: m/z 491.2 [M+H]$^+$ |
| 115 | 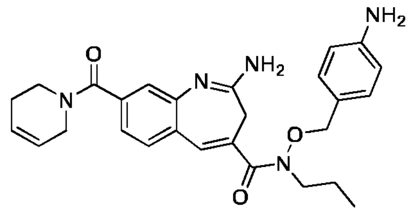 | MS-ESI: m/z 474.2 [M+H]$^+$ |
| 116 | 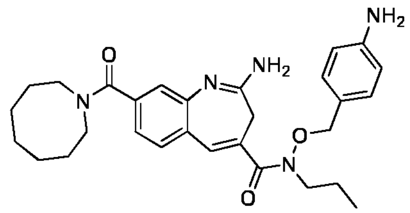 | MS-ESI: m/z 504.3 [M+H]$^+$ |
| 117 | 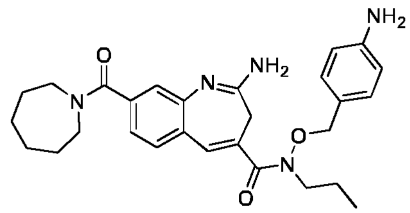 | MS-ESI: m/z 490.3 [M+H]$^+$ |
| 118 | 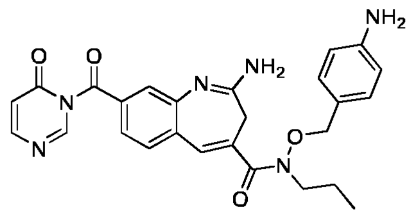 | MS-ESI: m/z 487.2 [M+H]$^+$ |

(continued)

| No. | Structure | LC-MS |
|---|---|---|
| 119 | 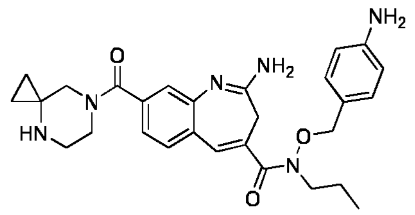 | MS-ESI: m/z 503.3 [M+H]+ |
| 120 | 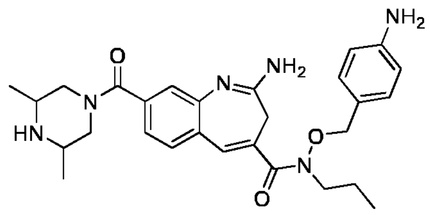 | MS-ESI: m/z 505.3 [M+H]+ |
| 121 | 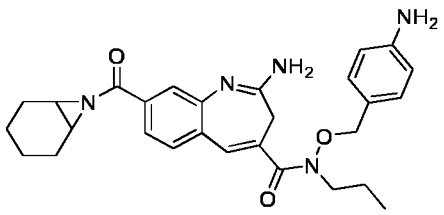 | MS-ESI: m/z 488.3 [M+H]+ |
| 122 | 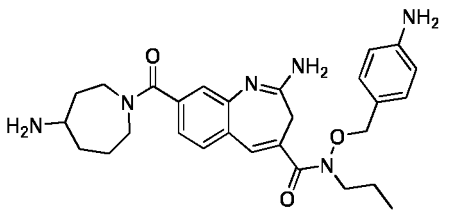 | MS-ESI: m/z 505.3 [M+H]+ |
| 123 | 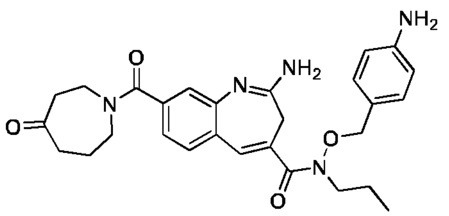 | MS-ESI: m/z 504.3 [M+H]+ |
| 124 | 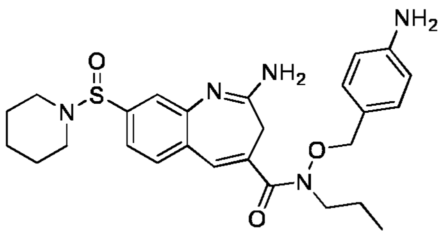 | MS-ESI: m/z 496.2 [M+H]+ |
| 125 | 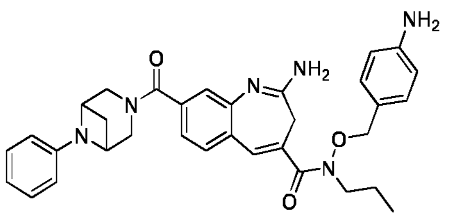 | MS-ESI: m/z 565.3 [M+H]+ |

(continued)

| No. | Structure | LC-MS |
|---|---|---|
| 126 | 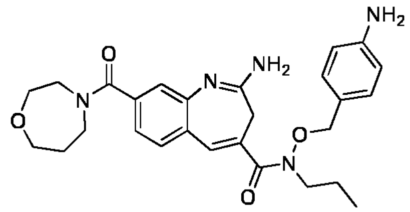 | MS-ESI: m/z 492.3 [M+H]$^+$ |
| 127 | 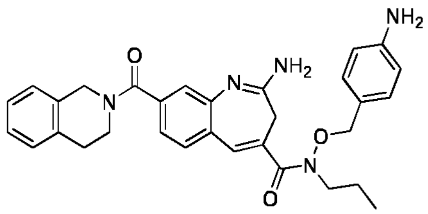 | MS-ESI: m/z 524.3 [M+H]$^+$ |
| 128 | 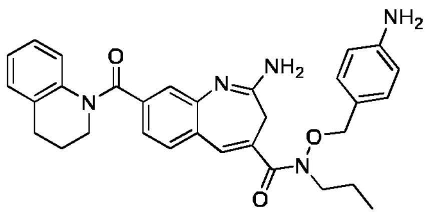 | MS-ESI: m/z 524.3 [M+H]$^+$ |
| 129 | 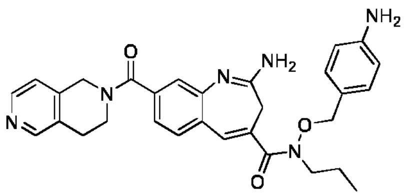 | MS-ESI: m/z 525.3 [M+H]$^+$ |
| 130 | 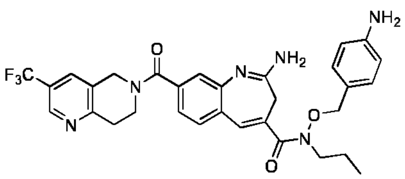 | MS-ESI: m/z 593.2 [M+H]$^+$ |
| 131 | 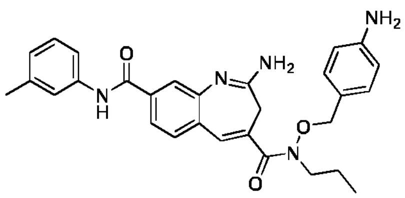 | MS-ESI: m/z 498.2 [M+H]$^+$ |
| 132 | 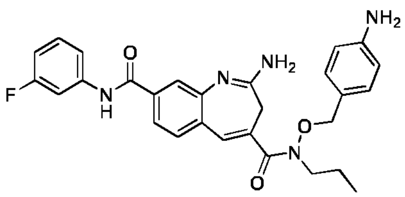 | MS-ESI: m/z 502.2 [M+H]$^+$ |

(continued)

| No. | Structure | LC-MS |
|---|---|---|
| 133 | 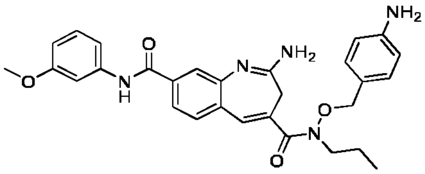 | MS-ESI: m/z 514.2 [M+H]+ |
| 134 | 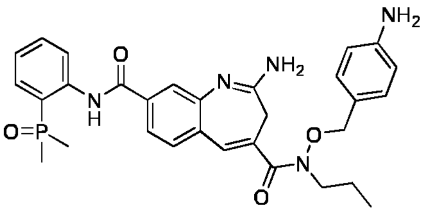 | MS-ESI: m/z 560.2 [M+H]+ |
| 135 | 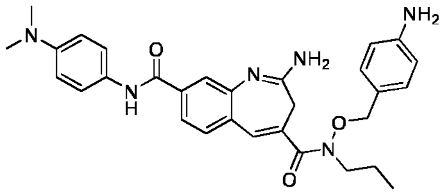 | MS-ESI: m/z 527.3 [M+H]+ |
| 136 | 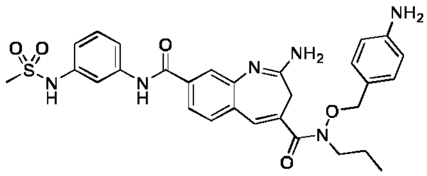 | MS-ESI: m/z 577.2 [M+H]+ |
| 137 | 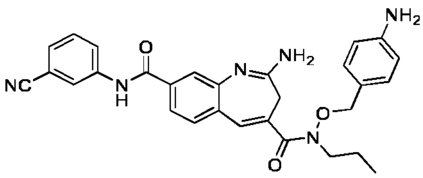 | MS-ESI: m/z 509.2 [M+H]+ |
| 138 | 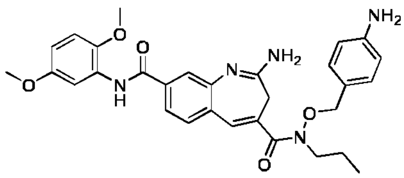 | MS-ESI: m/z 544.3 [M+H]+ |
| 139 | 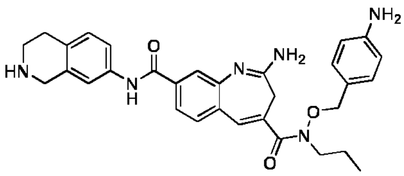 | MS-ESI: m/z 539.3 [M+H]+ |
| 140 | 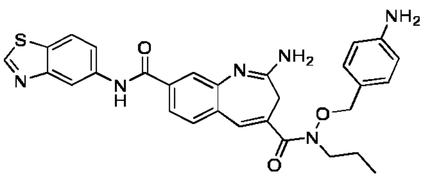 | MS-ESI: m/z 541.2 [M+H]+ |

(continued)

| No. | Structure | LC-MS |
|---|---|---|
| 141 | 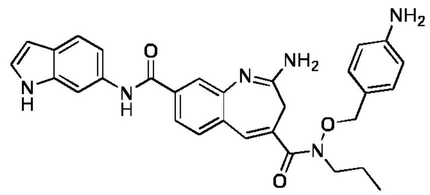 | MS-ESI: m/z 523.2 $[M+H]^+$ |
| 142 | 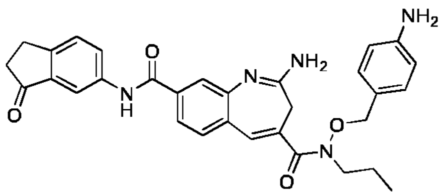 | MS-ESI: m/z 538.2 $[M+H]^+$ |
| 143 | 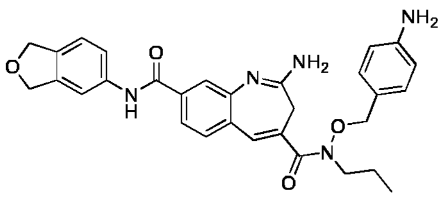 | MS-ESI: m/z 526.2 $[M+H]^+$ |
| 144 | 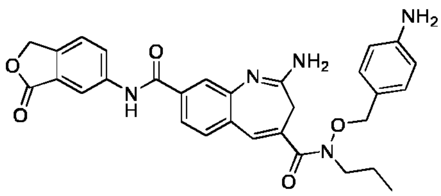 | MS-ESI: m/z 540.2 $[M+H]^+$ |
| 145 | 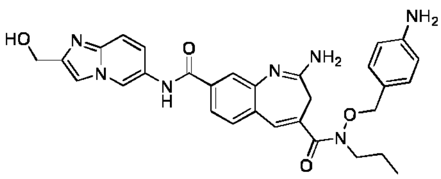 | MS-ESI: m/z 554.2 $[M+H]^+$ |
| 146 | 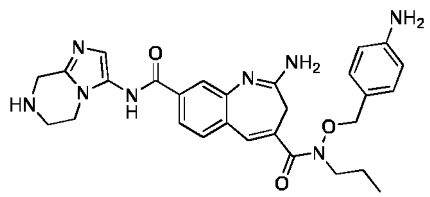 | MS-ESI: m/z 529.3 $[M+H]^+$ |
| 147 | 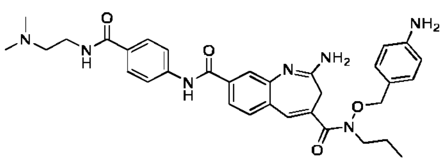 | MS-ESI: m/z 598.3 $[M+H]^+$ |
| 148 | 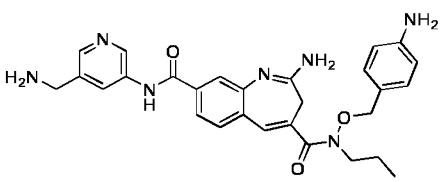 | MS-ESI: m/z 514.3 $[M+H]^+$ |

(continued)

| No. | Structure | LC-MS |
|---|---|---|
| 149 | 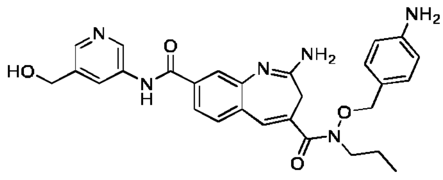 | MS-ESI: m/z 515.2 $[M+H]^+$ |
| 150 | 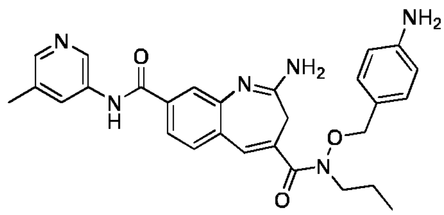 | MS-ESI: m/z 499.2 $[M+H]^+$ |
| 151 | 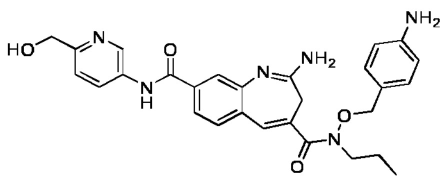 | MS-ESI: m/z 515.2 $[M+H]^+$ |
| 152 | 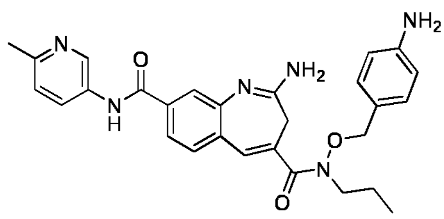 | MS-ESI: m/z 499.2 $[M+H]^+$ |
| 153 | 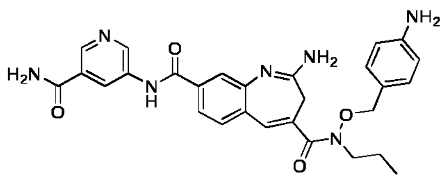 | MS-ESI: m/z 528.2 $[M+H]^+$ |
| 154 | 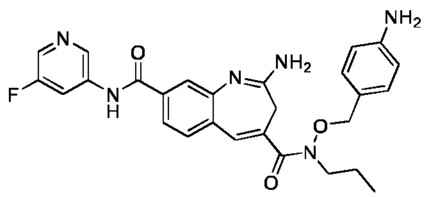 | MS-ESI: m/z 503.2 $[M+H]^+$ |
| 155 | 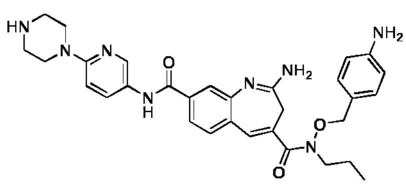 | MS-ESI: m/z 569.3 $[M+H]^+$ |
| 156 | 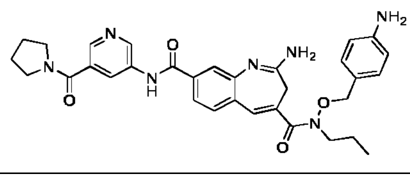 | MS-ESI: m/z 582.3 $[M+H]^+$ |

(continued)

| No. | Structure | LC-MS |
|---|---|---|
| 157 | 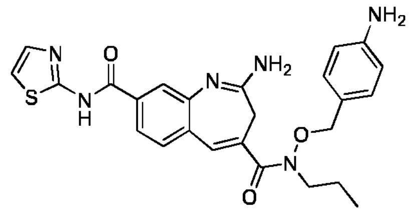 | MS-ESI: m/z 491.2 [M+H]$^+$ |
| 158 | 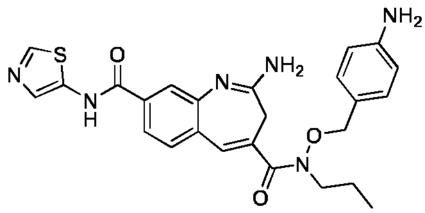 | MS-ESI: m/z 491.2 [M+H]$^+$ |
| 159 | 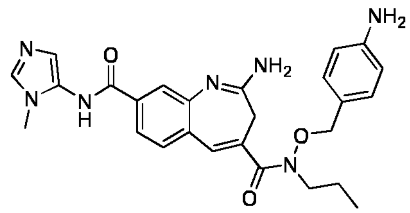 | MS-ESI: m/z 488.2 [M+H]$^+$ |
| 160 | 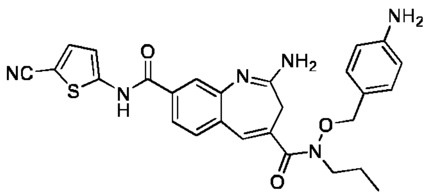 | MS-ESI: m/z 515.2 [M+H]$^+$ |
| 161 | 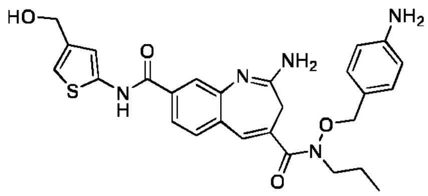 | MS-ESI: m/z 520.2 [M+H]$^+$ |
| 162 | 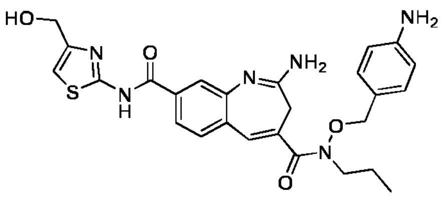 | MS-ESI: m/z 521.2 [M+H]$^+$ |
| 163 | 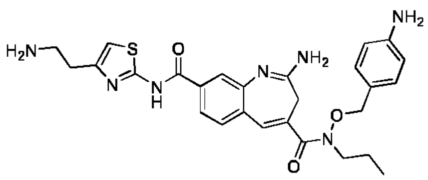 | MS-ESI: m/z 534.2 [M+H]$^+$ |

(continued)

| No. | Structure | LC-MS |
|---|---|---|
| 164 | 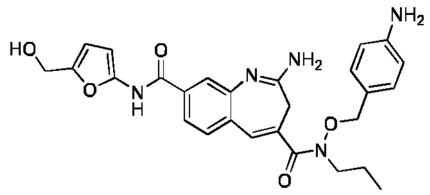 | MS-ESI: m/z 504.2 [M+H]+ |
| 165 | 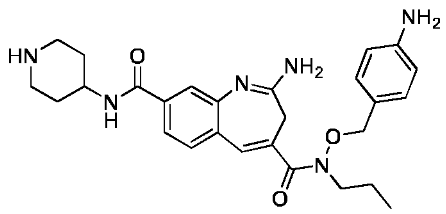 | MS-ESI: m/z 491.3 [M+H]+ |
| 166 | 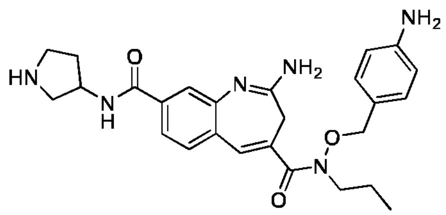 | MS-ESI: m/z 477.3 [M+H]+ |
| 167 | 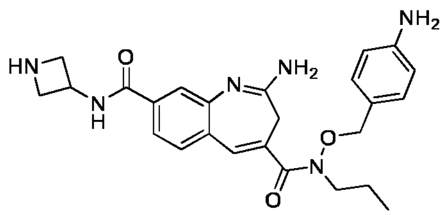 | MS-ESI: m/z 462.2 [M+H]+ |
| 168 | 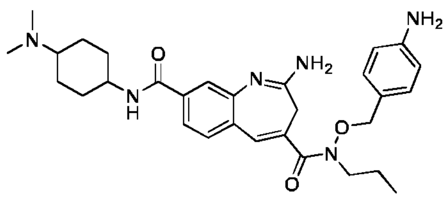 | MS-ESI: m/z 533.3 [M+H]+ |
| 169 | 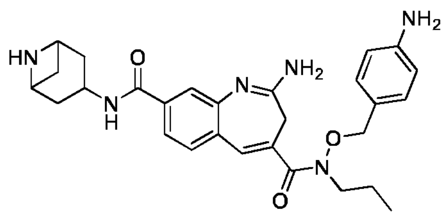 | MS-ESI: m/z 503.3 [M+H]+ |
| 170 | 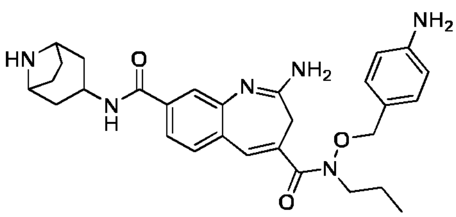 | MS-ESI: m/z 517.3 [M+H]+ |

(continued)

| No. | Structure | LC-MS |
|---|---|---|
| 171 | 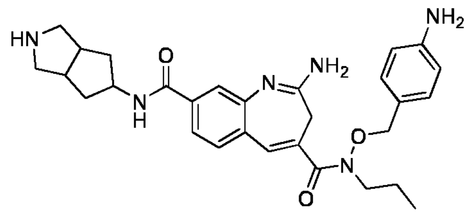 | MS-ESI: m/z 517.3 $[M+H]^+$ |
| 172 | 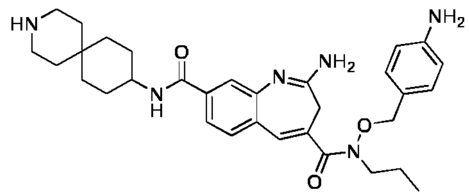 | MS-ESI: m/z 559.3 $[M+H]^+$ |
| 173 | 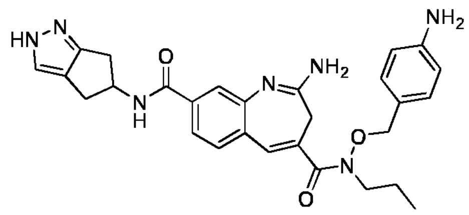 | MS-ESI: m/z 514.3 $[M+H]^+$ |
| 174 | 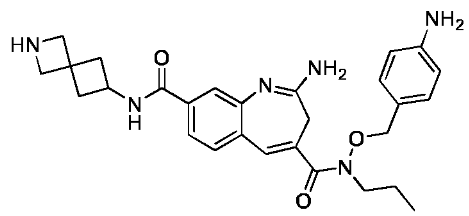 | MS-ESI: m/z 503.3 $[M+H]^+$ |
| 175 | 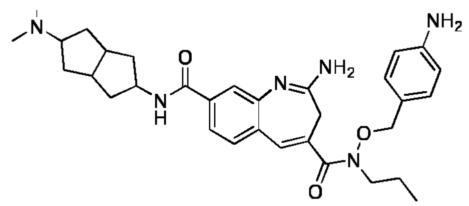 | MS-ESI: m/z 559.3 $[M+H]^+$ |
| 176 | 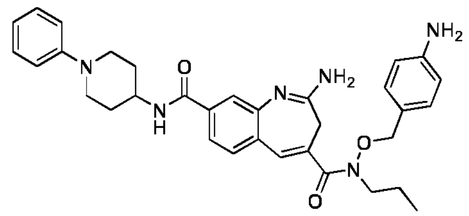 | MS-ESI: m/z 567.3 $[M+H]^+$ |
| 177 | 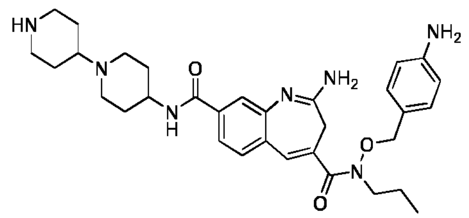 | MS-ESI: m/z 574.3 $[M+H]^+$ |

(continued)

| No. | Structure | LC-MS |
|---|---|---|
| 178 | 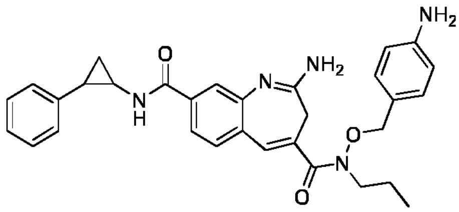 | MS-ESI: m/z 524.3 $[M+H]^+$ |
| 179 | 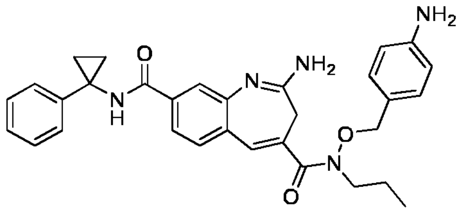 | MS-ESI: m/z 524.3 $[M+H]^+$ |
| 180 | 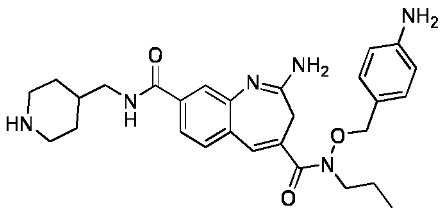 | MS-ESI: m/z 505.3 $[M+H]^+$ |
| 181 | 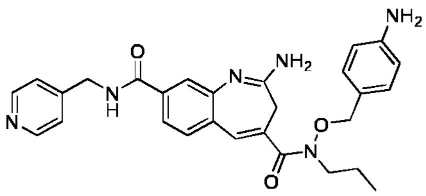 | MS-ESI: m/z 499.2 $[M+H]^+$ |
| 182 | 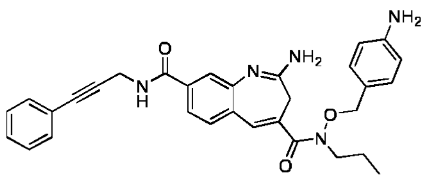 | MS-ESI: m/z 522.2 $[M+H]^+$ |
| 183 | 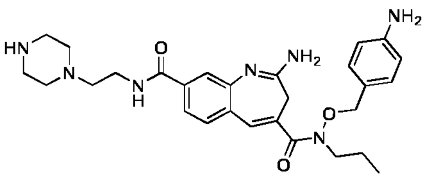 | MS-ESI: m/z 520.3 $[M+H]^+$ |
| 184 | 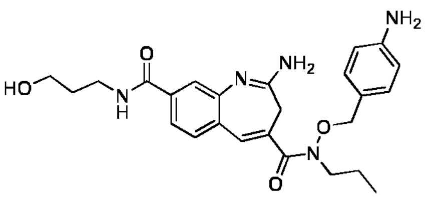 | MS-ESI: m/z 466.2 $[M+H]^+$ |

(continued)

| No. | Structure | LC-MS |
|---|---|---|
| 185 | 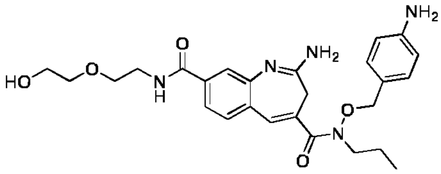 | MS-ESI: m/z 496.3 [M+H]+ |
| 186 | 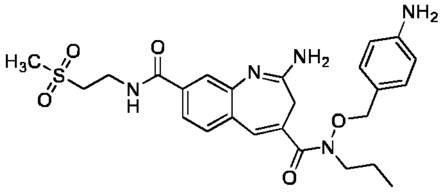 | MS-ESI: m/z 514.2 [M+H]+ |
| 187 | 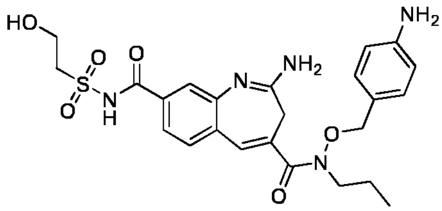 | MS-ESI: m/z 516.2 [M+H]+ |
| 188 | 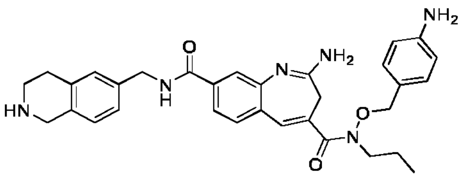 | MS-ESI: m/z 553.3 [M+H]+ |
| 189 | 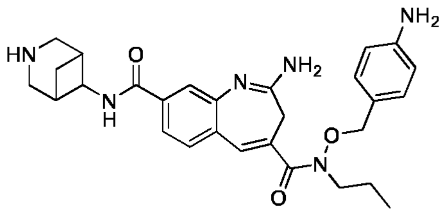 | MS-ESI: m/z 503.3 [M+H]+ |
| 190 | 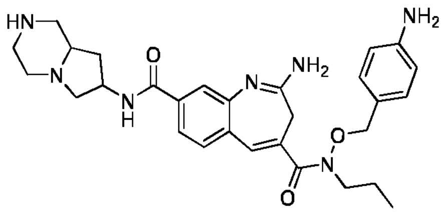 | MS-ESI: m/z 532.3 [M+H]+ |
| 191 | 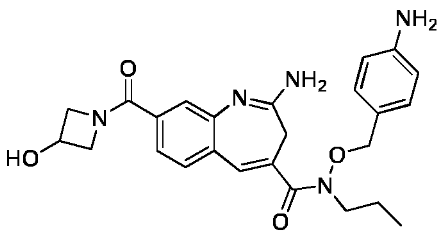 | MS-ESI: m/z 464.2 [M+H]+ |

(continued)

| No. | Structure | LC-MS |
|---|---|---|
| 192 | 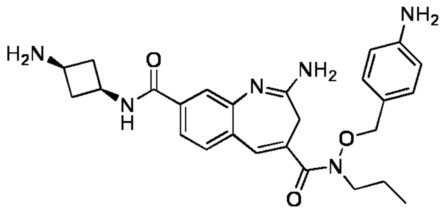 | MS-ESI: m/z 477.3 [M+H]+ |
| 193 | 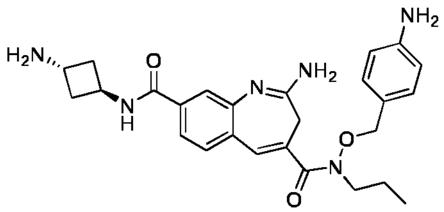 | MS-ESI: m/z 477.3 [M+H]+ |
| 194 | 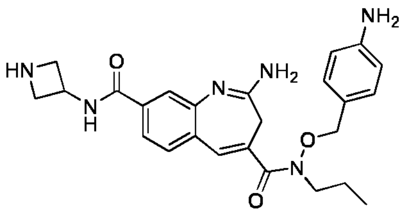 | MS-ESI: m/z 463.2 [M+H]+ |
| 195 | 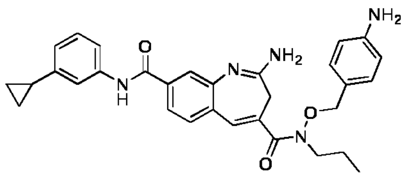 | MS-ESI: m/z 524.3 [M+H]+ |
| 196 | 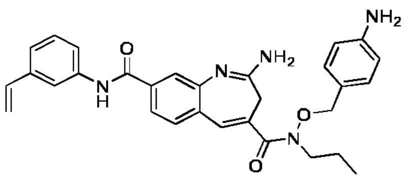 | MS-ESI: m/z 510.2 [M+H]+ |
| 197 | 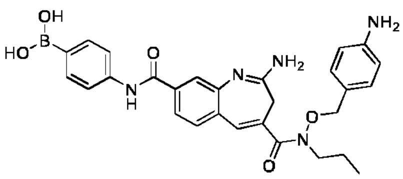 | MS-ESI: m/z 528.2 [M+H]+ |
| 198 | 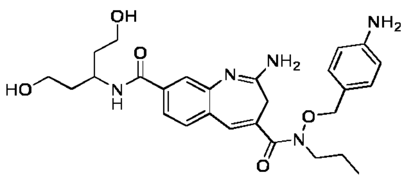 | MS-ESI: m/z 510.3 [M+H]+ |
| 199 | 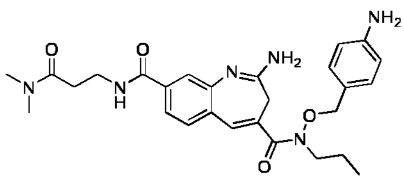 | MS-ESI: m/z 507.3 [M+H]+ |

**Synthesis of compounds 200-287: the following compounds were synthesized using a synthetic method similar to that of compound 45.**

| No. | Structure | LC-MS |
|---|---|---|
| 200 | 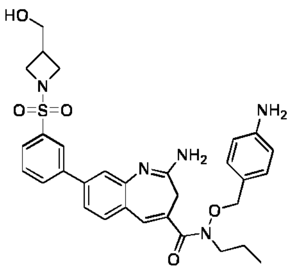 | MS-ESI: m/z 590.2 [M+H]$^+$ |
| 201 | 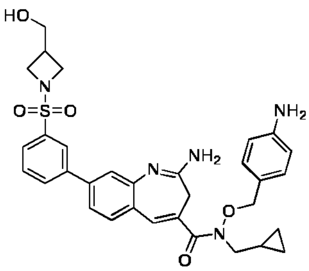 | MS-ESI: m/z 602.2 [M+H]$^+$ |
| 202 | 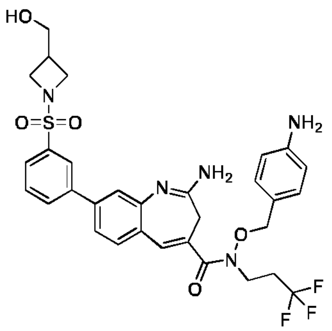 | MS-ESI: m/z 643.2 [M+H]$^+$ |
| 203 | 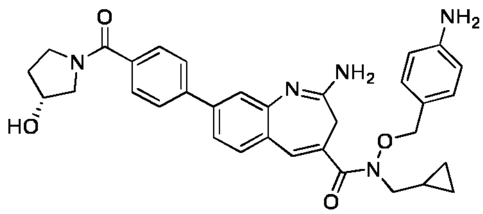 | MS-ESI: m/z 566.3 [M+H]$^+$ |
| 204 | 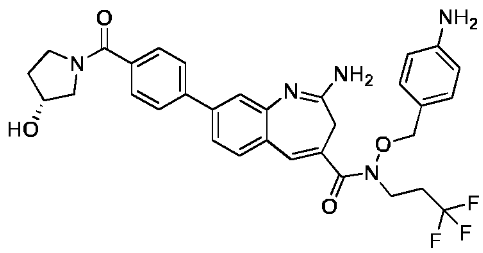 | MS-ESI: m/z 608.2 [M+H]$^+$ |

(continued)

| No. | Structure | LC-MS |
|---|---|---|
| 205 | 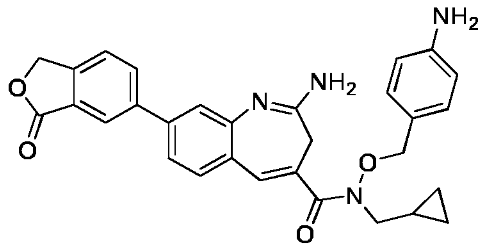 | MS-ESI: m/z 509.2 $[M+H]^+$ |
| 206 | 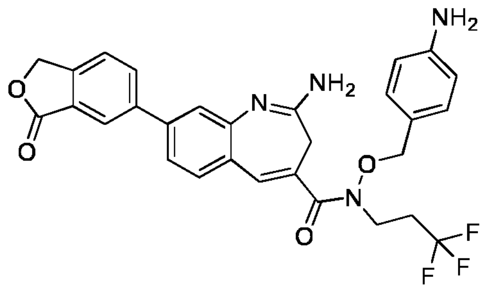 | MS-ESI: m/z 551.2 $[M+H]^+$ |
| 207 | 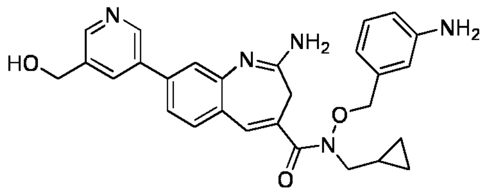 | MS-ESI: m/z 484.2 $[M+H]^+$ |
| 208 | 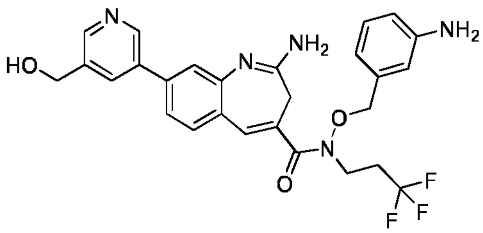 | MS-ESI: m/z 526.2 $[M+H]^+$ |
| 209 | 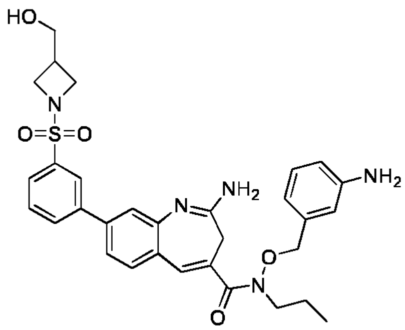 | MS-ESI: m/z 590.2 $[M+H]^+$ |

(continued)

| No. | Structure | LC-MS |
|---|---|---|
| 210 | 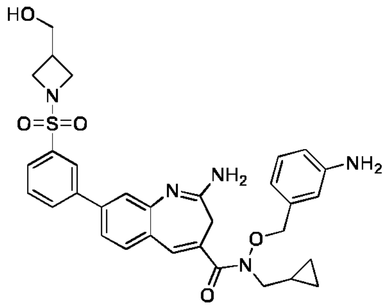 | MS-ESI: m/z 602.2 [M+H]+ |
| 211 | 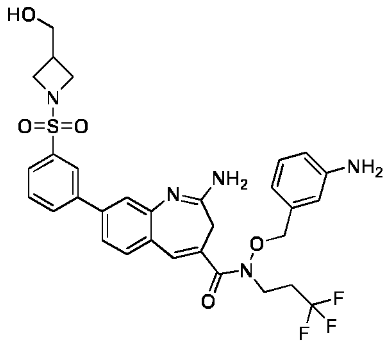 | MS-ESI: m/z 644.2 [M+H]+ |
| 212 | 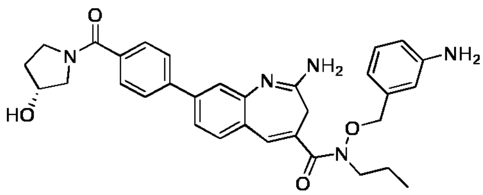 | MS-ESI: m/z 554.3 [M+H]+ |
| 213 | 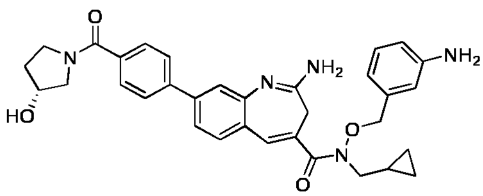 | MS-ESI: m/z 566.3 [M+H]+ |
| 214 | 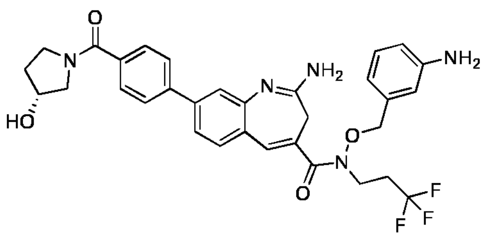 | MS-ESI: m/z 608.3 [M+H]+ |
| 215 | 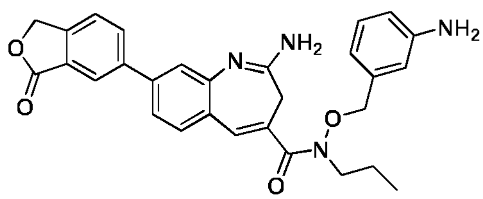 | MS-ESI: m/z 497.2 [M+H]+ |

(continued)

| No. | Structure | LC-MS |
|---|---|---|
| 216 | 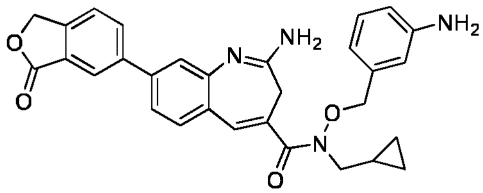 | MS-ESI: m/z 509.2 [M+H]$^+$ |
| 217 | 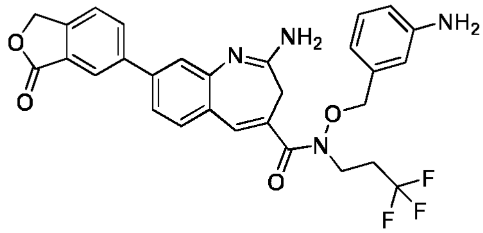 | MS-ESI: m/z 551.2 [M+H]$^+$ |
| 218 | 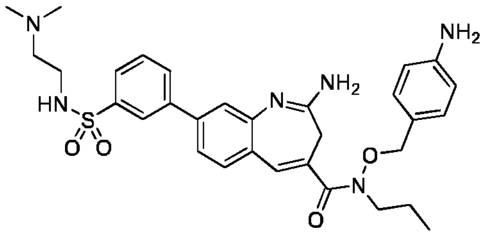 | MS-ESI: m/z 591.3 [M+H]$^+$ |
| 219 | 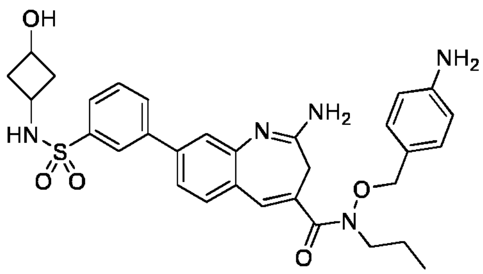 | MS-ESI: m/z 590.2 [M+H]$^+$ |
| 220 | 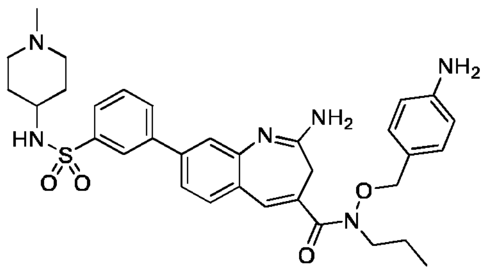 | MS-ESI: m/z 617.3 [M+H]$^+$ |
| 221 | 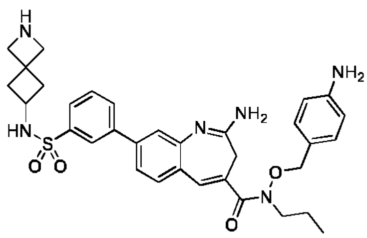 | MS-ESI: m/z 615.3 [M+H]$^+$ |

(continued)

| No. | Structure | LC-MS |
|---|---|---|
| 222 | 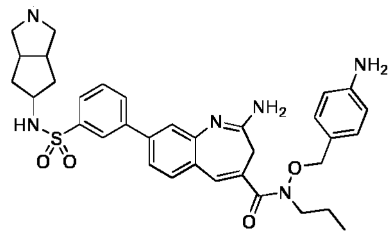 | MS-ESI: m/z 643.3 $[M+H]^+$ |
| 223 | 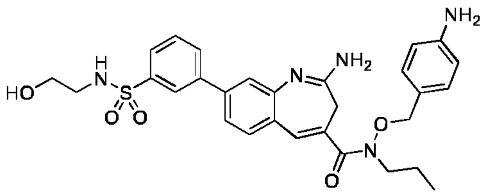 | MS-ESI: m/z 564.2 $[M+H]^+$ |
| 224 | 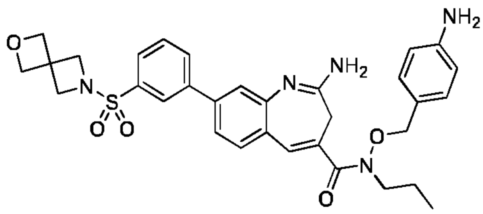 | MS-ESI: m/z 602.2 $[M+H]^+$ |
| 225 | 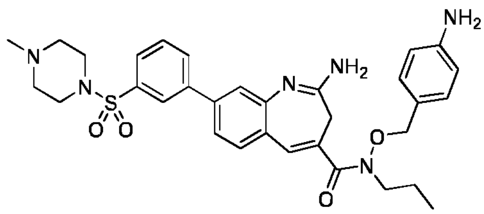 | MS-ESI: m/z 603.3 $[M+H]^+$ |
| 226 | 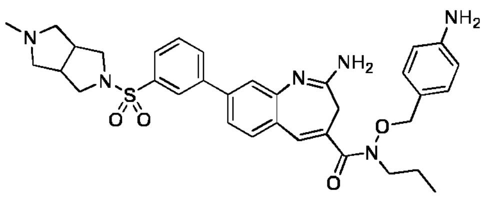 | MS-ESI: m/z 629.3 $[M+H]^+$ |
| 227 | 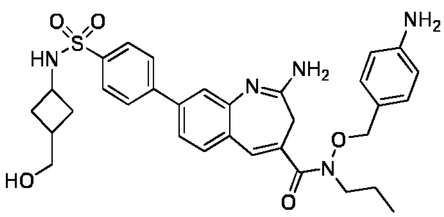 | MS-ESI: m/z 604.3 $[M+H]^+$ |
| 228 | 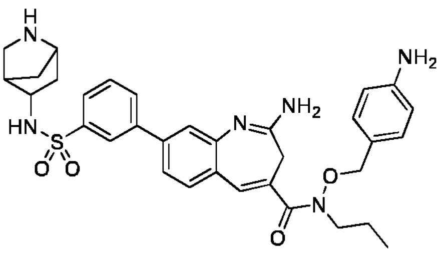 | MS-ESI: m/z 615.3 $[M+H]^+$ |

(continued)

| No. | Structure | LC-MS |
| --- | --- | --- |
| 229 | 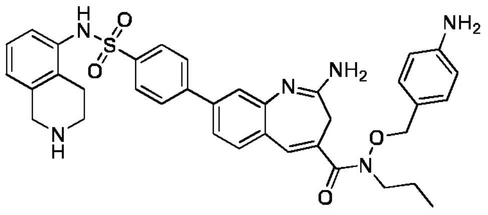 | MS-ESI: m/z 651.3 [M+H]+ |
| 230 | 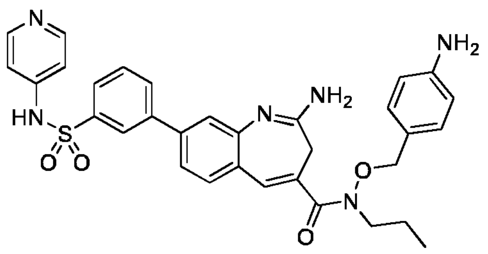 | MS-ESI: m/z 597.2 [M+H]+ |
| 231 | 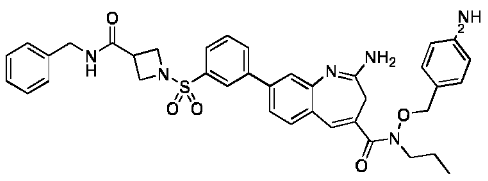 | MS-ESI: m/z 693.3 [M+H]+ |
| 232 | 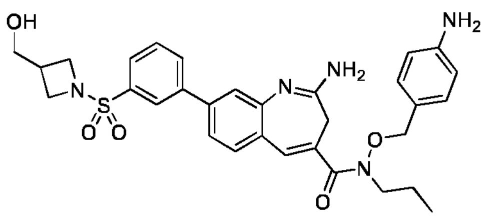 | MS-ESI: m/z 590.2 [M+H]+ |
| 233 | 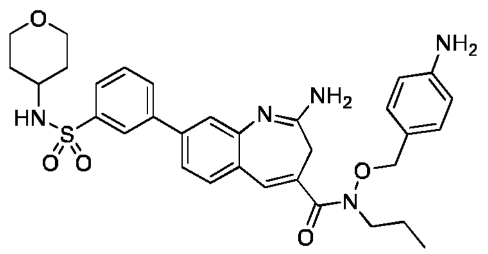 | MS-ESI: m/z 604.3 [M+H]+ |
| 234 | 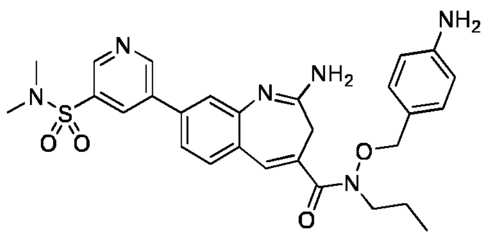 | MS-ESI: m/z 549.2 [M+H]+ |

(continued)

| No. | Structure | LC-MS |
|---|---|---|
| 235 | 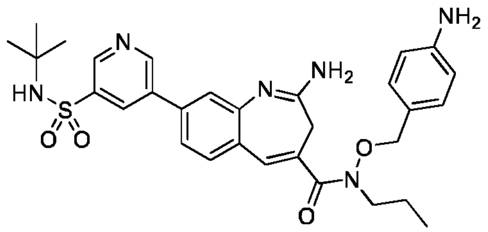 | MS-ESI: m/z 577.3 [M+H]+ |
| 236 | 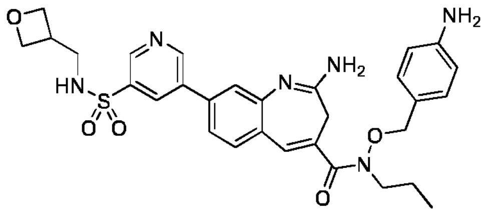 | MS-ESI: m/z 591.2 [M+H]+ |
| 237 | 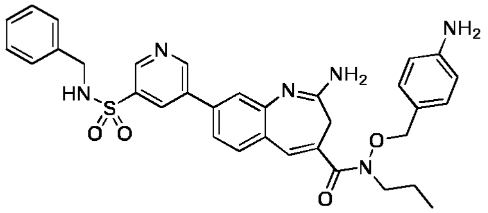 | MS-ESI: m/z 611.2 [M+H]+ |
| 238 | 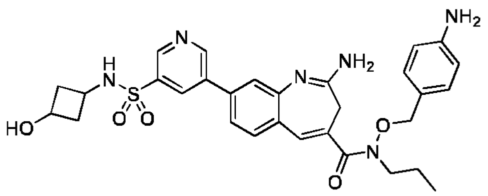 | MS-ESI: m/z 591.2 [M+H]+ |
| 239 | 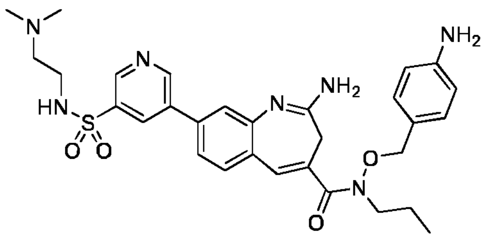 | MS-ESI: m/z 592.3 [M+H]+ |
| 240 | 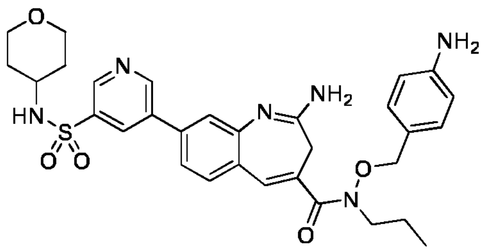 | MS-ESI: m/z 605.3 [M+H]+ |

(continued)

| No. | Structure | LC-MS |
|---|---|---|
| 241 | 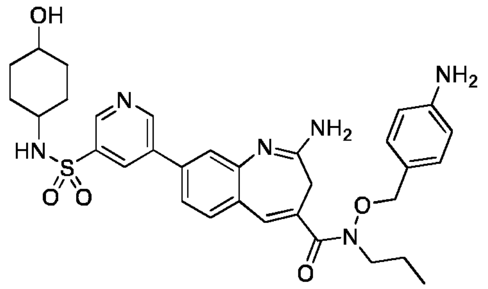 | MS-ESI: m/z 619.3 [M+H]+ |
| 242 | 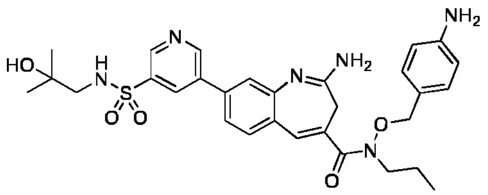 | MS-ESI: m/z 593.3 [M+H]+ |
| 243 | 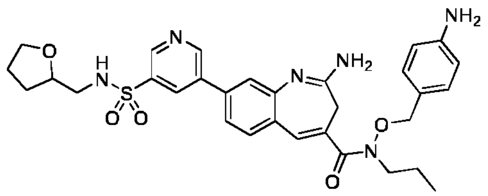 | MS-ESI: m/z 605.3 [M+H]+ |
| 244 | 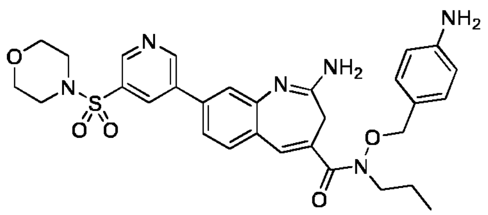 | MS-ESI: m/z 591.2 [M+H]+ |
| 245 | 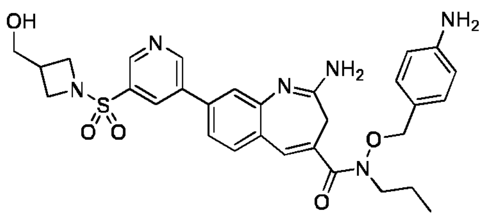 | MS-ESI: m/z 591.2 [M+H]+ |
| 246 | 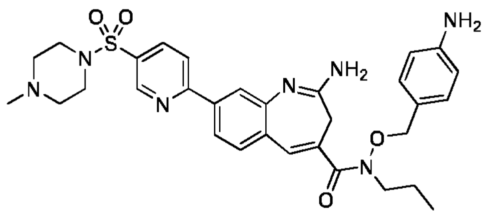 | MS-ESI: m/z 604.3 [M+H]+ |
| 247 | 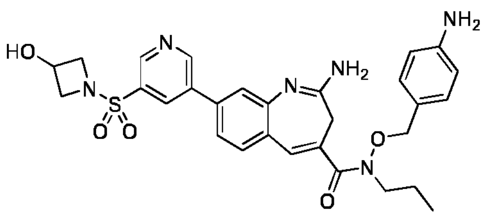 | MS-ESI: m/z 577.2 [M+H]+ |

(continued)

| No. | Structure | LC-MS |
|---|---|---|
| 248 | 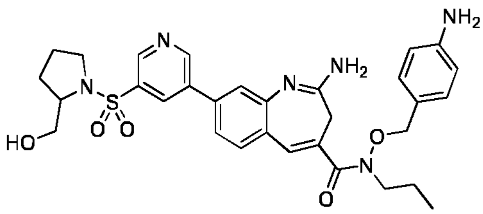 | MS-ESI: m/z 605.3 [M+H]+ |
| 249 | 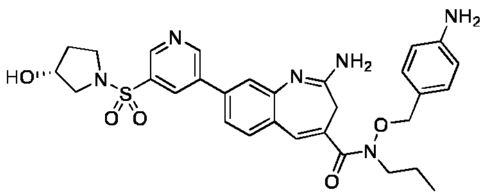 | MS-ESI: m/z 591.2 [M+H]+ |
| 250 | 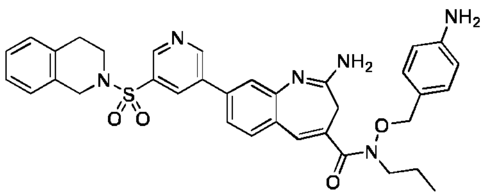 | MS-ESI: m/z 637.3 [M+H]+ |
| 251 | 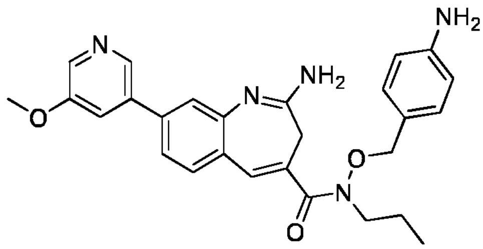 | MS-ESI: m/z 472.2 [M+H]+ |
| 252 | 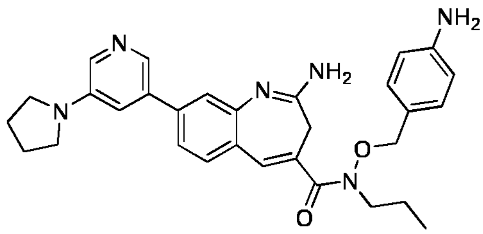 | MS-ESI: m/z 511.3 [M+H]+ |
| 253 | 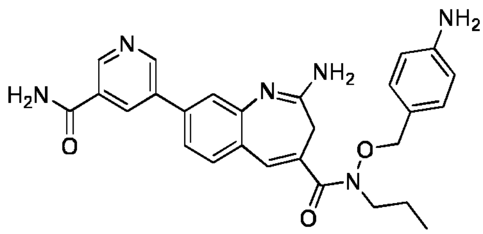 | MS-ESI: m/z 485.2 [M+H]+ |
| 254 | 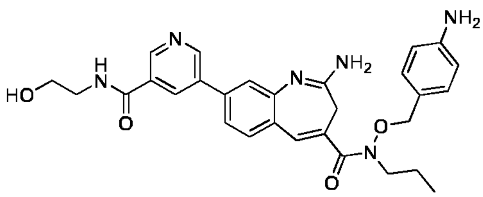 | MS-ESI: m/z 529.3 [M+H]+ |

(continued)

| No. | Structure | LC-MS |
|---|---|---|
| 255 | 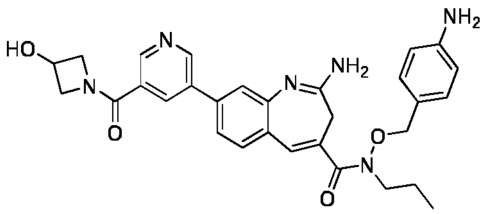 | MS-ESI: m/z 541.3 $[M+H]^+$ |
| 256 | 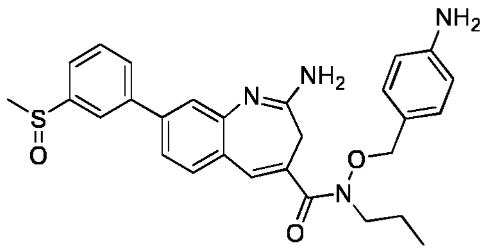 | MS-ESI: m/z 503.2 $[M+H]^+$ |
| 257 | 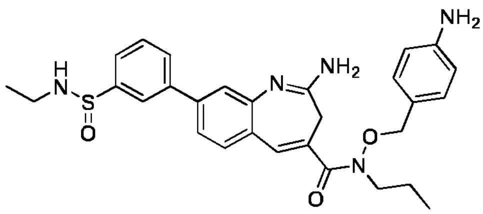 | MS-ESI: m/z 532.2 $[M+H]^+$ |
| 258 | 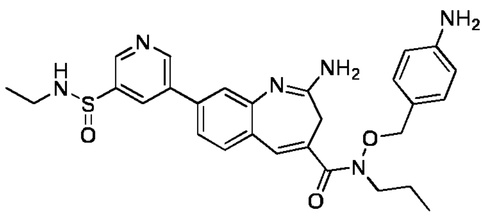 | MS-ESI: m/z 533.2 $[M+H]^+$ |
| 259 | 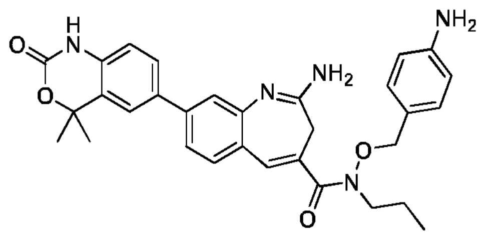 | MS-ESI: m/z 540.3 $[M+H]^+$ |
| 260 | 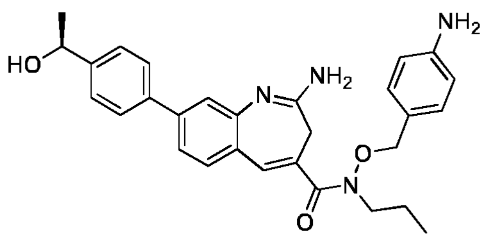 | MS-ESI: m/z 485.3 $[M+H]^+$ |

(continued)

| No. | Structure | LC-MS |
|---|---|---|
| 261 | 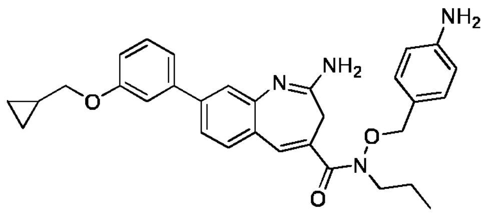 | MS-ESI: m/z 511.3 [M+H]$^+$ |
| 262 | 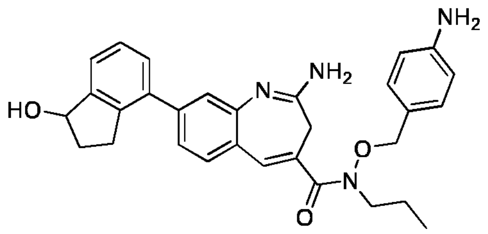 | MS-ESI: m/z 497.3 [M+H]$^+$ |
| 263 | 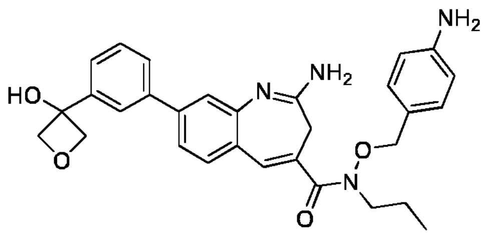 | MS-ESI: m/z 513.2 [M+H]$^+$ |
| 264 | 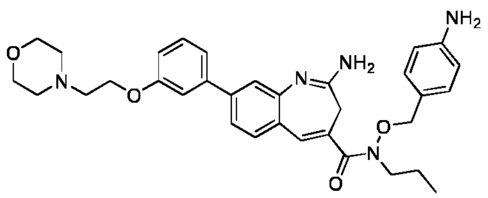 | MS-ESI: m/z 570.3 [M+H]$^+$ |
| 265 | 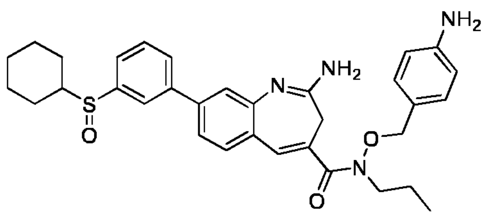 | MS-ESI: m/z 571.3 [M+H]$^+$ |
| 266 | 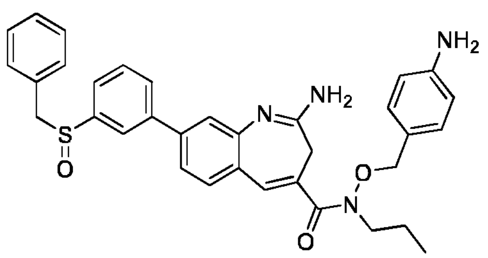 | MS-ESI: m/z 579.2 [M+H]$^+$ |
| 267 | 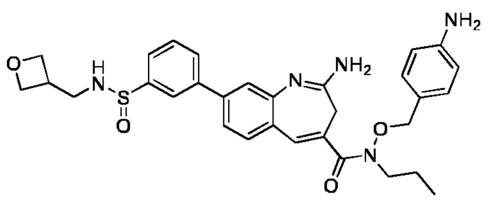 | MS-ESI: m/z 574.2 [M+H]$^+$ |

(continued)

| No. | Structure | LC-MS |
|---|---|---|
| 268 | 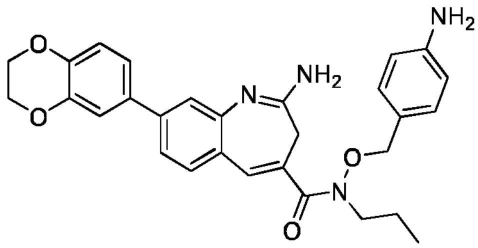 | MS-ESI: m/z 499.2 [M+H]+ |
| 269 | 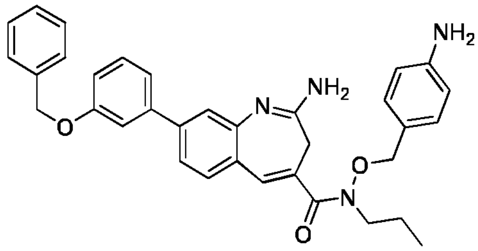 | MS-ESI: m/z 547.3 [M+H]+ |
| 270 | 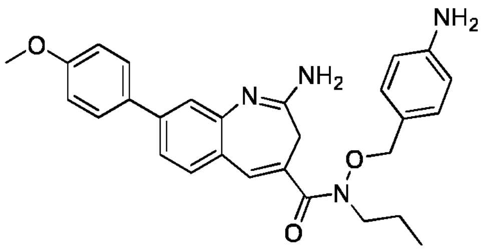 | MS-ESI: m/z 471.2 [M+H]+ |
| 271 | 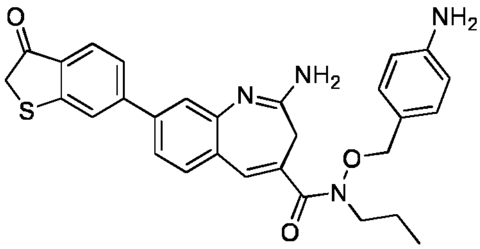 | MS-ESI: m/z 513.2 [M+H]+ |
| 272 | 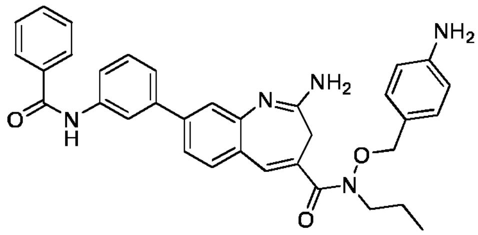 | MS-ESI: m/z 560.3 [M+H]+ |
| 273 | 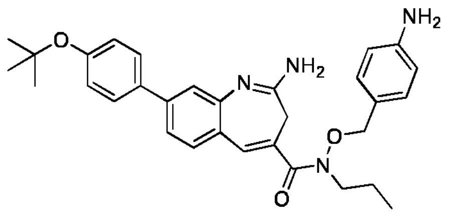 | MS-ESI: m/z 513.3 [M+H]+ |

(continued)

| No. | Structure | LC-MS |
|---|---|---|
| 274 | 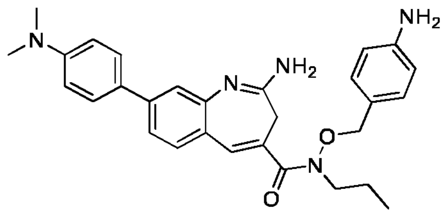 | MS-ESI: m/z 484.3 [M+H]+ |
| 275 | 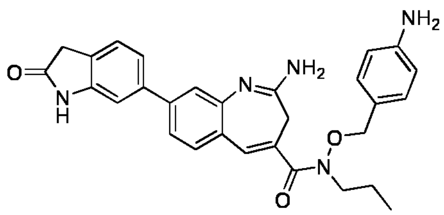 | MS-ESI: m/z 496.2 [M+H]+ |
| 276 | 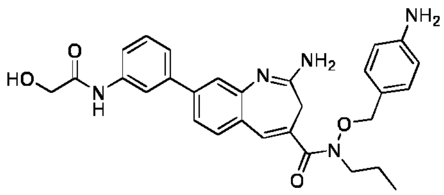 | MS-ESI: m/z 514.2 [M+H]+ |
| 277 | 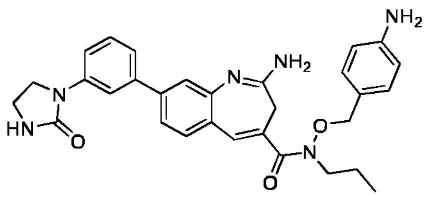 | MS-ESI: m/z 525.3 [M+H]+ |
| 278 | 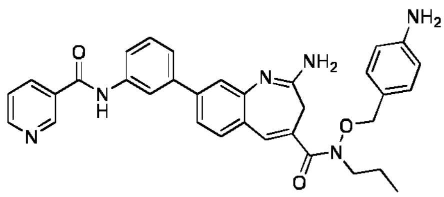 | MS-ESI: m/z 561.3 [M+H]+ |
| 279 | 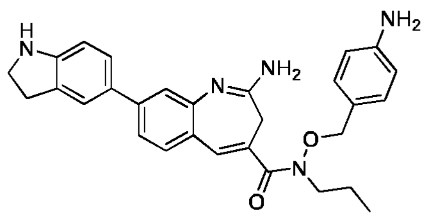 | MS-ESI: m/z 482.3 [M+H]+ |
| 280 | 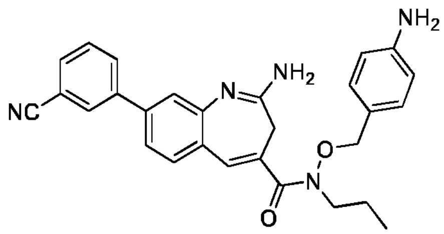 | MS-ESI: m/z 466.2 [M+H]+ |

(continued)

| No. | Structure | LC-MS |
|---|---|---|
| 281 | 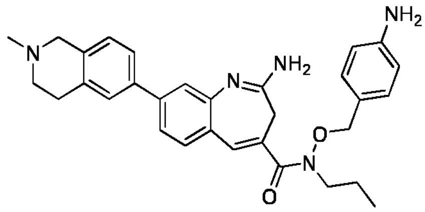 | MS-ESI: m/z 510.3 [M+H]$^+$ |
| 282 | 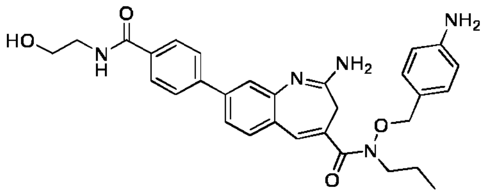 | MS-ESI: m/z 528.3 [M+H]$^+$ |
| 283 | 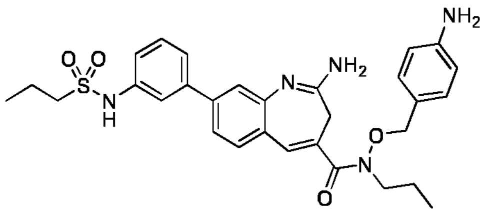 | MS-ESI: m/z 562.2 [M+H]$^+$ |
| 284 | 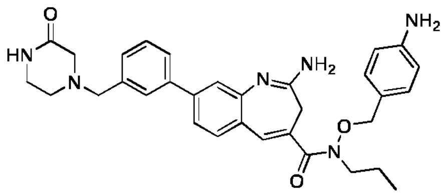 | MS-ESI: m/z 553.3 [M+H]$^+$ |
| 285 | 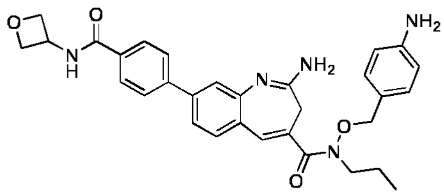 | MS-ESI: m/z 540.3 [M+H]$^+$ |
| 286 | 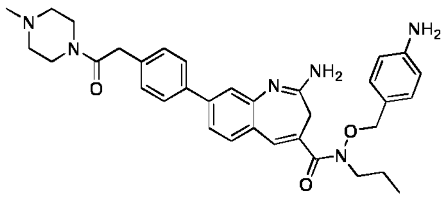 | MS-ESI: m/z 581.3 [M+H]$^+$ |
| 287 | 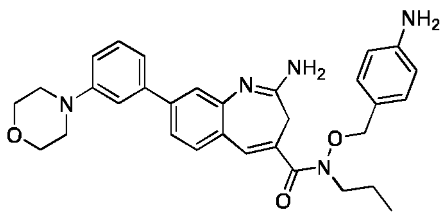 | MS-ESI: m/z 526.3 [M+H]$^+$ |

## Example 2

In this example, the agonistic activity of the compounds of the present application on hTLR7 (human Toll-like

receptor 7) and hTLR8 (human Toll-like receptor 8) was assessed; the induction activity of the compounds on human PBMCs for TNF-α and IFN-α production was assessed in human PBMCs (human peripheral blood mononuclear cells).

All compounds were formulated in DMSO into 30 mM stock solutions and preserved at 4 °C. The reference compound (WuXi AppTec, Cat. No. tlrl-r848-5) was purchased from InvivoGen and formulated into a 5 mg/mL stock solution in DMSO and preserved in a freezer at -20 °C. HEK-Blue hTLR7 and HEK-Blue hTLR8 cell lines were purchased from InvivoGen, and human PBMCs were purchased from Shanghai Saily Biotechnology Co., Ltd. (Cat. No. XFB-HP100B).

Other common reagents are commercially available, for example:

| Abbreviation | Reagent | Supplier | Cat. No. |
|---|---|---|---|
| QUANTI-Blue™ | QUANTI-Blue™ detection agent | InvivoGen | rep-qbs |
| CellTiter-Glo | Fluorescent cell viability detection kit | Promega | G7573 |
| RPMI 1640 | RPMI 1640 medium | Invitrogen | 22400-089 |
| DMEM | DMEM medium | Invitrogen | 11960-051 |
| HI FBS | Thermally inactivated serum | Gibco | 10100-147C |
| L-Glutamine | L-glutaminase | Gibco | 25030-081 |
| PS | Antibiotics (penicillin and streptomycin) | Hyclone | SV30010 |
| Blasticidin | Blasticidin | Invivogen | ant-bl-1 |
| Normocin | Neomycin | Invivogen | ant-nr-1 |
| Zeocin | Bleomycin | Invivogen | ant-zn-05 |
| TrypLE Express | Pancreatin | Invitrogen | 12605-010 |
| DPBS | Dulbecco's phosphate-buffered saline | Corning | 21-031-CVC |
| Human TNF-α ELISA kit | Human TNF-α ELISA kit | DAKEWE | 1117202 |
| Human IFN-α ELISA kit | Human IFN-α ELISA kit | DAKEWE | 1110012 |

The major instruments used in this study were multifunctional microplate reader Flexstation III (Molecular Device), Envision (Perkin Elmer), M2e (Molecular Device), and Echo555 (Labcyte).

2.1 Agonistic activity of compounds on hTLR7 (human Toll-like receptor 7) and hTLR8 (human Toll-like receptor 8)

Compound dilution: The compounds of the present application are diluted in DMSO, for example, to about 15 mM. The reference compound was added to the plates in sequence after dilution. The compounds were serially 3-fold diluted to 10 concentrations and added to 96-well plates in duplicate. For the negative control wells, DMSO was added at 0.2 μL/well, and the reference compound was added to the positive control wells. The final concentration of DMSO was 0.2%.

Cell plating: When an 80% confluence of HEK Blue hTLR7&8 cells in the culture flask was observed under a microscope, the culture medium was discarded, 10 mL of detection medium was added, and the cells were separated by pipetting. The cells were counted on a cytometer and diluted to 500,000 cells/mL. The cells were plated at 100 μL/50,000 cells/well in the 96-well plates containing the compounds. The compounds and cells were co-incubated at 37 °C in a 5% $CO_2$ incubator for 24 hours.

Compound activity detection: 20 μL of cell supernatant was taken from each well and added to a plate containing QUANTI-Blue™ reagent at 180 μL/well. The plate was incubated at 37 °C for 1 hour, and the absorbance at 650 nm ($OD_{650}$) was measured on the multifunctional microplate reader Flexstation III.

Cell viability detection: The chemiluminescent signal (RLU) was detected on the multifunctional microplate reader Flexstation III according to the instructions of Celltiter-Glo.

Compound activity data analysis: The $OD_{650}$ values were analyzed by GraphPad Prism software and fitted to give dose-response curves of the compounds. The $EC_{50}$ values of compounds were calculated.

Cell viability data analysis: The cell viability% was calculated as follows. The cell viability% was analyzed by GraphPad Prism software and fitted to give dose-response curves of the compounds. The $CC_{50}$ values of the compounds in the cells were calculated.

$$\text{Cell viability\%} = RLU_{compound}/RLU_{DMSO\ Control} \times 100\%$$

The agonistic activity of the compounds of the present application on hTLR7 (human Toll-like receptor 7) and hTLR8 (human Toll-like receptor 8) is summarized in Tables 1-1 and 1-2.

**Table 1-1**

| | | Reporter assay | |
|---|---|---|---|
| **Structure** | **Compound No.** | **TLR8 EC50 (μM)** | **TLR7 EC50 (μM)** |
| 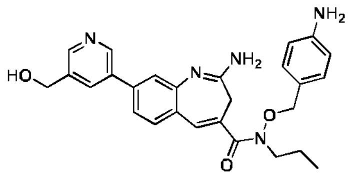 | Compound 15 | <0.0015 | 0.712 |
| 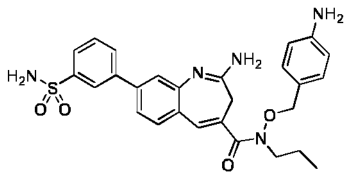 | Compound 35 | <0.0015 | 0.7576 |
| 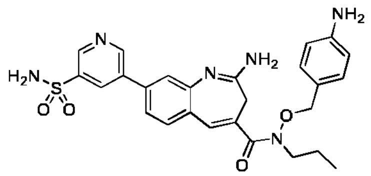 | Compound 36 | 0.00171 | 1.082 |
| 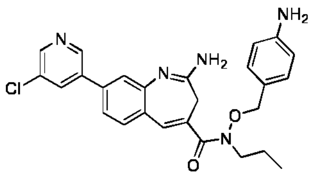 | Compound 49 | 0.0066 | 0.245 |
| 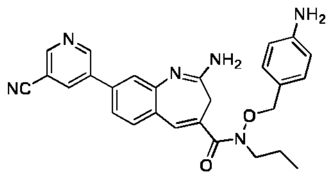 | Compound 50 | 0.0015 | 0.274 |
| 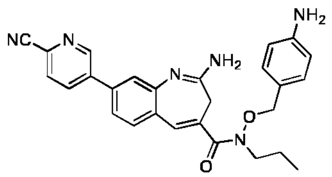 | Compound 51 | 0.0056 | 0.428 |
| 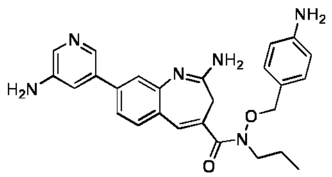 | Compound 52 | 0.0044 | 0.355 |

(continued)

| | | Reporter assay | |
|---|---|---|---|
| Structure | Compound No. | TLR8 EC50 (μM) | TLR7 EC50 (μM) |
| 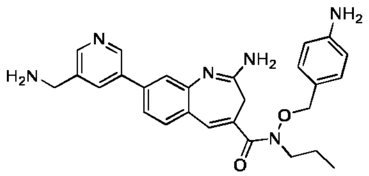 | Compound 54 | <0.0015 | 0.528 |
| 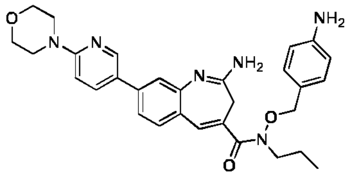 | Compound 55 | 0.0023 | 0.685 |
| 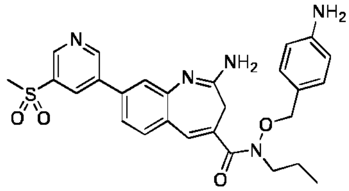 | Compound 56 | <0.0015 | 0.664 |
| 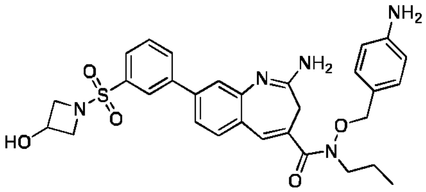 | Compound 41 | 0.005369 | 2.242 |
| 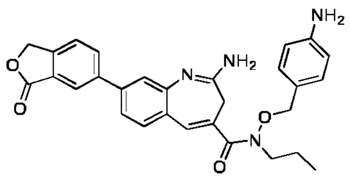 | Compound 43 | <0.0015 | 0.1796 |
| 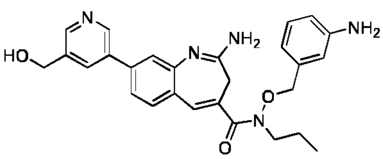 | Compound 44 | 0.0015 | 0.104 |
| 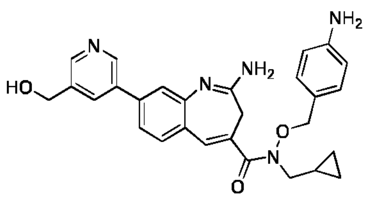 | Compound 45 | 0.0023 | 1.165 |
| 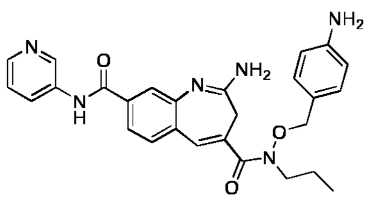 | Compound 40 | 0.0025 | 11.67 |

(continued)

| | | Reporter assay | |
|---|---|---|---|
| Structure | Compound No. | TLR8 EC50 (μM) | TLR7 EC50 (μM) |
| 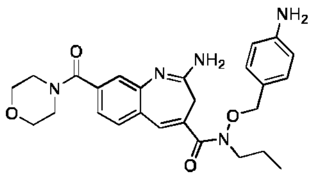 | Compound 39 | 0.0028 | 3.554 |

**Table 1-2**

| | | Reporter assay | |
|---|---|---|---|
| Structure | Compound No. | TLR8 EC50 | TLR7 EC50 |
| 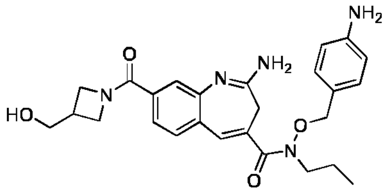 | 37 | +++ | + |
| 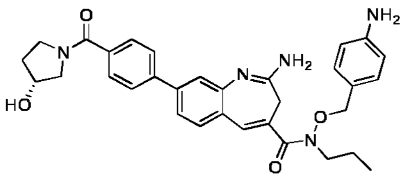 | 42 | +++ | + |
| 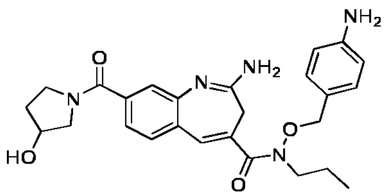 | 46 | +++ | + |
| 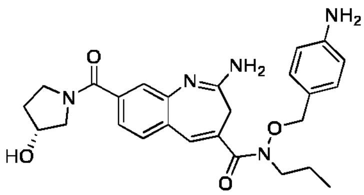 | 46A | +++ | + |
| 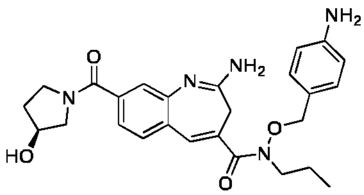 | 46B | +++ | + |

(continued)

| | | Reporter assay | |
|---|---|---|---|
| Structure | Compound No. | TLR8 EC50 | TLR7 EC50 |
| 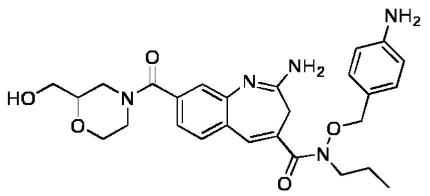 | 47 | +++ | + |
| 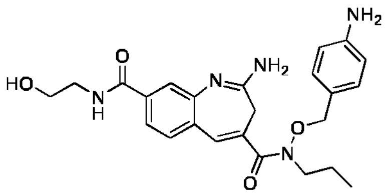 | 48 | +++ | + |
| 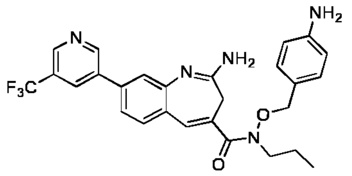 | 53 | +++ | ++ |
| 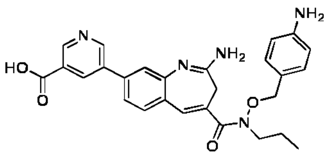 | 32 | +++ | + |
| 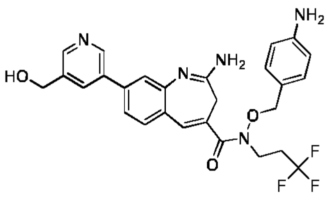 | 58 | ++++ | ++ |
| 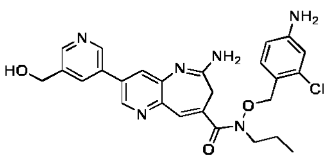 | 59 | ++++ | ++ |
| 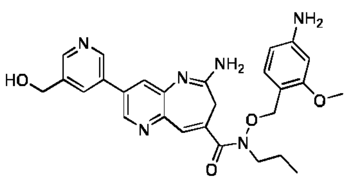 | 60 | ++++ | ++ |
| 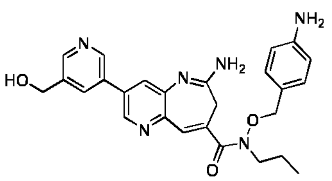 | 61 | ++++ | ++ |

(continued)

| | | Reporter assay | |
|---|---|---|---|
| Structure | Compound No. | TLR8 EC50 | TLR7 EC50 |
| 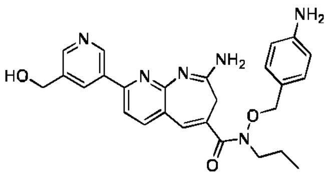 | 62 | ++++ | ++ |
| 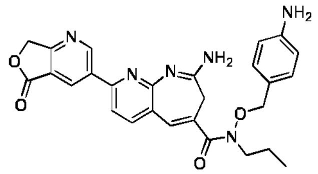 | 63 | ++++ | ++ |
| 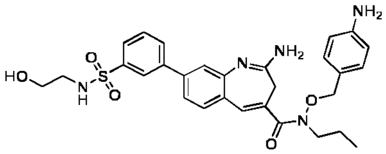 | 64 | ++++ | ++ |
| 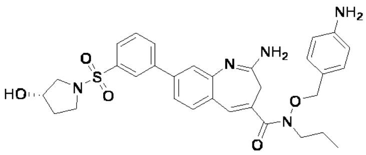 | 65 | ++++ | ++ |
| 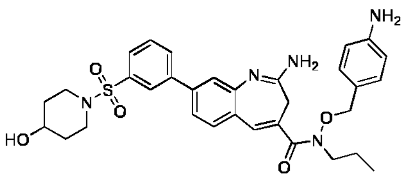 | 66 | ++++ | ++ |
| 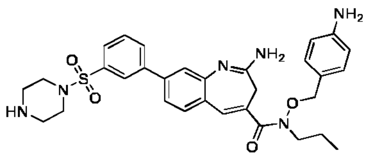 | 67 | ++++ | ++ |
| 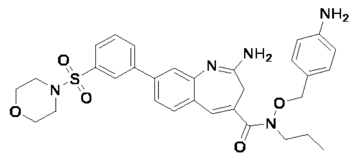 | 68 | ++++ | ++ |
| 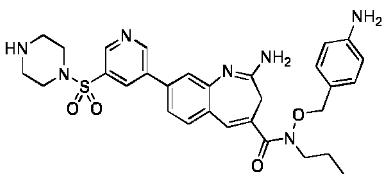 | 69 | ++++ | ++ |

(continued)

| | | Reporter assay | |
|---|---|---|---|
| Structure | Compound No. | TLR8 EC50 | TLR7 EC50 |
| 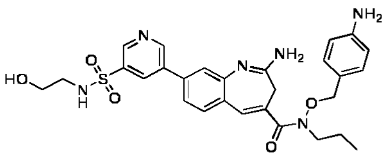 | 70 | ++++ | ++ |
| 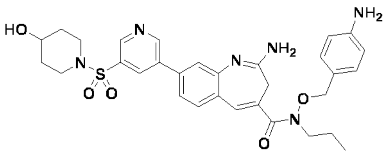 | 71 | ++++ | ++ |
| 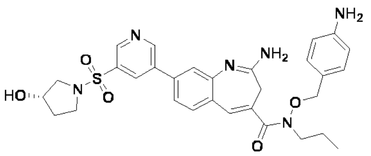 | 72 | ++++ | ++ |
| 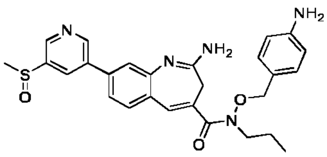 | 73 | ++++ | ++ |
| 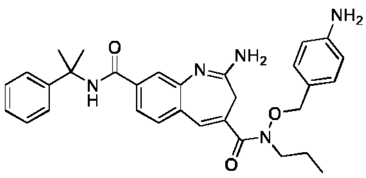 | 76 | ++++ | ++ |
| 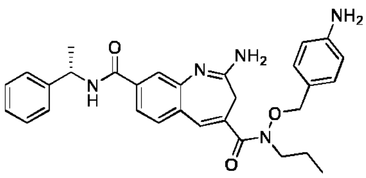 | 77 | ++++ | ++ |
| 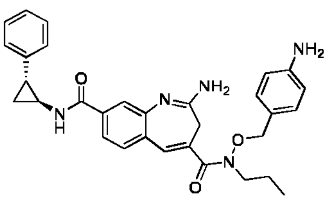 | 78 | ++++ | ++ |
| 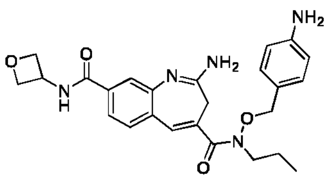 | 81 | +++ | + |

(continued)

| | | Reporter assay | |
|---|---|---|---|
| Structure | Compound No. | TLR8 EC50 | TLR7 EC50 |
| 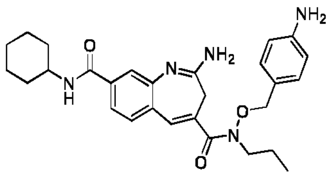 | 82 | ++++ | + |
| 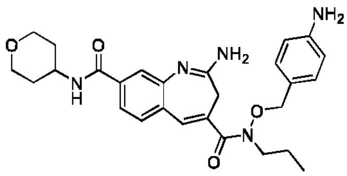 | 84 | +++ | + |
| 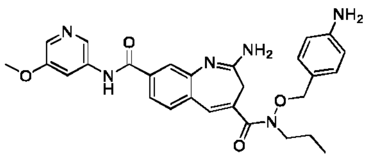 | 85 | ++++ | + |
| 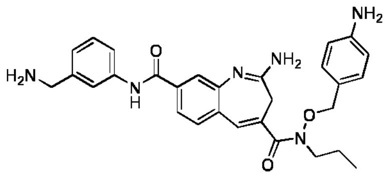 | 86 | ++++ | + |
| 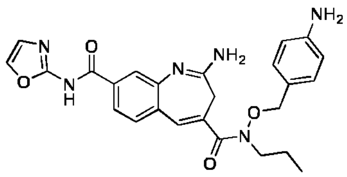 | 87 | ++++ | + |
| 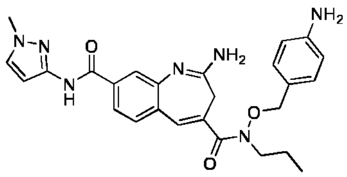 | 88 | ++++ | + |
| 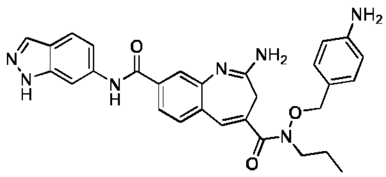 | 89 | +++ | + |
| 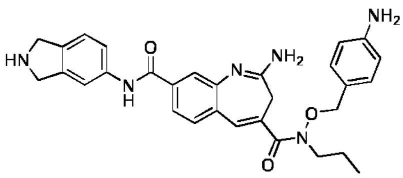 | 90 | ++++ | + |

(continued)

| | | Reporter assay | |
|---|---|---|---|
| Structure | Compound No. | TLR8 EC50 | TLR7 EC50 |
| 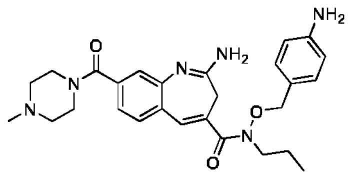 | 93 | ++++ | + |
| 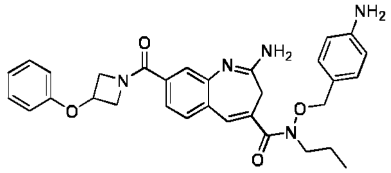 | 94 | ++++ | ++ |
| 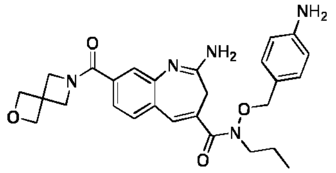 | 95 | ++++ | + |
| 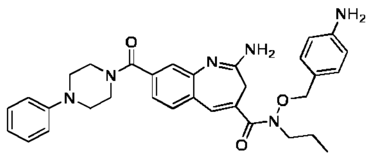 | 97 | ++++ | + |
| Note: "+" indicates $EC_{50}$ > 1000 nM; "++" indicates 1000 nM ≥ $EC_{50}$ > 100 nM; "+++" indicates 100 nM ≥ $EC_{50}$ > 10 nM; "++++" indicates $EC_{50}$ ≤ 10 nM. | | | |

The results show that the compounds of the present application have significant agonistic activity on TLRs, such as TLR7 and/or TLR8.

2.2 Induction activity of compounds on TNF-α and IFN-α in human PBMCs

In this example, the induction activity of the compounds of the present application on human PBMCs for TNF-α and IFN-α production was assessed in human PBMCs (human peripheral blood mononuclear cells). The human PBMCs were purchased from Shanghai Saily Biotechnology Co., Ltd. (Cat. No. XFB-HP100B). PBMC cells were treated with the compounds. The compounds of the present application are diluted in DMSO, for example, to about 15 mM. The reference compound was added to the plates in sequence after diluted to 5 mg/mL. The compounds were serially 3 - or 4-fold diluted to 8 concentrations and added to plates in duplicate (Table 2-1 for serial 3-fold dilutions and Table 2-2 for serial 4-fold dilutions). For the negative control wells, DMSO was added at 0.4 μL/well, and a 5 mg/mL reference compound solution was added to the positive control wells at 0.4 μL/well. The final concentration of DMSO was 0.2%. Frozen human PBMCs in cryopreservation tubes were quickly thawed in a 37 °C water bath and transferred into 50-mL centrifuge tubes. Prewarmed RPMI1640 medium was gradually added and the mixture was well mixed and centrifuged for 15 min before the supernatant was discarded. The cells were resuspended in a culture medium and counted. The cell suspension was adjusted to 2.5 × $10^6$ cells/mL with the culture medium. Diluted cells were added at 200 μL/500,000 cells/well in 96-well plates containing the compounds. The cell plates were incubated at 37 °C/5% $CO_2$ for 24 h.

TNF-α content assay:

Sample loading: 100 μL of serially diluted Cytokine standard was added to each well. 100 μL of the sample to be tested 10-fold diluted was added to the sample well, and 100 μL of 1 × dilution buffer R was added to the blank control well. Addition of detection antibody: 50 μL of biotinylated antibody working solution was added to each well. After

mixing well, the plate was covered with a plate-sealing film, incubated at room temperature for 3 hours, and washed thrice. 100 μL of streptavidin-HRP working solution was added to each well. The plate was covered with a plate-sealing film and incubated at room temperature for 20 min. The plate was then washed thrice. Color development: 100 μL of TMB solution was added to each well, and the plate was incubated at room temperature (18-25 °C) in the dark for 25 min. Termination of reaction: The reaction was terminated by quickly adding the stop solution at 100 μL/well. Plate reading: The reading at wavelength 450 nm was detected by an M2e instrument within 10 min after the termination.

IFN-α content assay:

Sample loading: 100 μL of serially diluted Cytokine standard was added to each well. 100 μL of the sample to be tested 2-fold diluted was added to the sample well, and 100 μL of 1 × dilution buffer R was added to the blank control well. After mixing well, the plate was covered with a plate-sealing film and incubated at room temperature for 2 h. The plate was washed 4 times, then the detection antibody was added. 100 μL of biotinylated antibody working solution was added to each well. After mixing well, the plate was covered with a plate-sealing film and incubated at room temperature for 1 h. The plate was washed 4 additional times, then the enzyme was added. 100 μL of streptavidin-HRP working solution was added to each well. The plate was covered with a plate-sealing film and incubated at room temperature for 30 min. The plate was washed 4 additional times, then the color development: 100 μL of TMB solution was added to each well, and the plate was incubated at room temperature (18-25 °C) in the dark for 25 min. Termination of reaction: The reaction was terminated by quickly adding the stop solution at 100 μL/well. Plate reading: The reading at wavelength 450 nm was detected by an M2e instrument within 10 min after the termination.

Cell viability detection:

The chemiluminescent signal (RLU) was detected on the multifunctional microplate reader Envision according to the instructions of Celltiter-Glo.

Compound activity data analysis: The $OD_{450}$ values were converted to TNF-α and IFN-α contents by GraphPad Prism software analysis and fitted to give dose-response curves of the compounds. The $EC_{50}$ values for the compounds were calculated, where the Emax values are the maximal values for TNF-α and IFN-α.

Cell viability data analysis: The cell viability% was calculated as follows. The cell viability% was analyzed by GraphPad Prism software and fitted to give dose-response curves of the compounds. The $CC_{50}$ values of the compounds in the cells were calculated. Cell viability% = $RLU_{compound}/RLU_{DMSO}$ Control × 100%.

The results of the induction activity of the compounds of the present application on TNF-α and IFN-α in human PBMCs are shown in Tables 2-1 and 2-2.

**Table 2-1**

| | | PBMC assay | |
|---|---|---|---|
| Structure | Compound No. | TNFa EC50 (nM) | IFNa EC50 (nM) |
| 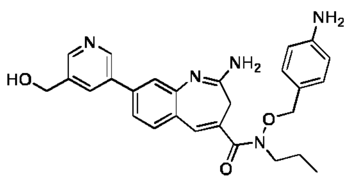 | Compound 15 | 0.0030 | 0.003022 |
| 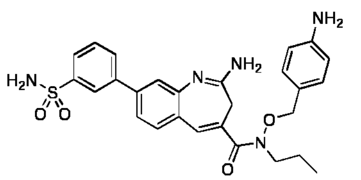 | Compound 35 | 0.0050 | 0.002945 |
| 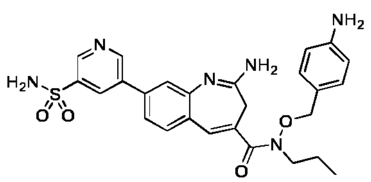 | Compound 36 | 0.01223 | 0.006048 |

(continued)

| | | PBMC assay | |
|---|---|---|---|
| Structure | Compound No. | TNFa EC50 (nM) | IFNa EC50 (nM) |
| 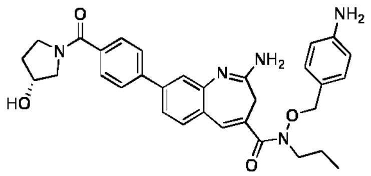 | Compound 42 | 0.03705 | 0.04833 |
| 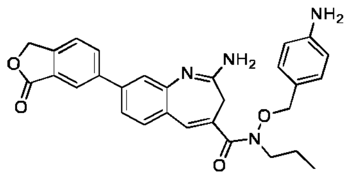 | Compound 43 | 0.00804 | 0.02961 |

| | | PBMC assay | |
|---|---|---|---|
| Structure | Compound No. | TNFa EC50 (nM) | IFNa EC50 (nM) |
| 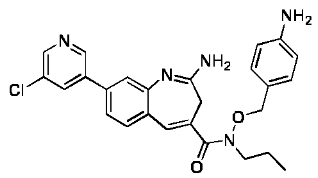 | Compound 49 | 0.02606 | 0.04823 |
| 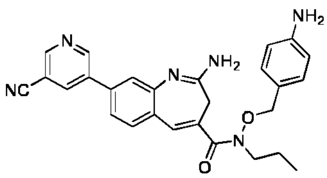 | Compound 50 | 0.0077 | 0.04151 |
| 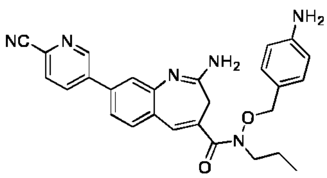 | Compound 51 | 0.01734 | 0.1415 |
| 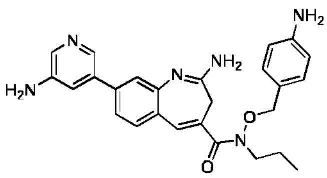 | Compound 52 | 0.008606 | 0.006576 |
| 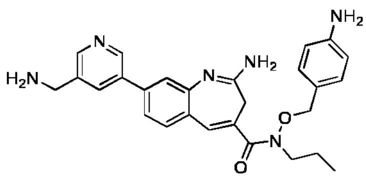 | Compound 54 | 0.003977 | 0.005349 |

(continued)

| | | PBMC assay | |
|---|---|---|---|
| **Structure** | **Compound No.** | **TNFa EC50 (nM)** | **IFNa EC50 (nM)** |
| 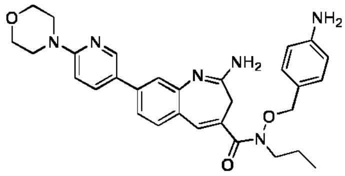 | Compound 55 | 0.03345 | 0.04336 |
| 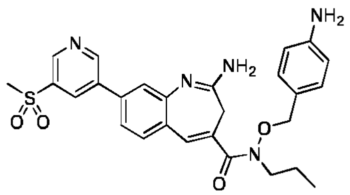 | Compound 56 | 0.007352 | 0.02846 |
| 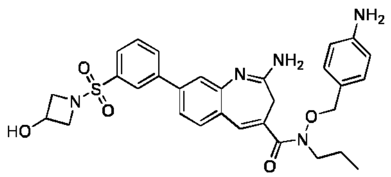 | Compound 41 | 0.01526 | 0.03108 |
| 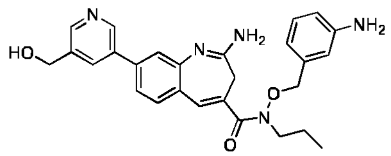 | Compound 44 | 0.007284 | 0.001493 |
| 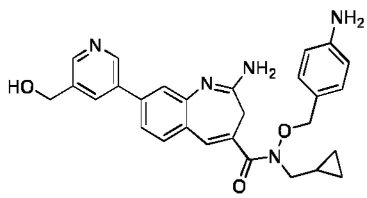 | Compound 45 | 0.007333 | 0.007245 |
| 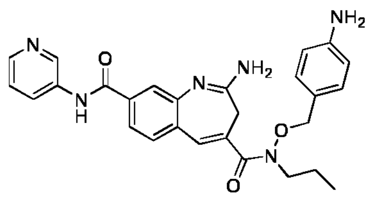 | Compound 40 | 0.009082 | 1.317 |

**Table 2-2**

| | | PBMC assay | |
|---|---|---|---|
| **Structure** | **Compound No.** | **TNFa EC50** | **IFNa EC50** |
| 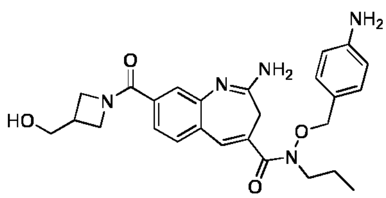 | 37 | +++ | + |

(continued)

| | | PBMC assay | |
|---|---|---|---|
| Structure | Compound No. | TNFa EC50 | IFNa EC50 |
| 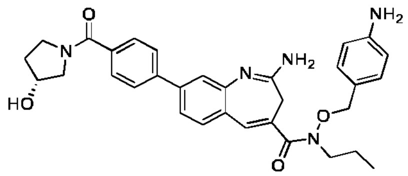 | 42 | +++ | + |
| 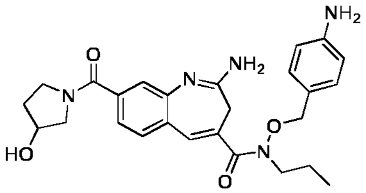 | 46 | +++ | ++ |
| 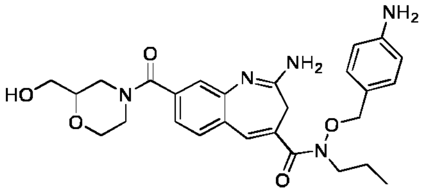 | 47 | +++ | ++ |
| 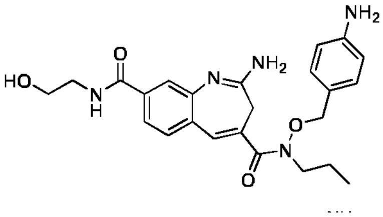 | 48 | +++ | + |
| 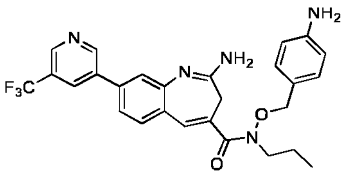 | 53 | +++ | ++ |
| 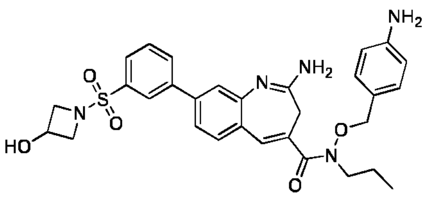 | 41 | ++++ | ++ |
| 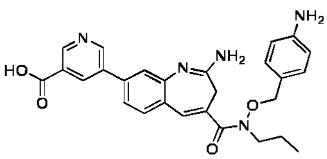 | 32 | +++ | ++ |

(continued)

| | | PBMC assay | |
|---|---|---|---|
| Structure | Compound No. | TNFa EC50 | IFNa EC50 |
| 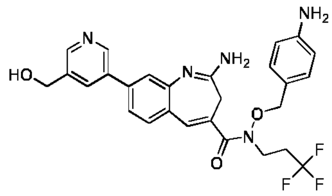 | 58 | ++++ | +++ |
| 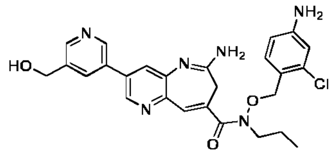 | 59 | ++++ | ++ |
| 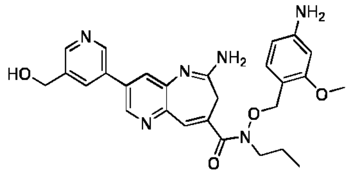 | 60 | ++++ | ++ |
| 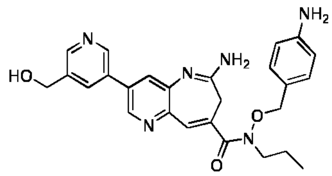 | 61 | ++++ | +++ |
| 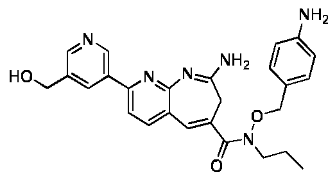 | 62 | ++++ | ++++ |
| 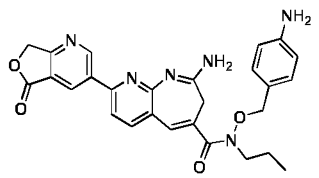 | 63 | ++++ | +++ |
| 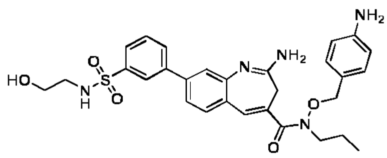 | 64 | ++++ | +++ |
| 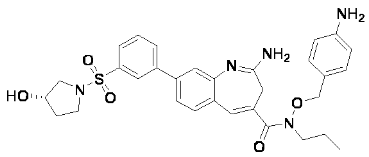 | 65 | ++++ | +++ |

(continued)

| | | PBMC assay | |
|---|---|---|---|
| Structure | Compound No. | TNFa EC50 | IFNa EC50 |
| 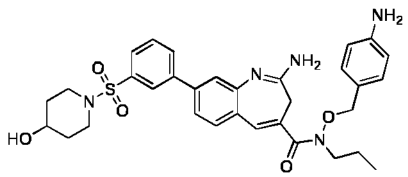 | 66 | ++++ | ++ |
| 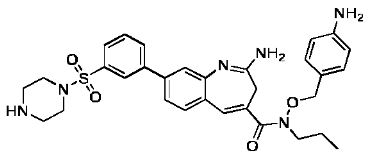 | 67 | ++++ | + |
| 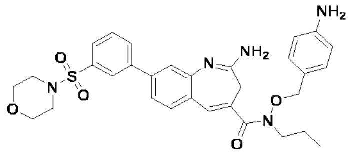 | 68 | ++++ | +++ |
| 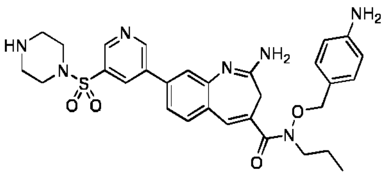 | 69 | ++++ | ++ |
| 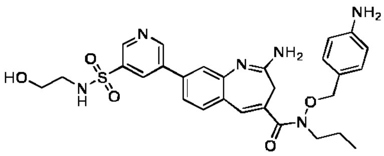 | 70 | ++++ | +++ |
| 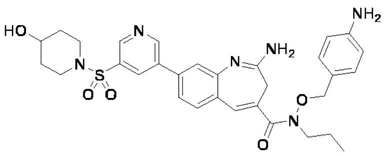 | 71 | ++++ | +++ |
| 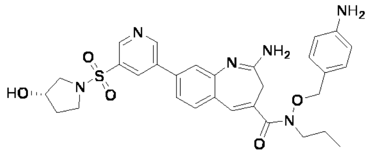 | 72 | ++++ | +++ |
| 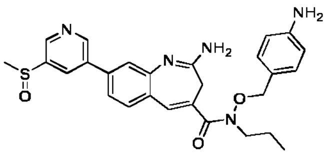 | 73 | ++++ | ++++ |
| 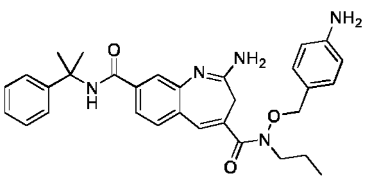 | 76 | ++++ | +++ |

(continued)

| | | PBMC assay | |
|---|---|---|---|
| Structure | Compound No. | TNFa EC50 | IFNa EC50 |
| 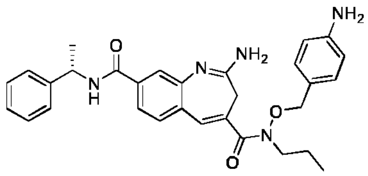 | 77 | ++++ | +++ |
| 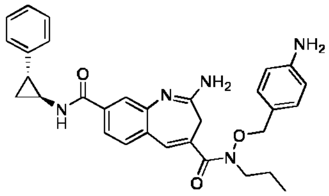 | 78 | ++++ | ++++ |
| 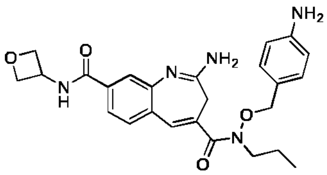 | 81 | +++ | +++ |
| 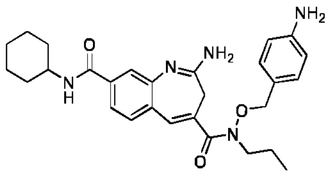 | 82 | ++++ | ++ |
| 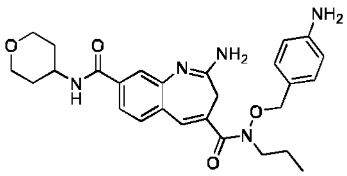 | 84 | ++++ | ++ |
| 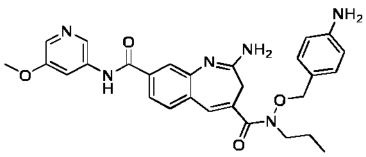 | 85 | ++++ | ++ |
| 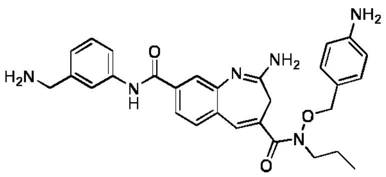 | 86 | ++++ | ++ |
| 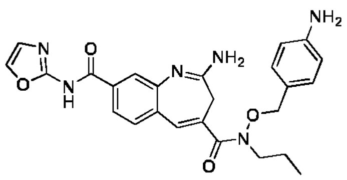 | 87 | ++++ | ++ |

(continued)

| | | PBMC assay | |
|---|---|---|---|
| **Structure** | **Compound No.** | **TNFa EC50** | **IFNa EC50** |
| 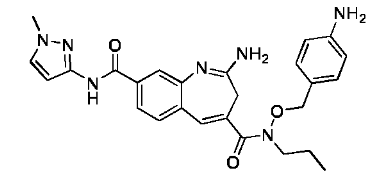 | 88 | ++++ | ++ |
| 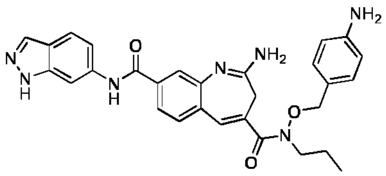 | 89 | +++ | ++ |
| 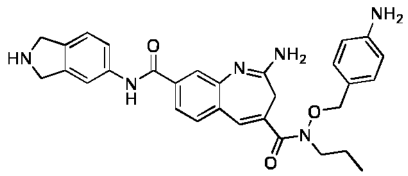 | 90 | ++++ | ++ |
| 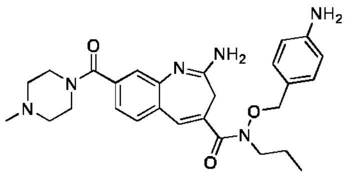 | 93 | ++++ | ++ |
| 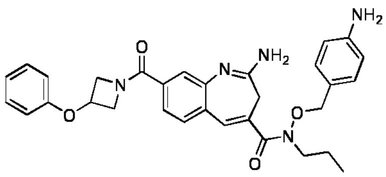 | 94 | ++++ | ++ |
| 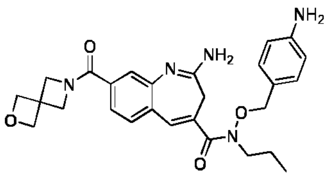 | 95 | ++++ | ++ |
| 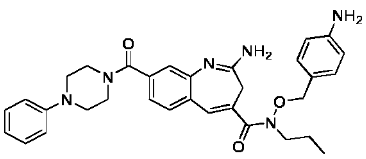 | 97 | ++++ | + |
| Note: "+" indicates $EC_{50}$ > 500 nM; "++" indicates 500 nM ≥ $EC_{50}$ > 50 nM; "+++" indicates 50 nM ≥ $EC_{50}$ > 10 nM; "++++" indicates $EC_{50}$ ≤ 10 nM. | | | |

The results show that the compounds of the present application can induce a significant increase in the expression level of cytokines, such as immune cell cytokines (e.g., TNF-α and/or IFN-α) in cells, such as immune cells (e.g., PBMCs). The results also show that the compounds of the present application can significantly increase the activity of cells, such as immune cells (e.g., PBMCs).

The foregoing detailed description is provided by way of illustration and instances, and is not intended to limit

the scope of the appended claims. Various modifications of the embodiments currently enumerated in the present application will be apparent to those of ordinary skill in the art and shall fall within the scope of the appended claims and their equivalents.

## Claims

1. A compound or a tautomer, a mesomer, a racemate, an enantiomer or a diastereoisomer thereof, or a mixture thereof, or a pharmaceutically acceptable salt thereof, wherein the compound comprises the structure of formula (II-a):

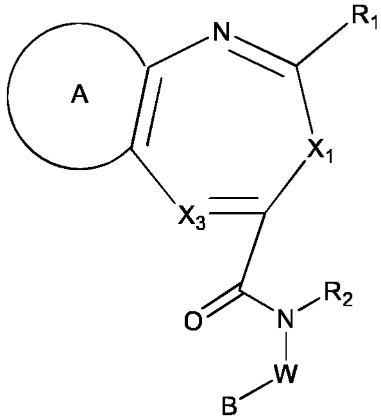

(II-a),

wherein $R_1$ and $R_2$ are each independently any group, $X_1$, $X_2$, and $X_3$ are each independently any atom optionally substituted by any group, A is an optionally substituted cyclic structure, W is absent or is any group, and B is an optionally substituted aromatic ring,
wherein B is substituted with one or more optionally substituted amino groups.

2. The compound or the tautomer, the mesomer, the racemate, the enantiomer or the diastereoisomer thereof, or the mixture thereof, or the pharmaceutically acceptable salt thereof according to claim 1,
wherein $R_1$ is an optionally substituted amino.

3. The compound or the tautomer, the mesomer, the racemate, the enantiomer or the diastereoisomer thereof, or the mixture thereof, or the pharmaceutically acceptable salt thereof according to claim 1,
wherein, $X_1$ is selected from optionally substituted $-CH_2-$.

4. The compound or the tautomer, the mesomer, the racemate, the enantiomer or the diastereoisomer thereof, or the mixture thereof, or the pharmaceutically acceptable salt thereof according to claim 3,
wherein $X_1$ is substituted with one or more $R_{X1-1}$, the one or more $R_{X1-1}$ being each independently selected from any group.

5. The compound or the tautomer, the mesomer, the racemate, the enantiomer or the diastereoisomer thereof, or the mixture thereof, or the pharmaceutically acceptable salt thereof according to claim 4,
wherein the one or more $R_{X1-1}$ are each independently selected from hydrogen and optionally substituted $C_1$-$C_6$ alkyl; when the one or more $R_{X1-1}$ comprise methylene units, the methylene units of the one or more $R_{X1-1}$ are each independently unreplaced, or are each independently replaced by any structure.

6. The compound or the tautomer, the mesomer, the racemate, the enantiomer or the diastereoisomer thereof, or the mixture thereof, or the pharmaceutically acceptable salt thereof according to claim 4 or 5,
wherein the two $R_{X1-1}$, together with the atom to which they are connected, form an optionally substituted cyclic structure.

7. The compound or the tautomer, the mesomer, the racemate, the enantiomer or the diastereoisomer thereof, or the mixture thereof, or the pharmaceutically acceptable salt thereof according to any one of claims 4-6,
wherein the two $R_{X1-1}$, together with the atom to which they are connected, form an optionally substituted alcyl.

**8.** The compound or the tautomer, the mesomer, the racemate, the enantiomer or the diastereoisomer thereof, or the mixture thereof, or the pharmaceutically acceptable salt thereof according to any one of claims 4-7, wherein the two $R_{X1-1}$, together with the atom to which they are connected, form an optionally substituted cyclopropyl.

**9.** The compound or the tautomer, the mesomer, the racemate, the enantiomer or the diastereoisomer thereof, or the mixture thereof, or the pharmaceutically acceptable salt thereof according to claim 1, wherein $X_3$ is selected from optionally substituted -CH- or -N-.

**10.** The compound or the tautomer, the mesomer, the racemate, the enantiomer or the diastereoisomer thereof, or the mixture thereof, or the pharmaceutically acceptable salt thereof according to claim 9, wherein $X_3$ is substituted with $R_{X3-1}$, $R_{X3-1}$ being selected from any group.

**11.** The compound or the tautomer, the mesomer, the racemate, the enantiomer or the diastereoisomer thereof, or the mixture thereof, or the pharmaceutically acceptable salt thereof according to claim 10, wherein $R_{X3-1}$ is selected from hydrogen and optionally substituted $C_1$-$C_6$ alkyl; when $R_{X3-1}$ comprises methylene units, the methylene units of $R_{X3-1}$ are each independently unreplaced, or are each independently replaced by any structure.

**12.** The compound or the tautomer, the mesomer, the racemate, the enantiomer or the diastereoisomer thereof, or the mixture thereof, or the pharmaceutically acceptable salt thereof according to claim 10 or 11, wherein $R_{X3-1}$ comprises an optionally substituted methyl.

**13.** The compound or the tautomer, the mesomer, the racemate, the enantiomer or the diastereoisomer thereof, or the mixture thereof, or the pharmaceutically acceptable salt thereof according to claim 1, wherein $R_2$ is each independently selected from hydrogen and optionally substituted $C_1$-$C_6$ alkyl; when $R_2$ comprises methylene units, the methylene units of $R_2$ are each independently unreplaced, or are each independently replaced by any structure.

**14.** The compound or the tautomer, the mesomer, the racemate, the enantiomer or the diastereoisomer thereof, or the mixture thereof, or the pharmaceutically acceptable salt thereof according to claim 13, wherein $R_2$ is each independently selected from optionally substituted methyl, optionally substituted ethyl, optionally substituted propyl, optionally substituted butyl, and optionally substituted pentyl.

**15.** The compound or the tautomer, the mesomer, the racemate, the enantiomer or the diastereoisomer thereof, or the mixture thereof, or the pharmaceutically acceptable salt thereof according to claim 13 or 14, wherein the methylene unit of $R_2$ is each independently unreplaced or replaced by -O-, -C(=O)-, and/or optionally substituted -NH-.

**16.** The compound or the tautomer, the mesomer, the racemate, the enantiomer or the diastereoisomer thereof, or the mixture thereof, or the pharmaceutically acceptable salt thereof according to any one of claims 13-15, wherein $R_2$ is selected from the group consisting of: optionally substituted

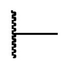,

optionally substituted

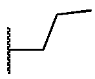,

optionally substituted

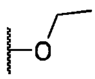,

optionally substituted

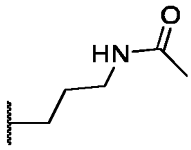,

optionally substituted

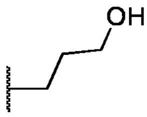,

optionally substituted

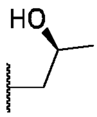,

optionally substituted

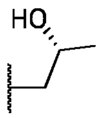,

optionally substituted

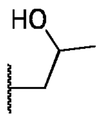,

optionally substituted

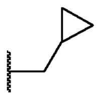,

optionally substituted

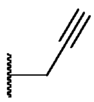,

optionally substituted

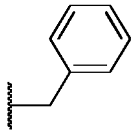

,

and optionally substituted

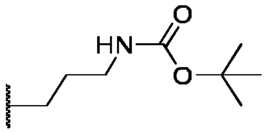

.

**17.** The compound or the tautomer, the mesomer, the racemate, the enantiomer or the diastereoisomer thereof, or the mixture thereof, or the pharmaceutically acceptable salt thereof according to any one of claims 13-16, wherein $R_2$ is selected from the group consisting of: optionally substituted

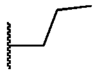

,

optionally substituted

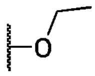

,

optionally substituted

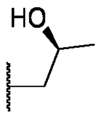

,

optionally substituted

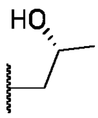

,

and optionally substituted

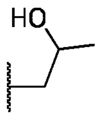

.

**18.** The compound or the tautomer, the mesomer, the racemate, the enantiomer or the diastereoisomer thereof, or the mixture thereof, or the pharmaceutically acceptable salt thereof according to any one of claims 13-17, wherein $R_2$ is selected from the group consisting of: optionally substituted

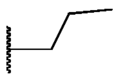

and optionally substituted

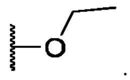

**19.** The compound or the tautomer, the mesomer, the racemate, the enantiomer or the diastereoisomer thereof, or the mixture thereof, or the pharmaceutically acceptable salt thereof according to any one of claims 13-18, wherein $R_2$ is each independently substituted with one or more $R_{2-1}$, the one or more $R_{2-1}$ being selected from any group.

**20.** The compound or the tautomer, the mesomer, the racemate, the enantiomer or the diastereoisomer thereof, or the mixture thereof, or the pharmaceutically acceptable salt thereof according to claim 19, wherein the one or more $R_{2-1}$ are selected from the group consisting of: hydrogen, halogen, optionally substituted alkyl, optionally substituted alcyl, and an optionally substituted aryl; when the one or more $R_{2-1}$ comprise methylene units, the methylene units of the one or more $R_{2-1}$ are each independently unreplaced, or are each independently replaced by any structure.

**21.** The compound or the tautomer, the mesomer, the racemate, the enantiomer or the diastereoisomer thereof, or the mixture thereof, or the pharmaceutically acceptable salt thereof according to claim 19 or 20, wherein the one or more $R_{2-1}$ are selected from the group consisting of: hydrogen, fluorine, optionally substituted methyl, optionally substituted cyclopropyl, and optionally substituted phenyl.

**22.** The compound or the tautomer, the mesomer, the racemate, the enantiomer or the diastereoisomer thereof, or the mixture thereof, or the pharmaceutically acceptable salt thereof according to claim 1, wherein W is absent, or is each independently selected from hydrogen and optionally substituted $C_1$-$C_6$ alkyl; when W comprises methylene units, the methylene units of W are each independently unreplaced, or are each independently replaced by any structure.

**23.** The compound or the tautomer, the mesomer, the racemate, the enantiomer or the diastereoisomer thereof, or the mixture thereof, or the pharmaceutically acceptable salt thereof according to claim 22, wherein W is each independently selected from optionally substituted methylene, optionally substituted ethylene, optionally substituted propylene, optionally substituted butylene, and optionally substituted pentylene.

**24.** The compound or the tautomer, the mesomer, the racemate, the enantiomer or the diastereoisomer thereof, or the mixture thereof, or the pharmaceutically acceptable salt thereof according to claim 22 or 23, wherein the methylene unit of W is each independently unreplaced or replaced by -O-, -C(=O)-, and/or optionally substituted -NH-.

**25.** The compound or the tautomer, the mesomer, the racemate, the enantiomer or the diastereoisomer thereof, or the mixture thereof, or the pharmaceutically acceptable salt thereof according to any one of claims 22-24, wherein W is selected from the group consisting of: optionally substituted

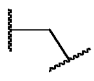,

optionally substituted

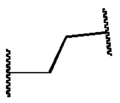,

optionally substituted

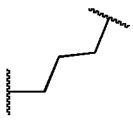,

optionally substituted

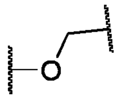,

and optionally substituted

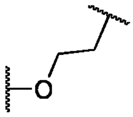.

26. The compound or the tautomer, the mesomer, the racemate, the enantiomer or the diastereoisomer thereof, or the mixture thereof, or the pharmaceutically acceptable salt thereof according to any one of claims 22-25, wherein W is selected from the group consisting of: optionally substituted

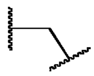

and optionally substituted

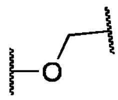.

27. The compound or the tautomer, the mesomer, the racemate, the enantiomer or the diastereoisomer thereof, or the mixture thereof, or the pharmaceutically acceptable salt thereof according to claim 1, wherein B is selected from the group consisting of: optionally substituted aryl and optionally substituted heteroaryl.

28. The compound or the tautomer, the mesomer, the racemate, the enantiomer or the diastereoisomer thereof, or the mixture thereof, or the pharmaceutically acceptable salt thereof according to claim 27, wherein B is selected from the group consisting of: optionally substituted phenyl, optionally substituted naphthyl, optionally substituted pyridinyl, optionally substituted pyrrolyl, optionally substituted thienyl, optionally substituted furanyl, and optionally substituted pyrazinyl.

29. The compound or the tautomer, the mesomer, the racemate, the enantiomer or the diastereoisomer thereof, or the mixture thereof, or the pharmaceutically acceptable salt thereof according to claim 27 or 28, wherein B is selected from optionally substituted phenyl.

30. The compound or the tautomer, the mesomer, the racemate, the enantiomer or the diastereoisomer thereof, or the mixture thereof, or the pharmaceutically acceptable salt thereof according to any one of claims 27-29, wherein B is further substituted with one or more $R_{B-1}$, the one or more $R_{B-1}$ being each independently selected from any group.

31. The compound or the tautomer, the mesomer, the racemate, the enantiomer or the diastereoisomer thereof, or the mixture thereof, or the pharmaceutically acceptable salt thereof according to claim 30, wherein the one or more $R_{B-1}$ are each independently selected from the group consisting of: hydrogen, halogen, and optionally substituted $C_1$-$C_6$ alkyl; when the one or more $R_{B-1}$ comprise methylene units, the methylene units

of the one or more $R_{B-1}$ are each independently unreplaced, or are each independently replaced by any structure.

**32.** The compound or the tautomer, the mesomer, the racemate, the enantiomer or the diastereoisomer thereof, or the mixture thereof, or the pharmaceutically acceptable salt thereof according to claim 30 or 31, wherein the one or more $R_{B-1}$ are each independently selected from the group consisting of: hydrogen, fluorine, and optionally substituted methyl.

**33.** The compound or the tautomer, the mesomer, the racemate, the enantiomer or the diastereoisomer thereof, or the mixture thereof, or the pharmaceutically acceptable salt thereof according to any one of claims 27-32, wherein B is substituted with one or more optionally substituted amino groups, the one or more optionally substituted amino groups being each independently substituted with any structure.

**34.** The compound or the tautomer, the mesomer, the racemate, the enantiomer or the diastereoisomer thereof, or the mixture thereof, or the pharmaceutically acceptable salt thereof according to any one of claims 27-33, wherein B is substituted with one or more optionally substituted amino groups, the one or more amino groups being each independently substituted with hydrogen or optionally substituted $C_1$-$C_6$ alkyl.

**35.** The compound or the tautomer, the mesomer, the racemate, the enantiomer or the diastereoisomer thereof, or the mixture thereof, or the pharmaceutically acceptable salt thereof according to claim 1, wherein A is selected from the group consisting of: optionally substituted alcyl, optionally substituted aliphatic heterocyclyl, an optionally substituted aryl, and optionally substituted heteroaryl.

**36.** The compound or the tautomer, the mesomer, the racemate, the enantiomer or the diastereoisomer thereof, or the mixture thereof, or the pharmaceutically acceptable salt thereof according to claim 35, wherein A is selected from the group consisting of: optionally substituted phenyl, optionally substituted pyridinyl, optionally substituted pyrrolyl, optionally substituted thienyl, optionally substituted furanyl, and optionally substituted pyrazinyl.

**37.** The compound or the tautomer, the mesomer, the racemate, the enantiomer or the diastereoisomer thereof, or the mixture thereof, or the pharmaceutically acceptable salt thereof according to claim 35 or 36, wherein A is substituted with one or more $R_{A-1}$, the one or more $R_{A-1}$ being each independently selected from any group.

**38.** The compound or the tautomer, the mesomer, the racemate, the enantiomer or the diastereoisomer thereof, or the mixture thereof, or the pharmaceutically acceptable salt thereof according to claim 37, wherein $R_{A-1}$ is absent or is selected from optionally substituted $C_1$-$C_6$ alkyl; when $R_{A-1}$ comprises methylene units, the methylene units of $R_{A-1}$ are each independently unreplaced, or are each independently replaced by any structure.

**39.** The compound or the tautomer, the mesomer, the racemate, the enantiomer or the diastereoisomer thereof, or the mixture thereof, or the pharmaceutically acceptable salt thereof according to claim 38, wherein $R_{A-1}$ is selected from optionally substituted methyl, optionally substituted ethyl, optionally substituted propyl, or optionally substituted butyl.

**40.** The compound or the tautomer, the mesomer, the racemate, the enantiomer or the diastereoisomer thereof, or the mixture thereof, or the pharmaceutically acceptable salt thereof according to any one of claims 37-39, wherein the methylene units that $R_{A-1}$ comprises are unreplaced, or are replaced by a group selected from the group consisting of: -S(=O)-, -C(=O)-, $-S(=O)_2-$, -O-, -S-, optionally substituted -NH-, optionally substituted alkylene, optionally substituted alkenylene, optionally substituted alkynylene, optionally substituted alcylene, optionally substituted aliphatic heterocyclylene, optionally substituted arylene, and optionally substituted heteroarylene.

**41.** The compound or the tautomer, the mesomer, the racemate, the enantiomer or the diastereoisomer thereof, or the mixture thereof, or the pharmaceutically acceptable salt thereof according to any one of claims 37-40, wherein $R_{A-1}$ is absent, or is selected from the group consisting of: optionally substituted $CH_3$-, optionally substituted $CH(=CH_2)$-, optionally substituted HC(=O)-, optionally substituted HOC(=O)-, optionally substituted HC(=O)O-, optionally substituted NH-, optionally substituted HO-, optionally substituted HS-, optionally substituted HS(=O)-, optionally substituted $HS(=O)_2$-, optionally substituted $CH_3CH_2$-, optionally substituted $CH_2$=CH-, optionally substituted HC=C-, optionally substituted $HOCH_2$-, optionally substituted $CH_3O$-, optionally substituted $CH_3NH$-, optionally substituted $NH_2CH_2$-, optionally substituted $HS(=O)_2$-NH-, optionally substituted $NH_2$-$S(=O)_2$-, optionally substituted HS-$CH_2$-, optionally substituted $CH_3S$-, optionally substituted HS(=O)-$CH_2$-, optionally substituted $CH_3$-S(=O)-, op-

tionally substituted $HS(=O)_2-CH_2-$, optionally substituted $CH_3-S(=O)_2-$, optionally substituted $NH_2C(=O)-$, optionally substituted $HC(=O)NH-$, optionally substituted $HC(=O)CH_2-$, optionally substituted $HC(=O)NHCH_2-$, optionally substituted $CH_3NHC(=O)-$, optionally substituted

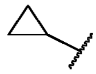,

optionally substituted

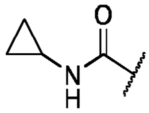,

optionally substituted

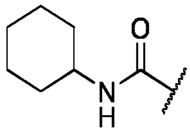,

optionally substituted

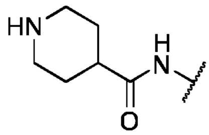,

optionally substituted

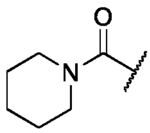,

optionally substituted

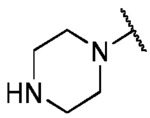,

optionally substituted

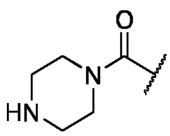,

optionally substituted

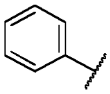,

optionally substituted

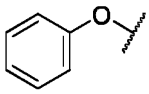,

optionally substituted

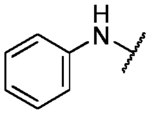,

optionally substituted

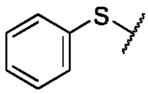,

optionally substituted

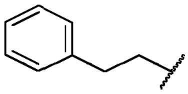,

optionally substituted

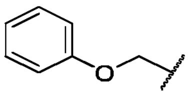,

optionally substituted

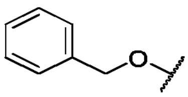,

optionally substituted

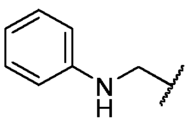,

optionally substituted

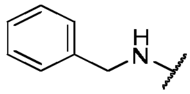,

optionally substituted

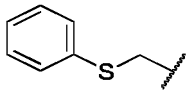,

optionally substituted

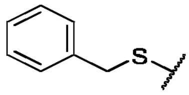,

optionally substituted

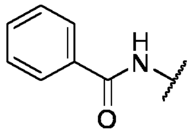,

optionally substituted

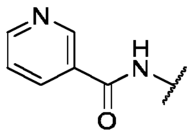,

optionally substituted

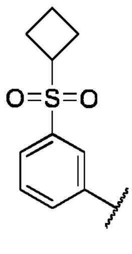,

optionally substituted

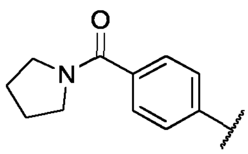,

optionally substituted

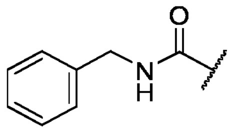

,

optionally substituted

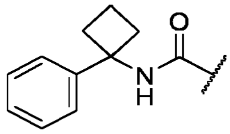

,

optionally substituted

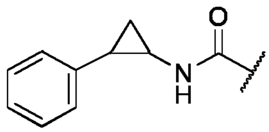

,

optionally substituted

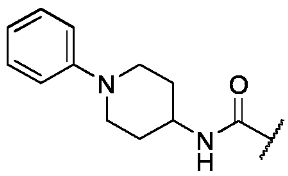

,

optionally substituted

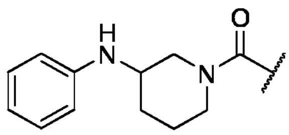

,

optionally substituted

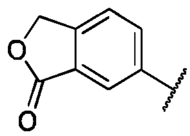

,

optionally substituted

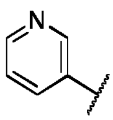

and optionally substituted

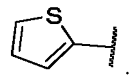

**42.** The compound or the tautomer, the mesomer, the racemate, the enantiomer or the diastereoisomer thereof, or the mixture thereof, or the pharmaceutically acceptable salt thereof according to any one of claims 37-41, wherein $R_{A-1}$ comprises optionally substituted

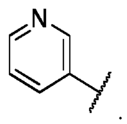

**43.** The compound or the tautomer, the mesomer, the racemate, the enantiomer or the diastereoisomer thereof, or the mixture thereof, or the pharmaceutically acceptable salt thereof according to any one of claims 37-42, wherein the one or more $R_{A-1}$ are each independently substituted with one or more $R_{A-2}$, the one or more $R_{A-2}$ being each independently selected from any group.

**44.** The compound or the tautomer, the mesomer, the racemate, the enantiomer or the diastereoisomer thereof, or the mixture thereof, or the pharmaceutically acceptable salt thereof according to claim 43, wherein $R_{A-2}$ is absent, or is selected from optionally substituted $C_1$-$C_6$ alkyl; when $R_{A-2}$ comprises methylene units, the methylene units of $R_{A-2}$ are each independently unreplaced, or are each independently replaced by any structure.

**45.** The compound or the tautomer, the mesomer, the racemate, the enantiomer or the diastereoisomer thereof, or the mixture thereof, or the pharmaceutically acceptable salt thereof according to claim 43 or 44, wherein $R_{A-2}$ comprises hydrogen, halogen, optionally substituted methyl, and/or optionally substituted hydroxy.

**46.** The compound or the tautomer, the mesomer, the racemate, the enantiomer or the diastereoisomer thereof, or the mixture thereof, or the pharmaceutically acceptable salt thereof according to any one of claims 43-45, wherein the one or more $R_{A-2}$ are each independently substituted with one or more $R_{A-3}$, the one or more $R_{A-3}$ being each independently selected from any group.

**47.** The compound or the tautomer, the mesomer, the racemate, the enantiomer or the diastereoisomer thereof, or the mixture thereof, or the pharmaceutically acceptable salt thereof according to claim 46, wherein $R_{A-3}$ comprises optionally substituted methyl and/or optionally substituted hydroxy.

**48.** The compound according to claim 1, wherein the compound is a compound of formula (A), or a tautomer, a mesomer, a racemate, an enantiomer or a diastereoisomer thereof, or a mixture thereof, or a pharmaceutically acceptable salt thereof,

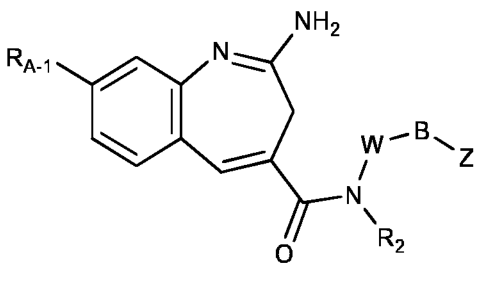

(A)

wherein $R_{A-1}$ is selected from $R_{A2-1}$-Lx-;

Lx is selected from single bond, $C_1$-$C_6$ alkylene, -O-, -S-, -C(O)-, -NH-, -C(O)NH-, -NHC(O)-, - C(O)-N($C_1$-$C_6$ alkyl)-, or -N($C_1$-$C_6$ alkylene)-C(O)-;

$R_{A2-1}$ is aryl, heteroaryl, or heterocyclyl, preferably phenyl, pyridinyl, or isobenzofuranonyl, and $R_{A2-1}$ is optionally substituted with one or more substituents $R_{A2-2}$, wherein $R_{A2-2}$ are each independently selected from $C_1$-$C_6$ alkyl, amino, -NH($C_1$-$C_6$ alkyl), -N($C_1$-$C_6$ alkyl)$_2$, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, -$C_1$-$C_6$ alkylhydroxy, -$C_1$-$C_6$ alkyl-$C_1$-$C_6$ alkoxy, halogen, halogenated $C_1$-$C_6$ alkyl, halogenated $C_1$-$C_6$ alkoxy, or -C(O)N$R_e$$R_f$, and wherein $R_e$e and $R_f$ are each independently selected

from H or $C_1$-$C_6$ alkyl, or $R_e$ and $R_f$, together with the nitrogen atom to which they are connected, form a 5- or 6-membered nitrogen-containing heterocyclyl, such as tetrahydropyrrolyl or piperidinyl, the 5- or 6-membered nitrogen-containing heterocyclyl being optionally substituted with one or more substituents independently selected from $C_1$-$C_6$ alkyl, $C_1$-$C_6$ alkoxy, hydroxy, oxo, amino, - NH($C_1$-$C_6$ alkyl), or -N($C_1$-$C_6$ alkyl)$_2$, or

$R_{A2-1}$ is phenyl, 5- to 6-membered heteroaryl, or isobenzofuranonyl, and $R_{A2-1}$ is optionally substituted with one or more substituents $R_{A2-2}$, wherein $R_{A2-2}$ is each independently selected from $C_1$-$C_6$ alkyl, cyano, amino, -COOH, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, -$C_1$-$C_6$ alkylhydroxy, -$C_1$-$C_6$ alkylphenyl, -phenyl, -O-phenyl, $C_{3-6}$ cycloalkyl, -$C_1$-$C_6$ alkyl-$C_1$-$C_6$ alkoxy, halogen, halogenated $C_1$-$C_6$ alkyl, halogenated $C_1$-$C_6$ alkoxy, -S(O)$C_1$-$C_6$ alkyl, -S(O)$_2$$C_1$-$C_6$ alkyl, -S(O)$_2$NR$_e$R$_f$, -C(O)NR$_e$R$_f$, or - NR$_e$R$_f$, wherein $R_e$ and $R_f$ are each independently selected from H, $C_1$-$C_6$ alkyl or -$C_1$-$C_6$ alkylhydroxy, or $R_e$ and $R_f$, together with the nitrogen atom to which they are connected, form a 4- to 8-membered nitrogen-containing heterocyclyl, e.g., tetrahydropyrrolyl, piperidinyl, morpholinyl, piperazinyl, and azetidinyl, the 4- to 8-membered nitrogen-containing heterocycle being optionally substituted with one or more substituents independently selected from halogen, hydroxy, oxo, amino, $C_1$-$C_6$ alkyl, $C_1$-$C_6$ alkoxy, -$C_1$-$C_6$ alkylhydroxy, -NH($C_1$-$C_6$ alkyl), -N($C_1$-$C_6$ alkyl)$_2$, -O-phenyl, and phenyl; or

$R_{A-1}$ is selected from N($R_{AN-1}$)($R_{AN-2}$)C(O)-, wherein $R_{AN-1}$ and $R_{AN-2}$ are each independently selected from H, $C_1$-$C_6$ alkyl, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, -$C_1$-$C_6$ alkylhydroxy, halogenated $C_1$-$C_6$ alkyl, -$C_1$-$C_6$ alkyl-$C_1$-$C_6$ alkoxy, -Ci-$C_6$ alkyl-phenyl, -phenyl, 5- to 10-membered heteroaryl, $C_{3-6}$ cycloalkyl, -$C_{3-6}$ cycloalkyl-phenyl, 7- to 10-membered bicyclic heterocyclyl, and 3- to 8-membered heterocyclyl, wherein the phenyl, 5- to 10-membered heteroaryl, $C_{3-6}$ cycloalkyl, 7- to 10-membered bicyclic heterocyclyl, and 3-to 8-membered heterocyclyl are each independently optionally substituted with one or more substituents selected from $C_1$-$C_6$ alkyl, cyano, amino, -NH($C_1$-$C_6$ alkyl), -N($C_1$-$C_6$ alkyl)$_2$, -$C_1$-$C_6$ alkylamino, -$C_1$-$C_6$ alkyl-NH($C_1$-$C_6$ alkyl), -$C_1$-$C_6$ alkyl-N($C_1$-$C_6$ alkyl)$_2$, hydroxy, -$C_1$-$C_6$ alkoxy, -$C_1$-$C_6$ alkylhydroxy, -$C_1$-$C_6$ alkyl-$C_1$-$C_6$ alkoxy, halogen, halogenated $C_1$-$C_6$ alkyl, halogenated $C_1$-$C_6$ alkoxy, -S(O)$C_1$-$C_6$ alkyl, -S(O)$_2$$C_1$-$C_6$ alkyl, -S(O)$_2$NR$_e$R$_f$, or -C(O)NR$_e$R$_f$, $R_e$ and $R_f$ being each independently selected from H, $C_1$-$C_6$ alkyl, or -$C_1$-$C_6$ alkylhydroxy; or

$R_{AN-1}$ and $R_{AN-2}$, together with the nitrogen atom to which they are connected, form a 4- to 10-membered nitrogen-containing heterocycle, e.g., tetrahydropyrrolyl, piperidinyl, morpholinyl, piperazinyl, azetidinyl,

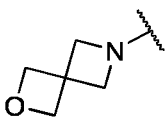, 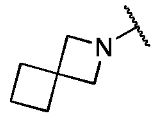, 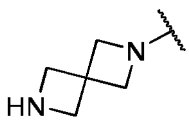, 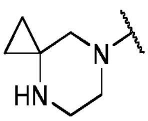, 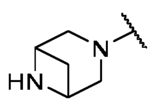,

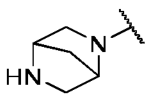, 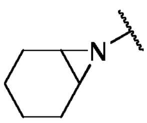, 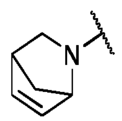, 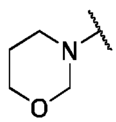, 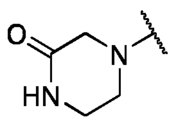, 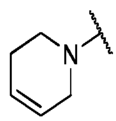, 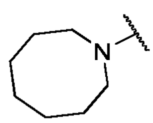,

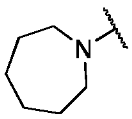, 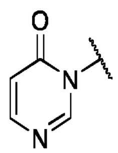, 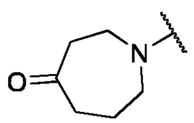, 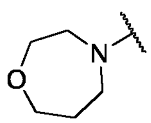, 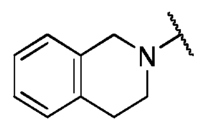, 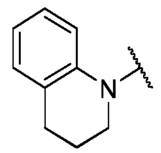,

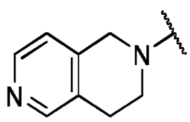 and 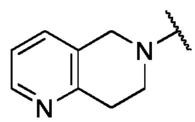,

wherein the 4- to 10-membered nitrogen-containing heterocycle is optionally substituted with one or more substituents independently selected from hydrogen, halogen, $C_1$-$C_6$ alkyl, $C_1$-$C_6$ alkoxy, -$C_1$-$C_6$ alkylhydroxy, hydroxy, oxo, amino, - NH($C_1$-$C_6$ alkyl), -N($C_1$-$C_6$ alkyl)$_2$, -O-phenyl, and phenyl;

$R_2$ is selected from -$C_1$-$C_6$ alkyl, -$C_1$-$C_6$ alkoxy, -$C_1$-$C_6$ alkylenehydroxy, or halogenated $C_1$-$C_6$ alkyl; or $R_2$ is

$-C_1-C_6$ alkyl-cyclopropyl;

W is selected from $-C_1-C_6$ alkylene- and $-O-C_1-C_6$ alkylene-; preferably, W is selected from

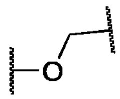

;

B is phenyl, and is optionally substituted with one or more substituents selected from: halogen, $C_1-C_6$ alkyl, halogenated $C_1-C_6$ alkyl, and $C_1-C_6$ alkoxy; and

Z is $-NH(R_{Z-1})$, $R_{Z-1}$ being selected from H, $-C_1-C_6$ alkyl, or halogenated $C_1-C_6$ alkyl.

**49.** The compound or the tautomer, the mesomer, the racemate, the enantiomer or the diastereoisomer thereof, or the mixture thereof, or the pharmaceutically acceptable salt thereof according to any one of claims 1-48, wherein,

$R_{A2-1}$ is selected from

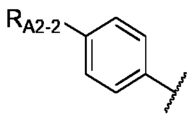 , 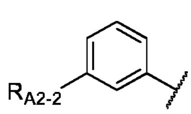 , 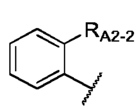 , 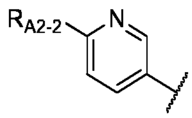 ,

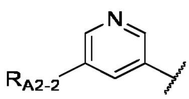 , 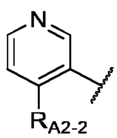 , 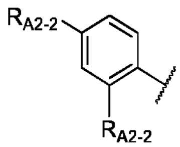 , 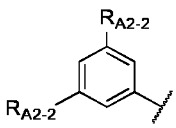 , 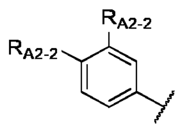 ,

or $N(R_{AN-1})(R_{AN-2})$ C(O)-; $R_{A2-2}$, $R_{AN-1}$, and $R_{AN-2}$ are as described in claim 48.

**50.** The compound or the tautomer, the mesomer, the racemate, the enantiomer or the diastereoisomer thereof, or the mixture thereof, or the pharmaceutically acceptable salt thereof according to any one of claims 1-49, wherein $R_{A-1}$ is selected from

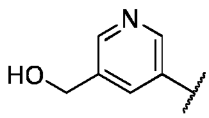 , 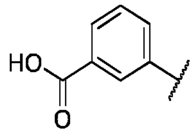 , 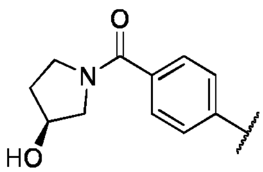 ,

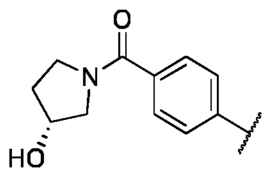 , 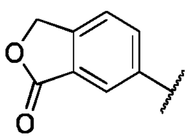 , 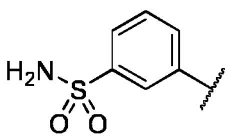 , 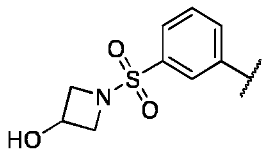 ,

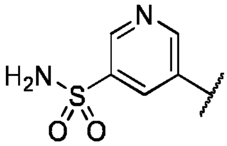 , 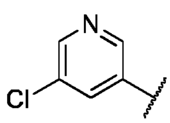 , 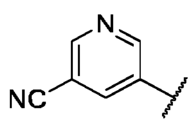 , 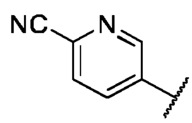 , 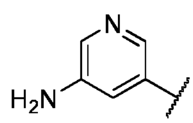 ,

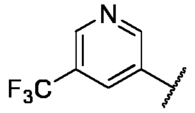 , 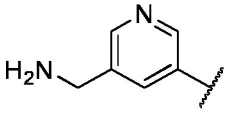 , 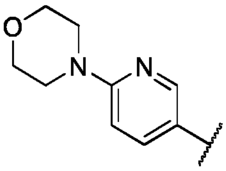 , 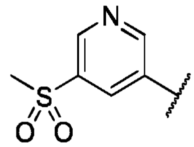 ,

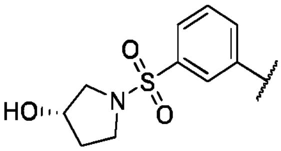 , 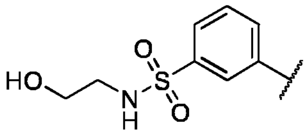 , 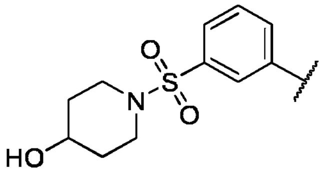 ,

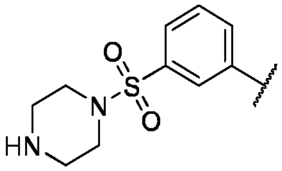 , 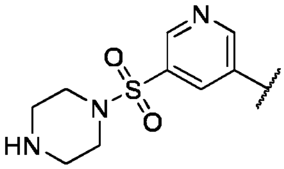 , 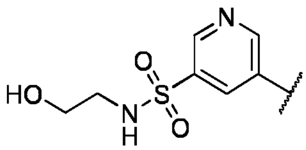 ,

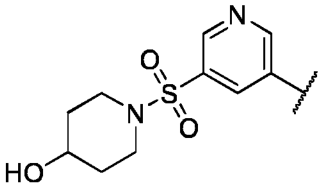 , 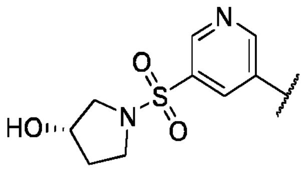 , 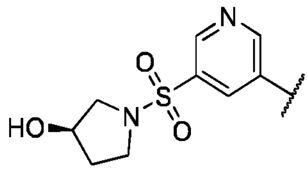 ,

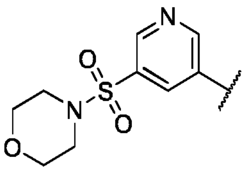 , 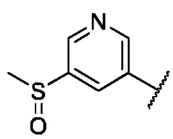 , 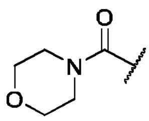 , 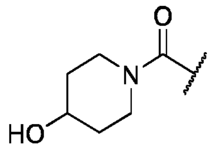 , 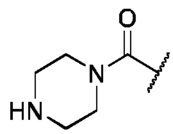 ,

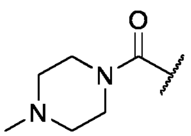 , 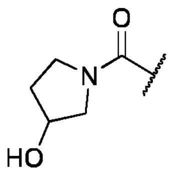 , 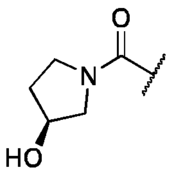 , 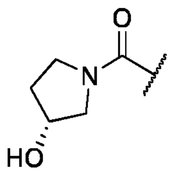 , 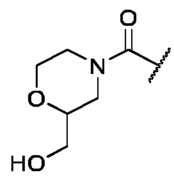 ,

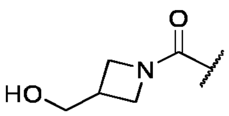 , 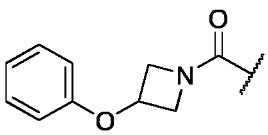 , 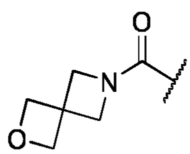 , 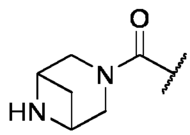 ,

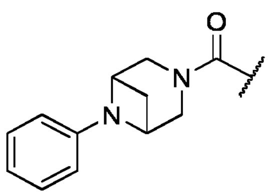 , 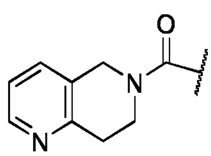 , 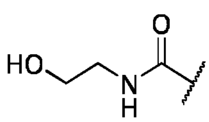 , 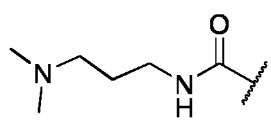 ,

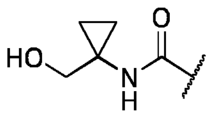, 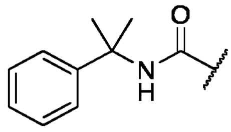, 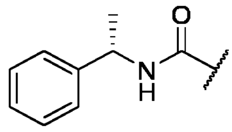, 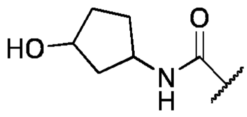, 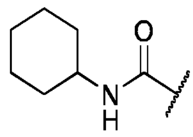,

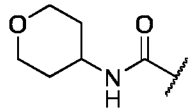, 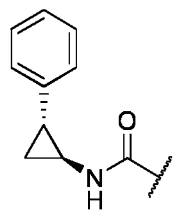, 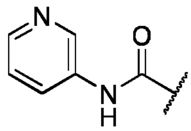, 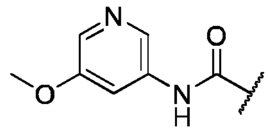, 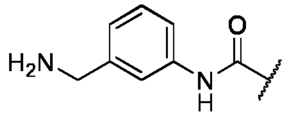,

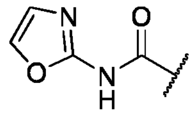, 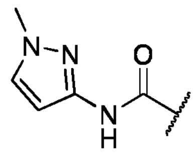, 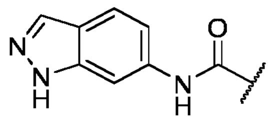, and 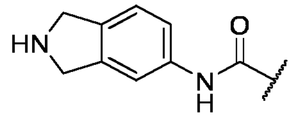.

**51.** The compound or the tautomer, the mesomer, the racemate, the enantiomer or the diastereoisomer thereof, or the mixture thereof, or the pharmaceutically acceptable salt thereof according to any one of claims 1-48, wherein B is selected from

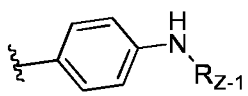, 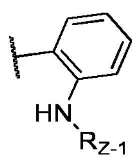, and 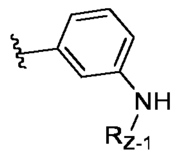,

$R_{Z-1}$ being selected from H, -$C_1$-$C_6$ alkyl, and halogenated $C_1$-$C_6$ alkyl.

**52.** The compound or the tautomer, the mesomer, the racemate, the enantiomer or the diastereoisomer thereof, or the mixture thereof, or the pharmaceutically acceptable salt thereof according to any one of claims 1-48, wherein the compound is selected from:

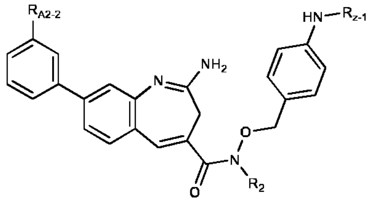,

(II-a-1)

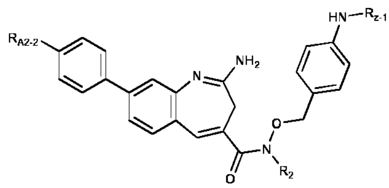,

(II-a-2)

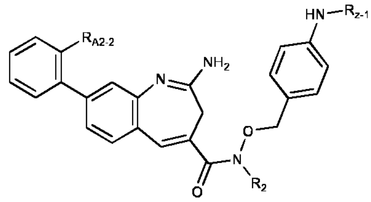,

(II-a-3)

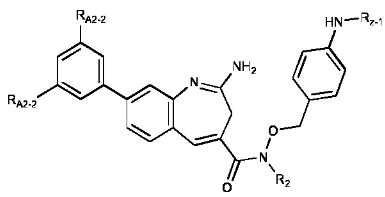,

(II-a-4)

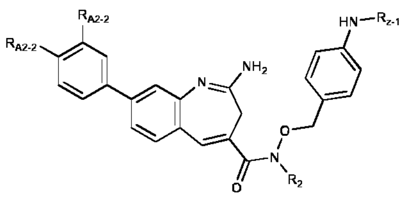,

(II-a-5)

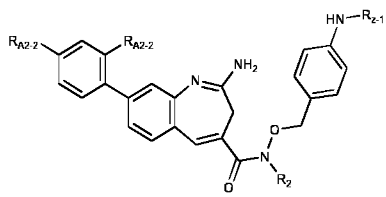,

(II-a-6)

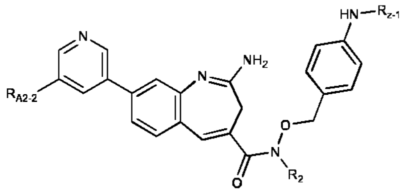,

(II-a-7)

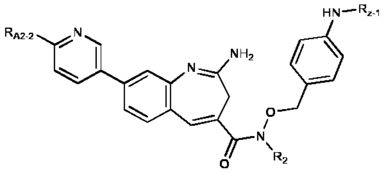,

(II-a-8)

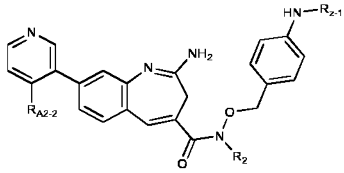,

(II-a-9)

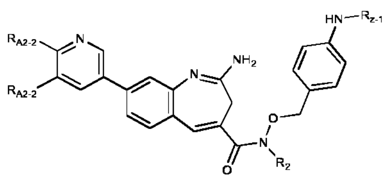,

(II-a-10)

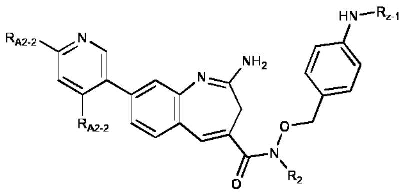,

(II-a-11)

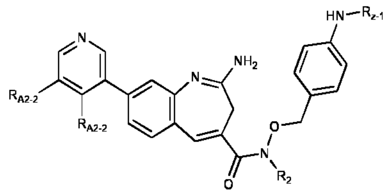,

(II-a-12)

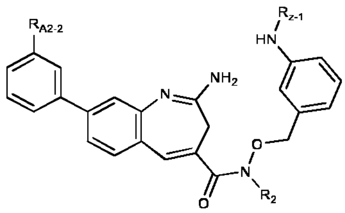,

(II-a-13)

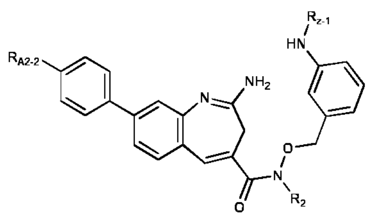,

(II-a-14)

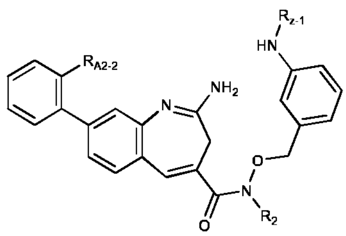,

(II-a-15)

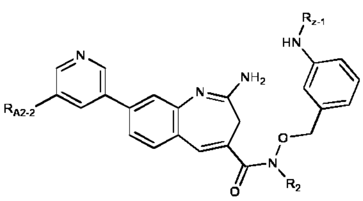,

(II-a-16)

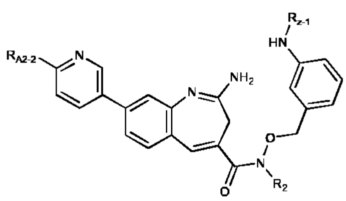,

(II-a-17)

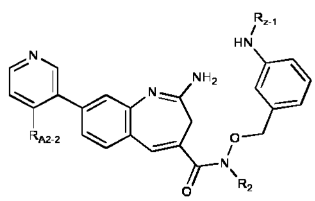,

(II-a-18)

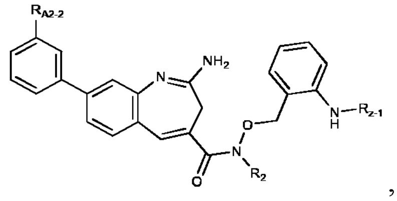,
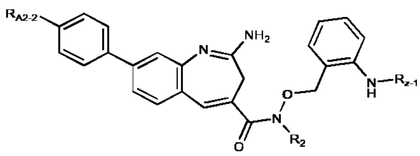,
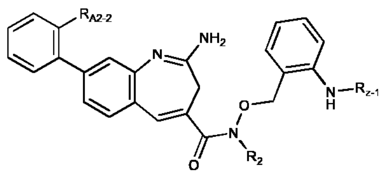,

(II-a-1) (II-a-2) (II-a-3)

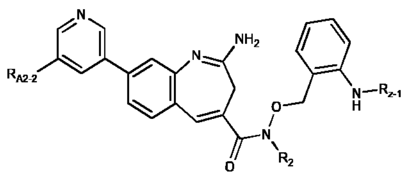,
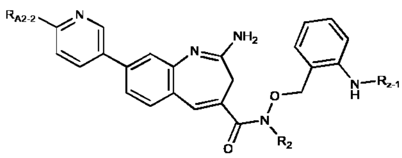,
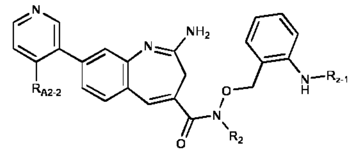,

(II-a-19) (II-a-20) (II-a-21)

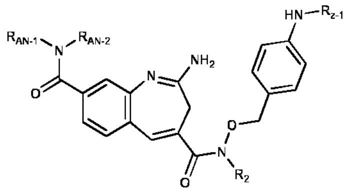,
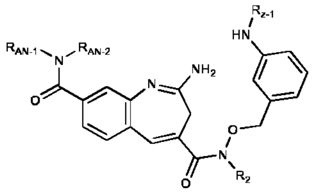,
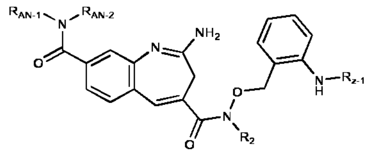,

(II-a-22) (II-a-23) (II-a-24)

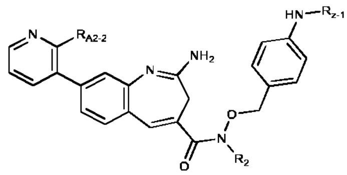,

(II-a-25)

wherein $R_{AN-1}$, $R_{AN-2}$, $R_{A2-2}$, and $R_2$ are as described in claim 48.

**53.** A compound or a tautomer, a mesomer, a racemate, an enantiomer or a diastereoisomer thereof, or a mixture thereof, or a pharmaceutically acceptable salt thereof, wherein the compound comprises a structure selected from the group consisting of:

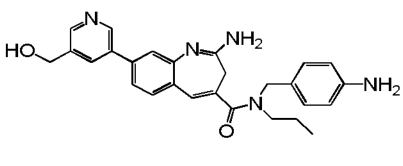
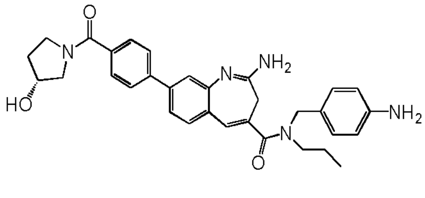
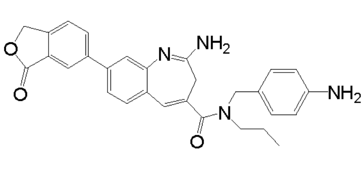

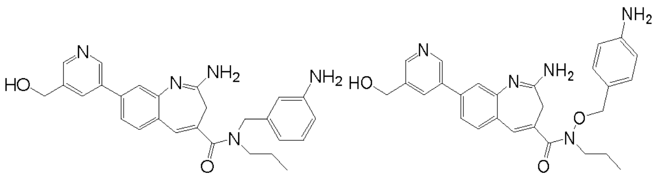
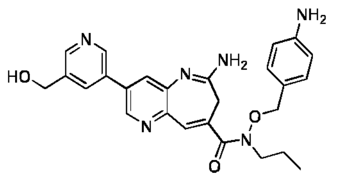

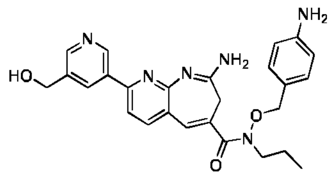
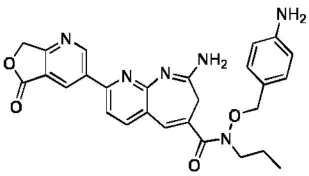
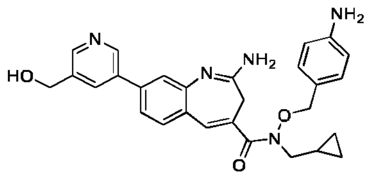

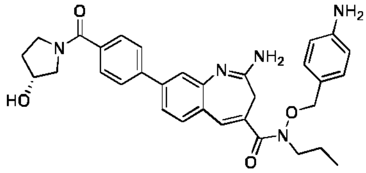
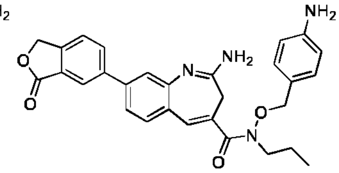
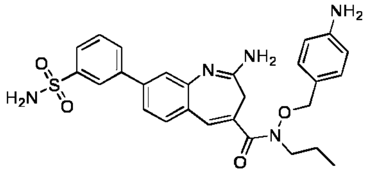

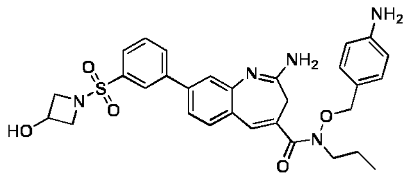
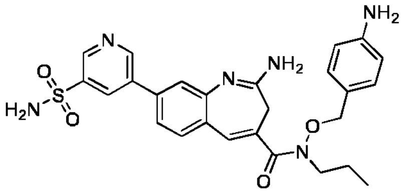
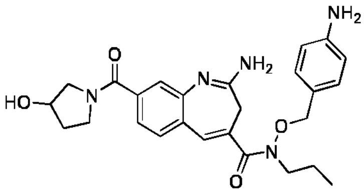

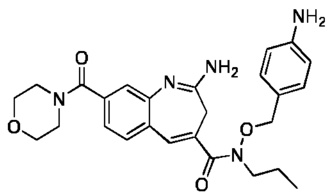
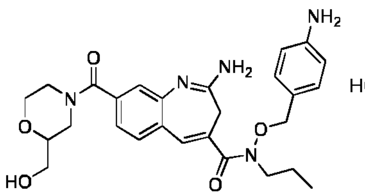
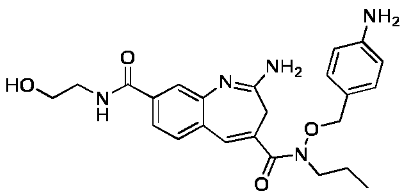

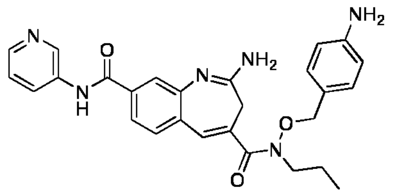
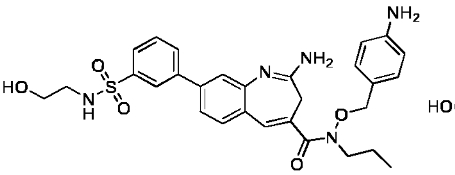
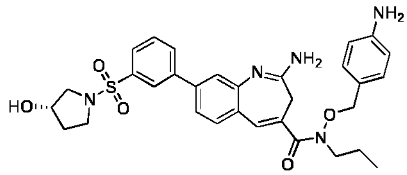

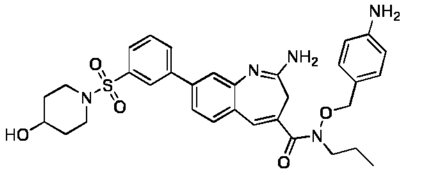
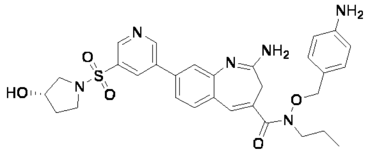
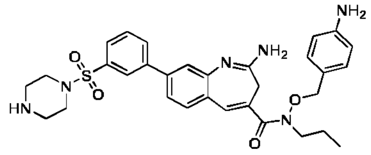

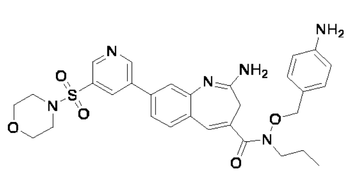
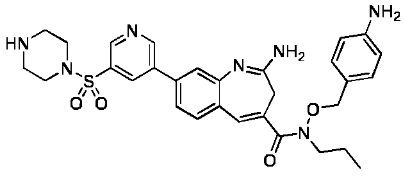
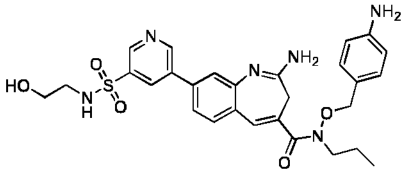

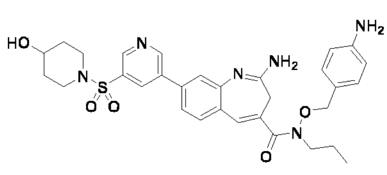
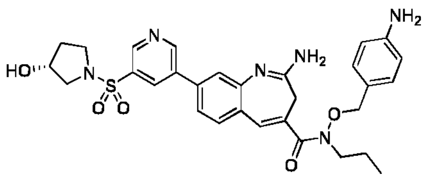
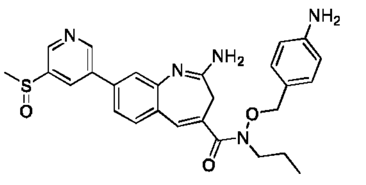

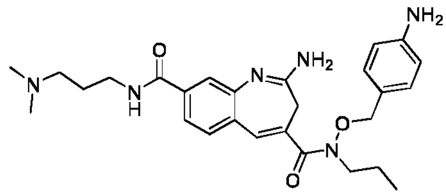
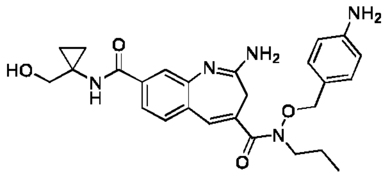
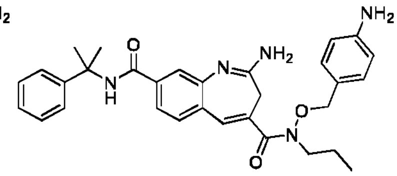

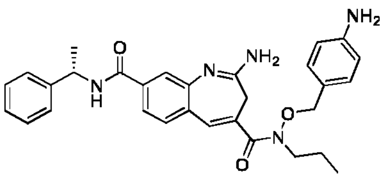
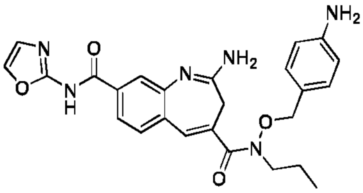
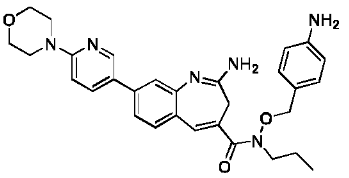

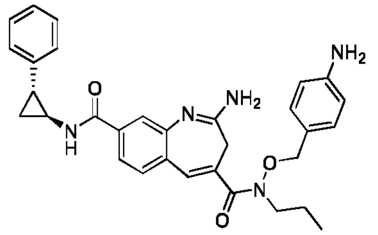
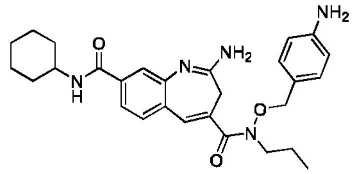
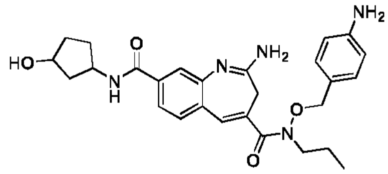

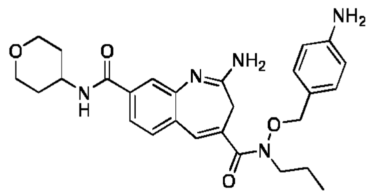
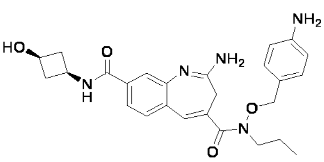
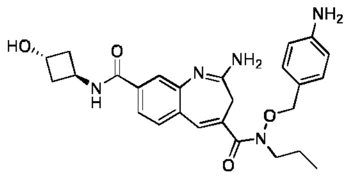

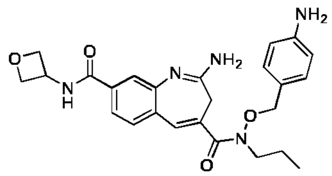
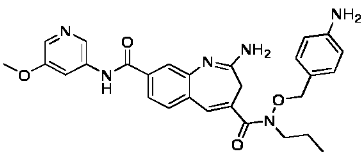
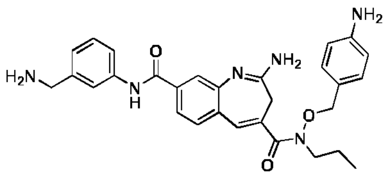

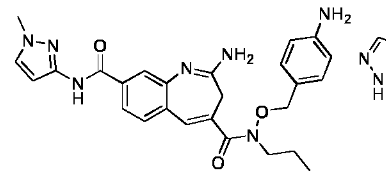
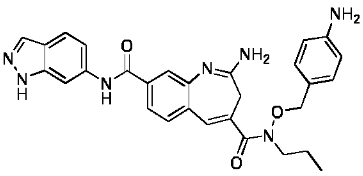
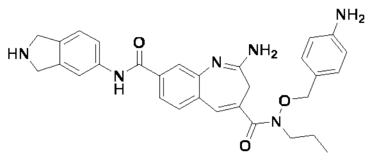

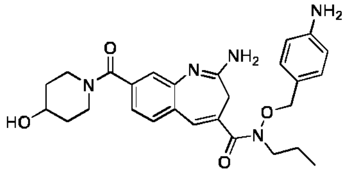
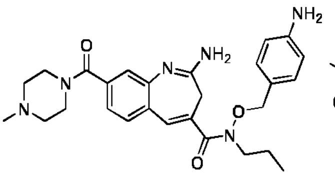
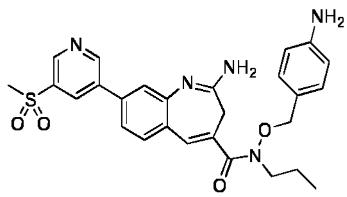

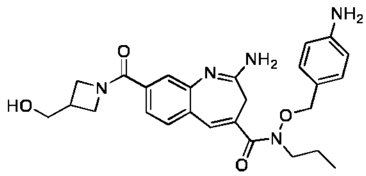 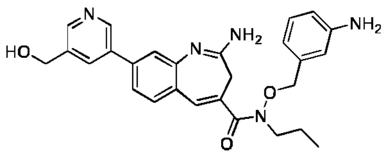 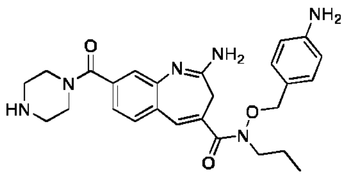

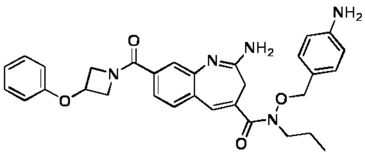 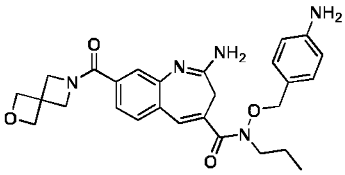 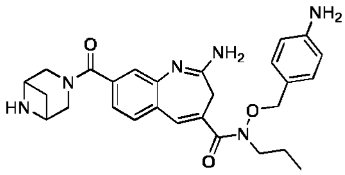

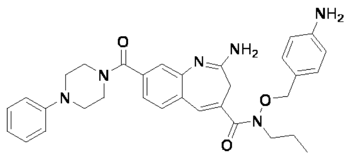 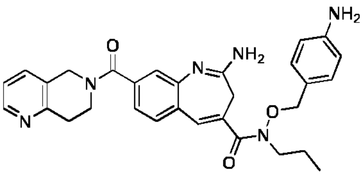 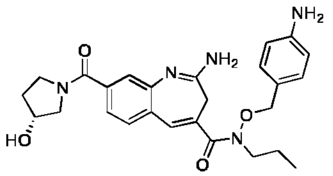

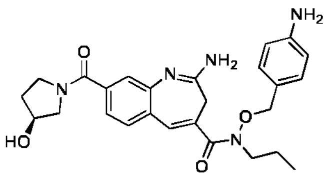 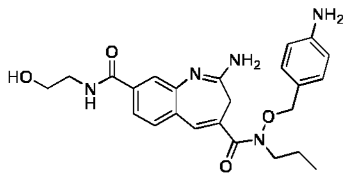 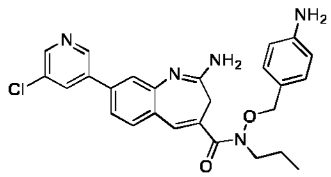

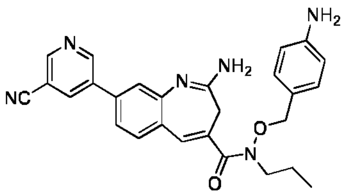 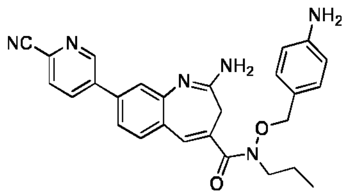 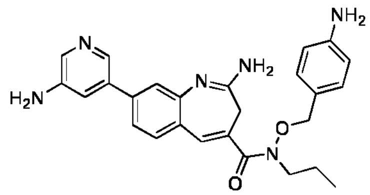

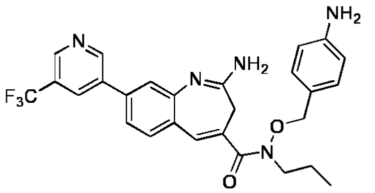 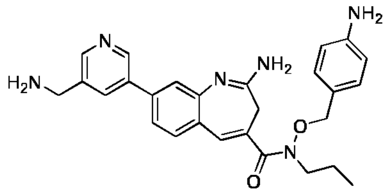 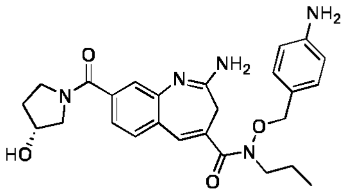

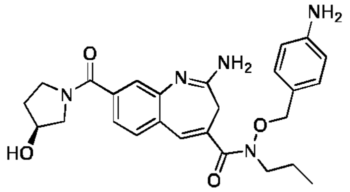 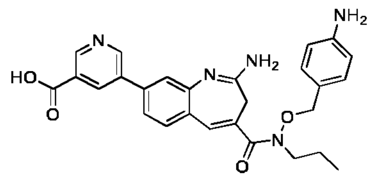 .

**54.** A conjugate, comprising the compound or the tautomer, the mesomer, the racemate, the enantiomer or the diastereoisomer thereof, or the mixture thereof, or the pharmaceutically acceptable salt thereof according to any one of claims 1-53.

**55.** The conjugate according to claim 54, comprising an antibody-drug conjugate.

**56.** A pharmaceutical composition, comprising the compound or the tautomer, the mesomer, the racemate, the enantiomer or the diastereoisomer thereof, or the mixture thereof, or the pharmaceutically acceptable salt thereof according to any one of claims 1-53 and/or the conjugate according to claim 54 or 55, and optionally a pharmaceutically acceptable carrier.

**57.** A method for influencing Toll-like receptor (TLR) functionality, comprising administering the compound or the tautomer, the mesomer, the racemate, the enantiomer or the diastereoisomer thereof, or the mixture thereof, or the pharmaceutically acceptable salt thereof according to any one of claims 1-53, the conjugate according to claim 54 or 55, or the pharmaceutical composition according to claim 56, wherein TLR includes TLR7 and/or TLR8.

**58.** A method for modulating immune system functionality, comprising administering the compound or the tautomer, the mesomer, the racemate, the enantiomer or the diastereoisomer thereof, or the mixture thereof, or the pharmaceutically acceptable salt thereof according to any one of claims 1-53, the conjugate according to claim 54 or 55, or the pharmaceutical composition according to claim 56.

**59.** Use of the compound or the tautomer, the mesomer, the racemate, the enantiomer or the diastereoisomer thereof, or the mixture thereof, or the pharmaceutically acceptable salt thereof according to any one of claims 1-53, the conjugate according to claim 54 or 55, or the pharmaceutical composition according to claim 55 in preparing a medicament for preventing and/or treating a disease and/or a symptom, wherein the disease and/or the symptom includes a disease and/or a symptom associated with Toll-like receptor (TLR) signaling.

**60.** The use according to claim 59, wherein the disease and/or the symptom is selected from the group consisting of: tumor, autoimmune disease, inflammation, septicemia, allergy, asthma, transplant rejection, graft-versus-host disease, immunodeficiency, and viral infection.

**61.** The use according to claim 60, wherein the disease and/or the symptom is selected from the group consisting of: melanoma, lung cancer, liver cancer, basal cell carcinoma, kidney cancer, myeloma, biliary tract cancer, brain cancer, breast cancer, cervical cancer, choriocarcinoma, colon cancer, rectal cancer, head and neck cancer, peritoneal tumor, fallopian tube cancer, endometrial cancer, esophageal cancer, gastric cancer, leukemia, lymphoma, sarcoma, neuroblastoma, oral cancer, ovarian cancer, pancreatic cancer, prostate cancer, testicular cancer, skin cancer, and thyroid cancer.

**62.** The use according to claim 60, wherein the disease and/or the symptom is a viral infection selected from the group consisting of: dengue virus, yellow fever virus, West Nile virus, Japanese encephalitis virus, tick-borne encephalitis virus, Kunjin virus, Murray Valley encephalitis virus, St. Louis encephalitis virus, Omsk hemorrhagic fever virus, bovine viral diarrhea virus, Zika virus, HIV (human immunodeficiency virus), HBV (hepatitis B virus), HCV (hepatitis C virus), HPV (human papillomavirus), RSV (respiratory syncytial virus), SARS-CoV (severe acute respiratory syndrome coronavirus), SARS-CoV-2 (severe acute respiratory syndrome coronavirus 2), MERS-CoV (middle east respiratory syndrome coronavirus), and influenza virus.

**INTERNATIONAL SEARCH REPORT**

International application No.
**PCT/CN2022/139570**

**A. CLASSIFICATION OF SUBJECT MATTER**

C07D403/12(2006.01)i;A61K31/4427(2006.01)i;A61P35/00(2006.01)i

According to International Patent Classification (IPC) or to both national classification and IPC

**B. FIELDS SEARCHED**

Minimum documentation searched (classification system followed by classification symbols)

C07D403/-; A61K31/-; A61P35/-

Documentation searched other than minimum documentation to the extent that such documents are included in the fields searched

Electronic data base consulted during the international search (name of data base and, where practicable, search terms used)

CNPAT, STN: 结构式, TLR, 抑制剂, 癌症, inhibitor, cancer

**C. DOCUMENTS CONSIDERED TO BE RELEVANT**

| Category* | Citation of document, with indication, where appropriate, of the relevant passages | Relevant to claim No. |
|---|---|---|
| X | CN 102753542 A (VENTIRX PHARMACEUTICALS INC. et al.) 24 October 2012 (2012-10-24)<br>claims 1, 11, 15 and 17-19, and description, paragraphs [0073]-[0075] and [0226] | 1-62 |
| X | CN 107295798 A (F. HOFFMANN-LA ROCHE AG) 24 October 2017 (2017-10-24)<br>claims 1 and 19-21, and description, paragraphs [0237]-[0245] and embodiment 60 | 1-62 |
| X | WO 2020252294 A1 (BOLT BIOTHERAPEUTICS, INC.) 17 December 2020 (2020-12-17)<br>claim 1, and description, page 51, table 1a | 1-62 |
| X | CN 102781933 A (VENTIRX PHARMACEUTICALS INC. et al.) 14 November 2012 (2012-11-14)<br>claims 1, 11, 15 and 17-19, and description, paragraphs [0061]-[0066] and [0210] | 1-62 |
| X | WO 2020252254 A1 (BOLT BIOTHERAPEUTICS, INC.) 17 December 2020 (2020-12-17)<br>claim 1, and description, paragraph [0076], table 1a | 1-62 |
| A | WO 2017202704 A1 (F. HOFFMANN-LA ROCHE AG et al.) 30 November 2017 (2017-11-30)<br>claims 1-23, and embodiment 1 | 1-62 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 March 2023** | **07 March 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No.
**PCT/CN2022/139570**

**C. DOCUMENTS CONSIDERED TO BE RELEVANT**

| Category* | Citation of document, with indication, where appropriate, of the relevant passages | Relevant to claim No. |
|---|---|---|
| A | WO 2021067242 A1 (BOLT BIOTHERAPEUTICS, INC.) 08 April 2021 (2021-04-08) claim 1, and description, pages 31-32, table 1a | 1-62 |

Form PCT/ISA/210 (second sheet) (July 2022)

**INTERNATIONAL SEARCH REPORT**

International application No.
**PCT/CN2022/139570**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **57-58**
because they relate to subject matter not required to be searched by this Authority, namely:

Claims 57-58 relate to a method for treating a disease. The present search is carried out on the basis of the use of a compound in the preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2022/139570**

| Patent document cited in search report | Publication date (day/month/year) | Patent family member(s) | Publication date (day/month/year) |
|---|---|---|---|
| CN 102753542 A | 24 October 2012 | BR 112012003757 A2 | 11 August 2020 |
| | | US 2014234376 A1 | 21 August 2014 |
| | | US 9126940 B2 | 08 September 2015 |
| | | US 2016129010 A1 | 12 May 2016 |
| | | US 9655904 B2 | 23 May 2017 |
| | | EP 3225623 A1 | 04 October 2017 |
| | | RU 2016125705 A | 04 December 2018 |
| | | RU 2012110251 A | 27 September 2013 |
| | | RU 2593261 C2 | 10 August 2016 |
| | | AU 2010284240 A1 | 08 March 2012 |
| | | AU 2010284240 A8 | 12 April 2012 |
| | | AU 2010284240 B2 | 10 November 2016 |
| | | AU 2017200937 A1 | 02 March 2017 |
| | | IL 218193 A0 | 30 April 2012 |
| | | IL 218193 A | 31 October 2017 |
| | | EP 2467377 A2 | 27 June 2012 |
| | | EP 2467377 A4 | 02 January 2013 |
| | | EP 2467377 B1 | 28 December 2016 |
| | | ES 2620629 T3 | 29 June 2017 |
| | | HRP 20170404 T1 | 19 May 2017 |
| | | JP 2015155471 A | 27 August 2015 |
| | | WO 2011022508 A2 | 24 February 2011 |
| | | WO 2011022508 A3 | 23 June 2011 |
| | | US 2011092485 A1 | 21 April 2011 |
| | | US 8691809 B2 | 08 April 2014 |
| | | HUE 032626 T2 | 30 October 2017 |
| | | DK 2467377 T3 | 03 April 2017 |
| | | PL 2467377 T3 | 31 October 2017 |
| | | JP 2013502430 A | 24 January 2013 |
| | | JP 5756804 B2 | 29 July 2015 |
| | | PT 2467377 T | 04 April 2017 |
| | | CA 2771609 A1 | 24 February 2011 |
| | | CA 2771609 C | 02 October 2018 |
| CN 107295798 A | 24 October 2017 | CO 2017006523 A2 | 20 October 2017 |
| | | HK 1245766 A1 | 31 August 2018 |
| | | DK 3265458 T3 | 18 March 2019 |
| | | PL 3265458 T3 | 31 May 2019 |
| | | SG 11201705732 SA | 30 August 2017 |
| | | HRP 20190282 T1 | 05 April 2019 |
| | | RU 2017134866 A | 09 April 2019 |
| | | RU 2017134866 A3 | 13 August 2019 |
| | | RU 2712248 C2 | 27 January 2020 |
| | | EP 3265458 A1 | 10 January 2018 |
| | | EP 3265458 B1 | 19 December 2018 |
| | | RS 58356 B1 | 29 March 2019 |
| | | PH 12017501591 A1 | 12 February 2018 |
| | | ES 2712488 T3 | 13 May 2019 |
| | | JP 2018508535 A | 29 March 2018 |
| | | JP 6728208 B2 | 22 July 2020 |
| | | MA 41625 A | 10 January 2018 |

Form PCT/ISA/210 (patent family annex) (July 2022)

**INTERNATIONAL SEARCH REPORT**
**Information on patent family members**

International application No.
**PCT/CN2022/139570**

| Patent document cited in search report | | | Publication date (day/month/year) | Patent family member(s) | | | Publication date (day/month/year) |
|---|---|---|---|---|---|---|---|
| | | | | US | 9822065 | B1 | 21 November 2017 |
| | | | | PT | 3265458 | T | 13 February 2019 |
| | | | | UA | 121887 | C2 | 10 August 2020 |
| | | | | HUE | 042563 | T2 | 29 July 2019 |
| | | | | US | 2017014423 | A1 | 19 January 2017 |
| | | | | US | 9597333 | B2 | 21 March 2017 |
| | | | | AU | 2016231289 | A1 | 13 July 2017 |
| | | | | AU | 2016231289 | B2 | 16 July 2020 |
| | | | | WO | 2016142250 | A1 | 15 September 2016 |
| | | | | MY | 191185 | A | 04 June 2022 |
| | | | | CL | 2017002035 | A1 | 06 April 2018 |
| | | | | PE | 20171456 | A1 | 06 October 2017 |
| | | | | BR | 112017013806 | A2 | 09 January 2018 |
| | | | | LT | 3265458 | T | 12 March 2019 |
| | | | | NZ | 732796 | A | 30 April 2021 |
| | | | | MX | 2017010895 | A | 15 December 2017 |
| | | | | MX | 364223 | B | 16 April 2019 |
| | | | | KR | 20170123631 | A | 08 November 2017 |
| | | | | TW | 201639819 | A | 16 November 2016 |
| | | | | TWI | 680965 | B | 01 January 2020 |
| | | | | IL | 253170 | A0 | 31 August 2017 |
| | | | | US | 2016257653 | A1 | 08 September 2016 |
| | | | | US | 9475775 | B2 | 25 October 2016 |
| | | | | CA | 2972148 | A1 | 15 September 2016 |
| | | | | CR | 20170394 | A | 25 September 2017 |
| | | | | SI | 3265458 | T1 | 29 March 2019 |
| WO | 2020252294 | A1 | 17 December 2020 | AU | 2020291014 | A1 | 27 January 2022 |
| | | | | BR | 112021025034 | A2 | 26 April 2022 |
| | | | | TW | 202112752 | A | 01 April 2021 |
| | | | | EP | 3983082 | A1 | 20 April 2022 |
| | | | | JP | 2022537158 | A | 24 August 2022 |
| | | | | KR | 20220034784 | A | 18 March 2022 |
| | | | | CA | 3142887 | A1 | 17 December 2020 |
| | | | | IL | 288816 | A | 01 February 2022 |
| | | | | US | 2020390899 | A1 | 17 December 2020 |
| CN | 102781933 | A | 14 November 2012 | ES | 2617676 | T3 | 19 June 2017 |
| | | | | EP | 3159337 | A2 | 26 April 2017 |
| | | | | EP | 3159337 | A3 | 26 July 2017 |
| | | | | IL | 218155 | A0 | 31 July 2012 |
| | | | | IL | 218155 | A | 29 February 2016 |
| | | | | JP | 2015155472 | A | 27 August 2015 |
| | | | | HUE | 031797 | T2 | 28 August 2017 |
| | | | | JP | 2013502431 | A | 24 January 2013 |
| | | | | JP | 5756805 | B2 | 29 July 2015 |
| | | | | DK | 2467380 | T3 | 13 March 2017 |
| | | | | PL | 2467380 | T3 | 29 September 2017 |
| | | | | US | 2016137608 | A1 | 19 May 2016 |
| | | | | PT | 2467380 | T | 07 March 2017 |
| | | | | US | 2014142086 | A1 | 22 May 2014 |
| | | | | US | 9242964 | B2 | 26 January 2016 |

Form PCT/ISA/210 (patent family annex) (July 2022)

**INTERNATIONAL SEARCH REPORT**
**Information on patent family members**

International application No.
**PCT/CN2022/139570**

| Patent document cited in search report | Publication date (day/month/year) | Patent family member(s) | Publication date (day/month/year) |
|---|---|---|---|
| | | RU 2016108987 A | 26 November 2018 |
| | | CA 2771484 A1 | 24 February 2011 |
| | | CA 2771484 C | 15 January 2019 |
| | | BR 112012003703 A2 | 08 December 2020 |
| | | WO 2011022509 A2 | 24 February 2011 |
| | | WO 2011022509 A3 | 16 June 2011 |
| | | US 2011118235 A1 | 19 May 2011 |
| | | US 8524702 B2 | 03 September 2013 |
| | | HRP 20170268 T1 | 19 May 2017 |
| | | IL 243710 A0 | 21 April 2016 |
| | | IL 243710 A | 31 October 2017 |
| | | RU 2012110246 A | 27 September 2013 |
| | | RU 2580320 C2 | 10 April 2016 |
| | | AU 2010284241 A1 | 08 March 2012 |
| | | AU 2010284241 B2 | 10 November 2016 |
| | | AU 2017200936 A1 | 02 March 2017 |
| | | EP 2467380 A2 | 27 June 2012 |
| | | EP 2467380 A4 | 02 January 2013 |
| | | EP 2467380 B1 | 30 November 2016 |
| WO 2020252254 A1 | 17 December 2020 | EP 3983080 A1 | 20 April 2022 |
| | | JP 2022538773 A | 06 September 2022 |
| | | CA 3143156 A1 | 17 December 2020 |
| | | US 2022315537 A1 | 06 October 2022 |
| WO 2017202704 A1 | 30 November 2017 | EP 3464245 A1 | 10 April 2019 |
| | | EP 3464245 B1 | 14 October 2020 |
| | | JP 2019516731 A | 20 June 2019 |
| | | JP 6918838 B2 | 11 August 2021 |
| | | US 2019077763 A1 | 14 March 2019 |
| | | US 10370338 B2 | 06 August 2019 |
| WO 2021067242 A1 | 08 April 2021 | AU 2020359446 A1 | 21 April 2022 |
| | | CA 3152601 A1 | 08 April 2021 |
| | | KR 20220077131 A | 08 June 2022 |
| | | EP 4038053 A1 | 10 August 2022 |
| | | BR 112022006001 A2 | 12 July 2022 |
| | | JP 2022549510 A | 25 November 2022 |
| | | IL 291760 A | 01 June 2022 |
| | | US 2022347310 A1 | 03 November 2022 |

Form PCT/ISA/210 (patent family annex) (July 2022)